# EUROPEAN PATENT SPECIFICATION

(11) **EP 1 928 879 B1**
(45) Date of publication and mention of the grant of the patent: **13.02.2013**
(21) Application number: 06825074.5
(22) Date of filing: 22.09.2006
(51) Int. Cl.: C07D 487/04, A61K 31/437, A61P 29/00

(54) **FUSED HETEROCYCLIC COMPOUNDS USEFUL AS KINASE MODULATORS**
KONDENSIERTE HETEROCYCLISCHE VERBINDUNGEN ALS KINASEMODULATOREN
COMPOSES HETEROCYCLIQUES CONDENSES UTILISES COMME MODULATEURS DES KINASES

(30) Priority: 22.09.2005 US 719519 P; 21.09.2006 US 524996
(43) Date of publication of application: 11.06.2008
(73) Proprietor: Bristol-Myers Squibb Company, Princeton, NJ 08543-4000 (US)
(72) Inventor: VACCARO, Wayne, New Jersey 08540-4000 (US); CHEN, Zhong, NJ 08540-4000 (US); DODD, Dharmpal, S., NJ 08540-4000 (US); HUYNH, Tram, N., NJ 08540-4000 (US); LIN, James, NJ 08540-4000 (US); LIU, Chunjian, NJ 08540-4000 (US); MUSSARI, Christopher, P., NJ 08540-4000 (US); TOKARSKI, John, S., NJ 08540-4000 (US); TORTOLANI, David, R., NJ 08540-4000 (US); WROBLESKI, Stephen, T., NJ 08540-4000 (US)
(74) Representative: Hart-Davis, Jason
(86) International application number: PCT/US2006/037056
(87) International publication number: WO 2007/038314

(56) References cited:
- WO-A-2004/076458
- WO-A-2006/107784
- WO-A1-98/08847
- POLANC, S. ET AL: "Pyridazines. LXIIV. Synthesis of some tricyclic heterocycles fused at the nitrogen-carbon (N-1-C-8) bond of imidazo[1,2-b]pyridazines" SYNTHESIS , (3), 175-6 CODEN: SYNTBF; ISSN: 0039-7881, 1975, XP002420091

## Description

This application claims priority benefit under Title 3 5 § 119(e) of United States Provisional Application No. 60/719,519 filed September 22, 2005, the content of which is incorporated herein by reference.

### FIELD OF THE INVENTION

This invention relates to fused heterocyclic compounds useful as kinase modulators, including the modulation of MAPKAP kinase-2 (MK2). The invention further pertains to pharmaceutical compositions containing at least one compound according to the invention that are useful for the treatment of conditions related to kinase modulation and methods of inhibiting the activity of kinases, including MK2, in a mammal.

### BACKGROUND OF THE INVENTION

A large number of cytokines participate in the inflammatory response, including IL-1, IL-6, IL-8 and TNF-α. The overproduction of cytokines such as IL-1 and TNF-α is implicated in a wide variety of diseases, including inflammatory bowel disease, rheumatoid arthritis, psoriasis, multiple sclerosis, endotoxin shock, osteoporosis, Alzheimer's disease, and congestive heart failure. *See e.g.* Henry et al., Drugs Fut., Vol. 24 (1999), at pp. 1345-1354; and Salituro et al., Curr. Med. Chem., Vol. 6 (1999), at pp. 807-823. Evidence in human patients indicates that protein antagonists of cytokines are effective in treating chronic inflammatory diseases, such as, for example, monoclonal antibody to TNF-α (Enbrel) (*see* Rankin et al., Br. J. Rheumatol., Vol 34 (1995), at pp. 334-342), and soluble TNF-α receptor-Fc fusion protein (Etanercept) (*see* Moreland et al., Ann. Intern. Med., Vol. 130 (1999), at pp. 478-486).

The biosynthesis of TNF-α occurs in many cell types in response to an external stimulus, such as, for example, a mitogen, an infectious organism, or trauma. Important mediators of TNF-α production are the mitogen-activated protein (MAP) kinases, including p38 kinase (p38). Activation of p38 requires dual phosphorylation by an upstream MAP kinase (MKK3 and MKK6) on threonine and tyrosine within a Thr-Gly-Tyr motif characteristic of p38 isozymes. The p38 kinase is an upstream kinase of mitogen-activated protein kinase-activated protein kinase-2 (MAPKAP K2 or MK2). *See* Freshney et al., Cell, Vol. 78 (1994), at pp. 1039-1049.

MK2 is a protein that appears to be predominantly regulated by p3 8 in cells. In fact, MK2 was the first substrate of p38α to be identified, and *in vitro* phosphorylation of MK2 by p38α is required for MK2 activation. MK2, in turn, phosphorylates substrates including, but not limited to, heat shock protein 27 (HSP27), lymphocyte-specific protein 1 (LAP-1), leukocyte-specific protein-1 (LSP-1), 5-lipoxygenase (5-LO), cAMP response element-binding protein (CREB), ATF1, serum response factor (SRF), tyrosine hydroxylase, and most importantly, adenosine and uridine-rich element (ARE) binding proteins. ARE binding proteins regulate the mRNA stability of inflammatory mediators such as TNFα and COX-2.

Targeted mutations have been introduced into the mouse MK2 gene that resulted in the generation of MK2-deficient mice. See Kotlyarov et al, Nat. Cell Biol., Vol. 1 (1999), at pp. 94-97. These MK2-deficient mice exhibited increased stress resistance to LPS-induced endoxic shock and had a better survival rate compared to mice that retained the MK2 gene. *See id.* Isolated splenocytes from these mice challenged with LPS had reduced levels of TNFα, IL-1β, IL-6 and IFNγ. *See id.* More recently, Lehner et al. reported that MK2-deficient mice showed increased susceptibility to *Listeria moocytogenes* infection and concluded that MK2 had an essential role in host defense against intracellular bacteria, probably through the regulation of TNFα and IFNγ, two of the cytokines required for the activation of antibacterial effector mechanisms. *See* Lehner et al., J. Immunol., Vol. 168 (2002), at pp. 4667-4673. Moreover, since MK2 is located immediately downstream of p38 in the p38 signaling pathway, it is recognized that MK2 could act as a focal point for more selectively modulating the inflammatory pathway thereby reducing the possibility of undesirable side effects.

Pyrazolo[1,5-a]pyrimidine derivatives have been disclosed in WO2004076458(A1) and described as having kinase inhibiting activity.

New compounds and methods of modulating the activity of kinases, including MK2, would be desirable in the treatment of diseases and disorders that are mediated by cytokines, such as TNFα. It would be even more desirable to provide MK2 inhibitors that have improved potency and reduced undesirable side effects.

### SUMMARY OF THE INVENTION

The present invention provides compounds useful in treating inflammatory or immune disease having the formula (I): 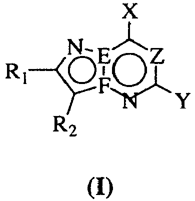 or an enantiomer, diastereomer, or a pharmaceutically-acceptable salt, thereof,
wherein:
E is C; F is N;
X is NR₄R₅;
Z is CR₃;
Y is selected from hydrogen, halogen, nitro, cyano, SR₈, S(O)*p*R₈, OR₈, NR₆R₇, CO₂R₈, C(=O)R₈, O-C(=O)R₈, C(=O)NR₈R₉, cycloalkyl, cycloalkenyl, cycloalkynyl, heterocyclo, aryl, and heteroaryl, provided that if Y is hydrogen then R₄ is phenyl substituted with a carboxamido group;
R₁ and R₂ are independently selected from (i) hydrogen, alkyl, halogen, nitro, cyano, SR₁₀, OR₁₀, NR₁₀R₁₁, NR₁₀C(=O)R₁₁, CO₂R₁₀, C(=O)R₁₀, -O-C(=O)R₁₀;
R₃ is selected from hydrogen, halogen, alkyl, substituted alkyl, alkenyl, substituted alkenyl, alkynyl, substituted alkynyl, nitro, cyano, SR₁₃, OR₁₃, NR₁₃R₁₄, NR₁₃C(=O)R₁₄, CO₂R₁₃, C(=O)R₁₃, -O-C(=O)R₁₃, -C(=O)NR₁₃R₁₄, cycloalkyl, heterocyclo, aryl, and heteroaryl;
R₄, R₅, R₆, and R₇ are independently selected from hydrogen, alkyl, substituted alkyl, alkenyl, substituted alkenyl, alkynyl, substituted alkynyl, OR₁₅, SR₁₅, C(=O)R₁₅, CO₂R₁₅, C(=O)NR₁₅R₁₆, C(W)OR₁₆, S(O)ₚR₁₇, SO₂NR₁₅R₁₆, cycloalkyl, heterocyclo, aryl, and heteroaryl; or (ii) R₄ is taken together with R₅ and the nitrogen atom to which they are both attached and/or R₆ is taken together with R₇ and the nitrogen atom to which they are both attached to form a heteroaryl or heterocyclo;
R₈, R₉, R₁₀, R₁₁, R₁₃, R₁₄, R₁₅, and R₁₆ at each occurrence are independently selected from (i) hydrogen, alkyl, substituted alkyl, alkenyl, substituted alkenyl, alkynyl, substituted alkynyl, cycloalkyl, aryl, heteroaryl, and heterocyclo; or (ii) together with the nitrogen atom to which they are attached, R₈ is taken together with R₉, and/or R₁₀ is taken together with R₁₁, and/or R₁₃ is taken together with R₁₄, and/or R₁₅ is taken together with R₁₆ to form a heteroaryl or heterocyclo;
R₁₇is selected from alkyl, substituted alkyl, alkenyl, substituted alkenyl, alkynyl, substituted alkynyl, cycloalkyl, aryl, heteroaryl, and heterocyclo;
W at each occurrence is O, S, N, CN, or NH; and
*p* is 1 or 2,
with the following provisos:
(1) if X is NH(Me), N(Me)₂, NH(unsubstituted phenyl), or NHNH₂, then Y is other than hydrogen or halogen; and
(2) the following compounds are excluded: 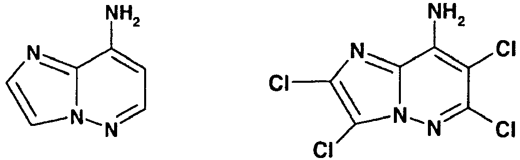
The left-hand and centre compound excluded under proviso (2) were disclosed in Polanc et al., Synthesis, March 1975, 175-176. The right-hand compound was disclosed in WO 2006/107784. Proviso (1) excludes the following compounds: 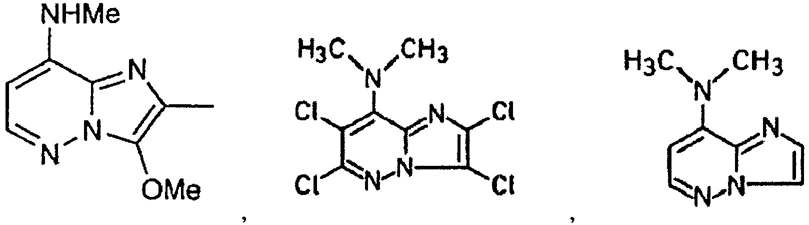 The right-hand and centre compound thus excluded were disclosed in Stanovnik, Synthesis, August 1971, 424-425. The left-hand compound was disclosed (as Compound 147) in Hajos et al., Science of Synthesis, 12, 613-678 (2002).

The present invention is also directed to pharmaceutical compositions useful in treating diseases associated with kinase modulation, including modulation (especially inhibition) of MK2, comprising compounds of formula (I), or pharmaceutically-acceptable salts thereof, and pharmaceutically-acceptable carriers or diluents. The invention further relates, for use in methods of treating diseases associated with the kinase modulation, including the modulation of MK2, by administering to a patient in need of such treatment a therapeutically-effective amount thereof, a compound according to formula (I).

### DETAILED DESCRIPTION OF THE INVENTION

The following are definitions of terms used in this specification and appended claims. The initial definition provided for a group or term herein applies to that group or term throughout the specification and claims, individually or as part of another group, unless otherwise indicated.

The term "alkyl" refers to straight or branched chain hydrocarbon groups having 1 to 12 carbon atoms, preferably 1 to 8 carbon atoms. Lower alkyl groups, that is, alkyl groups of 1 to 4 carbon atoms, are most preferred. When numbers appear in a subscript after the symbol "C", the subscript defines with more specificity the number of carbon atoms that a particular group may contain. For example, "C₁₋₆alkyl" refers to straight and branched chain alkyl groups with one to six carbon atoms, such as methyl, ethyl, n-propyl, isopropyl, n-butyl, t-butyl, n-pentyl, and so forth. The subscript "0" refers to a bond. Thus, the term hydroxy(C₀₋₂)alkyl or (C₀₋₂)hydroxyalkyl includes hydroxy, hydroxymethyl and hydroxyethyl.

The term "substituted alkyl" refers to an alkyl group as defined above having one, two, or three substituents selected from the group consisting of halo (*e.g*., trifluoromethyl), alkenyl, substituted alkenyl, alkynyl, nitro, cyano, oxo (=O), ORₐ, SRₐ, (=S), -NRₐR_{b}, -N(alkyl)₃⁺, -NRₐSO₂, -NRₐSO₂R_{c}, -SO₂R_{c} -SO₂NRₐR_{b}, -SO₂NRₐC(=O)R_{b}, SO₃H, -PO(OH)₂, -OC(O)Rₐ, -C(=O)Rₐ, -CO₂Rₐ, -C(=O)NRₐR_{b}, -C(=O)(C₁₋₄alkylene)NRₐR_{b}, -C(=O)NRₐ(SO₂)R_{b}, -CO₂(C₁₋₄alkylene)NRₐR_{b}, -NRₐC(=O)R_{b}, -NRₐCO₂R_{b}, -NRₐ(C₁₋₄alkylene)CO₂R_{b}, =N-OH, =N-O-alkyl, aryl, cycloalkyl, heterocyclo, and/or heteroaryl,wherein Rₐ and R_{b} are selected from hydrogen, alkyl, alkenyl, CO₂H, CO₂(alkyl), C₃₋₇cycloalkyl, phenyl, benzyl, phenylethyl, napthyl, a four to seven membered heterocylo, or a five to six membered heteroaryl, or when attached to the same nitrogen atom may join to form a heterocyclo or heteroaryl, and R_{c} is selected from same groups as Rₐ and R_{b} but is not hydrogen. Each group Rₐ and R_{b} when other than hydrogen, and each R_{c} group optionally has up to three further substituents attached at any available carbon or nitrogen atom of Rₐ, R_{b}, and/or R_{c}, said substituent(s) being selected from the group consisting of (C₁₋₆)alkyl, (C₂₋₆)alkenyl, hydroxy, halogen, cyano, nitro, =O (as valence allows), CF₃, O(C₁₋₆alkyl), OCF₃, C(=O)H, C(=O)(C₁₋₆alkyl), CO₂H, CO₂(C₁₋₆alkyl), NHCO₂(C₁₋₆alkyl), -S(C₁₋₆alkyl), -NH₂, NH(C₁₋₆alkyl), N(C₁₋₆alkyl)₂, N(CH₃)₃⁺, SO₂(C₁₋₆alkyl), C(=O)(C₁₋₄alkylene)NH₂, C(=O)(C₁₋₄alkylene)NH(alkyl), C(=O)(C₁₋₄alkylene)N(C₁-₄alkyl)₂, C₃₋₇cycloalkyl, phenyl, benzyl, phenylethyl, phenyloxy, benzyloxy, napthyl, a four to seven membered heterocyclo or cycloalkyl, or a five to six membered heteroaryl. When a substituted alkyl is substituted with an aryl (including, for example, phenyl and napthyl), heterocyclo, cycloalkyl, or heteroaryl group, said ringed systems are as defined below and thus may have zero, one, two, or three substituents, also as defined below.

One skilled in the field will understand that, when the designation "CO₂" is used herein, this is intended to refer to the group 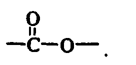

When the term "alkyl" is used together with another group, such as in "arylalkyl", this conjunction defines with more specificity at least one of the substituents that the substituted alkyl will contain. For example, "arylalkyl" refers to a substituted alkyl group as defined above where at least one of the substituents is an aryl, such as benzyl. Thus, the term aryl(C₀₋₄)alkyl includes a substituted lower alkyl having at least one aryl substituent and also includes an aryl directly bonded to another group, *i.e*., aryl(C₀)alkyl.

The term "alkenyl" refers to straight or branched chain hydrocarbon groups having 2 to 12 carbon atoms and at least one double bond. Alkenyl groups of 2 to 6 carbon atoms and having one double bond are most preferred.

The term "alkynyl" refers to straight or branched chain hydrocarbon groups having 2 to 12 carbon atoms and at least one triple bond. Alkynyl groups of 2 to 6 carbon atoms and having one triple bond are most preferred.

The term "alkylene" refers to bivalent straight or branched chain hydrocarbon groups having 1 to 12 carbon atoms, preferably 1 to 8 carbon atoms, *e.g*., {-CH₂-}ₙ, wherein *n* is 1 to 12, preferably 1-8. Lower alkylene groups, that is, alkylene groups of 1 to 4 carbon atoms, are most preferred. The terms "alkenylene" and "alkynylene" refer to bivalent radicals of alkenyl and alkynyl groups, respectively, as defined above.

When reference is made to a substituted alkenyl, alkynyl, alkylene, alkenylene, or alkynylene group, these groups are substituted with one to three substitutents as defined above for substituted alkyl groups.

The term "heteroalkylene" is used herein to refer to saturated and unsaturated bivalent straight or branched chain hydrocarbon groups having 2 to 12 carbon atoms, preferably 2 to 8 carbon atoms, wherein one or two carbon atoms in the straight chain are replaced by heteroatom(s) selected from -O-, -S-, -S(=O)-, -SO₂-, -NH-, and -NHSO₂-. Thus, the term "heteroalkylene" includes bivalent alkoxy, thioalkyl, and aminoalkyl groups, as defined below, as well as alkylene and alkenylene groups having a combination of heteroatoms in the alkyl chain. As an illustration, a "heteroalkylene" herein may comprise groups such as -S-(CH₂)₁₋₅NH-CH₂-, -O-(CH₂)₁₋₅S(=O)-CH₂-, -NHSO₂CH₂-, -CH₂-NH-, and so forth. Preferably, a heteroalkylene does not have two adjacent atoms simultaneously selected from -0- and -S-. When a subscript is used with the term heteroalkylene, *e.g*., as in C₂₋₃heteroalkylene, the subscript refers to the number of carbon atoms in the group in addition to heteroatoms. Thus, for example, a C₁₋₂heteroalkylene may include groups such as NH-CH₂-, -CH₂-NH-CH₂-, -CH₂-CH₂-NH-, -S-CH₂-, -CH₂-S-CH₂-, -O-CH₂-NH-CH₂-, CH₂-O-CH₂ and so forth.

The term "substituted heteroalkylene" refers to a heteroalkylene group as defined above wherein at least one of the nitrogen or carbon atoms in the heteroalkylene chain is bonded to (or substituted with) a group other than hydrogen. Carbon atoms in the heteroalkylene chain may be substituted with a group selected from those recited above for substituted alkyl groups, or with a further alkyl or substituted alkyl group. Nitrogen atoms of the heteroalkylene chain may be substituted with a group selected from alkyl, alkenyl, alkynyl, cyano, or A₁-Q-A₂-Rₕ, wherein A₁ is a bond, C₁₋₂alkylene, or C₂₋₃alkenylene; Q is a bond, -C(=O)-, -C(=O)NR_{d}-, -C(=S)NR_{d}-, -SO₂-, -SO₂NR_{d}-, -CO₂-, or - NR_{d}CO₂-; A₂ is a bond, C₁₋₃alkylene, C₂₋₃alkenylene, -C₁₋₄akylene-NR_{d}-, -C₁₋₄alkylene-NR_{d}C(=O)-, -C₁₋₄alkylene-S-, -C₁₋₄alkylene-SO₂-, or -C₁₋₄alkylene-O-, wherein said A₂ alkylene groups are branched or straight chain and optionally substituted as defined herein for substituted alkylene; Rₕ is hydrogen, alkyl, substituted alkyl, alkenyl, substituted alkenyl, aryl, heteroaryl, heterocyclo, or cycloalkyl; and R_{d} is selected from hydrogen, alkyl, and substituted alkyl, as defined herein, provided, however, that for a substituted heteroalkylene Rₕ is not hydrogen when A₁, Q and A₂ are each bonds. When Rₕ is aryl, heteroaryl, cycloalkyl or heterocyclo, these rings are, in turn, optionally substituted with one to three groups as defined below in the definitions for these terms.

The term "alkoxy" refers to an oxygen atom substituted by alkyl or substituted alkyl, as defined herein. For example, the term "alkoxy" or includes the group -O-C₁₋₆alkyl.

The term "alkylthio" refers toa sulfur atom that is substituted by an alkyl or substituted alkyl group as defined herein. For example, the term "thioalkyl" includes the group -S-C₁₋₆alkyl, and so forth.

The term "alkylamino" refers to an amino group substituted with an alkyl group or substituted alkyl group as defined above. For example, the term "alkylamino" includes the group -NR-C₁₋₁₂alkyl. (where R is preferably hydrogen but may include alkyl or substituted alkyl as defined above.)

When a subscript is used with reference to an alkoxy, thioalkyl or aminoalkyl, the subscript refers to the number of carbon atoms that the group may contain in addition to heteroatoms. Thus, for example, monovalent C₁₋₂aminoalkyl includes the groups -CH₂-N(CH₃)₂, and -(CH₂)₂-NH₂. A lower aminoalkyl comprises an aminoalkyl having one to four carbon atoms. The term (C₁₋₄alkyl)₀₋₂amino includes the groups NH₂, -NH(C₁₋₄alkyl), and -N(C₁₋₄alkyl)₂. "Amino" refers to the group NH₂. A "substituted amino" refers to an amino group substituted as described above for the nitrogen atom of a heteroalkylene chain and includes, for example, the terms alkylamino and acylamino (-NR_{d}C(O)Rₑ).

The alkoxy, thioalkyl, or aminoalkyl groups may be monovalent or bivalent. By "monovalent" it is meant that the group has a valency (*i.e*., ability to combine with another group), of one, and by "bivalent" it is meant that the group has a valency of two. Thus, for example, a monovalent alkoxy includes groups such as -O-C₁₋₁₂alkyl, whereas a bivalent alkoxy includes groups such as -O-C₁₋₁₂alkylene-.

It should be understood that the selections for all groups, including for examples, alkoxy, thioalkyl, and aminoalkyl, will be made by one skilled in the field to provide stable compounds. Thus, for example, in compounds of formula (I), when G is attached to a nitrogen atom (N*) of ring A and is selected from an alkoxy or alkylthio group, the alkoxy and alkylthio groups will have at least one carbon atom bonded directly to ring A (at N*), with the oxygen or sulfur atoms being at least one atom away from said nitrogen atom.

The term "carbonyl" refers to a bivalent carbonyl group -C(=O)-. When the term "carbonyl" is used together with another group, such as in "heterocyclocarbonyl", this conjunction defines with more specificity at least one of the substituents that the substituted carbonyl will contain. For example, "heterocyclocarbonyl" refers to a carbonyl group as defined above where at least one of the substituents is an heterocyclo, such as morpholinyl.

The term "acyl" refers to a carbonyl group linked to an organic radical, more particularly, the group C(=O)Rₑ. The group Rₑ can be selected from alkyl, alkenyl, alkynyl, aminoalkyl, substituted alkyl (i.e. substituted alkylene), substituted alkenyl, substituted alkenyl, cycloalkyl, heterocyclo, aryl, or heteroaryl, as defined herein. When Rₑ is aryl, heteroaryl, cycloalkyl, or heterocyclo, these rings are, in turn, optionally substituted with one to three groups as defined below in the definitions for these terms.

The term "alkoxycarbonyl" refers to a carboxy group 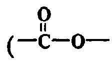 or 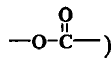 linked to an organic radical (CO₂Rₑ), as well as the bivalent groups -CO₂-, -CO₂Rₑ- which are linked to organic radicals in compounds of formula (I), wherein Rₑ is as defined above for acyl. The organic radical to which the carboxy group is attached may be monovalent (*e.g*., -CO₂-alkyl or -OC(=O)alkyl), or bivalent (*e.g*., -CO₂-alkylene, -OC(=O)alkylene, etc.) Accordingly, in compounds of formula (I), when it is recited that G can be "alkoxycarbonyl," this is intended to encompass a selection for G of-CO₂- and also the groups -CO₂Rₑ- or -RₑCO₂-, wherein in this instance, the group Rₑ will be selected from bivalent groups, e.g., alkylene, alkenylene, alkynylene, bivalent aminoalkyl, substituted alkylene, substituted alkenylene, or substituted alkynylene.

The term "carboxamide","carboxamidyl", or "carboxamido" refers to the group -NR_{d}C(=O)Rₑ, wherein the groups R_{d} and Rₑ are defined as recited above in the definitions for heteroalkyl, alkoxycarbonyl and acyl.. For example, the group 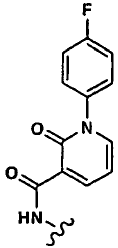 is a carboxamido group where Rₑ is a substituted heterocyclo according to the definitions herein.

The term "amide","amidyl", or "amido" refers to the group -C(=O)NRₐR_{b}, wherein the groups Rₐ and R_{b} are defined as recited above in the definition for substituted alkyl groups..

The term "urea" refers to the group -NR_{d}C(=O)NRₐR_{b}, wherein the groups Rₐ, R_{b}, and R_{d} are defined as recited above in the definition for substituted alkyl groups. Additionally, the urea group may be bivalent, in which case one of the groups Rₐ and R_{b} will be a bond. Thus, in compounds of formula (I), when it is stated that G may be urea, it can mean that G is a group -NR_{d}(C(=O)NRₐ- where appropriate.

The term "sulfonyl" refers to a sulphoxide group linked to an organic radical in compounds of formula (I), more particularly, the monovalent group -S(O)₂-Rₑ. Additionally, the sulfonyl group may be bivalent, in which case Rₑ is a bond. Accordingly, in compounds of formula (I), when it is recited that G canbe "sulfonyl," it can mean that G is a group -S(O) where appropriate. The group Rₑ is selected from those recited above for acyl and alkoxycarbonyl groups, with the exception that Rₑ is not hydrogen..

The terms "sulfonamide", "sulfonamidyl", or "sulfonamido" refers to the group -S(O)₂NRₐR_{b}, wherein Rₐ and R_{b} are as defined above for substituted alkyl groups.

The term "cycloalkyl" refers to fully saturated and partially unsaturated hydrocarbon rings (and therefore includeshydrocarbon rings also known as "cycloalkenyl rings") of 3 to 9, preferably 3 to 7 carbon atoms. The term "cycloalkyl" includes such rings having zero, one, two, or three substituents selected from the group consisting of halogen, trifluoromethyl, trifluoromethoxy, alkyl, substituted alkyl, alkenyl, substituted alkenyl, alkynyl, nitro, cyano, oxo (=O), ORₐ, SRₐ, (=S), - NRₐR_{b}, -N(alkyl)₃⁺, -NRₐSO₂, -NRₐSO₂R_{c}, -SO₂R_{c} -SO₂NRₐR_{b}, -SO₂NRₐC(=O)R_{b}, SO₃H, -PO(OH)₂, -C(=O)Rₐ, -CO₂R_{b} -C(=O)NRₐR_{b}, -C(=O)(C₁₋₄alkylene)NRₐR_{b},-C(=O)NRₐ(SO₂)R_{b}, -CO₂(C₁₋₄alkylene)NRₐR_{b}, -NRₐC(=O)R_{b}, -NRₐCO₂R_{b}, -NRₐ(C₁₋₄alkylene)CO₂R_{b}, =N-OH, =N-O-alkyl, aryl, cycloalkyl, heterocyclo, and/or heteroaryl, wherein Rₐ, R_{b} and R_{c} are as defined above for substituted alkyl groups, and are also in turn optionally substituted as recited above in the definition for substituted alkyl groups. The term "cycloalkyl" also includes such rings having a second ring fused thereto (*e.g*., including benzo, heterocyclo, or heteroaryl rings) or having a carbon-carbon bridge of 3 to 4 carbon atoms. When a cycloalkyl is substituted with a further ring (or has a second ring fused thereto), said ring in turn is optionally substituted with one to two of (C₁₋₄)alkyl, (C₂₋₄)alkenyl, (C₂₋₄)alkynyl, halogen, hydroxy, cyano, nitro, CF₃, O(C₁₋₄alkyl), OCF₃, C(=O)H, C(=O)(C₁₋₄alkyl), CO₂H, CO₂(C₁₋₄alkyl), NHCO₂(C₁₋₄alkyl), -S(C₁₋₄alkyl), -NH₂, NH(C₁₋₄alkyl), N(C₁-₄alkyl)₂, N(C₁₋₄akyl)₃⁺, SO₂(C₁₋₄akyl), C(=O)(C₁₋₄alkylene)NH₂, C(=O)(C₁-₄alkylene)NH(alkyl), C(=O)(C₁₋₄alkylene)N(C₁₋₄alkyl)₂ and/or phenyl optionally substituted with any of the preceeding groups. As valence allows, if said further ring is cycloalkyl or heterocyclo it is additionally optionally substituted with =O (oxo).

Accordingly, in compounds of formula (I), the term "cycloalkyl" includes cyclopropyl, cyclobutyl, cyclopentyl, cyclohexyl, cycloheptyl, bicyclooctyl, etc., as well as the following ring systems, 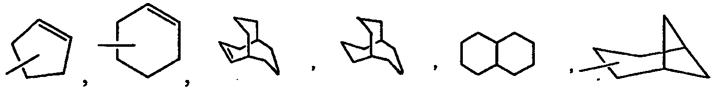 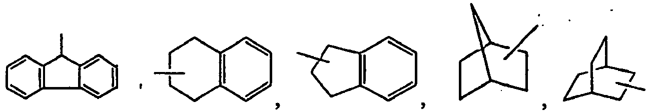 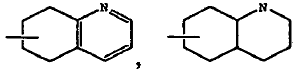 and the like, which optionally may be substituted at any available atoms of the ring(s). Preferred cycloalkyl groups include cyclopropyl, cyclopentyl, cyclohexyl, and

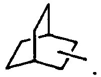

The term "halo" or "halogen" refers to chloro, bromo, fluoro and iodo.

The term "haloalkyl" means a substituted alkyl having one or more halo substituents. For example, "haloalkyl" includes mono, bi, and trifluoromethyl.

The term "haloalkoxy" means an alkoxy group having one or more halo substituents. For example, "haloalkoxy" includes OCF₃.

The term "aryl" refers to phenyl, biphenyl, fluorenyl, 1-naphthyl and 2-naphthyl. The term "aryl" includes such rings having zero, one, two or three substituents selected from the group consisting of halogen, trifluoromethyl, trifluoromethoxy, alkyl, substituted alkyl, alkenyl, substituted alkenyl, alkynyl, nitro, cyano, ORₐ, SRₐ, (=S), SO₃H, -NRₐR_{b}, -N(alkyl)₃⁺, -NRₐSO₂, -NRₐSO₂R_{c}, -SO₂R_{c}-SO₂NRₐR_{b}, -SO₂NRₐC(=O)R_{b}, SO₃H, -PO(OH)₂, -C(-O)Rₐ, -CO₂Rₐ, -C(=O)NRₐR_{b},-C(=O)(C₁₋₄alkylene)NRₐR_{b}, -C(=O)NRₐ(SO₂)R_{b}, -CO₂(C₁₋₄alkylene)NRₐR_{b}, - NRₐC(=O)R_{b}, -NRₐCO₂R_{b}, -NRₐ(C₁₋₄alkylene)CO₂R_{b}, aryl, cycloalkyl, heterocyclo, and/or heteroaryl, wherein Rₐ, R_{b} and R_{c} are as defined above for substituted alkyl groups, and are also in turn optionally substituted as recited above. Additionally, two substituents attached to an aryl, particularly a phenyl group, may join to form a further ring such as a fused or spiro-ring, *e.g*., cyclopentyl or cyclohexyl, or fused heterocyclo or heteroaryl. When an aryl is substituted with a further ring (or has a second ring fused thereto), said ring in turn is optionally substituted with one to two of (C₁₋₄)alkyl, (C₂₋₄)alkenyl, (C₂₋₄)alkynyl, halogen, hydroxy, cyano, nitro, CF₃, O(C₁₋₄alkyl), OCF₃, C(=O)H, C(=O)(C₁₋₄alkyl), CO₂H, CO₂(C₁₋₄alkyl), NHCO₂(C₁₋₄alkyl), -S(C₁₋₄alkyl), - NH₂, NH(C₁₋₄alkyl), N(C₁₋₄alkyl)₂, N(C₁₋₄alkyl)₃⁺, SO₂(C₁₋₄alkyl), C(=O)(C₁-₄alkylene)NH₂, C(=O)(C₁₋₄alkylene)NH(alkyl), C(=O)(C₁₋₄alkylene)N(C₁₋₄alkyl)₂ and/or phenyl optionally substituted with any of the preceeding groups. As valence allows, if said further ring is cycloalkyl or heterocyclo it is additionally optionally substituted with =O (oxo).

Thus, examples of aryl groups include: 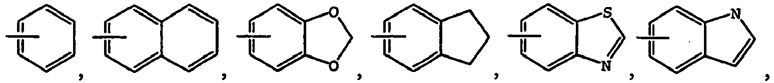 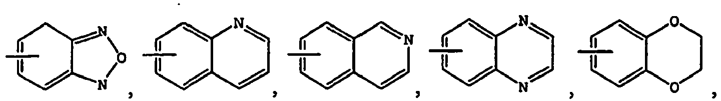 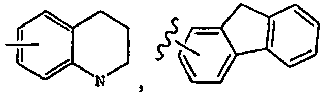 (fluorenyl) and the like, which optionally may be substituted at any available carbon or nitrogen atom. A preferred aryl group is optionally-substituted phenyl.

The terms "heterocycloalkyl", "heterocyclo" or "heterocyclic" may be used interchangeably and refer to substituted and unsubstituted non-aromatic 3-to 7-membered monocyclic groups, 7-to 11-membered bicyclic groups, and 10-to 15-membered tricyclic groups, in which at least one of the rings has at least one heteroatom (O, S or N), said heteroatom containing ring preferably having 1, 2, or 3 heteroatoms selected from O, S, and N. Each ring of such a group containing a heteroatom can contain one or two oxygen or sulfur atoms and/or from one to four nitrogen atoms provided that the total number of heteroatoms in each ring is four or less, and further provided that the ring contains at least one carbon atom. The nitrogen and sulfur atoms may optionally be oxidized and the nitrogen atoms may optionally be quaternized. The fused rings completing the bicyclic and tricyclic groups may contain only carbon atoms and may be saturated, partially saturated, or unsaturated. The heterocyclo group may be attached at any available nitrogen or carbon atom. The hetetocyclo ring may contain zero, one, two or three substituents selected from the group consisting of halogen, trifluoromethyl, trifluoromethoxy, alkyl, substituted alkyl, alkenyl, substituted alkenyl, alkynyl, nitro, cyano, oxo (=O), ORₐ, SRₐ, (=S), -NRₐR_{b}, -N(alkyl)₃⁺, -NRₐSO₂, -NRₐSO₂R_{c}, -SO₂R_{c} -SO₂NRₐR_{b}, - SO₂NRₐC(=O)R_{b}, SO₃H, -PO(OH)₂, -C(=O)Rₐ, -CO₂Rₐ, -C(=O)NRₐR_{b}, . -C(=O)(C₁₋₄alkylene)NRₐR_{b}, -C(=O)NRₐ(SO₂)R_{b}, -CO₂(C₁₋₄alkylene)NRₐR_{b}, -NRₐC(=O)R_{b}, -NRₐCO₂R_{b}, -NRₐ(C₁₋₄alkylene)CO₂R_{b}, =N-OH, =N-O-alkyl, aryl, cycloalkyl, heterocyclo, and/or heteroaryl, wherein Rₐ, R_{b} and R_{c} are as defined above for substituted alkyl groups, and are also in turn optionally substituted as recited above. When a heterocyclo is substituted with a further ring, said ring in turn is optionally substituted with one to two of (C₁₋₄)alkyl, (C₂₋₄)alkenyl, (C₂₋₄)alkynyl, halogen, hydroxy, cyano, nitro, CF₃, O(C₁₋₄alkyl), OCF₃, C(=O)H, C(=O)(C₁₋₄alkyl), CO₂H, CO₂(C₁₋₄alkyl), NHCO₂(C₁₋₄alkyl), -S(C₁₋₄alkyl), -NH₂, NH(C₁₋₄alkyl), N(C₁₋₄alkyl)₂, N(C₁₋₄alkyl)₃⁺, SO₂(C₁₋₄alkyl), C(=O)(C₁₋₄alkylene)NH₂, C(=O)(C₁₋₄alkylene)NH(alkyl), C(=O)(C₁₋₄alkylene)N(C₁₋₄alkyl)₂ and/or phenyl optionally substituted with any of the preceeding groups. As valence allows, if said further ring is cycloalkyl or heterocyclo it is additionally optionally substituted with =O (oxo).

Exemplary monocyclic groups include azetidinyl, pyrrolidinyl, oxetanyl, imidazolinyl, oxazolidinyl, isoxazolinyl, thiazolidinyl, isothiazolidinyl, tetrahydrofuranyl, piperidyl, piperazinyl, 2-oxopiperazinyl, 2-oxopiperidyl, 2-oxopyrrolodinyl, 2-oxoazepinyl, azepinyl, 4-piperidonyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl and the like. Exemplary bicyclic heterocyclo groups include quinuclidinyl.

Preferred heterocyclo groups in compounds of formula (I) include 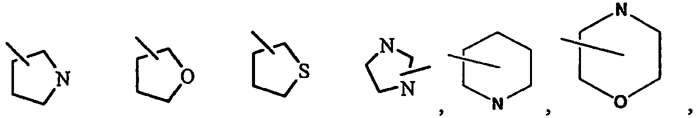 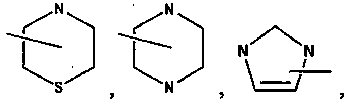 and 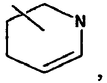 which optionally may be substituted.

The term "heteroaryl" refers to substituted and unsubstituted aromatic 5-or 6-membered monocyclic groups, 9- or 10-membered bicyclic groups, and 11- to 14-membered tricyclic groups which have at least one heteroatom (O, S or N) in at least one of the rings, said heteroatom-containing ring preferably having 1, 2, or 3 heteroatoms selected from O, S, and N. Each ring of the heteroaryl group containing a heteroatom can contain one or two oxygen or sulfur atoms and/or from one to four nitrogen atoms provided that the total number of heteroatoms in each ring is four or less and each ring has at least one carbon atom. The fused rings completing the bicyclic and tricyclic groups may contain only carbon atoms and may be saturated, partially saturated, or unsaturated. The nitrogen and sulfur atoms may optionally be oxidized and the nitrogen atoms may optionally be quatemized. Heteroaryl groups which are bicyclic or tricyclic must include at least one fully aromatic ring but the other fused ring or rings may be aromatic or non-aromatic. The heteroaryl group may be attached at any available nitrogen or carbon atom of any ring. The heteroaryl ring system may contain zero, one, two or three substituents selected from the group consisting of halogen, trifluoromethyl, trifluoromethoxy, alkyl, substituted alkyl, alkenyl, substituted alkenyl, alkynyl, nitro, cyano, ORₐ, SRₐ, (=S), -NRₐR_{b}, - N(alkyl)₃⁺, -NRₐSO₂, -NRₐSO₂R_{c}, -SO₂R_{c} -SO₂NRₐR_{b}, -SO₂NRₐC(=O)R_{b}, SO₃H, - PO(OH)₂, -C(=O)Rₐ,-CO₂Rₐ, -C(=O)NRₐR_{b}, -C(=O)(C₁₋₄alkylene)NRₐR_{b}, - C(=O)NRₐ(SO₂)R_{b}, -CO₂(C₁₋₄alkylene)NRₐR_{b}, -NRₐC(=O)R_{b}, -NRₐCO₂R_{b}, -NRₐ(C₁-₄alkylene)CO₂R_{b}, aryl, cycloalkyl, heterocyclo, and/or heteroaryl, wherein Rₐ, R_{b} and R_{c} are as defined above for substituted alkyl groups, and are also in turn optionally substituted as recited above. When a heteroaryl is substituted with a further ring, said ring in turn is optionally substituted with one to two of (C₁₋₄)alkyl, (C₂-₄)alkenyl, (C₂-₄)alkynyl, halogen, hydroxy, cyano, nitro, CF₃, O(C₁₋₄alkyl), OCF₃, C(=O)H, C(=O)(C₁₋₄alkyl), CO₂H, CO₂(C₁₋₄alkyl), NHCO₂(C₁₋₄alkyl), -S(C₁₋₄alkyl), -NH₂, NH(C₁₋₄alkyl), N(C₁₋₄alkyl)₂, N(C₁₋₄alkyl)₃⁺, SO₂(C₁₋₄alkyl), C(=O)(C₁-₄alkylene)NH₂, C(=O)(C₁₋₄alkylene)NH(alkyl), C(=O)(C₁₋₄alkylene)N(C₁₋₄alkyl)₂ and/or phenyl optionally substituted with any of the preceeding groups. As valence allows, if said further ring is cycloalkyl or heterocyclo it is additionally optionally substituted with =O (oxo).

Exemplary monocyclic heteroaryl groups include pyrrolyl, pyrazolyl, pyrazolinyl, imidazolyl, oxazolyl, isoxazolyl, thiazolyl, thiadiazolyl, isothiazolyl, furanyl, thienyl, oxadiazolyl, pyridyl, pyrazinyl, pyrimidinyl, pyridazinyl, triazinyl and the like.

Exemplary bicyclic heteroaryl groups include indolyl, benzothiazolyl, benzodioxolyl, benzoxazolyl, benzothienyl, quinolinyl, tetrahydroisoquinolinyl, isoquinolinyl, benzimidazolyl, benzopyranyl, indolizinyl, benzofuranyl, chromonyl, coumarinyl, benzopyranyl, cinnolinyl, quinoxalinyl, indazolyl, pyrrolopyridyl, furopyridyl, dihydroisoindolyl, tetrahydroquinolinyl and the like.

Exemplary tricyclic heteroaryl groups include carbazolyl, benzidolyl, phenanthrollinyl, acridinyl, phenanthridinyl, xanthenyl and the like.

In compounds of formula (I), preferred heteroaryl groups include 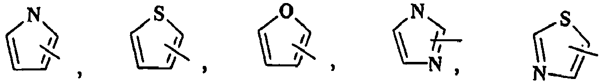 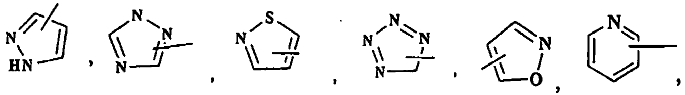 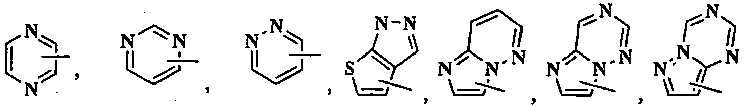 and 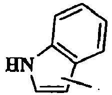 and the like, which optionally may be substituted at any available carbon or nitrogen atom. Aromatic rings may also be designated by an unbroken circle in the ring. For example the core ring of formula (I), 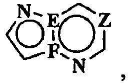 represents a bicyclic heteroaryl group.

Unless otherwise indicated, when reference is made to a specifically-named aryl (*e.g*., phenyl), cycloalkyl (*e.g*., cyclohexyl), heterocyclo (*e.g*., pyrrolidinyl, piperidinyl, and morpholinyl) or heteroaryl (*e.g*., tetrazolyl, imidazolyl, pyrazolyl, triazolyl, thiazolyl, and furyl,) unless otherwise specifically indicated the reference is intended to include rings having 0 to 3, preferably 0-2, substituents selected from those recited above for the aryl, cycloalkyl, heterocyclo and/or heteroaryl groups, as appropriate.

Generally, for a non-formula substituent listing a combination of groups, unless specifically designated otherwise, the last group of the combination is the point of attachment with adjacent groups attached sequentially. Accordingly, for example, the term "aminocyclohexylmethyl is intended" to mean 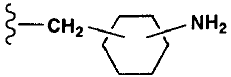 and N-(n-propyl)sulfonamido is intended to mean

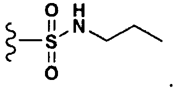

The term "heteroatoms" shall include oxygen, sulfur and nitrogen.

The term "carbocyclic" means a saturated or unsaturated monocyclic or bicyclic ring in which all atoms of all rings are carbon. Thus, the term includes cycloalkyl and aryl rings. The carbocyclic ring may be substituted in which case the substituents are selected from those recited above for cycloalkyl and aryl groups.

When the term "unsaturated" is used herein to refer to a ring or group, the ring or group may be fully unsaturated or partially unsaturated.

When the term "optionally substituted" is used herein to refer to a ring or group, the ring or group may be substituted or unsubstituted.

Throughout the specification, groups and substituents thereof may be chosen by one skilled in the field to provide stable moieties and compounds and compounds useful as pharmaceutically-acceptable compounds and/or intermediate compounds useful in making pharmaceutically-acceptable compounds.

According to the foregoing definitions, the instant invention provides compounds within the scope of formula (I) having the formula (Ia): 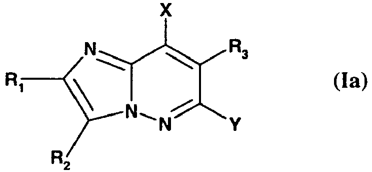 wherein the groups R₁, R₂, R₃, X and Y, are as defined herein.

The compounds of formula (I) can form salts which are also within the scope of this invention. Unless otherwise indicated, reference to an inventive compound is understood to include reference to salts thereof. The term "salt(s)" denotes acidic and/or basic salts formed with inorganic and/or organic acids and bases. In addition, the term "salt(s) may include zwitterions (inner salts), *e.g*., when a compound of formula (I) contains both a basic moiety, such as an amine or a pyridine or imidazole ring, and an acidic moiety, such as a carboxylic acid. Pharmaceutically acceptable (*i.e*., non-toxic, physiologically acceptable) salts are preferred, such as, for example, acceptable metal and amine salts in which the cation does not contribute significantly to the toxicity or biological activity of the salt. However, other salts may be useful, *e.g*., in isolation or purification steps which may be employed during preparation, and thus, are contemplated within the scope of the invention. Salts of the compounds of the formula (I) may be formed, for example, by reacting a compound of the formula (I) with an amount of acid or base, such as an equivalent amount, in a medium such as one in which the salt precipitates or in an aqueous medium followed by lyophilization.

Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides (formed with hydrochloric acid), hydrobromides (formed with hydrogen bromide), hydroiodides, 2-hydroxyethanesulfonates; lactates, maleates (formed with maleic acid), methanesulfonates (formed with methanesulfonic acid), 2-naphthalenesulfonates, nicotinates, nitrates, oxalates, pectinates, persulfates, 3-phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

Exemplary basic salts include ammonium salts, alkali metal salts such as sodium, lithium, and potassium salts; alkaline earth metal salts such as calcium and magnesium salts; barium, zinc, and aluminum salts; salts with organic bases (for example, organic amines) such as trialkylamines such as triethylamine, procaine, dibenzylamine, N-benzyl-β-phenethylamine, 1-ephenamine, N,N-dibenzylethylenediamine, dehydroabietylamine, N-ethylpiperidine, benzylamine, dicyclohexylamine or similar pharmaceutically acceptable amines and salts with amino acids such as arginine, lysine and the like. Basic nitrogen-containing groups may be quaternized with agents such as lower alkyl halides (*e.g*., methyl, ethyl, propyl, and butyl chlorides, bromides and iodides), dialkyl sulfates (*e.g*., dimethyl, diethyl, dibutyl, and diamyl sulfates), long chain halides (*e.g*., decyl, lauryl, myristyl and stearyl chlorides, bromides and iodides), aralkyl halides (*e.g*., benzyl and phenethyl bromides), and others. Preferred salts include monohydrochloride, hydrogensulfate, methanesulfonate, phosphate or nitrate salts.

Prodrugs and solvates of the inventive compounds are also contemplated. The term "prodrug" denotes a compound which, upon administration to a subject, undergoes chemical conversion by metabolic or chemical processes to yield a compound of the formula (I), and/or a salt and/or solvate thereof. Any compound that will be converted *in vivo* to provide the bioactive agent (*i.e*., the compound for formula I) is a prodrug within the scope and spirit of the invention. For example, compounds containing a carboxy group can form physiologically hydrolyzable esters which serve as prodrugs by being hydrolyzed in the body to yield formula (I) compounds *per se.* Such prodrugs are preferably administered orally since hydrolysis in many instances occurs principally under the influence of the digestive enzymes. Parenteral administration may be used where the ester *per se* is active, or in those instances where hydrolysis occurs in the blood. Examples of physiologically hydrolyzable esters of compounds of formula (I) include C₁₋₆alkylbenzyl, 4-methoxybenzyl, indanyl, phthalyl, methoxymethyl, C₁₋₆alkanoyloxy-C₁₋₆alkyl, *e.g*. acetoxymethyl, pivaloyloxymethyl or propionyloxymethyl, C₁₋₆alkoxycarbonyloxy-C₁₋₆alkyl, *e.g*. methoxycarbonyl-oxymethyl or ethoxycarbonyloxymethyl, glycyloxymethyl, phenylglycyloxymethyl, (5-methyl-2-oxo-1,3-dioxolen-4-yl)-methyl and other well known physiologically hydrolyzable esters used, for example, in the penicillin and cephalosporin arts. Such esters may be prepared by conventional techniques known in the art.

Various forms of prodrugs are well known in the art. For examples of such prodrug derivatives, see:
a) Design of Prodrugs, edited by H. Bundgaard, (Elsevier, 1985) and Methods in Enzymology, Vol. 112, pp. 309-396, edited by K. Widder, et al. (Acamedic Press, 1985);
b) A Textbook of Drug Design and Development, edited by Krosgaard-Larsen and H. Bundgaard, Chapter 5, "Design and Application of Prodrugs," by H. Bundgaard, pp. 113-191 (1991); and
c) H. Bundgaard, Advanced Drug Delivery Reviews, Vol. 8, pp. 1-38 (1992), each of which is incorporated herein by reference.

Compounds of the formula (I) and salts thereof may exist in their tautomeric form, in which hydrogen atoms are transposed to other parts of the molecules and the chemical bonds between the atoms of the molecules are consequently rearranged. It should be understood that the all tautomeric forms, insofar as they may exist, are included within the invention. Additionally, inventive compounds may have *trans* and *cis* isomers and may contain one or more chiral centers, therefore existing in enantiomeric and diastereomeric forms. The invention includes all such isomers, as well as mixtures of *cis* and *trans* isomers, mixtures of diastereomers and racemic mixtures of enantiomers (optical isomers). When no specific mention is made of the configuration (*cis*, *trans* or R or S) of a compound (or of an asymmetric carbon), then any one of the isomers or a mixture of more than one isomer is intended. The processes for preparation can use racemates, enantiomers or diastereomers as starting materials. When enantiomeric or diastereomeric products are prepared, they can be separated by conventional methods for example, chromatographic or fractional crystallization. The inventive compounds may be in the free or hydrate form.

It should further be understood that solvates (*e.g.*, hydrates) of the compounds of Formula (I) are also with the scope of the present invention. Methods of solvation are generally known in the art.

### PREFERRED COMPOUNDS

Preferred compounds are those within the scope of formula (I) (above) have the following formula (Ia): 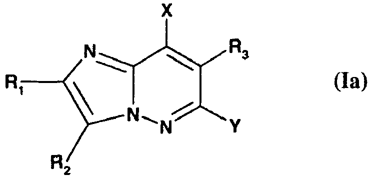 their enantiomers, diastereomers, a pharmaceutically-acceptable salt, or hydrate, thereof.

Other preferred compounds, including enantiomers, diastereomers, a pharmaceutically-acceptable salt, or hydrate, thereof, within the scope of formula (Ia), are those in which:
R₄ is -AM;
R₅ is hydrogen or C₁₋₄alkyl (more preferably R₅ is hydrogen or methyl);
or R₄ and R₅ together with the nitrogen atom to which they are attached form a 5-, 6-or 7-membered monocyclic heteroaryl or heterocyclo ring, or a 7- to 11-membered bicyclic heteroaryl or heterocyclo ring, each ring optionally substituted with one to three groups, T₁, T₂; and/or T₃;
A is a bond, C₁₋₃alkylene, C₂₋₄alkenylene, C₂₋₄alkynylene, -C(O)-, or -SO₂-;
M is (i) hydrogen, alkyl, alkoxy, or alkenyl; or (ii) cycloalkyl, heterocyclo, aryl, or heteroaryl, each group optionally substituted by one to three groups, T₁, T₂, and/or T₃;
T₁, T₂, and T₃ are independently selected from (i) halogen, alkyl, substituted alkyl, alkenyl, substituted alkenyl, alkynyl, substituted alkynyl, nitro, cyano, SO₃H SR₁₉, S(O)*ₚ*R₂₁, S(O)*ₚ*NR₁₉R₂₀, NR₁₉S(O)*ₚ*R₂₁, OR₁₉, NR₁₉R₂₀, NR₁₉C(=O)₂₀, NR₁₉C(=O)NR₁₉R₂₀, CO₂R₁₉, C(=O)R₁₉, -O-C(=O)R₁₉, -C(=O)NR₁₉R₂₀, cycloalkyl, heterocyclo, aryl, and heteroaryl, wherein *p* is one or 2; and/or (ii) two groups, T₁ and T₂, located on adjacent ring atoms are taken together with the ring atoms to which they are attached to form a fused cycloalkyl, aryl, heteroaryl, or heterocyclo;
R₁₉, R₂₀, and R₂₁ at each occurrence, are selected independently from (i) hydrogen, alkyl, substituted alkyl, alkenyl, substituted alkenyl, cycloalkyl, aryl, heteroaryl, and heterocyclo; or (ii) R₁₉ and R₂₀ together with the nitrogen atom to which they are both attached form a heteroaryl or heterocyclo; and
R₂₁ at each occurrence, is selected from alkyl, substituted alkyl, alkenyl, substituted alkenyl, alkynyl, substituted alkynyl, cycloalkyl, aryl, heteroaryl, and heterocyclo.

More preferred compounds, including enantiomers, diastereomers, a pharmaceutically-acceptable salt, or hydrate, thereof, within the scope of formula (I) are those in which
R₄ is -AM;
A is a bond, -C(O)-, or -S(O)₂-, or C₁₋₃alkylene (A is more preferably a bond; methylene, or ethylene, especially a bond);
M is (i) hydrogen, -NH(aryl), C₁₋₆alkyl, C₂₋₄alkenyl, or -OC₁₋₄alkyl or (ii) C₃₋₆cycloakyl, phenyl, fluorenyl, 1-naphthyl, or 2-naphthyl, each group optionally substituted by one to three groups, T₁, T₂, and/or T₃; or (iii) a 5-, 6-or 7-membered monocyclic or a 7- to 11-membered bicyclic heteroaryl or heterocyclo ring, each ring optionally substituted by one to three groups, T₁, T₂, and/or T₃ (is more preferably a C₃₋₆cycloalkyl, or a 5-, 6-, or 7-membered aryl, heteroaryl, or heteroaryl ring, each ring optionally substituted by 1 to 3 groups, T₁, T₂, and/or T₃, especially a 5-, 6-, or 7-membered aryl, heteroaryl, or heteroaryl ring, each ring optionally substituted by 1 to 2 groups, T₁ and/or T₂); and
T₁, T₂, and T₃ are independently selected from (i) C₁₋₄alkyl, substituted C₁₋₄alkyl, C₁₋₄alkyloxy, substitutedC₁₋₄alkyloxy, C₁₋₄alkylthio, phenoxy, -NR₁₉R₂₀, halogen, hydroxy, cyano, SO₃H, COOH, -C(O)(R₁₉), C(O)NR₁₉R₂₀, NR₁₉C(O)R₂₀, S(O)₂R₂₁, S(O)₂NR₁₉R₂₀ and NR₁₉(C(O)NR₁₉R₂₀; and/or (ii) phenyl, cyclopropyl, cyclohexyl, tetrazolyl, imidazolyl, pyrazolyl, triazolyl, thiazolyl, furyl, and morpholinyl, each group of which is optionally substituted as valence allows from one to three groups, R₂₂, R₂₃ and/or R₂₄; and/or (iii) two groups, T₁ and T₂, substituted on adjacent ring atoms are taken together with the ring atoms to which they are attached to form, a fused five- to seven-membered cycloalkyl, a fused phenyl or a fused 5- or 6-membered heterocyclo or heteroaryl, each group of which is optionally substituted as valence allows from one to three groups, R₂₂, R₂₃ and/or R₂₄; and
R₁₉ and R₂₀ at each occurrence are selected independently from (i) hydrogen, - (CH₂)ᵥOH, and C₁₋₄alkyl; or (ii) -(CH₂)ᵥcyclohexyl, -(CH₂)ᵥphenyl, - (CH₂)ᵥmorpholinyl, -(CH₂)ᵥpyridyl, -(CH₂)ᵥpyrazolyl, -(CH₂)ᵥcyclopropyl, - (CH₂)ᵥpyrrolidinyl, -(CH₂)ᵥpiperidinyl, -(CH₂)ᵥfuryl, -(CH₂)ᵥimidazolyl, - (CH₂)ᵥpyrimidinyl, -(CH₂)ᵥpiperazinyl, and -(CH₂)ᵥ pyradizinyl, each group of which is optionally substituted as valence allows from one to three groups, R₂₂, R₂₃ and/or R₂₄; or R₁₉ and R₂₀ are taken together with the nitrogen atom to which they are both attached to form a pyrrolindyl, morpholinyl, piperidinyl, pyradazinyl, or piperazinyl, each group of which is optionally substituted as valence allows from one to three groups, R₂₂, R₂₃ and/or R₂₄;
R₂₁ at each occurrence is selected from (i) -(CH₂)ᵥOH, and C₁₋₄alkyl; or (ii) - (CH₂)ᵥcyclohexyl, -(CH₂)ᵥphenyl, -(CH₂)ᵥmorpholinyl, -(CH₂)ᵥpyridyl, - (CH₂)ᵥpyrazolyl, -(CH₂)ᵥcyclopropyl, -(CH₂)ᵥpyrolidinyl, -(CH₂)ᵥpiperidinyl, -(CH₂)ᵥfuryl, -(CH₂)ᵥimidazolyl, -(CH₂)ᵥpyrimidinyl, -(CH₂)ᵥpiperazinyl, and - (CH₂)ᵥ pyradizinyl, each group of which is optionally substituted as valence allows from one to three groups, R₂₂, R₂₃ and/or R₂₄;R₂₂, R₂₃, and R₂₄ at each occurrence, are selected independently from (C₁₋₄)alkyl, (C₂₋₄)alkenyl, halogen, hydroxy, cyano, nitro, CF₃, =O, O(C₁₋₄alkyl), OCF₃, C(=O)H, C(=O)(C₁₋₄alkyl), CO₂H, CO₂(C₁₋₄alkyl), NHCO₂(C₁₋₄alkyl), -S(C₁₋₄alkyl), - NH₂, NH(C₁₋₄alkyl), N(C₁₋₄alkyl)₂, N(C₁₋₄alkyl)₃⁺, SO₂(C₁₋₄alkyl), C(=O)(C₁₋₄alkylene)NH₂, C(=O)(C₁₋₄alkylene)NH(alkyl), C(=O)(C₁₋₄alkylene)N(C₁₋₄alkyl)₂, and optionally substituted phenyl; and v is 0, 1, 2, or 3.

Other more preferred compounds, including enantiomers, diastereomers, a pharmaceutically-acceptable salt, or hydrate, thereof, within the scope of formula (I) are those in which
A is a bond, methylene, or ethylene;
M is hydrogen, methoxy, phenyl, fluorenyl, pyridyl, cyclopropyl, cyclohexyl, isopropyl, ethyl, n-propenyl, isopentyl, n-propyl, n-butyl, pyrazolyl, or pyrimidinyl, each group optionally substituted by one to two groups selected from T₁ and T₂; and
T₁ and T₂ are independently selected from ethoxy, methoxy, methyl, n-butoxy, phenyl, benzyloxy, dimethylamino, chloro, iodo, trifluoromethyl, fluoro, hydroxy, cyano, carboxylic acid, N-methyl-N-(pyridinylethyl)amido, ethyltetrazole, phenoxy, chlorophenyl, methylphenyl, benzyl, morpholinyl, isopropyl, n-propyl, n-butyl, ethyl, isopropoxy, n-propoxy, methylthio, cyclohexyl, t-butyl, trifluoromethoxy, amino, triazolyl, dichloroimidazolyl, dimethylpyrazolyl, methyltriazolyl, methylimidazolyl, methylthiazolyl, methylfuryl, N,N-dimethylamido, phenylsulfonyl, morpholinylsulfonyl, pyrrolidinylsulfonyl, N,N-diethylamido, N-methylamido, N-methylsulfonamido, N-methylsulfonamido, methanesulfonamido, N,N-dimethylsulfonamido, N, N-diethylsulfonamido, N-propylsulfonamido, N-ethylsulfonamido, N-methylsulfonamido, sulfonamido, aminomethyl, amido, N-(furylmethyl)amido, N-(imidazolylmethyl)amido; N-(pyridylmethyl)amido, (phenylpiperidinyl)carbonyl, piperidinylcarbonyl, N-benzylamido, N-methoxyphenylamido, N-phenylamido, N-(hydroxyethyl)amido, 1-morpholinylcarbonyl, N-(pyridinyl)amido, N-(pyridinylmethyl)amido, N-(pyridinylethyl)amido, N,N-diethylamido, N-cyclopropylamido, N-(cyclohexylmethyl)amido, N-(cyclohexyl)amido N-(methylpyrazolyl)amido, N-((oxopyrrolidinyl)propyl)amido, 3-phenylurea, and 1-(fluorophenyl)-N-methyl-oxo-dihydropyridine-3-carboxamido;
or T₁ and T₂ substituted on adjacent atoms of M combine with the atoms to which they are attached to form a fused ring thereby forming a ring system selected from indolyl, methylbenzothiazolyl, napthyl, methylindolyl, tetrahydroquinolinyl, fluorenyl, quinolinyl, and dihydroindazol-one-yl.

Other preferred compounds, including enantiomers, diastereomers, a pharmaceutically-acceptable salt, or hydrate, thereof, within the scope of formula (I) are those in which
Y is hydrogen, halogen, OR₈, or NR₆R₇ (Y is more preferably NR₆R₇);
R₆ is selected from hydrogen or C₁₋₄alkyl optionally substituted by one to three groups selected from halogen, C₁₋₄alkyl, nitro, cyano, amino, C₁₋₄alkoxy, and OH (R6 is more preferably hydrogen or C₁₋₄alkyl);
R₇ and R₈ are independently selected from alkyl, cycloalkyl, heterocyclo, aryl, and heteroaryl, each group of which is optionally substituted by one to three groups, T₄, T₅, and/or T₆ (R₇ is more preferably C₁₋₄alkyl, C₃₋₆cycloalkyl, or a 5-, 6-, or 7-membered heterocyclo, each group optionally substituted by one to three groups T₄, T₅, and/or T₆; R₈ is more preferably C₃₋₆cycloalkyl, especially cyclohexyl substituted by C₁₋₄alkyl, amino, amino substituted with C₁₋₄alkyl, substituted C₁₋₄alkyl, furyl, or piperidinyl);
or R₆ and R₇ together with the nitrogen atom to which they are attached form a heteroaryl or heterocyclo ring (more preferably 5-, 6-, or 7-membered rings), each ring is optionally substituted by one to three groups, T₄, T₅, and/or T₆;
T₄, T₅ and T₆ are independently selected from (i) halogen, alkyl, substituted alkyl, alkenyl, substituted alkenyl, alkynyl, substituted alkynyl, nitro, cyano, SR₁₉, OR₁₉, NR₁₉R₂₀, NR₁₉C(=O)R₂₀, CO₂R¹⁹, C(=O)R₁₉, -O-C(=O)R₁₉, - C(=O)NR₁₉R₂₀, cycloalkyl, heterocyclo, aryl, and heteroaryl; and/or (ii) two groups, T₄ and T₅, substituted on adjacent ring atoms are taken together with the ring atoms to which they are attached to form a fused cyclalkyl, heterocyclo, aryl, or heteroaryl (T₄, T₅ and T₆ are more preferably C₁₋₄alkyl, and NR₁₉R₂₀); and
R₁₉ and R₂₀, at each occurrence are selected independently from (i) hydrogen, alkyl, substituted alkyl, alkenyl, substituted alkenyl, cycloalkyl, aryl, heteroaryl, and heterocyclo; or (ii) R₁₉ with R₂₀ together with the nitrogen atom to which they are both attached combine to form a heteroaryl or heterocyclo.

Other more preferred compounds, including enantiomers, diastereomers, a pharmaceutically-acceptable salt, or hydrate, thereof, within the scope of formula (I) are those in which
Y is NR₆R₇;
R₆ is selected from hydrogen or C₁₋₄alkyl (R₆ is more preferably hydrogen);
R₇ is selected from C₁₋₄alkyl, cyclopentyl, cyclohexyl, bicyclo[2.2.2]octyl, pyrrolidinyl, and piperidinyl, each group of which is optionally substituted by one to three groups, T₄, T₅, and/or T₆ (R₇ is more preferably cyclohexyl and bicycle {222]octyl, each group substituted by one group, T₄);
or R₆ and R₇ together with the nitrogen atom to which they are attached form piperazinyl, piperidinyl, pyrrolidinyl, or diazepanyl, each group of which is optionally substituted by one to three groups, T₄, T₅, and/or T₆ (more preferably R₆ and R₇ are taken together with the nitrogen atom to which they are both attached form an unsubstituted piperidinyl ring); and
T₄, T₅, and T₆ are independently selected from (i) C₁₋₄alkyl, OH, NH₂, NH(C₁₋₄alkyl), furyl, and N(C₁₋₄alkyl)₂, and NH(pyrimidinyl) wherein the pyrimidinyl is substituted by halogen; or (ii) C₁₋₄alkyl substituted by cyclohexyl or OH, wherein the cyclohexyl is substituted by NH₂. (More preferably T₄, T₅, and T₆ are selected from NH₂, NH(C₁₋₄alkyl), and (4-NH₂-cyclohexyl)methyl).

Yet other preferred compounds, including enantiomers, diastereomers, a pharmaceutically-acceptable salt, or hydrate, thereof, within the scope of formula (I) are those in which
R₆ is hydrogen;
R₇ is methyl, ethyl, n-propyl, n-butyl, cyclopentyl, cyclohexyl, bicyclo[2.2.2]octane, pyrrolidinyl, or piperidinyl, each group of which is optionally substituted by T₄ selected from amino, methyl, aminocyclohexylmethyl, dimethylamino, furyl, ethylamino, methylamino, piperidinyl, and (chloropyrimidinyl)amino; or
R₆ and R₇ together with the nitrogen atom to which they are attached form a piperazinyl, piperidinyl, pyrrolidinyl, and diazepanyl ring, each ring optionally substituted by T₄ selected from amino, hydroxyethyl, aminopyrrolidinyl, and methyl.

Other preferred compounds, including enantiomers, diastereomers, a pharmaceutically-acceptable salt, or hydrate, thereof, are those within the scope of formula (I) (above) having the formula (Ia), in which: 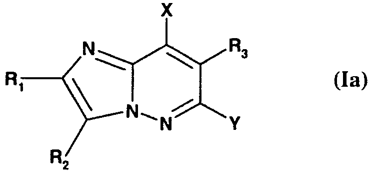 or an enantiomer, diastereomer, or a pharmaceutically-acceptable salt, thereof,
wherein:
X is NR₄R₅;
Y is hydrogen, halogen, OR₈, or NR₆R₇ (Y is more preferably NR₆R₇);
R₁ and R₂ are independently selected from hydrogen, halogen, OR₁₀, cyano, C₁₋₄alkyl, CO₂R₁₀, and C(O)NR₁₀R₁₁;
R₃ is selected from (i) hydrogen, halogen, nitro, cyano, OR₁₀, NR₁₀R₁₁, CO₂R₁₀, and C(=O)R₁₀, (ii) C₁₋₄alkyl, substituted C₁₋₄alkyl, cycloalkyl, aryl, and heteroaryl;
R₄ is -AM;
R₅ is hydrogen or C₁₋₄alkyl;
or R₄ and R₅ together with the nitrogen atom to which they are attached form a 5-, 6-or 7-membered monocyclic heteroaryl or heterocyclo ring, or a 7- to 11-membered bicyclic heteroaryl or heterocyclo ring, each ring optionally substituted with one to three groups, T₁, T₂; and/or T₃;
A is a bond, C₁₋₃alkylene, C₂₋₄alkenylene, C₂₋₄alkynylene, -C(O)-, or -SO₂-;
M is (i) hydrogen, NR₁₅R₁₆, alkyl, alkoxy, or alkenyl; or (ii) cycloalkyl, heterocyclo, aryl, or heteroaryl, each ring optionally substituted by one to three groups, T₁, T₂, and/or T₃;
R₆ is selected from hydrogen or C₁₋₄alkyl optionally substituted by one to three groups selected from halogen, C₁₋₄alkyl, nitro, cyano, amino, C₁₋₄alkoxy, and OH;
R₇ and R₈ are independently selected from alkyl, cycloalkyl, heterocyclo, aryl, and heteroaryl, each group of which is optionally substituted by one to three groups, T₄, T₅, and/or T₆ (R₈ is preferably C₃₋₆cycloalkyl, especially cyclohexyl optionally substituted by NH₂, NH(C₁₋₄alkyl), and (4-NH₂-cyclohexyl)methyl);
or R₆ and R₇ together with the nitrogen atom to which they are attached form a heteroaryl or heterocyclo ring, each ring is optionally substituted by one to three groups, T₄, T₅, and/or T₆;
R₁₀ and R₁₁ at each occurrence are independently selected from (i) hydrogen, C₁₋₄alkyl, and substituted C₁₋₄alkyl; or (ii) R₁₀ and R₁₁ together with the nitrogen atom they are both attached combine to form an optionally substituted 5-, 6-, or 7-membered heteroaryl or heterocyclo;
R₁₅ and R₁₆ are independently selected from (i) hydrogen, alkyl, substituted alkyl, alkenyl, substituted alkenyl, alkynyl, substituted alkynyl, cycloalkyl, aryl, heteroaryl, and heterocyclo; or (ii) together with the nitrogen atom to which they are attached R₁₅ is taken together with R₁₆ to form a heteroaryl or heterocyclo;
T₁, T₂, and T₃ are independently selected from (i) halogen, alkyl, substituted alkyl, alkenyl, substituted alkenyl, alkynyl, substituted alkynyl, nitro, cyano, SO₃H SR₁₉, S(O)*ₚ*R₂₁, S(O)*ₚ*NR₁₉R₂₀, NR₁₉S(O)*ₚ*R₂₁, OR₁₉, NR₁₉R₂₀, NR₁₉C(=O)₂₀, NR₁₉C(=O)NR₁₉R₂₀, CO₂R₁₉, C(=O)R₁₉, -O-C(=O)R₁₉, -C(=O)NR₁₉R₂₀, cycloalkyl, heterocyclo, aryl, and heteroaryl, wherein p is one or 2; and/or (ii) two groups, T₁ and T₂, located on adjacent ring atoms are taken together with the ring atoms to which they are attached to form a fused cycloalkyl, aryl, heteroaryl, or heterocyclo;
T₄, T₅ and T₆ are independently selected from (i) halogen, alkyl, substituted alkyl, alkenyl, substituted alkenyl, alkynyl, substituted alkynyl, nitro, cyano, SR₁₉, OR₁₉, NR₁₉R₂₀, NR₁₉C(=O)R₂₀, CO₂R₁₉, C(=O)R₁₉, -O-C(=O)R₁₉, - C(=O)NR₁₉R₂₀, cycloalkyl, heterocyclo, aryl, and heteroaryl; and/or (ii) two groups, T₄ and T₅, substituted on adjacent ring atoms are taken together with the ring atoms to which they are attached to form a fused cyclalkyl, heterocyclo, aryl, or heteroaryl; and
R₁₉ and R₂₀ at each occurrence are selected independently from (i) hydrogen, alkyl, substituted alkyl, alkenyl, substituted alkenyl, cycloalkyl, aryl, heteroaryl, and heterocyclo; or (ii) R₁₉ and R₂₀ together with the nitrogen atom to which they are both attached form a heteroaryl or heterocyclo ring; and
R₂₁ at each occurrence, is selected from alkyl, substituted alkyl, alkenyl, substituted alkenyl, alkynyl, substituted alkynyl, cycloalkyl, aryl, heteroaryl, and heterocyclo;
with the following provisos:
(1) if X is NH(Me), N(Me)₂, NH(unsubstituted phenyl), or NHNH₂. then Y is other than hydrogen or halogen; and
(2) the following compounds are excluded:

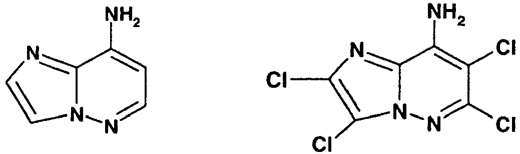

Particularly preferred compounds, including enantiomers, diastereomers, a pharmaceutically-acceptable salt, or hydrate, thereof, are those within the scope of formula (I) (above) having the formula (Ia), in which:
R₁ and R₂ are independently selected from hydrogen, halogen, OR₁₀, cyano, C₁₋₄alkyl, CO₂R₁₀, and -C(O)NR₁₀R₁₁ (more preferred R₁ groups are selected from hydrogen and C₁₋₄alkyl, especially hydrogen and methyl; more preferred R₂ groups are selected from hydrogen, halogen, cyano, nitro, alkyoxy, hydroxy, C(O)NH₂ and substituted amido (N, N- di-substituted by (i) one hydrogen and/or (ii) one to two groups selected from alkyl, alkenyl, C₃₋₇cycloalkyl, phenyl, benzyl, phenylethyl, napthyl, a four to seven membered heterocylo, and a five to six membered heteroaryl; or (iii) when attached to the same nitrogen atom two groups may join to form a heterocyclo or heteroaryl); R₂ is especially hydrogen, cyano, fluoro, or C(O)NH₂).
R₃ is selected from (i) hydrogen, halogen, nitro, cyano, OR₁₀, NR₁₀R₁₁, CO₂R₁₀, and C(=O)R₁₀; or (ii) C₁₋₄alkyl, cycloalkyl, aryl, and heteroaryl, each group of which is optionally substituted (more preferred R₃ groups: hydrogen, halogen, C₁₋₄alkyl, and aryl, especially: hydrogen, chloro, methyl, ethyl, isopropyl, and phenyl); and
R₁₀ and R₁₁ are independently selected from (i) hydrogen, C₁₋₄alkyl, and substituted C₁₋₄alkyl (preferred substitutions: one to three groups independently selected from hydrogen, halogen, nitro, cyano, OH, phenyl, and OC₁₋₄alkyl); or (ii) R₁₀ and R₁₁ together with the nitrogen atom to which they are both attached combine to form an optionally substituted 5-, 6-, or 7-membered heteroaryl or heterocyclo..

Even more particularly preferred compounds, including enantiomers, diastereomers, a pharmaceutically-acceptable salt, or hydrate, thereof, are those within the scope of formula (I) (above) having the formula (Ia), in which NR₄R₅ is selected from:

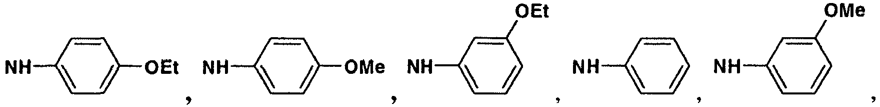

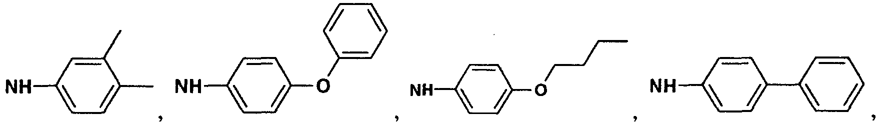

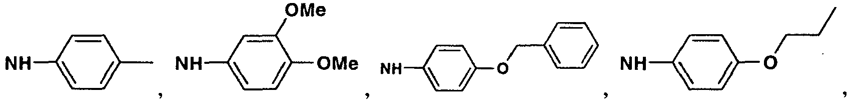

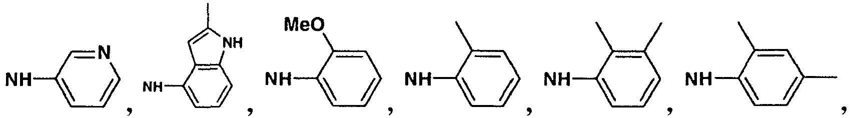

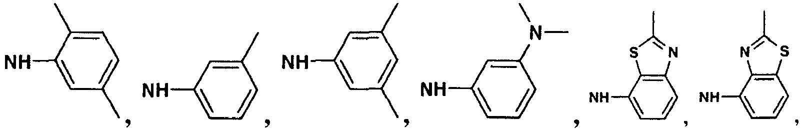

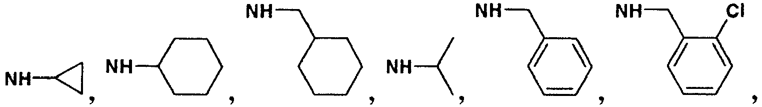

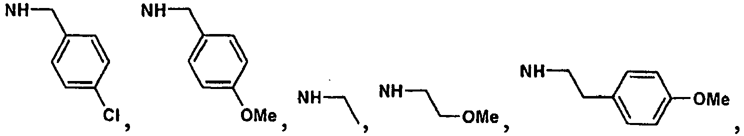

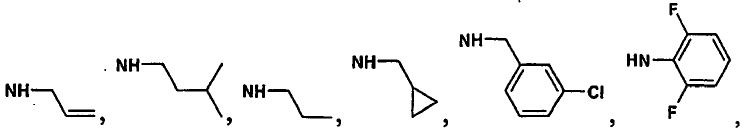

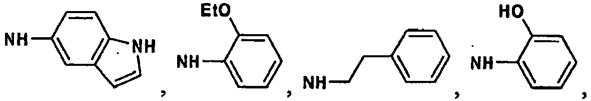

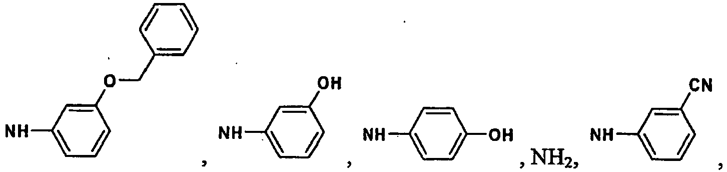

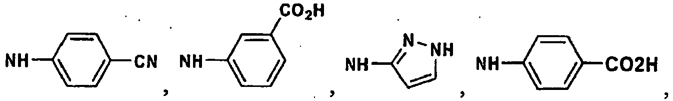

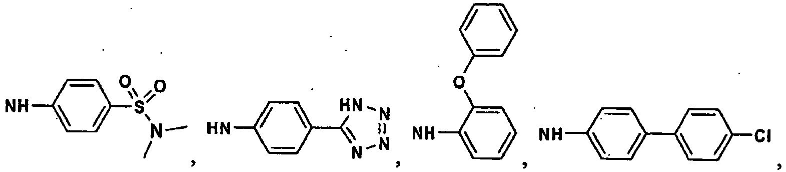

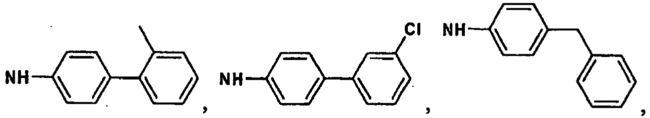

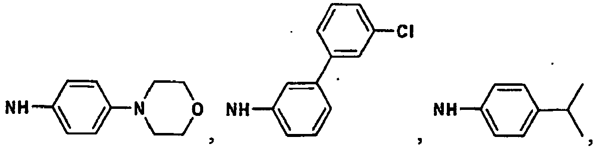

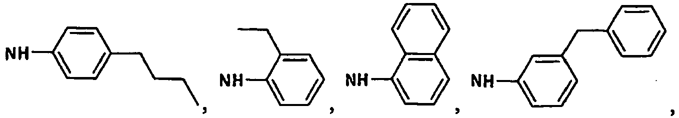

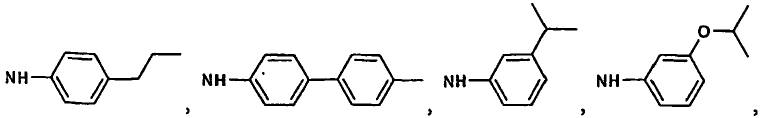

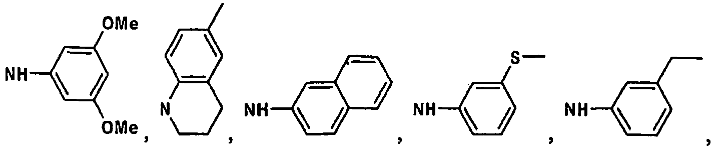

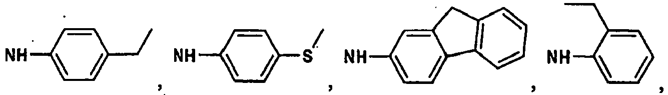

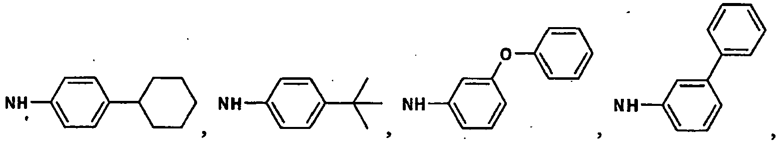

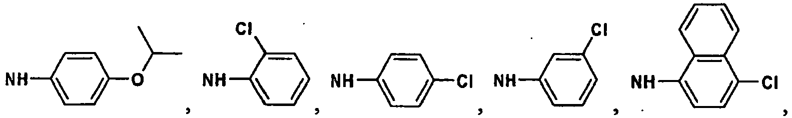

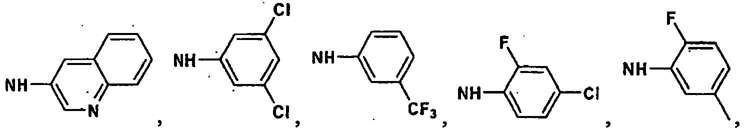

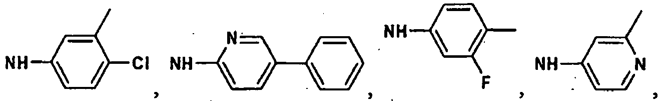

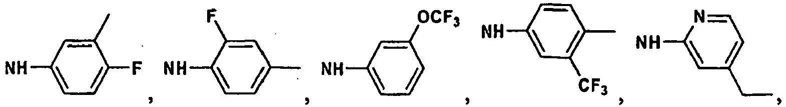

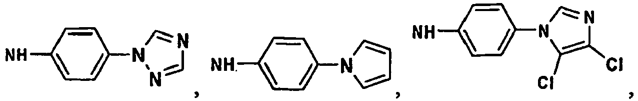

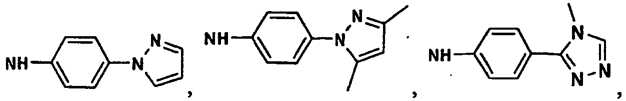

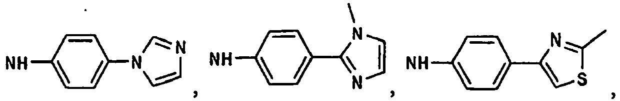

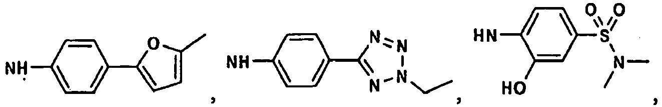

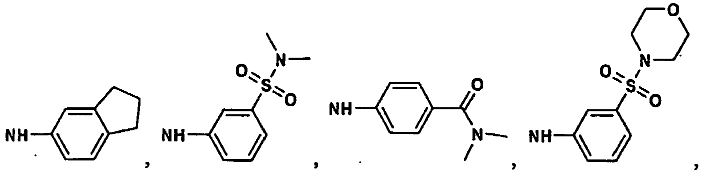

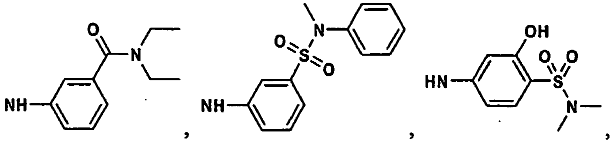

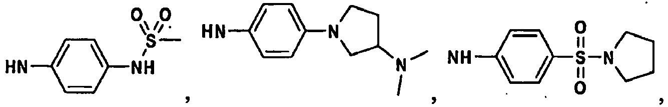

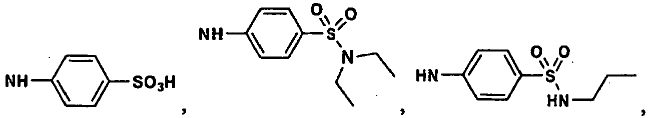

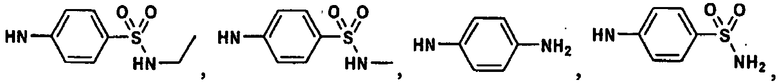

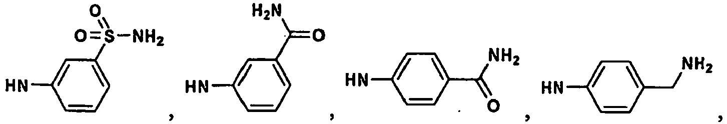

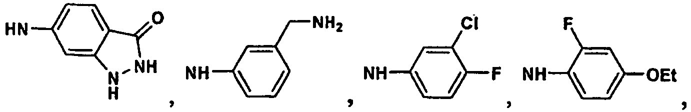

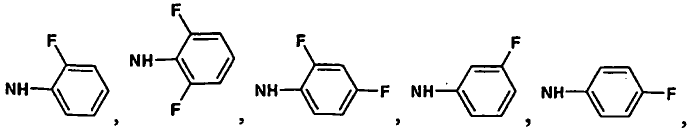

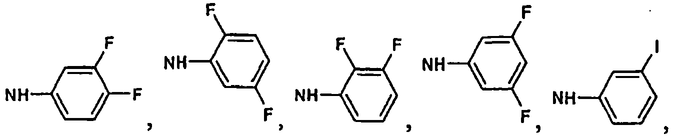

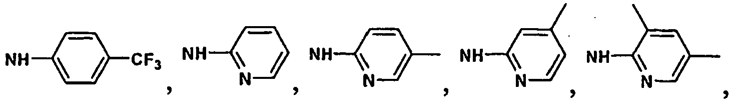

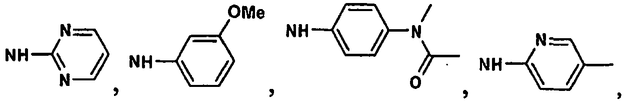

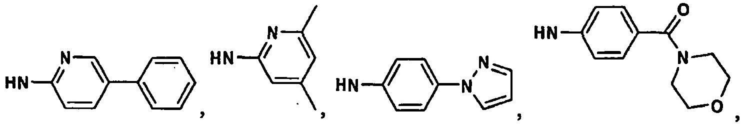

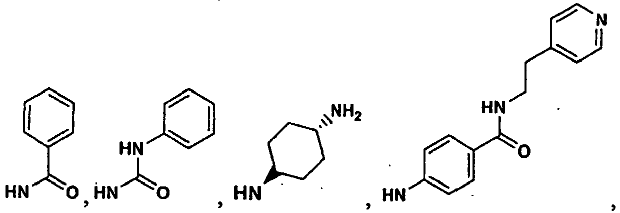

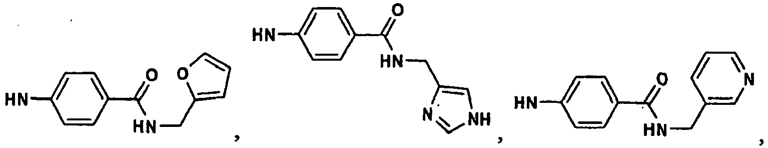

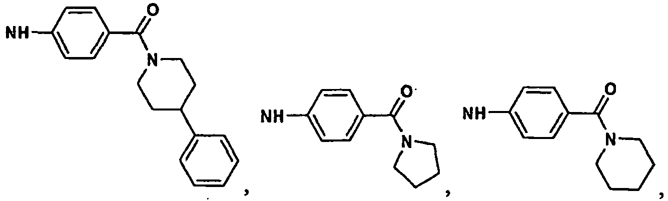

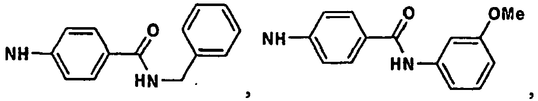

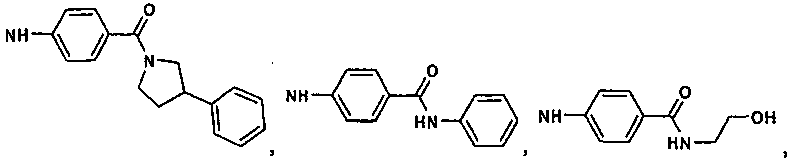

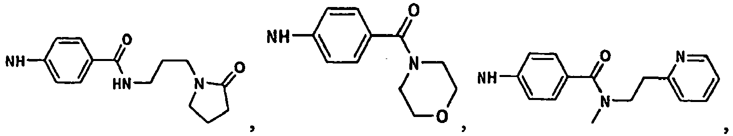

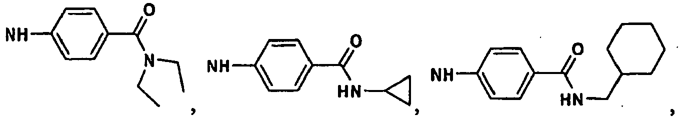

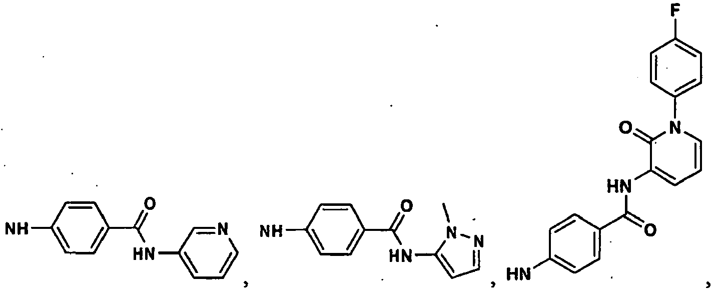

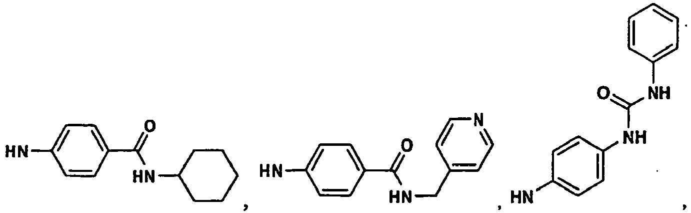

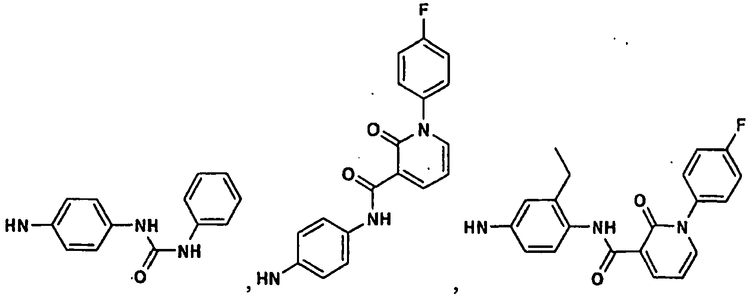

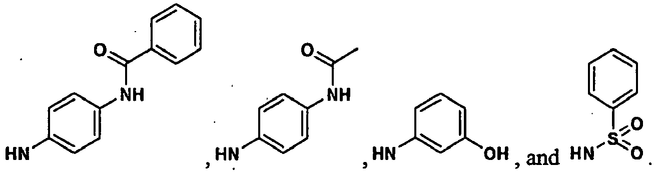

Alternative even more particularly preferred compounds, including enantiomers, diastereomers, a pharmaceutically-acceptable salt, or hydrate, thereof, are those within the scope of formula (I) in which NR₆R₇ is selected from: NH-(CH₂)₂-NH₂, NH-(CH₂)₄-NH₂,

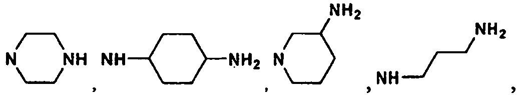

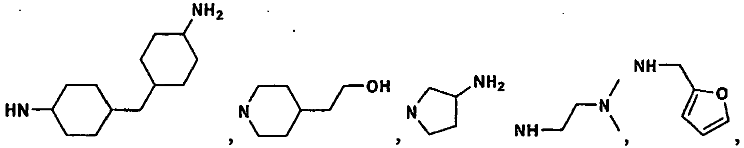

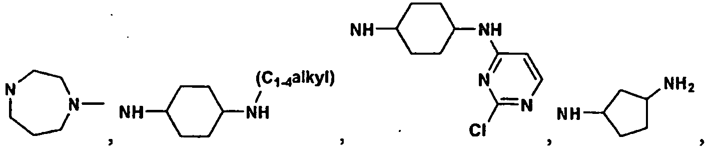

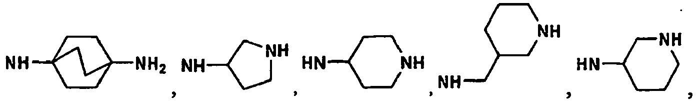

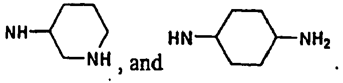

Other preferred compounds within the scope of formula (I), or an enantiomer, diastereomer, or a pharmaceutically-acceptable salt, thereof wherein:
R₄ is selected from a C₃₋₆cycloalkyl, a 5-, 6-, or 7-membered aryl, heteroaryl, or a heteroaryl ring optionally substituted by 1 to 3 groups selected from T₁, T₂, and T₃, (More preferably, R₄ is selected from phenyl, pyridyl, pyrimidinyl, cyclohexyl, and piperidinyl, each group optionally substituted by 1 to 2 groups, T₁ and/or T₂. T₁ and/or T₂ are preferably selected from ethoxy, methoxy, methyl, ethyl, n-butoxy, phenyl, benzyloxy, dimethylamino, chloro, iodo, trifluoromethyl, fluoro, hydroxyl, cyano, carboxylic acid, N-methyl-N-(pyridinylethyl)amido, ethyltetrazole, phenoxy, chlorophenyl, methylphenyl, benzyl, morpholinyl, isopropyl, n-propyl, n-butyl, ethyl, isopropoxy, n-propoxy, methylthio, cyclohexyl, t-butyl, chloro, trifluoromethoxy, amino, triazolyl, dichloroimidazolyl, dimethylpyrazolyl, methyltriazolyl, methylimidazolyl, methylthiazolyl, methylfuryl, N,N-dimethylamido, morpholinylsulfonyl, pyrrolidinylsulfonyl, N,N-diethylamido, N-methylamido, N-methylsulfonamido, N-methylsulfonamido, methanesulfonamido, N,N-dimethylsulfonamido, N, N-diethylsulfonamido, N-propylsulfonamido, N-ethylsulfonamido, N-methylsulfonamido, sulfonamido, aminomethyl, amido, N-(furylmethyl)amido, N-(imidazolylmethyl)amido; N-(pyridylinethyl)amido, (phenylpiperidinyl)carbonyl, piperidinylcarbonyl, N-benzylamido, N-methoxyphenylamido, N-phenylamido, N-(hydroxyethyl)amido, 1-morpholinylcarbonyl, N-(pyridinyl)amido, N-(pyridinylmethyl)amido, N-(pyridinylethyl)amido, N,N-diethylamido, N-cyclopropylamido, N-(cyclohexylmethyl)amido, N-(cyclohexyl)amido N-(methylpyrazolyl)amido, N-((oxopyrrolidinyl)propyl)amido, phenylurea, and 1-(fluorophenyl)-N-methyl-oxo-dihydropyridine-3-carboxamido);
or T₁ and T₂ located on adjacent atoms of M together with the atoms to which they are attached combine to form a fused ring thereby forming a fused ring system selected from indolyl, methylbenzothiazolyl, napthyl, methylindolyl, tetrahydroquinolinyl, fluorenyl, quinolinyl, and dihydroindazol-one-yl.

Yet other preferred compounds, including enantiomers, diastereomers, a pharmaceutically-acceptable salt, or hydrate, thereof, are those within the scope of formula (I) in which NR₆R₇ is selected from: 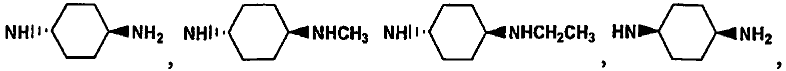 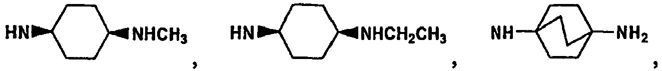 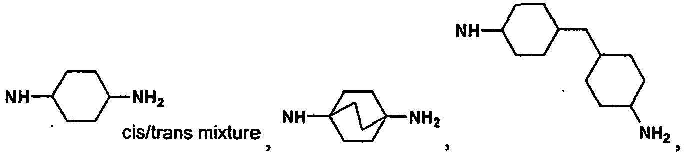 and

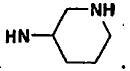

All aspects of the preferred compounds, including individual variable definitions, may be combined with other aspects to form other preferred compounds.

### METHODS OF PREPARATION

Compounds of the present invention may be prepared by the exemplary processes described in the following reaction schemes, A to E. Exemplary reagents and procedures for these reactions appear hereinafter. Starting materials are commercially available or can be readily prepared by one of ordinary skill in the art. Modifications can be made to the methods of schemes by one skilled in the art using known methods. For all of the schemes, the groups R₁, R₂, are as described herein for a compound of formula (I), unless otherwise indicated. Groups designated generally as R', R", Z, P' and P" as well as appropriate solvents, temperatures, pressures, starting materials (having the desired substituents), and other reaction conditions, may be readily selected by one of ordinary skill in the art. It is anticipated that, where possible, the products of the reaction schemes described below may be further elaborated by one of ordinary skill in the art

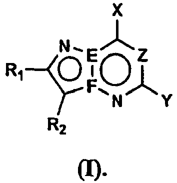

Compounds of general formula (I) where **E** = **C** and **F** = **N**, and **Z** = **CR3** (i.e. formula (Ia)) may be prepared as described below in Schemes A, B and C.

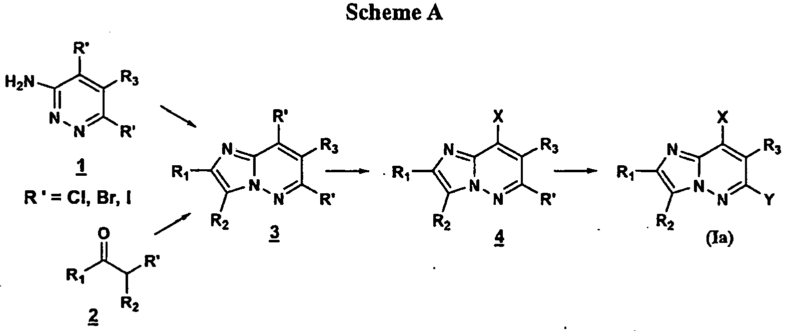

Readily prepared 3-amino-4,6-dihalopyridazines **1** are condensed with commercially available or readily prepared 2-haloaldehydes or 2-haloketones **2** or their equivalents to provide 6,8-dihaloimidazo[1,2-b]pyridazines **3** in an alcoholic solvent (such as ethanol). The reaction of **3** with an amine in a suitable solvent (such as N-methylpryrrolidinone or alcohols) in the presence of a suitable base (such as triethylamine or cesium carbonate) provides 6-hatoimidazo[1,2-b]pyridazines **4**. Alternatively, the reaction of **3** with non-reactive nucleophiles (such as electron deficient anilines) in a suitable solvent (such as dimethylformamide or tetrahydrofuran) with a suitable base (such as sodium hydride) may provide compounds having the formula **4**. Reaction of 6-haloimidazo[1,2-b]pyridazines **4** with nucleophiles (such as amines) provides imidazo[1,2-b]pyridazines **(Ia)** under either neat conditions or in a solvent (such as N-methylpryrrolidinone) in the presence of a suitable base (such as cesium carbonate).

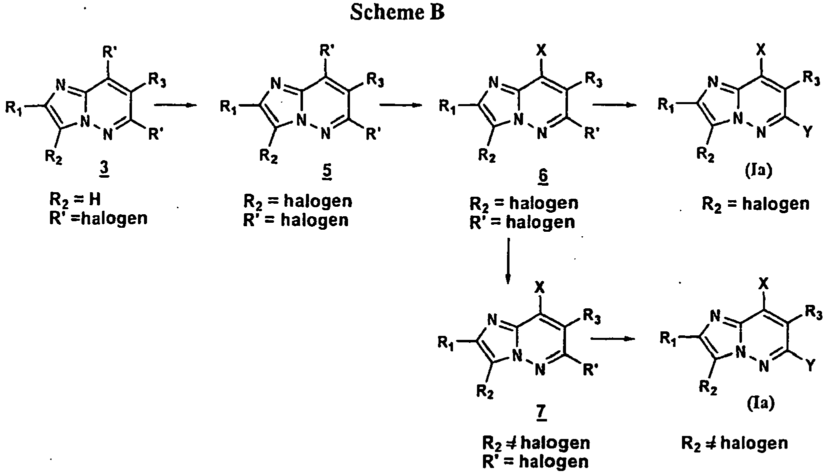

Compounds having the formula (Ia) can also be obtained via treatment of 6,8-dihaloimidazo[1,2-b]pyridazines **3** prepared as described in Scheme A with halogenating agents (such as NBS, NCS, NIS, selectfluor) in a suitable solvent (such as chloroform or acetonitrile) to provide the corresponding 3,6,8-trihaloimidazo[1,2-b]pyridazines **5**. The reaction of **5** with an amine in a suitable solvent (such as N-methylpryrrolidinone or alcohols) in the presence of a suitable base (such as triethylamine or cesium carbonate) provides 3,6-dihaloimidazo[1,2-b]pyridazines **6.** Alternatively, the reaction of **5** with non-reactive nucleophiles (such as electron deficient anilines) in a suitable solvent (such as dimethylformamide or tetrahydrofuran) with a suitable base (such as sodium hydride) may provide compounds having the formula **6**. The reaction of 6-haloimidazo[1,2-b]pyridazines such as **6** with amines provides imidazo[1,2-b]pyridazines **(Ia)** under either neat conditions or in a solvent (such as N-methylpryrrolidinone, dioxane) in the presence of a suitable base (such as cesium carbonate) with or without a catalyst (such as palladium acetate). Alternately, The 3-position of 3,6-dihaloimidazo[1,2-b]pyridazines **6** may be readily converted by one skilled in the art employing one of the many procedures of converting aryl halides into other functional groups to provide of 6-haloimidazo[1,2-b]pyridazines such as **7** where R₂ is other than halogen. The newly introduced functionality at R₂ can be further elaborated by known methods to prepare additional analogs. The reaction of 6-haloimidazo[1,2-b]pyridazines such as **7** with nucleophiles (such as amines or alcohols) provides imidazo[1,2-b]pyridazines (**Ia**) under either neat conditions or in a solvent (such as N-methylpryrrolidinone, dioxane) in the presence of a suitable base (such as cesium carbonate) with or without a catalyst (such as palladium acetate).

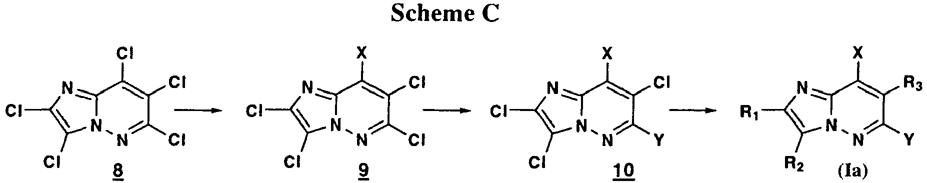

Compounds having the formula (Ia) can also be obtained via treatment of imidazo[1,2-b]pyridazine **8** with nucleophiles (such as amines) in a suitable solvent (such as ethanol) with a suitable base (such as triethylamine) to provide imidazo[1,2-b]pyridazines **9**. *See e.g.* Synthesis Vol. 8 (1971) at pp 424. Reaction of imidazo[1,2-b]pyridazines **9** with nucleophiles (such as amines) provides imidazo[1,2-b]pyridazines **7**. Treatment of imidazo[1,2-b]pyridazines **10** with a suitable catalyst (such as platinum oxide) in a suitable solvent (such as ethanol) under hydrogen pressure (such as 55 psi) provides imidazo[1,2-b]pyridazines (Ia) where R₁, R₂, and R₃ are independently selected from H and Cl.

### UTILITY

The compounds of the invention modulate kinase activity, including the modulation of MAPKAP kinase-2 (MK2). Other types of kinase activity may be modulated by the compounds of the invention including, but not limited to AKT1, AKT2, AKT3, DMPK1, MRCKA, GPRK4, GPRK5, GPRK6, NDR2, PKACA, PKACB, PRKX, PKACA, PDKI, PKCA,PKCD, PKCT, PKCH, PKCI, PKCZ, PKG1, PKG2, PKN2, MSK1, MSK2, RSK1, RSK2, RSK4, YANK2, YANK3, ADCK3, ADCK4, CAMK1A, CAMK1D, CAMK1G, CAMK2A, CAMK2B, CAMK2D, CAMK2G, AMPKA1, AMPKA2, BRSK2, LKB1, MARK1, MARK2, MARK4, QIK, STK33, DAPK2, DAPK3, DRAK1, DRAK2, DCAMKL3, MNK2, SKMLCK, PHKG1, PHKG2, PIM1, PIM2, CK1A2, CKID, CK1E, CK1G1, CK1G2, CDK2, CDK2, CDK5, CDK5, PCTAIRE1, CLK1, CLK2, CLK3, CLK4, GSK3A, GSK3B, GSK3B, ERK1, ERK2, JNK1, JNK2, JNK3, NLK, P38A, P38B, P38G, SRPK1, AURA,AURB, AURC, CAMKK1, CAMKK2, CK2A1, CK2A2, IKKB, AAK1, BIKE, GAK, MPSK1, NEK2, NEK6, NEK7, NEK9, GCN2, PLK1, PLK3, PLK4, TLK1, TLK2, TTK, FUSED, ULK3, MYT1, MAP3K4, MAP3K5, HPK1, KHS1, KHS2, ZC1/HGK, ZC2/TNIK, MST1, MST2, PAK1, PAK2, PAK3, PAK4, PAK5, PAK6, LOK, SLK, MST3, MST4, YSKI, ABL, ARG, ACK, TNKI, ALK, LTK, AXL, MER, TYRO3, CSK, DDR2, EGFR, HER2/ERBB2, HER4/ERBB4, EPHA1, EPHA2, EPHA3, EPHA4, EPHA5, EPHA6, EPHA7, EPHA8, EPHB1, EPHB2, EPHB3, EPHB4, FAK, PYK2, FER, FES, FGFR1, FGFR2, FGFR3, FGFR4, IGF1R, INSR, IRR, IGF1R, INSR, JAK1, JAK2, TYK2, JAK2, MET, MUSK, FLT3, FMS, KIT, PDGFRA, PDGFRB, FLT3, RET, ROS, BLK, BRK, FGR, FRK, FYN, HCK, LCK, LYN, SRC, YES, LCK, SYK, ZAP70, BMX, BTK, ITK, TXK, TIE2, TRKA, TRKB, TRKC, TRKA, TRKB, FLT1, FLT4, KDR, LIMK1, LIMK2, TESK1, HH498, MLK3, BRAF, BRAF, RAF1, RIPK2, ALK1, ALK2, ALK4, BMPR1A, TGFBR1, ACTR2, ACTR2B, and mutants thereof.

Accordingly, compounds of formula (I) have utility in treating conditions associated associated with the modulation of kinase activity, and particularly the selective inhibition of MK2 activity. Such conditions include diseases in which cytokine levels are modulated as a consequence of intracellular signaling via the p38 pathway, with MK2 as the downstream kinase substrate, and in particular, diseases that are associated with an overproduction of cytokines IL-1, IL-6, IL-8, IFNγ and TNF-α. As used herein, the terms "treating" or "treatment" encompass either or both responsive and prophylaxis measures, *e.g*., measures designed to inhibit or delay the onset of the disease or disorder, achieve a full or partial reduction of the symptoms or disease state, and/or to alleviate, ameliorate, lessen, or cure the disease or disorder and/or its symptoms.

In view of their activity as selective inhibitors of MK2, compounds of Formula (I) are useful in treating cytokine-associated conditions including, but not limited to, inflammatory diseases such as Crohn's and ulcerative colitis, asthma, graft versus host disease, chronic obstructive pumonary disease; autoimmune diseases such as Grave's disease, rheumatoid arthiritis, systemic lupus erythematosis, psoriasis; destructive bone disorders such as bone resorption disease, osteoarthritis, osteoporosis, multiple myeloma-related bone disorder ; proliferative disorders such as acute myelogenous leukemia, chronic myelogenous leukemia; angiogenic disorders such as angiogenic disorders including solid tumors, ocular neovasculization, and infantile haemangiomas; infectious diseases such as sepsis, septic shock, and Shigellosis; neurodegenerative diseases such as Alzheimer's disease, Parkinson's disease, cerebral ischemias or neurodegenerative disease caused by traumatic injury, oncologic and viral diseases such as metastatic melanoma, Kaposi's sarcoma, multiple myeloma, and HIV infection and CMV retinitis, AIDS, respectively.

More particularly, the specific conditions or diseases that may be treated with the inventive compounds include, without limitation, pancreatitis (acute or chronic), asthma, allergies, adult respiratory distress syndrome, chronic obstructive pulmonary disease, glomerulonephritis, rheumatoid arthritis, systemic lupus erythematosis, scleroderma, chronic thyroiditis, Grave's disease, autoimmune gastritis, diabetes, autoimmune hemolytic anemia, autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, graft vs. host disease, inflammatory reaction induced by endotoxin, tuberculosis, atherosclerosis, muscle degeneration, cachexia, psoriatic arthritis, Reiter's syndrome, gout, traumatic arthritis, rubella arthritis, acute synovitis, pancreatic β-cell disease; diseases characterized by massive neutrophil infiltration; rheumatoid spondylitis, gouty arthritis and other arthritic conditions, cerebral malaria, chronic pulmonary inflammatory disease, silicosis, pulmonary sarcoisosis, bone resorption disease, allograft rejections, fever and myalgias due to infection, cachexia secondary to infection, meloid formation, scar tissue formation, ulcerative colitis, pyresis, influenza, osteoporosis, osteoarthritis, acute myelogenous leukemia, chronic myelogenous leukemia, metastatic melanoma, Kaposi's sarcoma, multiple myeloma, sepsis, septic shock, and Shigellosis; Alzheimer's disease, Parkinson's disease, cerebral ischemias or neurodegenerative disease caused by traumatic injury; angiogenic disorders including solid tumors, ocular neovasculization, and infantile haemangiomas; viral diseases including acute hepatitis infection (including hepatitis A, hepatitis B and hepatitis C), HIV infection and CMV retinitis, AIDS, ARC or malignancy, and herpes; stroke, myocardial ischemia, ischemia in stroke heart attacks, organ hyposia, vascular hyperplasia, cardiac and renal reperfusion injury, thrombosis, cardiac hypertrophy, thrombin-induced platelet aggregation, endotoxemia and/or toxic shock syndrome, conditions associated with prostaglandin endoperoxidase syndase-2, and pemphigus vulgaris. Preferred methods of treatment are those wherein the condition is selected from Crohns and ulcerative colitis, allograft rejection, rheumatoid arthritis, psoriasis, ankylosing spondylitis, psoriatic arthritis, and pemphigus vulgaris. Alternatively preferred methods of treatment are those wherein the condition is selected from ischemia reperfusion injury, including cerebral ischemia reperfusions injury arising from stroke and cardiac ischemia reperfusion injury arising from myocardial infarction. Another preferred method of treatment is one in which the condition is multiple myeloma.

In addition, the MK2 inhibitors of the present invention inhibit the expression of inducible pro-inflammatory proteins such as prostaglandin endoperoxide synthase-2 (PGHS-2), also referred to as cyclooxygenase-2 (COX-2). Accordingly, additional MK2-associated conditions include edema, analgesia, fever and pain, such as neuromuscular pain, headache, pain caused by cancer, dental pain and arthritis pain. The inventive compounds also may be used to treat veterinary viral infections, such as lentivirus infections, including, but not limited to equine infectious anemia virus; or retro virus infections, including feline immunodeficiency virus, bovine immunodeficiency virus, and canine immunodeficiency virus.

When the terms "MK2-associated condition" or "MK2-associated disease or disorder" are used herein, each is intended to encompass all of the conditions identified above as if repeated at length, as well as any other condition that is affected by MK2 kinase activity.

The present invention thus provides methods for treating such conditions, comprising administering to a subject in need thereof a therapeutically-effective amount of at least one compound of Formula (I) or a salt thereof. Therapeutically effective amount" is intended to include an amount of a compound of the present invention that is effective when administered alone or in combination to inhibit MK2.

The methods of treating MK2 knase-associated conditions may comprise administering compounds of Formula (I) alone or in combination with each other and/or other suitable therapeutic agents useful in treating such conditions. Accordingly, "therapeutically effective amount" is also intended to include an amount of the combination of compounds claimed that is effective to inhibit MK2. The combination of compounds is preferably a synergistic combination. Synergy, as described, for example, by Chou and Talalay, Adv. Enzyme Regul. 1984, 22:27-55, occurs when the effect (in this case, inhibition of P2Y₁) of the compounds when administered in combination is greater than the additive effect of the compounds when administered alone as a single agent. In general, a synergistic effect is most clearly demonstrated at sub-optimal concentrations of the compounds. Synergy can be in terms of lower cytotoxicity, increased antithrombotic effect, or some other beneficial effect of the combination compared with the individual components.

Exemplary of such other therapeutic agents include corticosteroids, rolipram, calphostin, cytokine-suppressive anti-inflammatory drugs (CSAIDs), 4-substituted imidazo [1,2-A]quinoxalines as disclosed in US Pat. No. 4,200,750; Interleukin-10, glucocorticoids, salicylates, nitric oxide, and other immunosuppressants; nuclear translocation inhibitors, such as deoxyspergualin (DSG); non-steroidal antiinflammatory drugs (NSAIDs) such as ibuprofen, celecoxib and rofecoxib; steroids such as prednisone or dexamethasone; antiviral agents such as abacavir; antiproliferative agents such as methotrexate, leflunomide, FK506 (tacrolimus, Prograf); cytotoxic drugs such as azathiprine and cyclophosphamide; TNF-α inhibitors such as tenidap, anti-TNF antibodies or soluble TNF receptor, and rapamycin (sirolimus or Rapamune) or derivatives thereof.

The above other therapeutic agents, when employed in combination with the compounds of the present invention, may be used, for example, in those amounts indicated in the Physicians' Desk Reference (PDR) or as otherwise determined by one of ordinary skill in the art. In the methods of the present invention, such other therapeutic agent(s) may be administered prior to, simultaneously with, or following the administration of the inventive compounds. The present invention also provides pharmaceutical compositions capable of treating MK2 kinase-associated conditions, including IL-1, IL-6, IL-8, IFNγ and TNF-α-mediated conditions, as described above.

The inventive compositions may contain other therapeutic agents as described above and may be formulated, for example, by employing conventional solid or liquid vehicles or diluents, as well as pharmaceutical additives of a type appropriate to the mode of desired administration (*e.g*., excipients, binders, preservatives, stabilizers, flavors, etc.) according to techniques such as those well known in the art of pharmaceutical formulation.

Accordingly, the present invention further includes compositions comprising one or more compounds of Formula I and a pharmaceutically acceptable carrier.

A "pharmaceutically acceptable carrier" refers to media generally accepted in the art for the delivery of biologically active agents to animals, in particular, mammals. Pharmaceutically acceptable carriers are formulated according to a number of factors well within the purview of those of ordinary skill in the art. These include, without limitation: the type and nature of the active agent being formulated; the subject to which the agent-containing composition is to be administered; the intended route of administration of the composition; and, the therapeutic indication being targeted. Pharmaceutically acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, e.g., stabilization of the active agent, binders, etc., well known to those of ordinary skill in the art. Descriptions of suitable pharmaceutically acceptable carriers, and factors involved in their selection, are found in a variety of readily available sources such as, for example, Remington's Pharmaceutical Sciences, 17th ed., 1985, which is incorporated herein by reference in its entirety.

The compounds of Formula (I) may be administered by any means suitable for the condition to be treated, which may depend on the need for site-specific treatment or quantity of drug to be delivered. Topical administration is generally preferred for skin-related diseases, and systematic treatment preferred for cancerous or pre-cancerous conditions, although other modes of delivery are contemplated. For example, the compounds may be delivered orally, such as in the form of tablets, capsules, granules, powders, or liquid formulations including syrups; topically, such as in the form of solutions, suspensions, gels or ointments; sublingually; bucally; parenterally, such as by subcutaneous, intravenous, intramuscular or intrasternal injection or infusion techniques (*e.g*., as sterile injectable aq. or non-aq. solutions or suspensions); nasally such as by inhalation spray; topically, such as in the form of a cream or ointment; rectally such as in the form of suppositories; or liposomally. Dosage unit formulations containing non-toxic, pharmaceutically acceptable vehicles or diluents may be administered. The compounds may be administered in a form suitable for immediate release or extended release. Immediate release or extended release may be achieved with suitable pharmaceutical compositions or, particularly in the case of extended release, with devices such as subcutaneous implants or osmotic pumps.

Exemplary compositions for topical administration include a topical carrier such as PLASTIBASE® (mineral oil gelled with polyethylene).

Exemplary compositions for oral administration include suspensions which may contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which may contain, for example, microcrystalline cellulose, dicalcium phosphate, starch, magnesium stearate and/or lactose and/or other excipients, binders, extenders, disintegrants, diluents and lubricants such as those known in the art. The inventive compounds may also be orally delivered by sublingual and/or buccal administration, *e.g.*, with molded, compressed, or freeze-dried tablets. Exemplary compositions may include fast-dissolving diluents such as mannitol, lactose, sucrose, and/or cyclodextrins. Also included in such formulations may be high molecular weight excipients such as celluloses (AVICEL®) or polyethylene glycols (PEG); an excipient to aid mucosal adhesion such as hydroxypropyl cellulose (HPC), hydroxypropyl methyl cellulose (HPMC), sodium carboxymethyl cellulose (SCMC), and/or maleic anhydride copolymer (*e.g*., GANTREZ®); and agents to control release such as polyacrylic copolymer (*e.g*., CARBOPOL 934®). Lubricants, glidants, flavors, coloring agents and stabilizers may also be added for ease of fabrication and use.

Exemplary compositions for nasal aerosol or inhalation administration include solutions which may contain, for example, benzyl alcohol or other suitable preservatives, absorption promoters to enhance absorption and/or bioavailability, and/or other solubilizing or dispersing agents such as those known in the art.

Exemplary compositions for parenteral administration include injectable solutions or suspensions which may contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid.

Exemplary compositions for rectal administration include suppositories which may contain, for example, suitable non-irritating excipients, such as cocoa butter, synthetic glyceride esters or polyethylene glycols, which are solid at ordinary temperatures but liquefy and/or dissolve in the rectal cavity to release the drug.

The therapeutically-effective amount of a compound of the present invention may be determined by one of ordinary skill in the art, and includes exemplary dosage amounts for a mammal of from about 0.05 to 1000 mg/kg; 1-1000 mg/kg; 1-50 mg/kg; 5-250 mg/kg; 250-1000 mg/kg of body weight of active compound per day, which may be administered in a single dose or in the form of individual divided doses, such as from 1 to 4 times per day. It will be understood that the specific dose level and frequency of dosage for any particular subject may be varied and will depend upon a variety of factors, including the activity of the specific compound employed, the metabolic stability and length of action of that compound, the species, age, body weight, general health, sex and diet of the subject, the mode and time of administration, rate of excretion, drug combination, and severity of the particular condition. Preferred subjects for treatment include animals, most preferably mammalian species such as humans, and domestic animals such as dogs, cats, horses, and the like. Thus, when the term "patient" is used herein, this term is intended to include all subjects, most preferably mammalian species, that are affected by mediation of MK2 enzyme levels.

Examples of formula (I) as specified in the "Examples" section below, have been tested in one or more of the assays described below and have activity as inhibitors of MK2 enzymes at an IC₅₀ of less than 30 uM and preferably less than 10 *u*M; and inhibit TNF-α at an IC₅₀ of less than 100 uM and preferably less than 30 uM.

### BIOLOGICAL ASSAYS

### Generation of activated MK2 Kinase

DNA oligonucleotide PCR primers were synthesized and used to amplify from template DNA the MK2 DNA sequence (NCBI Refseq NM_032960.2) encoding native amino acid residues 47-400. The PCR primers were designed such that the amplified DNA also encoded an N-terminal (His)₆-affinity purification tag followed by a thrombin-cleavable linker. This amplified product was inserted into the pET28N vector. *E*. *coli* strain BL21(DE3) was transformed with the MK2(47-400)-pET28N plasmid and cultured at 37°C in a defined medium. IPTG (0.5 mM) was added to the medium to induce recombinant protein expression at 20°C for 18 hours. The cell paste was harvested by sedimentation and frozen at -80°C.

Frozen cell paste was thawed and lysed in buffer at 4°C using a microfluidizer. The MK2 protein was purified by sequential chromatography on columns of SP-Sepharose Fast Flow and Ni-NTA Superflow. The N-terminal (His)₆-tag was removed from the purified MK2 protein by digestion with thrombin followed by sequential benzamidine-Sepharose and Superdex 200 size exclusion chromatography.

MK2(47-400) was dialyzed and diluted into a final reaction buffer of 0.5 mg/ml MK2(47-400) in 20 mM HEPES pH 7.5, 5% glycerol, 2 mM DTT, 20 mM MgCl₂, 1 mM ATP, and 8 µg/ml activated (His)₅-p38alpha. The reaction was incubated at 25°C for 1 hour, after which an additional 1 mM fresh ATP was added. After an additional 30 minute incubation at 25°C the reaction was stopped by placing it on ice and adding NaCl and EDTA to 200 mM and 30 mM, respectively.

The protein in the activation reaction was concentrated, filtered, and buffer exchanged into 25 mM HEPES pH 7.2, 400 mM NaCl, 20 mM imidazole, 5% glycerol, 10 mM 2-mercaptoethanol, 0.5 mM TCEP. The void volume peak from this column was concentrated and loaded onto a Ni-NTA column to capture the (His)₅-p38 protein. The activated MK2(47-400) protein was not retained and eluted in the flow-through fractions. Fractions containing activated MK2(47-400) were pooled, supplemented with 10 mM EDTA, concentrated, and loaded onto a Superdex 200 column equilibrated with 20 mM HEPES pH 7.5, 100 mM NaCl, 10% (v/v) glycerol, 0.1 mM EDTA, 2 mM DTT). The activated MK2(47-400) protein eluted as a single, large peak, and fractions from the center of this peak were pooled, divided into aliquots, and frozen at -80°C.

### MK2 Assay

The MK2 radioactive assay was performed in a 96 well round bottom non-binding polystyrene plates (Coming 3605). The final assay volume was 30 µl prepared from three 10 µl additions of enzyme, substrates (HSP-27 and ATP) and test compounds in assay buffer (20 mM HEPES pH 7.5, 25 mM β-glycerolphosphate, 15 mM MgCl₂, 1 mM DTT). The reaction was incubated at RT for 30 min. and terminated by adding 20 µl of 0.5 M EDTA to each sample. Then 40 µl of the reaction mixture was transferred onto a pre-wet (2% phosphoric acid) Millipore Multiscreen phosphocellulose filter plate (MAPHNOB50). This reaction mixture was filtered through a Millipore multiscreen resist vacuum manifold. The filterplate was washed 3x with 2% phosphoric acid and air dried. The filterplate is put into a Packard multiscreen adapter plate. 50 µl of Microscint 20 was added to each well and sealed with a plate sealer and counted on the Packard Top Count NXT. Inhibition data were analyzed in ABASE using excel fit. The final concentration of reagents in the assay are 5 µM ATP; 10 µCi/µl [γ-³³P]ATP, 5 ng MK2 enzyme, 30 µM HSP-27 and DMSO, 0.3% for screening.

The Molecular Devices IMAP MAPKAP K2 Assay Kit is performed in a HE black microplate (Molecular Devices 75-000-005). The final assay volume is 10 µl prepared from 2.5 µl compound, 5 µl ATP/Peptide and 2.5 µl MK2 enzyme. The final concentration of reagents in the assay are 1 µM ATP, 200 n Peptide and 0.070 nM MK2 enzyme (note: The MK2 enzyme concentration should produce approximately 70% of the maximal signal of 380mP =/-40mP). Prepare a 1x complete reaction buffer (CRB) using distilled water from a 5x stock and add DTT to a 1 mM final concentration. The CRB is used for the initial reaction preparation. Incubate the reaction covered in foil at room temperature for 30 minutes. Prepare 1x Buffer A using distilled water from the 5x Buffer A stock. Add IMAP reagent by diluting 400 times into Buffer A. Add 30 uL of IMAP reagent in buffer to each well. Incubate for 30 minutes at RT covered in foil. Read on LJL analyst using 485 excitation 530 emission.

Caliper LabChip 3000 Assay is performed in U-bottom 384-well plates. The final assay volume is 30 µl prepared from 15 µl additions of enzyme and substrates (MK2 peptide and ATP) and test compounds in assay buffer (100 mM HEPES pH 7.4, 10 mM MgCl₂, 0.015% Brij35 and 4 mM DTT). The reaction is initiated by the combination of MapKapK2 with substrates and test compounds. The reaction is incubated at room temperature for 60 min. and terminated by adding 30 µl of 35 mM EDTA to each sample. The reaction mixture is analyzed on the Caliper LabChip 3000 by electrophoretic separation of the fluorescent substrate and phosphorylated product. Inhibition data were calculated by comparison to no enzyme control reactions for 100% inhibition and vehicle-only reactions for 0% inhibition. The final concentration of reagents in the assays are ATP, 1 µM; MK2 peptide, 1.5 uM; MapKapK2, 0.08 nM; Brij35, 0.015% and DMSO, 1.6%. Dose response curves are generated to determine the concentration required inhibiting 50% of kinase activity (IC₅₀). Compounds are dissolved at 10 mM in dimethylsulfoxide (DMSO) and evaluated at eleven concentrations, each in duplicate. IC₅₀ values are derived by non-linear regression analysis.

### TNF-α Production by LPS-Stimulated PBMCs

EDTA-treated human whole blood was obtained from healthy volunteers. Peripheral blood mononuclear cells (PBMCs) were purified from human whole blood by Ficoll-Hypaque density gradient centrifugation (Lympho Separation Media Cellgro #25-072-CV) and resuspended at a concentration of 2.5 x 10⁶/ml in assay medium (RPMI medium .containing 10% fetal bovine serum). 100 ul of cell suspension was incubated with 50 ul of test compound (4X concentration in assay medium containing 0.3% DMSO) in 96-well tissue culture plates for 1 hour at 37°C. 50 ul of LPS (400 ng/ml stock) was then added to the cell suspension yielding a 100 ng/ml final concentration of LPS and the plate was incubated for 5 hours at 37°C. Following incubation, the culture medium was collected and assayed. TNF-α concentration in the medium was quantified using a standard ELISA kit (R&D Systems Cat#DY210). Concentrations of TNF-α and IC₅₀ values for test compounds (concentration of compound that inhibited LPS-stimulated TNF-α* production by 50%) were calculated using softmax software using a 4-parameter curve fit.

### EXAMPLES

The following Examples illustrate embodiments of the inventive compounds and starting materials, and are not intended to limit the scope of the claims. For ease of reference, the following abbreviations are used herein:

### ABBREVIATIONS

- BOC =: *tert*-butoxycarbonyl
- bp =: boiling point
- Bu =: butyl
- DMAP =: 4-dimethylaminopyridine
- DIPEA or DIEA =: *N,N*-diisopropylethylamine
- DME =: 1,2-dimethoxyethane
- DMF =: dimethyl formamide
- EDCI =: 1-3-dimethylaminopropyl)-3-ethylcarbodiimide
- Et =: ethyl
- Et₂O =: diethyl ether
- HOBT =: 1-hydroxybenzotriazole
- EtOAc =: ethyl acetate.
- EtOH =: ethanol
- g =: gram(s)
- H =: hydrogen
- I =: liter
- mCPBA -: *meta* chloro perbenzoic acid
- Me =: methyl
- MeCN =: acetonitrile
- MeOH =: methanol
- NMP =: 1-methyl-2-pyrrolidinone
- Ph =: phenyl
- Pr =: propyl
- PS =: polystyrene
- TEA =: triethylamine
- TFA =: trifluoroacetic acid
- mg =: milligram(s)
- ml or mL =: milliliter
- µl =: microliter
- mmol =: millimole
- µmol =: micromole
- mol =: mole
- mp =: melting point
- RT =: room temperature
- HPLC =: high pressure liquid chromatography
- LC/MS =: liquid chromatography/mass spectrometry

### EXAMPLE I(1)

### N⁶-(trans-4-aminocyclohexyl)-N⁸-[4-(ethyloxy)phenyl]imidazo[1,2-b]pyridazine-6,8-diamine

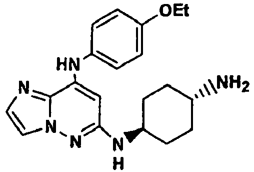

*(1a)* Bromine (9.71 g, 3.15 mL, 60.75 mmol) was added dropwise to a mixture of 3-amino-6-chloropyridazine (7.87 g, 60.75 mmol) in methanol (115 mL) and sodium bicarbonate (10.22 g, 121.67 mmol). The resultant mixture was stirred at room temperature for 16 hours and then filtered. Water (500 mL) was added to the filtrate and the solution was extracted with ethyl acetate (3 x 500 mL). The organic layers were combined and concentrated in vacuo. The resulting residue was purified by flash chromatography eluting with 1/1 hexane/ethyl acetate to give 5.40 g (43%) 3-amino-4-bromo-6-chloropyridazine.
*(1b)* Chloroacetaldehyde solution (50% in water, 13.2 mL, 16.32 g, 104.6 mmol) was added to 3-amino-4-bromo-6-chloropyridazine (4.2 g, 20.2 mmol) from *1a* in ethanol (28 mL). The solution was heated to 50 °C for 16 hours and then concentrated in vacuo. Acetone (22 mL) was added to the residue and the solid was collected by vacuum filtration and washed with cold acetone. Upon air drying 4.3 g (79%) of 8-bromo-6-chloroimidazo[1,2-b]pyridazine was obtained as a hydrochloride salt.
*(1c variation 1)* To a mixture of 8-bromo-6-chloroimidazo[1,2-b]pyridazine (257 mg, 0.956 mmol) from *1b* in THF (2.0 ml) was added p-phenetidine (131 mg, 0.956 mmol) and a 1.0 M solution of KOt-Bu in THF (2.5 eq, 2.4 ml, 2.39 mmol). The mixture was allowed to heat at 50 °C for 1 hour. The solution was then concentrated in vacuo to provide crude 6-chloro-*N*-(4-ethoxyphenyl)imidazo[1,2-*b*]pyridazin-8-amine as a solid. The solid was then used as is in the following step.
*(1c variation 2)* p-Phenetidine (1.0 eq, 0.149 mmol) and triethyamine (33 mg, 0.327 mmol) were added to a mixture of 8-bromo-6-chloroimidazo[1,2-*b*]pyridazine hydrochloride (40 mg, 0.149 mmol) from *1b* in EtOH (1.5 mL). The mixture was heated to 90 °C and stirred for 24-48 hours. The solution was then concentrated in vacuo to give crude 6-chloro-*N*-(4-ethoxyphenyl)imidazo[1,2-b]pyridazin-8-amine.
*(1d)* trans-1,4-Diaminocyclohexane (1000 mg, 8.77 mmol) was added to the crude 6-chloro-*N*-(4-ethoxyphenyl)imidazo[1,2-*b*]pyridazin-8-amine (0.149 mmol) from *1c*. The mixture was heated to 160 °C and allowed to melt. After stirring at 160 °C for 24-48 hrs, the liquid mixture was cooled to room temperature. Water was added, followed by extraction with dichloromethane. The organic layer was concentrated in vacuo. The resulting residue was purified by reverse phase preparative HPLC to provide the above titled compound as a TFA salt in approximately 35% yield. ¹H NMR (400 MHz, MeOH) δ ppm 7.59 (1 H, s), 7.29 (1 H, s), 7.23 (2 H, d, J=8.8 Hz), 6.95 (2 H, d, J=8.8 Hz), 5.85 (1 H, s), 4.04 (2 H, q, *J*=7 Hz), 3.51 - 3.61 (1 H, m), 2.65 - 2.73 (1 H, m), 2.10 (2 H, d, *J*=12.30 Hz), 1.92 (2 H, d, *J*=12.30 Hz),1.38 (3 H, t, *J*=7 Hz), 1.20-1.33 (4 H, m). LC/MS, *m*/*e* 367 (M+1). HPLC Rt, 2.11 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.

### Examples prepared in a similar manner are indicated in Table 1

Alternatively, Example I(1) may also be prepared by the following method.
*(1e)* To perchloroimidazo[1,2-*b*]pyridazine (Synthesis 1971, 8, 424) (213 mg, 0.731 mmol) in EtOH (5 mL) was added p-phenetidine (100 mg, 0.731 mmol) and triethyamine (86 mg, 0.804 mmol). The mixture was allowed to heat at 90 °C for 5 hours. The solution was then cooled to 0 °C. The resulting solid was collected by vacuum filtration and washed with cold EtOH. Upon air drying 191 mg (67%) of crude 2,3,6,7-tetrachloro-N-(4-ethoxyphenyl)imidazo[1,2-*b*]pyridazin-8-amine was isolated.
*(1f)* To 2,3,6,7-tetrachloro-*N*-(4-ethoxyphenyl)imidazo[1,2-*b*]pyridazin-8-amine (184 mg, 0.47 mmol) from *1e* was added trans-1,4-diaminocyclohexane (360 mg, 3.16 mmol). The mixture was allowed to melt at 120 °C for 1 1/2 hours. The melt was then cooled, diluted with water and extracted with ethyl acetate. The organic layer was then concentrated in vacuo to give 200 mg (91 %) of crude product. 20 mg of this product was then purified by preparative HPLC. This gave 8.0 mg *of N⁶*-(4-aminocyclohexyl)-2,3,7-trichloro-*N*⁸-(4-ethoxyphenyl)imidazo[1,2-*b*]pyridazine-6,8-diamine as a TFA salt.
*(1g)* To a mixture of crude *N*⁶-(4-aminocyclohexyl)-2,3,7-trichloro-*N*⁸-(4-ethoxyphenyl)imidazo[1,2-*b*]pyridazine-6,8-diamine from *1f* as a free base (140 mg, 0.300 mmol) in EtOH (10 ml) in a 500 ml PARR bottle was added 10% Palladium on carbon (175 mg). The PARR bottle was then charged with H₂ at 55 psi and allow to shake at room temperature for 24 hours. The catalyst was then filtered and the filtrate was concentrated in vacuo to give a crude mixture of 3 compounds. This mixture was purified by preparative HPLC to give 4.7 mg of Example I(1), *N*⁶-(4-aminocyclohexyl)-*N*⁸-(4-ethoxyphenyl)imidazo[1,2-*b*]pyridazine-6,8-diamine as a TFA salt, LC/MS, *m*/*e* 367 (M+1). 9.0 mg of N⁶-(4-aminocyclohexyl)-3-chloro-*N*⁸-(4-ethoxyphenyl)imidazo[1,2-*b*]pyridazine-6,8-diamine, LC/MS, *m*/*e* 401 (M+1) and 18.5 mg of N⁶-(4-aminocyclohexyl)-2,3-dichloro-*N*⁸-(4-ethoxyphenyl)imidazo[1,2-*b*]pyridazine-6,8-diamine, LC/MS, *m*/*e* 435 (M+1).

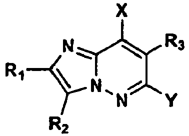

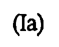

Compounds having the formula (Ia) were prepared according to procedures similar to Example I(1), where in R₁ R₂, R₃, X and Y have the values listed in Table 1, using the appropriate starting materials and substantially the same procedures as indicated.

**Table 1**

| **Exp** | **Name** | **R₁** | **R₂** | **R₃** | **X** | **Y** | **LC/MS m/z (M+1)** |
|---|---|---|---|---|---|---|---|
| **I(2)** | *N*⁶-(2-aminoethyl)-*N*⁸-(4-(ethyloxy)phenyl)midazo[1,2-*b*]pyridazine-6,8-diamine | **H** | **H** | **H** | 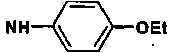 | NH-(CH₂)₂-NH₂ | 313 |
| **I(3** | *N*⁶-(4-aminobutyl)-*N*⁸-(4-(ethyloxy)phenyl)imidazo[,2-*b*]pyridazine-6,8-diamine | **H** | **H** | **H** | 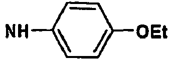 | NH-(CH₂)₄-NH₂ | 341 |
| **I(4)** | 7-chloro-*N*-(4-(ethy)oxy)phenyl)-6-(1-piperazinyl)imidazo[1,2-*b*]pyridazin-8-amine | **H** | **H** | **Cl** | 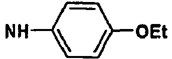 | 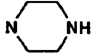 | 373 |
| **I(5)** | *N*⁶-(*trans*-4-aminocyclohexyl)-*N*⁶-(4-(methyloxy)phenyl)imidazo[1, 2-*b*]pyridazine-6,8-diamine | **H** | **H** | **H** | 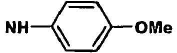 | 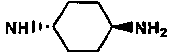 | 353 |
| **I(6)** | *N*⁶-(*trans*-4-aminocyclohexyl)-*N*⁸-(3-(ethyloxy)phenyl)imidazo[1,2-*b*]pyridazine-6,8-diamine | **H** | **H** | **H** | 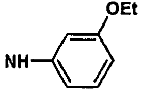 | 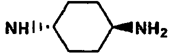 | 367 |
| **I(7)** | *N*⁶-(*trans*-4-aminocyclohexyl)-*N*⁸-phenylimidazo[1,2-*b*]pyridazine-6,8-diamine | **H** | **H** | **H** | 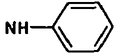 | 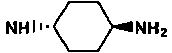 | 323 |
| **I(8)** | *N*⁶-(*trans*-4-aminocyclohexyl)-*N*⁸-(3-(methyloxy)phenyl)imidazo[1, 2-*b*]pyridazine-6,8-diamine | **H** | **H** | **H** | 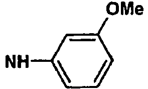 | 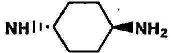 | 353 |
| **I(9)** | *N*⁶-(*trans*-4-aminocyclohexyl)-*N*⁸-(3,4-dimethylphenyl)imidazo[1,2-*b*]pyridazine-6,8-diamine | **H** | **H** | **H** | 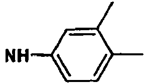 | 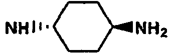 | 351 |
| **I(10)** | *N*⁶-(*trans*-4-aminocyclohexyl)-(phenyloxy)phenyl)imidazo[1, 2-*b*]pyridazine-6,8-diamine | **H** | **H** | **H** | 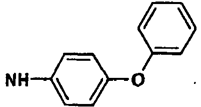 | 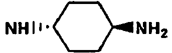 | 415 |
| **I(11)** | *N*⁶-(*trans*-4-aminocyclohexyl)-*N*⁶-(4-(butylooy)phenyl)imidazo[1,2-*b*]pyridazine-6,8-diamine | **H** | **H** | **H** | 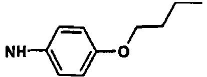 | 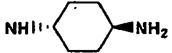 | 395 |
| **1(12)** | *N*⁶-(*trans*-4-aminocyclohexyl)-*N*⁸-4-biphenylylimidazo[1,2-*b*]pyridazine-6,8-diamine | **H** | **H** | **H** | 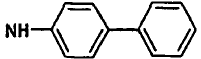 | 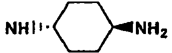 | 399 |
| **I(13)** | *N*⁶-(*trans*-4-aminocyclohexyl)-*N*⁶(4-methylphenyl)imidazo[1,2-*b*]pyridazine-6,8-diamine | **H** | **H** | **H** | 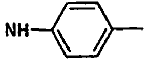 | 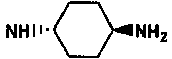 | 339 |
| **I(14)** | *N*⁶-(*trans*4-aminocyclohexyl)-*N*⁸-(3,4-bis(methyloxy)phenyl)imidazo[ 1,2-*b*]pyridazine-6,8-diamine | **H** | **H** | **H** | 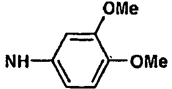 | 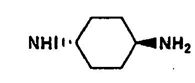 | 383 |
| **I(15)** | *N*⁶-(*trans*-4-aminocyclohexyl)-*N⁸-*(4-((phenylmethyl)oxy)phenyl)imi dazo[1,2-*b*]pyridazine-6,8-diamine | **H** | **H** | **H** | 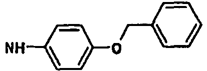 | 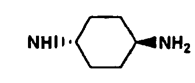 | 429 |
| **I(16)** | *N*⁶-(*trans*-4-3minocyclohexyl)-*N*⁸-(4-(propyloxy)phenyl)imidazo[1,2 -*b*]pyridazine-6,8-diamine | **H** | **H** | **H** | 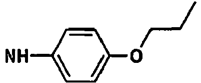 | 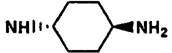 | 387 |
| **I(17)** | *N*⁶-(*trans*-4-aminocyclohexyl)-*N*⁶-pyridin-3-ylimidazo[1,2-*b*]pyridazine-6,8-diamine | **H** | **H** | **H** | 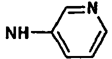 | 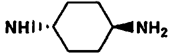 | 324 |
| **I(18)** | *N*⁶-(*trans*-aminocyclohexyl)-*N*⁸-(2-methyl-1*H*-indol-5-yl)imidazo[1,2-*b*]pyridazine-6,8-diamine | **H** | **H** | **H** | 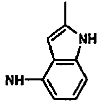 | 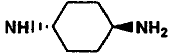 | 376 |
| **I(19)** | *N*⁶-(*trans*-4-aminocycloxexyl)-*N*⁶-methyl-*N*⁸-phenylmidazo[1,2-*b*]pyridazine-6,8-diamine | **H** | **H** | **H** | 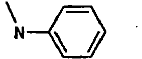 | 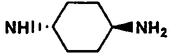 | 337 |
| | *N*⁶-(*trans*-4-aminocyclohexyl)-*N*⁸-[2-(methyloxy)phenyl]imidazo[1, 2-*b*]pyridazine-6,8-diamine | **H** | **H** | **H** | 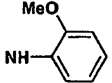 | 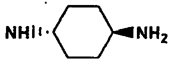 | 353 |
| **I(20)** | *N*⁶-(*trans*-4-aminocyclohexyl)-methylphenyl)i;mdazn[ol,2-*b*]pyridazine-6,8-diamine | **H** | **H** | **H** | 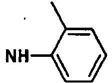 | 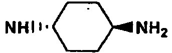 | 337 |
| **I(21)** | *N*⁶-(*trans*-4-aminocyclohexyl)-*N*⁶-(2,3-dimethylphenyl)imidazo[1,2-*b*]pyridazine-6,8-diamioc | **H** | **H** | **H** | 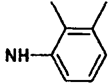 | 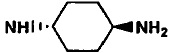 | 351 |
| **I(22)** | *N*⁶-(*trans*-4-aminocyclohexyl)-*N*⁸-(2,4-dimethylphenyl)imidazo[1,2-*b*]pyridazine-6,8-diamine | **H** | **H** | **H** | 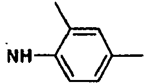 | 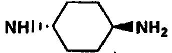 | 351 |
| **I(23)** | *N*⁶-(*trans*-4-aminocyclohexyl)-*N*⁶-(2,5-dimethylphenyl)imidazo[1,2-*b*]pyridazine-6,8-diamine | **H** | **H-** | **H** | 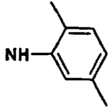 | 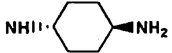 | 351 |
| **I(24)** | *N*⁶-(*trans*-4-aminocyclohexyl)-*N*⁸-(3-methylphenynimidazo[1,2-*b*]pyridazine-6,8-diamine | **H** | **H** | **H** | 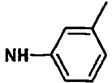 | 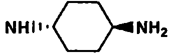 | 337 |
| **I(25)** | *N*⁶-(*trans*-4-aminocyclohexyl)-*N*⁸-(3,5-dimethylphenyl)imidazo[1,2-*b*]pyridazine-6,8-diamine | **H** | **H** | **H** | 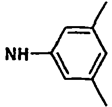 | 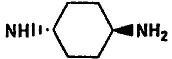 | 351 |
| **I(26)** | *N*⁶-(*trans*-4-aminocyclohexyl)-*N*⁸-[3-(dimethylamino)phenyl]imidaz o[1,2*-b*]pyridazine-6,8-diamine | **H** | **H** | **H** | 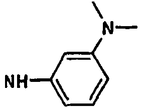 | 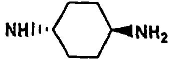 | 366 |
| **I(27)** | *N*⁶-(*trans*-4-aminocyclohexyl)-*N*³-(2-methyl-1,3-benzothiazol-6-yl)imidazo[1,2-*b*]pyridazine-6,8-diamine | **H** | **H** | **H** | 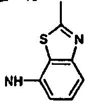 | 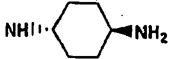 | 394 |
| **I(28)** | *N*⁶-(*trans*-4-aminocyclohexyl)-*N*⁸-(2-methy)-1,3-benzothiazol-5-yl)imidazo[1,2-*b*]pyridarine-6,8-diamine | **H** | **H** | **H** | 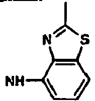 | 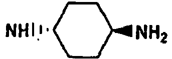 | 394 |
| **I(29)** | *N*⁶-(*trans*-4-aminocyclohexyl)-*N*⁸-cyclopropylimidazo[1,2_ *b*]pyridazine-6,8diamine | **H** | **H** | **H** | 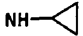 | 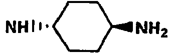 | 287 |
| **1(30)** | *N*⁶-(*trans*-4-aminocyclohexyl)-*N*⁸-cyclohexylimidazo[1,2-*b*]pyridazine-6,8-diamine | **H** | **H** | **H** | 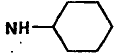 | 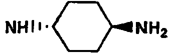 | 329 |
| **I(31)** | *N*⁶-(*trans*-4-aminocyclohexyl)-(cyclohexylmethyl)imidazo[1,2 -*b*]pyridazine-6,8-diamine | **H** | **H** | **H** | 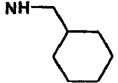 | 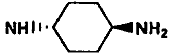 | 393 |
| **I(32)** | *N*⁶-(*trans*-4-aminocyclahexyl)-*N*⁸-(1-methylethyl)imidazo[1,2-*b*]pyridazine-6,8-diamine | **H** | **H** | **H** | 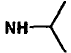 | 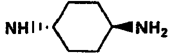 | 289 |
| **I(33)** | *N*⁶-(*trans*-4-aminocyclohexyl)-*N*⁶-(phenylmethyl)imidazo[1,2-*b*]pyridazine-6,8-diamine | **H** | **H** | **H** | 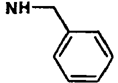 | 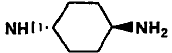 | 337 |
| **I(34)** | *N*⁶-(*trans*-4-aminocyclohexyl)-*N*⁶-[(2-chlorophenyl)methyl]imidazo[ 1,2-*b*]pyridazine-6,8-diamine | **H** | **H** | **H** | 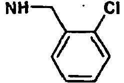 | 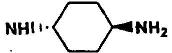 | 371 |
| **I(35)** | *N*⁶-(trans-4-aminocyclohcxy)r *N*⁸-((4-chlorophenyl)methyl)imidazo[ 1,2-*b*]pyridazine-6,8-diamine | **H** | **H** | **H** | 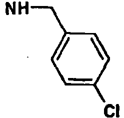 | 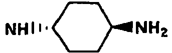 | 371 |
| **I(36)** | *N*⁶-(*trans*-4-aminocyclohexyl)-*N⁶-((4-*(methyloxy)phenyl)metbyl)imi dazo[1,2-*b*]pyridazine-6,8-diamine | **H** | **H** | **H** | 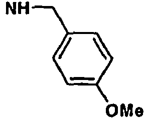 | 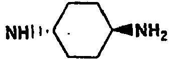 | 367 |
| **I(37)** | *N*⁶-(*trans*-4-aminocyclohexyl)-*N*⁸-ethylimidazo[1,2-*b*]pyridazine-6,8-diamine . | **H** | **H** | **H** | 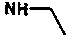 | 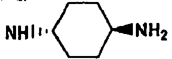 | 275 |
| I(38) | *N*⁶-(*trans*-4-aminocyelohwcyl)-*N*⁸-(2-(methyloxy)ethyl)imidazol[1,2-*b*]pyridazine-6,8-diamine | H | H | H | 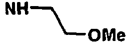 | 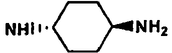 | 305 |
| **I(39)** | *N*⁶-(*trans*-4-aminocyclohexyl)-*N*⁸-(2-(4-(methyloxy)phenyl)ethyl)imida zo[1,2-*b*]pyridazine-6,8-diamine | **H** | **H** | **H** | 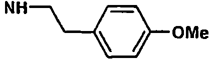 | 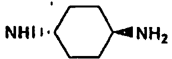 | 381 |
| **I(40)** | *N*⁶-(*trans*-4-aminocyclohexyl)-*N*⁸-2-propen-1-ylimidam[1,2-*b*]pyridazine-6,8-diamine | **H** | **H** | **H** | 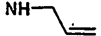 | 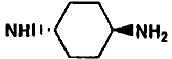 | 287 |
| **I(41)** | *N*⁶-(*trans*-4-aminocyclohexyl)-*N*⁸-(3-methylbutyl)imidazo[1,2-*b*]pyridazine-6,8-diamine | **H** | **H** | **H** | 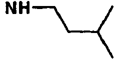 | 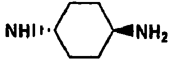 | 317 |
| **I(42)** | *N*⁶-(*trans*-4-aminocyclohexyl)-*N*⁸-propylimidazo[1,2-*b*]pyridazine-6,8-diamine | **H** | **H** | **H** | 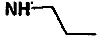 | 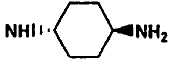 | 289 |
| **I(43)** | *N*⁶-(*trans*-4-aminocyclohexyl)-*N*⁸-(cyclopropylmethyl)imidazo[1, 2-*b*]pyridazine-6,8-diamine | **H** | **H** | **H** | 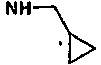 | 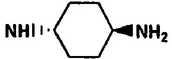 | 301 |
| **1(44)** | *N*⁶-(trans-4-aminocyclohexyl)-*N*⁸-((3-chlorophenyl)methyl)imidazo[ 1,2-*b*]pyridazine-6,8-diamine | **H** | **H** | **H** | 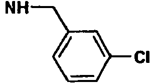 | 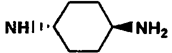 | 371 |
| **I(45)** | 6-(3-amino-1-piperidinyl)-*N*-(4-(ethyloxy)phenyl)imidazo[1,2-*b*]pyridazin-8-amine | **H** | **H** | **H** | 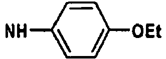 | 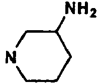 | 353 |
| **I(46)** | *N*⁶-(3-aminopropyl)-N⁸-(4-(ethyloxy)phenyl)imidazo[1,2-*b*]pyridazine-6,8-diamine | **H** | **H** | **H** | 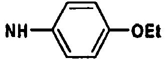 | 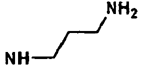 | 327 |
| **I(47)** | *N*⁶-(*trans*-4-aminocyclohexyl)-*N*⁸-(2,6-difluorophenyl)imidazo[1,2-*b*]pyridazine-6,8-diamine | **H** | **H** | **H** | 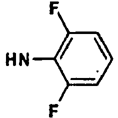 | 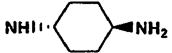 | 359 |
| **I(48)** | *N*⁶-(4-((4-aminocyclohexyl)methyl)cyclo hexyl)-*N*⁸-(4-(ethyloxy)phenyl)imidazo[1,2-*b*]pyridazine-6,8-dismine | **H** | **H** | **H** | 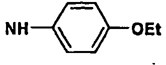 | 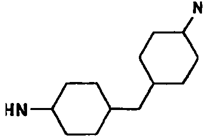 | 463 |
| **I(49)** | 2-(1-(8-((4-(ethyloxy)phenyl)amino)imida zo[1,2-*b*]pyridazin-6-yl)-4-piperidinyl)ethanol | **H** | **H** | **H** | 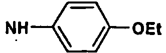 | 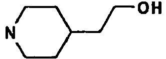 | 382 |
| **I(50)** | *N*⁶-(*trans*-4-aminocyclohexyl)-*N*⁶-1*H*-indol-5-ylimidazo[1,2-*b*]pyridazine-6,8-diamine | **H** | **H** | **H** | 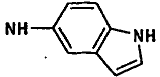 | 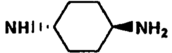 | 363 |
| **I(51** | *N*⁶-(trans-4-aminocyclohexyl)-*N*⁸-(2-(ethyloxy)phenyl)imidazo[1,2-*b*]pyridazine-6,8-diamine | **H** | **H** | **H** | 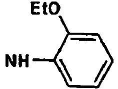 | 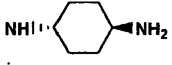 | 368 |
| **I(52)** | 6-(3-amino-1-pyrrolidinyl)-*N*-(4-(ethyloxy)phenyl)imidazo[1,2-*b*]pyridazin-8-amine | **H** | **H** | **H** | 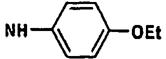 | 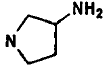 | 339 |
| **I(53)** | *N*⁶-(trans-4-aminocyclohexyl)-*N*³-(2-phenylethyl)imidazo[1,2-*b*]pyridazine-6,8-diamine | **H** | **H** | **H** | 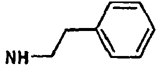 | 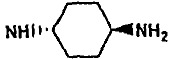 | 351 |
| **I(54)** | *N*-(4-ethyloxy)phenyl)-6-(1-piperazinyl)imidazo[1,2-6]pyridazin-8-amine | **H** | **H** | **H** | 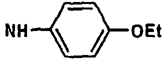 | 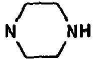 | 339 |
| **I(55)** | *N*⁶-(2-(dimethylamino)ethyl)-*N*⁸-(4-(ethyloxy)phenyl)imidazo[1,2-*b*]pyridazine-6,8-diamine | **H** | **H** | **H** | 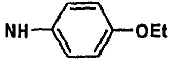 | 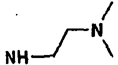 | 341 |
| **I(56)** | *N*⁶-(4-(ethyloxy)phenyl)-*N*⁶-(2-furanylmethyl)imidazo[1,2-*b*]pyridazine-6,8-diamine ' | **H** | **H** | **H** | 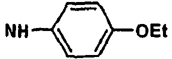 | 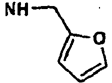 | 350 |
| **I(57)** | *N*-(4-(ethyloxy)phenyl)-6-(4-methyl-1,4-diazepan-1-yl)imidazo[1,2-*b*]pyridazin-8-amine | **H '** | **H** | **H** | 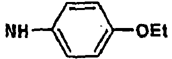 | 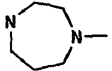 | 368 _{'} |
| **I(58)** | 2-((6-(*trans*-4-aminocyclohexyl)amino)imida zo[1,2-*b*]pyridazin-8-yl)amino)phenol | **H** | **H** | **H** | 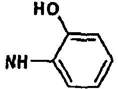 | 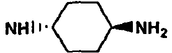 | 339 |
| **I(59)** | *N*⁶-(*trans*-4-aminocyclohexyl)-*N*⁶-(3-(phenylmethyl)oxy)phenyl)imi dazo[1,2-*b*]pyridazine-6,8-diamine | **H** | **H** | **H** | 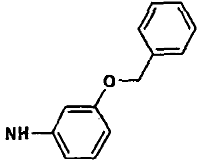 | 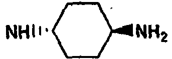 | 430 |
| **I(60)** | 3-((6-(*trans*-4-aminocyclohexyl)amino)imida zo[1,2-*b*]pyridazin-8-yl)amino)phenol | **H** | **H** | **H** | 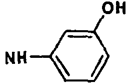 | 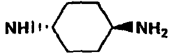 | 339 |
| **I(61)** | 4-((6-((*trans*-aminocyclohexyl)amino)imida zo[1,2-*b*]pyridazin-8-yl)amino)phenol | **H** | **H** | **H** | 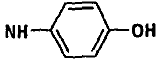 | 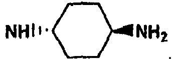 | 339 |
| **I(62)** | *N*⁶-(*trans*-4-aminocyclohexyl)imidazo[1,2-*b*]pyridazine-6,8-diamine | **H** | **H** | **H** | 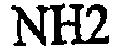 | 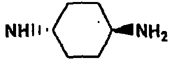 | 247 |
| **I(63)** | 3-((6-((*trans*-4-aminocyclohexyl)amino)imida zo[1,2*b*]pyridazin-8-yl)amino)benzonitrile | **H** | **H** | **H** | 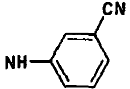 | 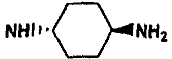 | 348 |
| **I(64)** | 4-((6-((*trans*-4-aminocyclohexyl)amino)imida zo[1,2-*b*]pyridazin-8 yl)amino)benzonitrile | **H** | **H** | **H** | 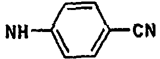 | 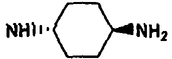 | 348 _{'} |
| **I(65)** | 3-((6-((*trans*-4-aminocyclohexyl)amino)imida zo[1,2-*b*]pyridazin-8-yl)amino)benzoic acid | **H** | **H** | **H** | 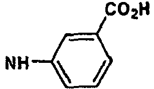 | 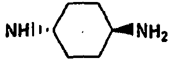 | 367 |
| **I(66)** | *N*⁶-(*trans*-4-aminocyclohexyl)-*N*⁸-1*H*-pyrazol-3-ylimidazo[1,2-*b*]pyridazine-6.8-diamine, | **H** | **H** | **H** | 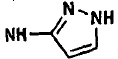 | 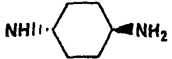 | 313 |
| **I(67)** | 4-((6-((*trans*-4-aminocyclohexyl)amino)imida zo[1,2-*b*]pyridazin-8-yl)amino)benzoic acid | **H** | **H** | **H** | 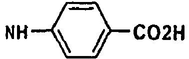 | 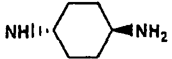 | 367 |
| **I(68)** | 4-((6-((*trans*-4-aminocyclohexyl)amino)imida zo[1,2-*b*]pyridazin-8-yl)amino)-*N,N*-dimethylbenzenesulfonamide | **H** | **H** | **H** | 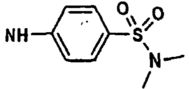 | 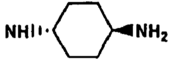 | 431 |
| **I(69)** | *N*⁶-(*trans*-4-aminocyclohexyl)-*N*⁸-(4-(1*H*-tetrazol-5-yl)phenyl)imidazo[1,2-*b*]pyridazine-6,8-diamine | **H** | **H** | **H** | 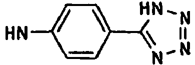 | 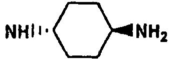 | 391 |
| **I(70)** | *N*⁶-(*trans*-4-(ethylamino)cyclobexyl)-*N*⁸-phenylimidazo[1,2-*b*]pyridazine-6,8-diamine | **H** | **H** | **H** | 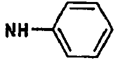 | 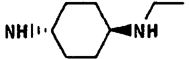 | 351 |
| **I(71)** | *N*⁶*-*(*trans*-4-(methylamino)cyclohexyl)-*N*⁸-phenylimidazo[1,2-*b*]pyridazine-6,8-diamine | **H** | **H** | **H** | 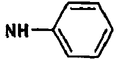 | 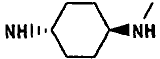 | 337 |
| **I(72)** | *N*⁶-(*trans*-4-aminocyclohexyl)-*N*⁸-(2-(phenyloxy)phenyl)imidazo[1, 2-*b*]pyridazine-6,8-diamine | **H** | **H** | **H** | 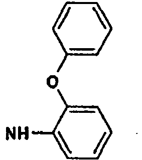 | 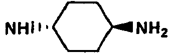 | 416 |
| **I(73)** | *N*⁶-(*trans*-4-aminocyclohexyl)-*N*⁸-(4'-chloro-4-biphenylyl)imidazo[1,2-*b*]pyridazine-6,8-diamine | **H** | **H** | **H** | 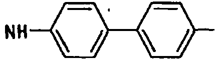 | 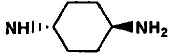 | 434 |
| **I(74)** | *N*⁶-(*trans*-4-aminocyclohexyl)-*N⁸*-(2'-methyl-4-biphenylyl)imidazo[1,2-*b*]pyridazine-6,8-diamine | **H** | **H** | **H** | 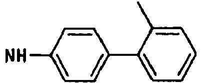 | 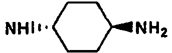 | 414 |
| **I(75)** | *N*⁶-(*trans*-4-aminocyclohexyl}-*N*⁸-(3'-chloro-4-biphenylyl)imidazo[1,2-*b*]pyridazine-6,8-diamine | **H** | **H** | H | 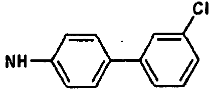 | 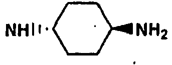 | 434 |
| **I(76)** | *N*⁶-(*trans*-4-aminocyclohexyl)-*N*⁸-(4-(phenylmethyl)phenyl)imidazo [1,2-*b*]pyridazine-6,8-diamine | **H** | **H** | **H** | 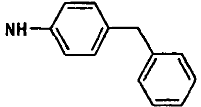 | 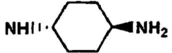 | 414 |
| **I(77)** | *N*⁶-(*trans*-4-aminocyclohexyl)-*N*⁸-(4-(4-morpholinyl)phenyl)imidazo[1, 2-*b*]pyridazine-6,8-diamine | **H** | **H** | **H** | 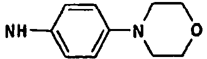 | 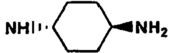 | 409 |
| **I(78)** | *N*⁶-(*trans*-4-aminocyclohexyl)-*N*⁶-(3'-chloro-3-biphenylyl)imidazo[1,2-*b*]pyridazine-6,8-diamine | **H** | **H** | **H** | 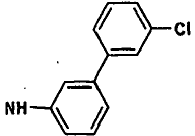 | 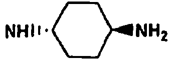 | 434 |
| **I(79)** | *N*⁶-(*trans*-4-aminocyclohexyl)-*N*⁸-(4-(1-methy)ethyl)phenyl)imidazo[1, 2-*b*]pyridazine-6,8-diamine | **H** | **H** | **H** | 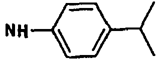 | 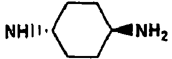 | 366 |
| I(80) | *N*⁶-(*trans*-4-aminocyclohexyl)-*N*⁸-(4-butylphenyl)imidazo[1,2-*b*]pyridazine-6,8-diamine | H | H | H | 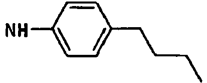 | 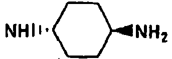 | 380 |
| 1(81) | *N*⁶-(*trans*-4-aminocyclohexyl)-*N*⁸-(5,6,7,8-tetrahydro-1-naphthalenyl)imidazo[1,2-*b*]pyridazine-6,8-diamine | H | H | H | 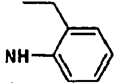 | 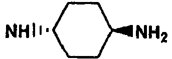 | 378 |
| I(82) | *N*⁶-(*trans*-4-aminocyclohexyl)-*N*⁸-1-naphthalenylimidazo[1,2-*b*]pyridazine-6,8-diamine | H | H | H | 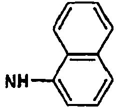 | 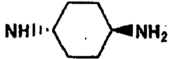 | 374 |
| I(83) | *N*⁶-(*trans*-4-aminocyclohexyl)-*N*⁸-(3-(phenylmethyl)phenyl)imidazo [1,2-*b*]pyridazine-6,8-diamine | H | H | H | 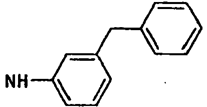 | 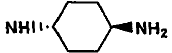 | 414 |
| 1(84) | *N*⁶-(*trans*-4-aminocyclohexyl)-*N*⁸-(4-propylphenyl)imidazo[1,2-*b*]pyridazine-6,8-diamine | H | H | H | 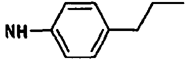 | 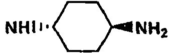 | 366 |
| I(85) | *N*⁶-(*trans*-4-aminocyclohexyl)-*N*⁸-(4'-methyl-4-biphenylyl)imidazo[1,2-*b*]pyridazine-6,8-diamine | H | H | H | 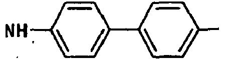 | 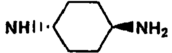 | 414 |
| I(86) | *N*⁶-*trans*-4-aminocyclohexyl)-methylethyl)phenyl)imidazo[1, 2-*b*]pyridazine-6,8-diamine | H | H | H | 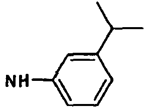 | 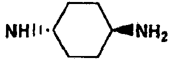 | 366 |
| 1(87) | *N*⁶-(*trans*-4-aminocyclohexyl)-*N*⁸-(3-((1-methylethyl)oxy)phenyl)imidaz o[1,2-*b*]pyridazine-6,8-diamine | H | H | H | 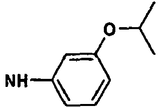 | 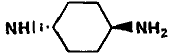 | 382 |
| I(88) | *N*⁶-(*trans*-4-aminocyclohexyl)-*N*⁸-(3,5-bis(methyloxy)phenyl)imidazo[ 1,2-*b*]pyridazine-6,8-diamine | H | H | H | 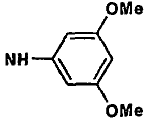 | 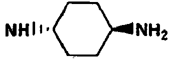 | 384 |
| I(89) | *trans-N*-(8-(6-methyl-3,4-dihydro-1(2*H*)-quinolinyl)imidazo[1,2-*b*]pyridazin-6-yl)-1,4-cyclohexanediamine | H | H | H | 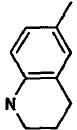 | 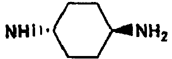 | 378 |
| I(90) | *N*⁶-(*trans*-4-aminocyclohexyl)-*N*⁸-2-naphthalenylimidazo[1,2-*b*]pyridazine-6,8-diamine | H | H | H | 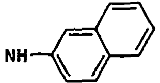 | 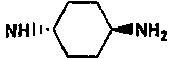 | 374 |
| I(91) | *N*⁶-(*trans*-4-aminocyclohexyl)-*N⁸-(3-*(methylsulfanyl)phenyl)imidaz o[1,2-*b*]pyridazine-6,8-diamine | H | H | H | 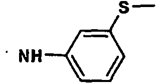 | 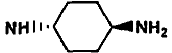 | 370 |
| I(92) | *N*⁶-(*trans*-4-aminocyclohexyl)-*N*⁸-(3-ethylphenyl)imidazo[1,2-*b*]pyridazine-6,8-diamine | H | H | H | 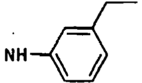 | 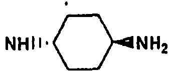 | 352 |
| I(93) | *N*⁶-(*trans*-4-aminocyclohexyl)-*N*⁸-(4-ethylphenyl)imidazo[1,2-*b*]pyridazine-6,8-diamine | H | H | H | 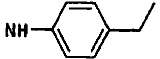 | 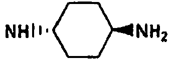 | 352 |
| I(94) | *N*⁶-(*trans*-4-aminocyclohexyl)-*N*⁸-(4-(methylsulfanyl)phenyl)imidaz o[1,2-*b*]pyridazine-6,8-diamine | H | H | H | 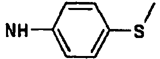 | 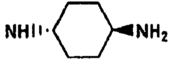 | 370 |
| I(95) | *N*⁶-(trans-4-aminocyclohexyl)-*N*⁸-9*H*-fluoren-2-ylimidazo[1,2-*b*]pyridazine-6,8-diamine | H | H | H | 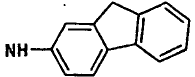 | 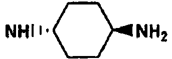 | 412 |
| I(96) | *N*⁶-(*trans*-4-aminocyclohexyl)-*N*⁸-(2-ethylphenyl)imidazo[1,2-*b*]pyridazine-6,8-diamine | H | H | H | 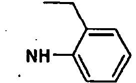 | 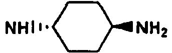 | 352 |
| I(97) | *N*⁶-(*trans*-4-iminocyclohexyl)-*N*⁶-(4-cyclohexylphenyl)imidazo[1,2-*b*]pyridazine-6,8-diamine | H | H | H | 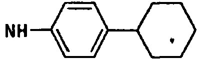 | 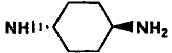 | 406 |
| I(98) | *N*⁶-(trans-4-aminocyclohexyl)-*N*⁸-(4-(1,1-dimethylethyl)phenyl)imidazo[ 1,2-*b*]pyridazine-6,8-diamine | H | H | H | 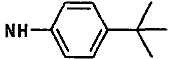 | 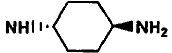 | 380 |
| I(99) | *N*⁶-(*trans*-4-aminocyclohexyl)-*N*⁸-(3-(phenyloxy)phenyl)imidazo[1, 2-*b*]pyridazine-6,8-diamine | H | H | H | 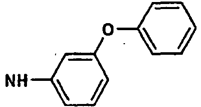 | 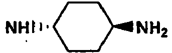 | 416 |
| I(100) | *N*⁶-(trans-4-aminocyclohexyl)-*N*⁸-3-biphenylylimidazo[1,2-*b*]pyridazine-6,8-diamine | H | H | H | 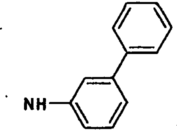 | 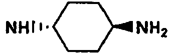 | 400 |
| I(101) | *N*⁶-(*trans*-4-aminocyclohexyl)-*N*⁸-(4-((1-methylethyl)oxy)phenyl)imidaz o[1,2-*b*]pyridazine-6,8-diamine | H | H | H | 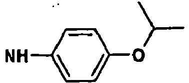 | 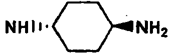 | 382 |
| I(102) | *N*⁶-(*trans*-4-aminocyclohexyl)-*N*⁸-(2-chlorophenyl)imidazo[1,2-*b*]pyridazine-6,8-diamine | H | H | H | 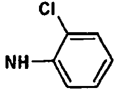 | 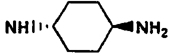 | 358 |
| I(103) | *N*⁶-(*trans*-4-aminocyclohexyl)-*N*⁸-(4-chlorophenyl)imidazo[1,2-*b*]pyridazine-6,8-diamine | H | H | H | 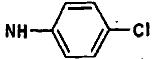 | 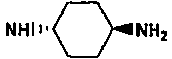 | 358 |
| I(104) | *N*⁶-(*trans*-4-aminocyclohexyl)-*N*⁸-(3-chlorophenyl)imidazo[1,2-*b*]pyridazine-6,8-diamine | H | H | H | 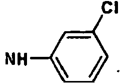 | 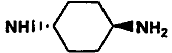 | 358 |
| 1(105) | *N*⁶-(*trans*-4-aminocyclohexyl)-*N*³-(4-chloro-1-naphthalenyl)imidazo[1,2-*b*]pyridazine-6,8-diamine | H | H | H | 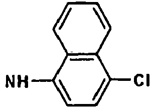 | 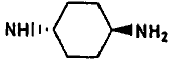 | 408 |
| I(106) | *N*⁶-(*trans*-4-aminocyclohexyl)-*N*⁸-3-quinolinylimidazo[1,2-*b*]pyridazine-6,8-diamine | H | H | H | 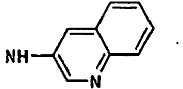 | 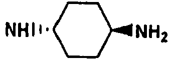 | 375 |
| I(107) | *N*⁶-(*trans*-4-aminocyclohexyl) *N*⁸-(3,5-dichlorophenyl)imidazo[1,2-*b*]pyridazine-6,8-diamine | H | H | H | 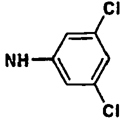 | 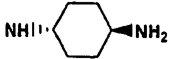 | 392 |
| I(108) | *N*⁶-(*trans*-4-aminocyclohexyl)-*N*⁸-(3-(trifluoromethyl)phenyl)imidaz o[1,2-*b*]pyridazine-6,8-diamine | H | H | H | 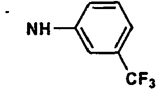 | 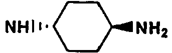 | 391 |
| I(109) | *N*⁶-(*trans*-4-aminocyclohexyl)-*N*⁸-(4-chloro-2-fluorophenylimidazo[1,2-*b*]pyridazine-6,8-diamine | H | H | H | 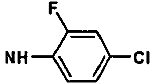 | 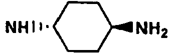 | 376 |
| I(110) | *N*⁶-(*trans*-4-aminocyclohexyl)-*N*⁸-(2-fluoro-5-methylphenyl)imidazo[1,2-*b*]pyridazine-6,8-diamine | H | H | H | 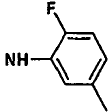 | 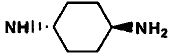 | 355 |
| I(111) | *N*⁶-(*trans*-4-aminocyclohexyl)-*N*⁸-(4-chloro-3-methylphenyl)imidazo[1,2-*b*]pyridazine-6,8-diamine | H | H | H | 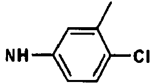 | 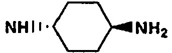 | 372 |
| I(112) | *N*⁶-(*trans*-4-aminocyclohexyl)-*N*⁸-(5-phenyl-2-pyridinyl)imidazo[1,2-*b*]pyridazine-6,8-diamine | H | H | H | 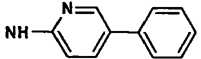 | 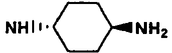 | 401 |
| I(113) | *N*⁶-(*trans*-4-aminocyclohexyl)-*N*⁸-(3-fluoro-4-methylphenyl)imidazo[1,2-*b*]pyridazine-6,8-diamine | H | H | H | 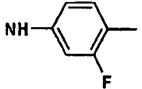 | 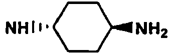 | 355 |
| I(114) | *N*⁶-(*trans*-4-aminocyclohexyl)-*N*⁸-(2-methyl-4-pyridinyl)imidazo[1,2-*b*]pyridazine-6,8-diamine | H | H | H | 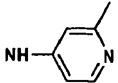 | 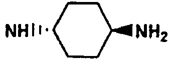 | 338 |
| I(115) | *N*⁶-(*trans*-4-aminocyclohexyl)-*N*⁸-(4-fluoro-3-methylphenyl)imidazo[1,2-*b*]pyridazine-6,8-diamine | H | H | H | 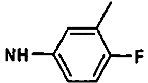 | 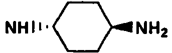 | 355 |
| *I(116)* | *N*⁶-(*trans*-4-aminocyclohexyl)-*N*⁸-(2-fluoro-4-methylphenyl)imidazo[1,2-*b*]pyridazine-6,8-diamine | H | H | H | 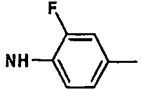 | 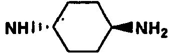 | 355 |
| I(117) | *N*⁶-(*trans*-4-aminocyclohexyl)-*N*⁸-(3-((trifluoromethyl)oxy)phenyl)i midazo[1,2-*b*]pyridazine-6,8-diamine | H | H | H | 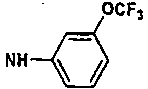 | 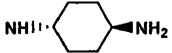 | 407 |
| I(118) | *N*⁶-(*trans*-4-aminocyclohexyl)-*N*⁸-(4-methyl-3-(trifluoromethyl)phenyl)imidaz o[1,2-*b*]pyridazine-6,8-diamine | H | H | H | 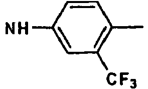 | 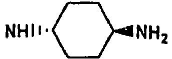 | 405 |
| I(119) | *N*⁶-(*trans*-4-aminocyclohexyl)-*N*⁸-(4-ethyl-2-pyridinyl)imidazo[1,2-*b*]pyridazine-6,8-diamine | H | H | H | 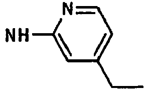 | 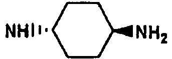 | 353 |
| I(120) | *N*⁶-(*trans*-4-aminocyclohexyl)-*N*⁸-(4-(1*H*-1,2,4-triazol-1-yl)phenyl)imidazo[1,2-*b*]pyridazine-6,8-diamine | H | H | H | 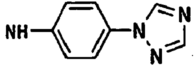 | 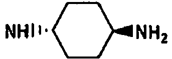 | 390 |
| I(121) | *N*⁶-(*trans*-4-aminocyclohexyl)-*N*⁸-(4-(1*H*-pyrrol-1-yl)phenyl)imidazo[1,2-*b*]pyridazine-6,8-diamine | H | H | H | 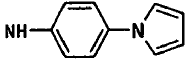 | 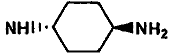 | 389 |
| I(122) | *N*⁶-(*trans*-4-aminocyclohexyl)-*N*⁸-(4-(4,5-dichloro-1*H*-imidazol-1-yl)phenyl)imidazo[1,2-*b*]pyridazine-6,8-diamine | H | H | H | 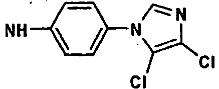 | 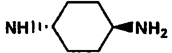 | 458 |
| I(123) | *N*⁶-(*trans*-4-aminocyclohexyl)-*N*⁸-(4-(1*H*-pyrazol-1-yl)phenyl)imidazo[1,2-*b*]pyridazine-6,8-diamine | H | H | H | 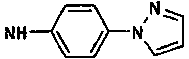 | 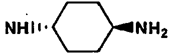 | 390 |
| I(124) | *N*⁶-(*trans*-4-aminocyclohexyl)-*N*⁸-(4-(3,5-dimethyl-1*H*-pyrazol-1-yl)phenyl)imidazo[1,2-*b*]pyridazine-6,8-diamine | H | H | H | 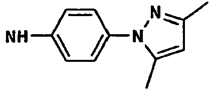 | 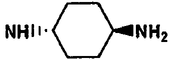 | 418 |
| 1(125) | *N*⁶-(*trans*-4-aminocyclohexyl)-*N*⁸-(4-(4-methyl-4*H*-1,2,4-triazol-3-yl)phenyl)imidazo[1,2-*b*]pyridazine-6,8-diamine | H | H | H | 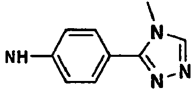 | 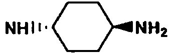 | 405 |
| I(126) | *N*⁶-(*trans*-4-aminocyclohexyl)-*N*⁸-(4-(1*H*-imidazol-1-yl)phenyl)imidazo[1,2-*b*]pyridazine-6,8-diamine | H | H | H | 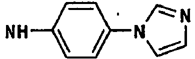 | 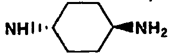 | 390 |
| I(127) | *N*⁶-(*trans*-4-aminocyclohexyl)-*N*⁸-(4-(1-methyl-1*H*-imidazol-2-yl)phenyl)imidazo[1,2-*b*]pyridazine-6,8-diamine | H | H | H | 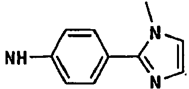 | 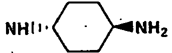 | 404 |
| I(128) | *N*⁶-(*trans*-4-aminocyclohexyl)-*N*⁸-(4-(2-methyl-1,3-thiazol-4-yl)phenyl)imidazo[1,2-*b*]pyridazine-6,8-diamine | H | H | H | 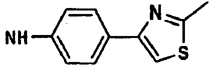 | 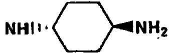 | 421 |
| I(129) | *N*⁶-(*trans*-4-aminocyclohexyl)-*N*⁸-(4-(5-methyl-2-furanyl)phenyl)imidazo[1,2-*b*]pyridazine-6,8-diamine | H | H | H | 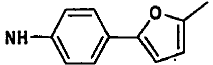 | 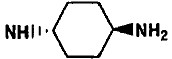 | 404 |
| I(130) | *N*⁶-(*trans*-4-aminocyclohexyl)-*N*⁸-(4-(2-ethyl-2*H*-tetrazol-5-yl)phenyl)imidazo[1,2-*b*]pyridazine-6,8-diamine | H | H | H | 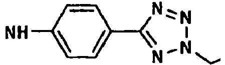 | 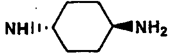 | 420 |
| I(131) | 4-((6-((*trans*-4-aminocyclohexyl)amino)imida zo[1,2-*b*]pyridazin-8-yl)amino)-3-hydroxy-*N*,*N*-dimethylbenzenesulfonamide | H | H | H | 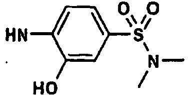 | 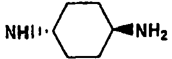 | 447 |
| I(132) | *N*⁶-(*trans*-4-aminocyclohexyl)-*N*⁸-2,3-dihydro-1*H*-inden-5-yl)imidazo[1,2-*b*]pyridazine-6,8-diamine | H | H | H | 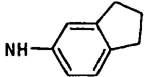 | 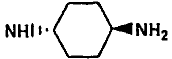 | 364 |
| I(133) | 3-((6-((*trans*-4-aminocyclohexyl)amino)imida zo[1,2-*b*]pyridazin-8-yl)amino)-*N*,*N*-dimethylbenzenenesulfonamide | H | H | H | 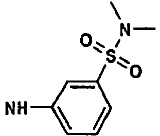 | 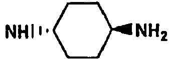 | 431 |
| I(134) | 4-((6-((*trans*-4-aminocyclohexyl)amino)imida zo[1,2-*b*]pyridazin-8-yl)amino)-*N,N-*dimethylbenzamide | H | H | H | 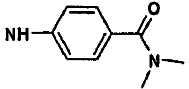 | 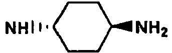 | 395 |
| I(135) | *N*⁶-(*trans*-4-((2-chloro-4-pyrimidinyl)amino)cyclohexyl) -*N*⁸-pheny)imidazo[1,2-*b*]pyridazine-6,8-diamine | H | H | H | 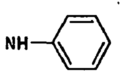 | 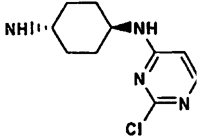 | 436 |
| I(136) | *N*⁶-(3-aminocyclopentyl)-*N*⁸-phenylimidazo[1,2-*b*]pyridazine-6,8-diamine | H | H | H | 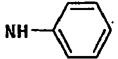 | 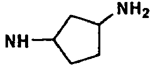 | 309 |
| I(137) | *N*⁶-(*trans*-4-aminocyclohexyl)-*N*⁶-(3-(4-morpholinylsulfonyl)phenyl)im idazo[1,2-*b*]pyridazine-6,8-diamine | H | H | H | 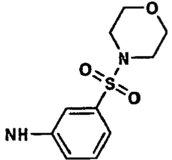 | 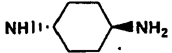 | 473 |
| I(138) | 3-((6-((*trans*-4-aminocyclohexyl)amino)imida zo[1,2-*b*]pyridazin-8-yl)amino)-*N*,*N*-diethylbenzamide | H | H | H | 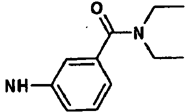 | 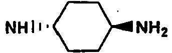 | 423 |
| I(139) | 3-((6-((*trans*-4-aminocyclohexyl)amino)imida zo[1,2-*b*]pyridazin-8-yl)amino)-*N*-methyl-*N*-phenylbenzenesulfonamide | H | H | H | 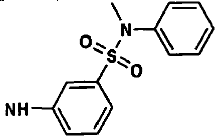 | 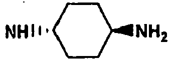 | 493 |
| I(140) | 4-((6-((*trans*-4-aminocyclohexyl)amino)imida zo[1,2-*b*]pyridazin-8-yl)amino)-2-hydroxy-*N*,*N*-dimethylbenzenesulfonamide | H | H | H | 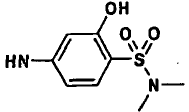 | 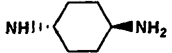 | 447 |
| I(141) | *N*⁶-(4-aminobicyclo[2.2.2]oct-1-yl)-*N*⁸-phenylimidazo[1,2-*b*]pyridazine-6,8-diamine | H | H | H | 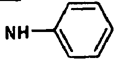 | 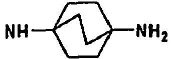 | 349 |
| I(42) | *N*-(4-((6-((*trans*-4-aminocyclohexyl)amino)imida zo[1,2-*b*]pyridazin-8-yl)amino)phenyl)methanesulfo namide | H | H | H | 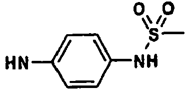 | 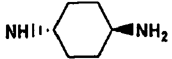 | 417 |
| I(143) | *N*⁶-(*trans*-4-aminocyclohexyl)-*N*⁸-(4-(3-(dimethylamino)-1-pyrrolidinyl)phenyl)imidazo[1, 2-*b*]pyridazine-6,8-diamine | H | H | H | 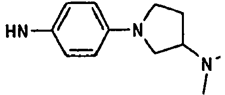 | 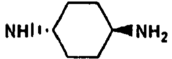 | 436 |
| I(144) | *N*⁶-(*trans*-4-aminocyclohexyl)-*N*⁸-(4-(1-pyrrolidinylsulfonyl)phenyl)im idazo[11,2-*b*]pyridazine-6,8-diamine | H | H | H | 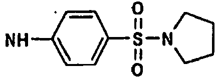 | 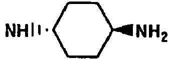 | 457 |
| I(145) | 4-((6-((*trans*-4-aminocyclohexyl)amino)imida zo[1,2-*b*]pyridazin-8-yl)amino)benzenesulfonic acid | H | H | H | 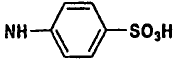 | 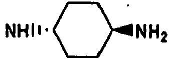 | 403 |
| I(146) | 4-((6-((*trans*-4-aminocyclohexyl)amino)imida zo[1,2-*b*]pyridazin-8-yl)amino)-*N*,*N*-diethylbenzenesulfonamide | H | H | H | 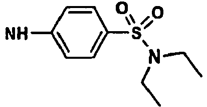 | 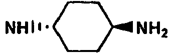 | 459 |
| I(147) | 4-((6-((*trans*-4-aminocyclohexyl)amino)imida zo[1,2-*b*]pyridazin-8-yl)amino)-*N*-propylbenzenesulfonamide | H | H | H | 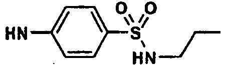 | 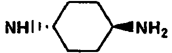 | 445 |
| I(148) | 4-((6-((*trans*-4-aminocyclohexyl)amino)imida zo[1,2-*b*]pyridazin-8-yl)amino)-*N*-ethylbenzenesulfonamide | H | H | H | 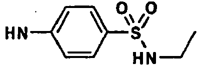 | 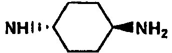 | 431 |
| I(149) | 4-((6-((*trans*-4-aminocyclohexyl)amino)imida zo[1,2-*b*]pyridazin-8-yl)amino)-*N*-methylbenzenesulfonamide | H | H | H | 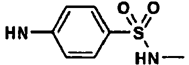 | 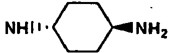 | 417 |
| I(150) | *N*⁶-(*trans*-4-aminocyclohexyl)-aminophenyl)imidazo[1,2-*b*]pyridazine-6,8-diamine | H | H | H | 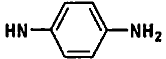 | 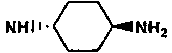 | 338 |
| I(151) | 4-((6-((*trans*-4-aminocyclohexyl)amino)imida zo[1,2-*b*]pyridazin-8-yl)amino)benzenesulfonamide | H | H | H | 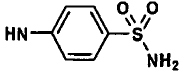 | 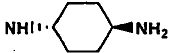 | 402 |
| I(152) | 3-((6-((*trans*-4-aminocyclohexyl)amino)imida zo[1,2-*b*]pyridazin-8-yl)amino)benzenesulfonamide | H | H | H | 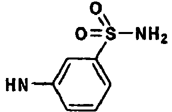 | 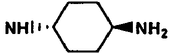 | 402 |
| I(153) | *3*-((6-((*trans*-4-aminocyclohexyl)amino)imida zo[1,2-*b*]pyridazin-8-yl)amino)benzamide | H | H | H | 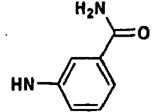 | 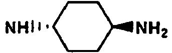 | 366 |
| I(154) | 4-((6-((*trans*-4-aminocyclohexyl)amino)imida zo[1,2-*b*]pyridazin-8-yl)amino)benzamide | H | H | H | 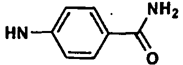 | 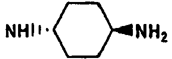 | 366 |
| I(155) | *N*⁶-(*trans*-4-aminocyclohexyl)-*N*⁶-(4-(aminomethyl)phenyl)imidazo[ 1,2-*b*]pyridazine-6,8-diamine | H | H | H | 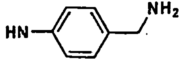 | 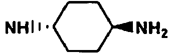 | 352 |
| I(156) | 6-((6-((*trans*-4-aminocyclohexyl)amino)imida zo[1,2-*b*]pyridazin-8-yl)amino)-1,2-dihydro-3*H*-indazol-3-one | H | H | H | 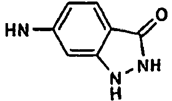 | 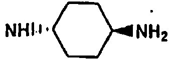 | 379 |
| I(157) | *N*⁶-(*trans*-4-aminocyclohexyl)-*N*⁹-(3-(aminomethyl)phenyl)imidazo[ 1,2-*b*]pyridazine-6,8-diamine | H | H | H | 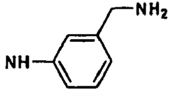 | 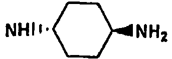 | 352 |

| | | | | | | | |
|---|---|---|---|---|---|---|---|
| *For substituents X and Y, substitution on the core (formula Ia) occurs at the available nitrogen atom | | | | | | | |

### EXAMPLE II(1)

### N⁶-(trans-4-aminocyclohexyl)-N⁸-(2,6-difluorophenyl)imidazo[1,2-b]pyridazine-6,8-diamine

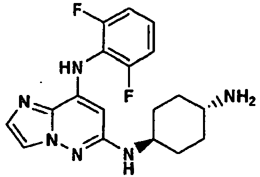

*(1a)* To 60% NaH (22.5 mg, 0.563 mmol) in DMF (400 µl) was added 2,6-difluoroaniline (24 mg, 0.186 mmol). After stirring at RT for 5 minutes THF (1000 µl) was added followed by 8-bromo-6-chloroimidazo[1,2-*b*]pyridazine (50 mg, 0.186 mmol, prepared as described in Example 1, step *(1b)*. The reaction was heated at 50 °C for 3 hours. The reaction was quenched with a few drops of water and methanol. The solution was then concentrated in vacuo to give crude 6-chloro-*N*-(2,6-difluorophenyl)imidazo[1,2-*b*]pyridazin-8-amine.
*(1b)* To crude 6-chloro-*N*-(2,6-difluorophenyl)imidazo[1,2-*b*]pyridazin-8-amine (0.186 mmol) from *1a* was added trans-1,4-diaminocyclohexane (1000 mg, 8.77 mmol). The mixture was allowed to melt at 160°C for 24 hrs. The melt was then cooled, water was added followed by extraction with dichloromethane. The organic layer was then concentrated in vacuo and the resulting residue purified by reverse phase preparative HPLC to provide 50.6 mg (46%) of the titled compound as a TFA salt. LC/MS, m/e 359 (M+1). HPLC Rt, 1.7 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min. Examples prepared in a similar manner are indicated in Table 2.

Compounds having the formula (Ia) were prepared according to procedures similar to Example II(1), where in R₁, R₂, R₃, X and Y have the values listed in Table 2, using the appropriate starting materials and substantially the same procedures as indicated.

**Table 2**

| **Exp** | **Name** | **R₁** | **R₂** | **R₃** | **X** | **Y** | **LC/MS m/z (M+1)** |
|---|---|---|---|---|---|---|---|
| II(2) | *N*⁶-(*trans*-4-aminocyclohexyl)-7-chloro-*N*⁸-[4-(ethyloxy)phenyl]imidazo[1,2-*b*]pyridazine-6,8-diamine | H | H | H | 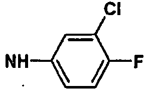 | 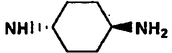 | 375 |
| II(3) | *N*⁶-(3-aminopropyl)-*N*⁸-[4-(ethyloxy)phenyl]imidazo[1,2-*b*]pyridazine-6,8-diamine | H | H | H | 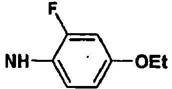 | 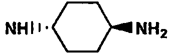 | 385 |
| II(4) | *N*⁶-(*trans*-4-aminocyclohexyl)-*N*⁸-(2-fluorophenyl)imidazo[1,2-*b*]pyridazine-6,8-diamine | H | H | H | 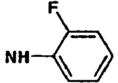 | 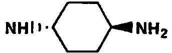 | 341 |
| II(5) | *N*⁶-(*trans*-4-aminocyclohexyl)-*N*⁸-(2,6-difluorophenyl)imidazo[1,2-*b*]pyridazine-6,8-diamine | H | H | H | 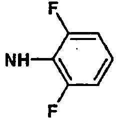 | 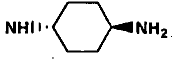 | 359 |
| II(6) | *N*⁶-(*trans*-4-aminocyclohexyl)-*N*⁸-(2,4-difluorophenyl)imidazo[1,2-*b*]pyridazine-6,8-diamine | H | H | H | 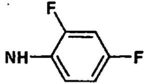 | 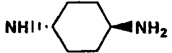 | 359 |
| II(7) | *N*⁶-(*trans*-4-aminocyclohexyl)-*N*⁸-(3-fluorophenyl)imidazo[1,2-*b*]pyridazine-6,8-diamine | H | H | H | 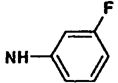 | 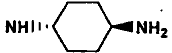 | 341 |
| II(8) | *N*⁶-(*trans*-4-aminocyclohexyl)-*N*⁸-(4-fluorophenyl)imidazo[1,2-*b*]pyridazine-6,8-diamine | H | H | H | 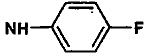 | 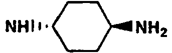 | 341 |
| II(9) | *N*⁶-(*trans*-4-aminocyclohexyl)-*N*⁸-(3,4-difluorophenyl)imidazo[1,2-*b*]pyridazine-6,8-diamine | H | H | H | 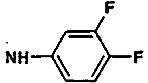 | 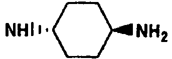 | 359 |
| II(10) | *N*⁶-(*trans*-4-aminocyclohexyl)-*N*⁸-(2,5-difluorophenyl)imidazo[1,2-*b*]pyridazine-6,8-diamine | H | H | H | 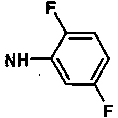 | 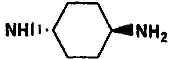 | 359 |
| II(11) | *N*⁶-(*trans*-4-aminocyclohexyl)-*N*⁶-(2,3-difluorophenyl)imidazo[1,2-*b*]pyridazine-6,8-diamine | H | H | H | 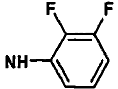 | 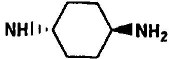 | 359 |
| II(12) ' | *N*⁶-(*trans*-4-aminocyclohexyl)-*N*⁸-(3,5-difluorophenyl)imidazo[1,2-*b*]pyridazine-6,8-diamine | H | H | H | 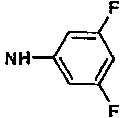 | 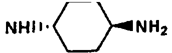 | 359 |
| II(13) | *N*⁶-(*trans*-4-aminocyclohexyl)-*N*⁸-(3-iodophenyl)imidazo[1,2-*b*]pyridazine-6,8-diamine | H | H | H | 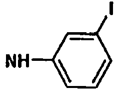 | 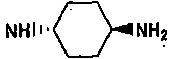 | 449 |
| II(14) | *N*⁶-*trans*-4-aminocyclohexyl)-*N*⁸-[4-(trifluoromethyl)phenyl]imidazo[1,2-*b*]pyridazine-6,8-diamine | H | H | H | 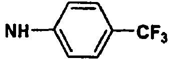 | 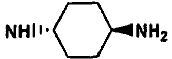 | 391 |
| II(15) | *N*⁶-(trans-4-aminocyclohexyl)-*N*⁸-pyridin-2-ylimidazo[1,2-*b*]pyridazine-6,8-diamine | H | H | H | 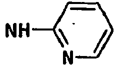 | 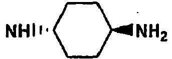 | 324 |
| II(16) | *N*⁶-(*trans*-4-aminocyclohexyl)-*N*⁸-(4-methylpyridin-2-yl)imidazo[1,2-*b*]pyridazine-6,8-diamine | H | H | H | 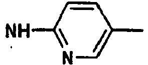 | 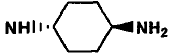 | 338 |
| II(17) | *N*⁶-(*trans*-4-aminocyclohexyl)-*N*⁸-(5-methylpyridin-2-yl)imidazo[1,2-*b*]pyridazine-6,8diamine | H | H | H | 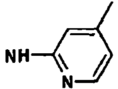 | 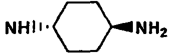 | 338 |
| II(18) | *N*⁶-(*trans*-4-aminocyclohexyl)-*N*⁸-(4,6-dimethylpyridin-2-yl)imidazo[1,2-*b*]pyridazine-6,8-diamine | H | H | H | 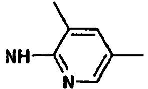 | 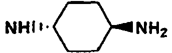 | 352 |
| II(19) | *N*⁶-*trans*-4-aminocyclohexyl)-*N*⁸-pyrimidin-2-ylimidazo[1,2-*b*]pyridazine-6,8-diamine | H | H | H | 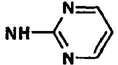 | 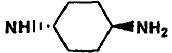 | 325 |

| | | | | | | | |
|---|---|---|---|---|---|---|---|
| *For substituents X and Y, substitution on the core (formula Ia) occurs at the available nitrogen atom | | | | | | | |

### EXAMPLE III(1)

### N⁶-(trans-4-aminocyclohexyl)-N⁸-[4-(ethyloxy)phenyl]-7-methylimidazo[1,2-b]pyridazine-6,8-diamine

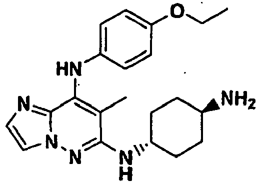

*(1a)* 3,6-Dichloro-4-methyl pyridizine (4.2 g, 26 mmol, Alfa) was suspended in aqueous 28% NH₄OH (14 mL) in a sealed microwave tube and heated at 155°C for 1.5 h. The microwave tube was uncapped and allowed to stir at room temperature for 30 min and in an ice bath for 30 min. The solid that crashed out was filtered, washed with ice water, and dried to give a mixture of 6-chloro-5-methylpyridazin-3-amine and 3-chloro-5-methylpyridazin-6-amine (3.4 g, 91 %).
*(1b)* The mixture of 6-chloro-5-methylpyridazin-3-amine and 3-chloro-5-methylpyridazin-6-amine (1.45 g, 10 mmol) from *1a* and NaHCO₃ (2.1g, 25 mmol) were suspended in MeOH (20 mL) and treated with Br₂ (0.57 mL, 11 mmol). The mixture was stirred at room temperature for 4h, then filtered. The filtrate was condensed in vacuo. The resulting residue was resuspended in EtOAc (100 mL) and washed sequentially with sat. aqueous NaHCO₃ solution (2X20 mL) and aqueous NaCl solution (1X20 mL). The solution was dried over MgSO₄. The solvent was removed in vacuo to give crude 4-bromo-6-chloro-5-methylpyridazin-3-amine (1 g).
(*1c*) Chloroacetaldehyde (1.6 ml, 10 mmol, 50% in H₂O) was added to a solution of crude 4-bromo-6-chloro-5-methylpyridazin-3-amine (0.5 g, 2 mmol) from *1b* in EtOH (5 mL). The mixture was heated in a sealed vial at 110°C for 2h. Solvent was removed in vacuo and the resulting solid was suspended in acetone/Et₂O (1/1, 5 mL), filtered, and then washed with Et₂O to give 8-bromo-6-chloro-7-methylimidazo[1,2-b]pyridazine HCl salt (0.5g, >90% pure).
*(1d)* A mixture of 8-bromo-6-chloro-7-methylimidazo[1,2-b]pyridazine HCl salt (30 mg, 0.1 mmol) from *1c*, *p*-methoxyaniline (20 pL, 0.15 mmol) and K₂CO₃ (75 mg) were suspended in NMP (600 µL) and heated in a microwave at 225°C for 15 min. The mixture was cooled to room temperature and then treated with H₂O (5 mL). The solid that precipitated out was filtered, washed with water and dried to provide 6-chloro-*N*-(4-ethoxyphenyl)-7-methylimidazo[1,2-*b*]pyridazin-8-amine (21 mg, >90 % pure by HPLC).
*(1e)* 6-chloro-*N*-(4-ethoxyphenyl)-7-methylimidazo[1,2-*b*]pyridazin-8-amine (20 mg, 0.067 mmol) from *1d* and trans-1,4-diaminocyclohexane (150 mg) were combined and heated at 165°C for 70 h. The mixture was cooled to room temperature, then diluted with water (10 mL) and extracted with EtOAc (4X5 mL). The organic layers were combined and concentrated in vacuo. The resulting residue was purified using preparative HPLC to give the above titled compound as a TFA salt (4.5 mg, 11%). ¹H NMR (400 MHz, CD₃OD) δ 7.97 (d, *J*=2.4 Hz, 1H), 7.67 (d, *J*=2.4 Hz, 1H), 6.90 (m, 4H), 4.05 (q, 2H, *J*=7.2 Hz), 4.00 (m, 1H), 3.20 (m, 1H), 2.30 (m, 2H), 2.20(m, 2H), 2.15 (s, 3H), 1.60 (m, 4H), 1.40 (t, *J*=7.2 H, 3H). LC/MS, *m*/*e* 381 (M+1). HPLC Rt, 2.1 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B =100, gradient time 4 min, hold at 100% B 1 min, flow rate 4 mL/min.

Compounds having the formula (Ia) were prepared according to procedures similar to Example III(1), where in R₁, R₂, R₃, X and Y have the values listed in Table 3, using the appropriate starting materials and substantially the same procedures as indicated.

**Table 3**

| **Exp** | **Name** | **R₁** | **R₂** | **R₃** | **X** | **Y** | **LC/MS m/z (M+1)** |
|---|---|---|---|---|---|---|---|
| III(2) | *N*⁶-(*trans*-4-aminocyclohexyl)-7-ethyl-*N*⁸-[4-(ethyloxy)phenyl]imidazo[1,2-*b*]pyridazine-6,8-diamine | H | H | Et | 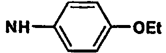 | 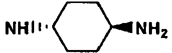 | 395 |
| III(3) | *N*⁶-(*trans*-4-aminocyclohexyl)-7-methyl-*N*⁸-phenylimidazo[1,2-*b*]pyridazine-6,8-diamine | H | H | CH3 | 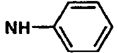 | 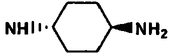 | 337 |
| III(4) | *N*⁶-(*trans*-4-aminocyclohexyl)-*N*⁸-(3,4-dimethylphenyl)-7-methylimidazo[1,2-*b*]pyridazine-6,8-diamine | H | H | CH3 | 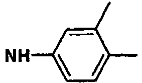 | 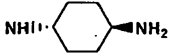 | 365 |
| III(5) | *N*⁶-(trans-4-aminocyclohexyl)-7-methyl-*N*⁸-(4-methylphenyl)imidazo[1,2-*b*]pyridazine-6,8-diamine | H | H | CH3 | 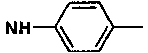 | 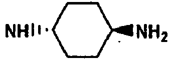 | 351 |
| III(6) | *N*⁶-(*trans*-4-aminocyclohexyl)-7-methyl-*N*⁸-[3-(methyloxy)phenyl]imidazo[1,2-b]pyridazine-6,8-diamine | H | H | CH3 | 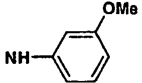 | 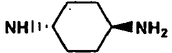 | 367 |
| III(7) | *N*⁶-(*trans*-4-aminocyclohexyl)-*N*⁸-biphenyl-4-yl-7-methylimidazo[1,2-*b*]pyridazine-6,8-diamine | H | H | CH3 | 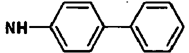 | 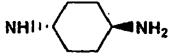 | 413 |
| III(8) | *N*⁶-(*trans*-4-aminocyclohexyl)-7-methyl-*N*⁸-[4-(propyloxy)phenyl]imidazo[ 1,2-*b*]pyridazine-6,8-diamine | H | H | CH3 | 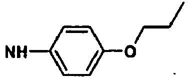 | 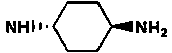 | 395 |
| III(9) | 4-((6-((*trans*-4-aminocyclohexyl)amino)-7-methylimidazo[1,2-*b*]pyridazin-8-yl)amino)benzoic acid | H | H | CH3 | 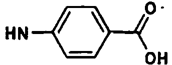 | 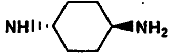 | 381 |
| III(10) | 4-((6-((4-aminocyclohexyl)amino)-7-methylimidazo[1,2-*b*]pyridazin-8-yl)amino)-*N*,*N*-dimethylbenzenesulfonamide | H | H | CH3 | 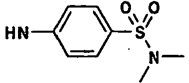 | 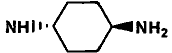 | 444 |
| III(11)1 | *N*-(4-((6-((*trans*-4-aminocyclohexyl)amino)-7-methylimidazo[1,2-*b*]pyridazin-8-yl)amino)phenyl)-*N*-methylacetamide | H | H | CH3 | 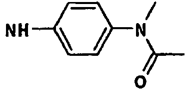 | 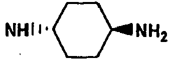 | 408 |
| III(12) , | *N*⁶-(*trans*-4-aminocyclohexyl)-7-ethyl-*N*⁸-phenylimidazo[1,2-*b*]pyridazine-6,8-diamine | H | H | Et | 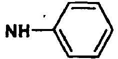 | 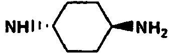 | 351 |

| | | | | | | | |
|---|---|---|---|---|---|---|---|
| *For substituents X and Y, substitution on the core (formula Ia) occurs at the available nitrogen atom | | | | | | | |

### EXAMPLE IV(1)

### N⁶-(trans-4-aminocyclohexyl)-7-chloro-N⁸-[4-(ethyloxy)phenyl]imidazo[1,2-b]pyridazine-6,8-diamine

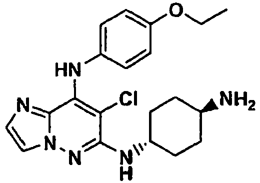

*(1a)* 3-Hydroxy-4,5-dichloropyridizine (5.34 g, 32.5 mmol) was added to a stirred solution of fuming H₂SO₄ (14.0 mL, 273 mmol) and conc. H₂SO₄ (7.3 mL, 137 mmol). KNO₃ (9.0 g, 88 mmol) was subsequently added slowly at room temperature. The reaction mixture was heated to 90 °C and the temperature was maintained at 90 °C for 18 hrs. The solution was cooled and poured over ice water. After stirring for one hour, the solid suspension was filtered to give 3-hydroxy-4,5-dichloro-6-nitropyridizine as a white solid (4.2 g, 62%).
(*1b*) Na₂S₂O₄ (0.65 g, 3.6 mmol) was added to a stirred solution of 3-hydroxy-4,5-dichloro-6-nitropyridizine (0.26 g, 1.2 mmol) from *1a* in THF (4.0 mL, 0.3 M) and H₂O (4.0 mL, 0.3 M). The reaction mixture was warmed to reflux, after 1 hr ethyl acetate was added. The layers were allowed to separate and the organic layer was washed with H₂O (10 mL), followed by brine (10 mL). The organic layer was dried over Na₂SO₄ and then concentrated *in vacuo.* The resulting residue was triterated with diethyl ether and filtered to give 3-hydroxy-4,5-dichloro-6-aminopyridizine as a white solid (0.165 g, 67%).
*(1c)* Chloroacetaldehyde (0.57 ml, 4.6 mmol, 50% in H₂O) was added to a solution of 3-hydroxy-4,5-dichloro-6-aminopyridizine (0.165 g, 0.92 mmol) from *1b* in EtOH (1.3 mL, 0.7 M). The reaction was heated in a sealed vial at 150 °C for 15 minutes in the microwave. The solvent was removed *in vacuo* and the solid was re-suspended in diethyl ether (10 mL), filtered and rinsed with diethyl ether to give 6-hydroxy-7,8-dichloroimidazo[1,2-*b*]pyridazine as an HCl salt (0.3 g, 60%).
(*1d*) 6-Hydroxy-7,8-dichloroimidazo[1,2-*b*]pyridazine (0.1 g, 0.5 mmol) from *1c* was added to POCl₃ (0.4 mL, 1.4 M) in a 1 dram vial. The mixture was heated to 120 °C for 2 days. Upon cooling CH₂Cl₂ was added and the mixture was poured onto ice water. The layers were separated and the aqueous layer was extracted with CH₂Cl₂ (15 mL). The organic layers were combined and washed with H₂O (5 mL), followed by brine (5 mL). The solution was dried over Na₂SO₄ and concentrated *in vacuo* to give 6,7,8-trichloroimidazo[1,2-*b*]pyridazine as a tan solid (0.04 g, 37%).
*(1e)* A mixture of 6,7,8-trichloroimidazo[1,2-*b*]pyridazine (0.04 g, 0.18 mmol) from *1d*, *p*-ethoxyaniline (0.025 g, 0.18 mmol) and triethylamine (0.055 mL, 0.4 mmol) were suspended in EtOH (1.0 mL) and heated to 90 °C for 2.5 hours. The reaction mixture was then cooled to room temperature and concentrated *in vacuo* to provide crude 6,7-dichloro-*N*-(4-ethoxyphenyl)imidazo[1,2-*b*]pyridazin-8-amine (0.05 g, 86%).
(*1f*) Crude 6,7-dichloro-*N*-(4-ethoxyphenyl)imidazo[1,2-*b*]pyridazin-8-amine (0.03 g, 0.09 mmol) from *1e* was mixed with *trans*-1,4-diaminocyclohexane (0.07 g, 0.6 mmol). The resulting mixture was heated to 120 °C for 1.5 days. Upon cooling, CH₂Cl₂ (10 mL) and H₂O (10 mL) were added and the layers separated. The organic layer was washed with brine (5 mL), dried over Na₂SO₄, and concentrated *in vacuo.* The resulting oil was purified by preparative HPLC to give the above titled compound as a TFA salt (0.010g, 29%); ¹H NMR (MeOH) δ 8.00 (s,1H), 7.70 (s,1H), 7.13 (d, J=8.8 Hz, 2H), 6.98 (d, J=8.8 Hz, 2H), 4.09 (q, J= 7.0 Hz, 2H), 4.00 - 3.90 (m, 1H), 3.24 - 3.15 (m, 1H), 2.30 - 2.23 (m, 2H), 2.20 - 2.10 (m, 2H), 1.65 1.57 (m, 4H), 1.50 (t, 3H). LC/MS, *m*/*e* 402 (M+1). HPLC Rt, 1.95 min. YMC ODSC18 column (4.6 x 100 mm). 20%-100%B. Solvent B: (90% MeOH, 10 % H₂O, 0.1%.TFA). Solvent A: (10% MeOH, 90 % H₂O, 0.1% TFA). Gradient, start %B = 20, final % B = 100, gradient time 10 min, hold at 100% B, 2 min, flow rate 4 mL/min.

### EXAMPLE V(I)

### N⁸-[4-(ethylozy)phenyl]-N⁶-piperidin-3-ylimidazo[1,2-b]pyridazine-6,8-diamine

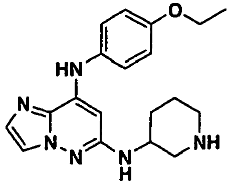

To a mixture of a *N⁶*-(1-benzylpiperidin-3-yl)-*N⁸*-(4-ethoxyphenyl)imidazo[1,2-*b*]pyridazine-6,8-diamine TFA salt (14 mg, 0.021 mmol), prepared by the method of example I(1) using 1-benzylpiperidin-3-amine in place of trans-1,4-diaminocyclohexane in step (*1d*), and MeOH (5 mL) in a 500 ml PARR bottle was added 10% Pd/C (20 mg) and 3 drops of glacial acetic acid. The PARR bottle was then charged with H₂ at 55 psi and allow to shake at room temperature for 5 hours. The reaction mixture was then filtered and the filtrate concentrated in vacuo. The residue was purified by preparative HPLC followed by neutralization with ion exchange resin to furnish 0.5 mg (7%) of the title compound ¹H NMR (500 MHz, MeOH) 8 ppm 7.57 (1 H, s), 7.31 (1 H, s), 7.24 (2 H, d, J=8.7 Hz), 6.97 (2 H, d, J=8.7Hz), 5.89 (1 H, s), 4.05 (2 H, q, J=6.9 Hz), 3.85 (1 H, m), 3.45 (1 H, m), 3.10 (1 H, m), 2.85 (1 H, m), 2.75 (1 H, m), 2.05 (1 H, m), 1.90 (1 H, m), 1.70 (1 H, m), 1.55 (1 H, m), 1.39 (3 H, t, J=6.9 Hz), LC/MS, *m*/*e* 353 (M+1). HPLC Rt, 2.04 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.

Compounds having the formula (Ia) were prepared according to procedures similar to Example V(1), where in R₁, R₂, R₃, X and Y have the values listed in Table 4, using the appropriate starting materials and substantially the same procedures as indicated.

**Table 4**

| **Exp** | **Name** | **R₁** | **R₂** | **R₃** | X | **Y** | **LC/MS m/z (M+1)** |
|---|---|---|---|---|---|---|---|
| **V(2)** | *N⁸*-[4-(ethyloxy)phenyl]-*N⁶*-pyrrolidin-3-ylimidazo[1,2-*b*]pyridazine-6,8-diamine | H | H | H | 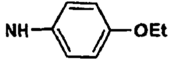 | 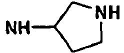 | 339 |
| **V(3)** | *N⁸*-[4-(ethyloxy)phenyl]-*N⁶*-piperidin-4-ylimidazo[1,2-*b*]pyridazine-6,8-diamine | H | H | H | 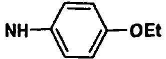 | 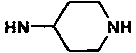 | 353 |
| **V(4)** | 6-(3-amino-1-piperidinyl)-*N*-(4-(ethyloxy)phenyl)imidazo[1,2-*b*]pyridazin-8-amine | H | H | H | 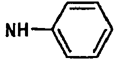 | 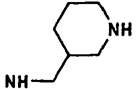 | 323 |
| **V(5)** | *N⁸*-(4-(ethyloxy)phenyl)-7-methyl-*N⁶*-3-piperidinylimidazo[1,2-*b*]pyridazine-6,8-diamine | H | H | Me | 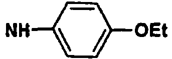 | 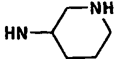 | 367 |
| **V(6)** | *N⁸*-phenyl-*N⁶*-3-piperidinylimidazo[1,2-*b*]pyridazine-6,8-diamine | **H** | **H** | **H** | 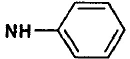 | 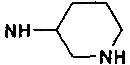 | 309 |

| | | | | | | | |
|---|---|---|---|---|---|---|---|
| *For substituents X and Y, substitution on the core (formula Ia) occurs at the available nitrogen atom | | | | | | | |

### EXAMPLE VI(1)

### 6-[(3S)-3-aminopyrrolidin-1-yl]-N-[4-(ethyloxy)phenyl]imidazo[1,2-b]pyridazin-8-amine

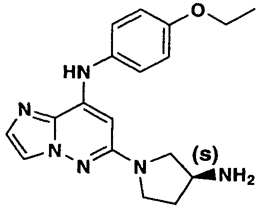

To 6-chloro-*N*-(4-ethoxyphenyl)imidazo[1,2-*b*]pyridazin-8-amine (26 mg, 0.090 mmol), prepared as described in example 1, step (1c) was added (*S*)-3-amino-1-*N*-boc-pyrrolidine (180 mg, 0.96 mmol). The mixture was microwaved at 225 °C for one hour. The melt was then cooled, water was added followed by extraction with dichloromethane. The organic layer was then concentrated in vacuo and purified by preparative HPLC to give 0.9 mg (2%) of the title compound as a TFA salt, (Note that during the reaction the Boc cleaves and the 1- nitrogen of the pyrrolidine adds). ¹H NMR (500 MHz, MeOH) δ ppm 7.99 (1 H, s), 7.88 (1 H, s), 7.31 (2 H, d, J=7.7 Hz), 7.03 (2 H, d, J=7.7Hz), 6.18 (1 H, s), 4.07 (2 H, q, J=6.9 Hz), 4.0 (1 H, m), 3.80 (1 H, m), 3.55 (3 H, m), 2.50 (1 H, m), 2.15 (1 H, m), 1.40 (3 H, t, J=6.9 Hz),. LC/MS, *m*/*e* 339 (M+1). HPLC Rt, 1.83 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1 % TFA). Gradient, start %B = 0, final % B = 100, gradient time 4 min, hold at 100% B 1 min, flow rate 4 mL/min.

### EXAMPLE IX(1)

### N⁶-(cis-4-aminocyclohexyl)-N⁸-[4-(ethyloxy)phenyl]-7-methylimidazo[1,2-b]pyridazine-6,8-diamine

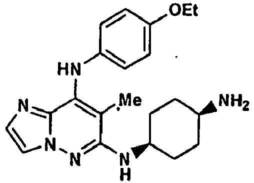

To 6-chloro-*N*-(4-ethoxyphenyl)-7-methylimidazo[1,2-*b*]pyridazin-8-amine (40 mg, 0.14 mmol) from Example III(1), step *1d* was added cis-1,4-diaminocyclohexane (250 mg, 2.19 mmol). The mixture was allowed to heat at 165 °C for 48 hrs. The reaction mixture then cooled, diluted with methanol and purified by preparative HPLC. The eluent was then concentrated in vacuo, diluted with methanol (2 mL), purified and neutrailized by passing through a 500 mg SCX (Cation exchange column). The eluent was concentrated to give 6.0 mg (11.3%) of the titled compound. ¹H NMR (500 MHz, MeOH) δ ppm 7.65 (1 H, s), 7.25 (1 H, s), 6.85 (4 H, m), 3.95 (2 H, d, J=7.2 Hz), 3.95 (1 H, m), 3.05 (1 H, m), 1.95 (2 H, m), 1.85 (3 H, s), 1.80 (4 H, m), 1.65 (2 H, m), 1.35 (3 H, t, J=7.2 Hz). LC/MS *m*/*e* 381 (M+1). HPLC, 1.91 min. Waters Sunfire C18 4.6 x 50. 0%-100%B. B: 90% MeOH, 10 % H₂O, 0.1% TFA. A: 10% MeOH, 90 % H₂O, 0.1% TFA, gradient time 4 min, hold at 100% B 1 min, flow rate 4 mL/min.

Compounds having the formula (Ia) were prepared according to procedures similar to Example IX(1), where in R₁, R₂, R₃, X and Y have the values listed in Table 5 using the appropriate starting materials and substantially the same procedures as indicated.

**Table 5**

| **Exp** | **Name** | **R₁** | **R₂** | **R₃** | **X** | **Y** | **LC/MS m/z (M+1)** |
|---|---|---|---|---|---|---|---|
| X(2) | *N⁶*-(*cis*-4-aminocyclohexyl)-*N⁸*-(5-methyl-2-pyridinyl)imidazo[1,2-*b*]pyridazine-6,8-diamine | **H** | **H** | **H** | 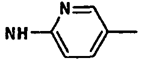 | 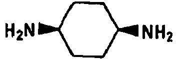 | 338 |
| X(3) | *N⁶*-(*cis*-4-aminocyclohexyl)-*N⁸*-phenylimidazo[1,2-*b*]pyridazine-6,8-diamine | **H** | **H** | **H** | 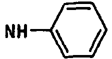 | 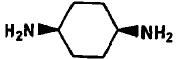 | 323 |
| IX(4) | *N⁶*-(*cis*-4-aminocyclohexyl)-7-methyl-*N³*-phenylimidazo[1,2-*b*]pyridazine-6,8-diamine | H | H | Me | 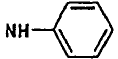 | 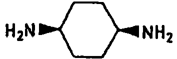 | 337 |
| IX(5) | *N⁶*-(*cis*-4-aminocyclohexyl)-*N⁸*-(4-(ethyloxy)phenyl)imidazo[1,2-*b*]pyridazine-6,8-diamine | H | H | H | 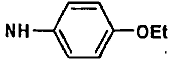 | 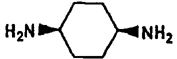 | 367 |
| IX(6) | *N⁶*-(cis-4-aminocyclohexyl)-*N⁸*-(3,4-dimethylphenyl)imidazo[1,2-*b*]pyridazine-6,8-diamine | H | H | H | 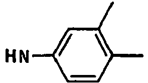 | 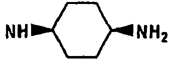 | 351 |
| IX(7) | *N⁶*-(*cis*-4-aminocyclohexyl)-*N⁸*-(5-phenyl-2-pyridinyl)imidazo[1,2-*b*]pyridazine-6,8-diamine | H | H | H | 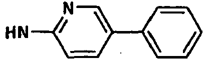 | 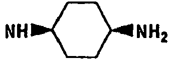 | 400 |
| IX(8) | 4-((6-((*cis*-4-aminocyclohexyl)amino)imidazo[ 1,2-*b*]pyridazin-8-yl)amino)-*N,N*-dimethylbenzenesulfonamide | H | H | H | 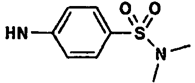 | 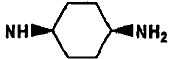 | 430 |
| IX(9) | -*N⁶*-(cis-4-aminocyclohexyl)-*N⁸*-(4-methylphenyl)imidazo[1,2-*b*]pyridazine-6,8-diamine | H | H | H | 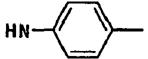 | 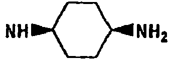 | 336 |
| IX(10) | *N⁶*-(*cis*-4-aminocyclohexyl)-*N⁸*-(4,6-dimethyl-2-pyridinyl)imidazo[1,2-*b*]pyridazine-6,8-diamine | H | H | H | 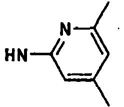 | 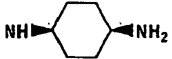 | 352 |
| IX(11) | *N⁶*-(*cis*-4-aminocyclohexyl)-*N⁸*-(4-(1*H*-pyrazol-1-yl)pheny)imidazo[1,2-*b*]pyridazine-6,8-diamine | H | H | H | 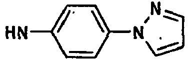 | 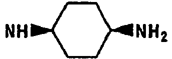 | 389 |
| IX(12) | *N⁶*-(*cis*-4-aminocyclohexyl)-*N⁸*-(4-(4-morpholinylcarbonyl)phenyl)imid azo[1,2-*b*]pyridazine-6,8-diamine | H | H | H | 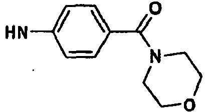 | 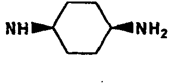 | 436 |
| IX(13) | *N⁶*-(*trans*-4-aminocyclohexyl)-7-methyl-*N⁸*-(2-methylphenyl)imidazo[1,2-*b*]pyridazine-6,8-diamine | H | H | Me | 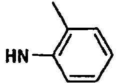 | 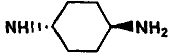 | 351 |
| IX(14) | *N⁶*-(*trans*-4-aminocyclohexyl)-*N⁸*-(2-fluorophenyl)-7-methylimidazo[1,2-*b*]pyridazine-6,8-diamine | H | H | Me | 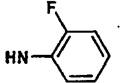 | 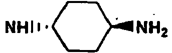 | 355 |
| IX(15) | *N⁶*-(*cis*-4-aminocyclohexyl)-7-methyl-*N⁸*-(2-methylphenyl)imidazo[1,2-*b*]pyridazine-6,8-diamine | H | H | Me | 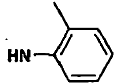 | 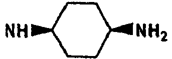 | 351 |
| IX(16) | *N⁶*-(*cis*-4-aminocyclohexyl)-*N⁸*-(2-fluorophenyl)-7-methylimidazo[1,2-*b*]pyridazine 6,8-diamine | H | H | Me | 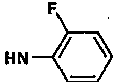 | 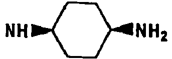 | 355 |

| | | | | | | | |
|---|---|---|---|---|---|---|---|
| *For substituents X and Y, substitution on the core (formula Ia) occurs at the available nitrogen atom | | | | | | | |

### EXAMPLE X(1)

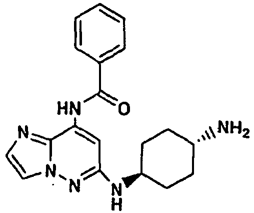

*(1a)* p-Methoxybenzyl amine (1.0 eq, 1.49 mmol) and triethylamine (330 mg, 3.27 mmol) were added to a mixture of 8-bromo-6-chloroimidazo[1,2-*b*]pyridazine hydrochloride (40 mg, 1.49 mmol) from Example 1, step *1b* in EtOH (15 mL). The mixture was heated to 90 °C and stirred for 24 hours. The solution was then concentrated in vacuo. The resulting residue was purified by reverse phase preparative HPLC to provide 6-chloro-N-(4-methoxybenzyl)imidazo[1,2-b]pyridazin-8-amine as a TFA salt LC/MS, *m*/*e* 288.97 (M+1). HPLC Rt, 2.84 min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.
(*1b*) trans-1,4-Diaminocyclohexane (1000 mg, 8.77 mmol) was added to 6-chloro-N-(4-methoxybenzyl)imidazo[1,2-b]pyridazin-8-amine (426 mg, 1.475 mmol) from *1a*. The mixture was heated to 160 °C and allowed to melt. After stirring at 160 °C for 7 days, the liquid mixture was cooled to room temperature. Water was added, followed by extraction with dichloromethane. The organic layer was concentrated *in vacuo.* The resulting residue was purified by reverse phase preparative HPLC to provide the N⁶-(trans-4-aminocyclohexyl)-N⁸-(4-methoxybenzyl)imidazo[1,2-b]pyridazine-6,8-diamine as a TFA salt in approximately 44% yield (0.315 g). LC/MS, *m*/*e* 367.27 (M+1). HPLC Rt, 1.81 min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1 % TFA). Gradient, start %B = 0, final % B = 100, gradient time 4 min, hold at 100% B 1 min, flow rate 4 mL/min.
*(1c)* To N⁶-(trans-4-aminocyclohexyl)-N⁸-(4-methoxybenzyl)imidazo[1,2-b]pyridazine-6,8-diaminefrom *1b* in dichloromethane (5 mL) was added TFA (2 mL). After stirring at RT for 2 hours, the reaction solution was concentrated *in vacuo* To provide crude N⁶-(trans-4-aminocyclohexyl)imidazo[1,2-b]pyridazine-6,8-diamine that was used without further purification. LC/MS, *m*/*e* 247.16 (M+1). HPLC Rt, 0.73 min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.
*(1d)* To N⁶-(trans-4-aminocyclohexyl)imidazo[1,2-b]pyridazine-6,8-diamine (70 mg, 0.284 mml, 1.0 eq.) from *1c* in THF (3 mL) was added triethylamine (31.6 mg, 0.313 mmol, 1.1 eq.) and boc anhydride (68.2 mg, 0.313 mmol, 1.1 eq). The reaction solution was stirred at RT for 2 hours. The solution was then concentrated *in vacuo* to give crude tert-butyl (trans)-4-(8-aminoimidazo[1,2-b]pyridazin-6-ylamino)cyclohexylcarbamate which was used as is in the next reaction. LC/MS, *m*/*e* 347.23 (M+1). HPLC Rt, 2.50 min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.
*(1e)* To 60% NaH (2.9 mg, 0.072 mmol) in THF (2 ml) was added tert-butyl (trans)-4-(8-aminoimidazo[1,2-b]pyridazin-6-ylamino)cyclohexylcarbamate (25 mg, 0.072 mmol) from *1d*. After stirring at RT for 1 hour, benzoyl chloride (20.3mg, 0.144 mg, 2.0 eq.) was added and the reaction solution was heated at 60°C for 3 days. The reaction was quenched with a few drops of water and methanol. The solution was then concentrated *in vacuo.* The resulting residue was purified by reverse phase preparative HPLC to provide tert-butyl (trans)-4-(8-benzamidoimidazo[1,2-b]pyridazin-6-ylamino)cyclohexylcarbamate as a TFA salt in approximately 10% yield (9.1 mg). LC/MS, *m*/*e* 451.26 (M+1). HPLC Rt, 2.99 min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.
(*1f*) To tert-butyl (trans)-4-(8-benzamidoimidazo[1,2-b]pyridazin-6-ylamino)cyclohexylcarbamate from *1e* in dichloromethane (2 mL) was added TFA (1 mL). After stirring at RT for 2 hours, the reaction solution was concentrated *in vacuo.* The residue was purified by SCX column (300 mg, eluting with 2M ammonia in methanol) to give the titled compound as a TFA salt in approximately 50% yield. ¹H NMR (500 MHz, MeOH) δ ppm 8.07 (2 H, d, J=7.7 Hz), 7.69 (2 H, s), 7.65 (1 H, t), 7.55 (2 H, t), 7.36 (1 H, s), 3.65 (1 H, m), 2.72 (1 H, m), 2.17 (2 H, m),1.95 (2 H, m),1.31 (4 H, t). LC/MS, *m*/*e* 351.21 (M+1). HPLC Rt, 1.70 min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.

### EXAMPLE XI(1)

### 1-(6-((trans-4-aminocyclohexyl)amino)imidazo[1,2-b]pyridazin-8-yl)-3-phenylurea

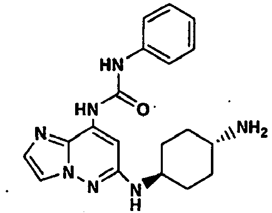

(*1a*) To 60% NaH (2.9 mg, 0.072 mmol) in THF (2 ml) was added tert-butyl (trans)-4-(8-benzamidoimidazo[1,2-b]pyridazin-6-ylamino)cyclohexylcarbamate (25 mg, 0.072 mmol, prepared in Example X step *1d*). After stirring at RT for 1 hour, phenyl isocyanate (17 mg, 0.144 mmol, 2.0 eq.) was added and the reaction solution was heated at 60 °C for 3 days. The reaction was quenched with a few drops of water and methanol. The solution was then concentrated *in vacuo.* The resulting residue was purified by reverse phase preparative HPLC to provide tert-butyl (trans)-4-(8-(3-phenylureido)imidazo[1,2-b]pyridazin-6-ylamino)cyclohexylcarbamate as a TFA salt in approximately less than 10% yield (6.5 mg). LC/MS, *m*/*e* 466.29 (M+1). HPLC Rt, 3.39 min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.
*(1b)* To tert-butyl (trans)-4-(8-(3-phenylureido)imidazo[1,2-b]pyridazin-6-ylamino)cyclohexylcarbamate (6.3mg, 0.135 mmol) from *1a* in dichloromethane (2 mL) was added TFA (1 mL). After stirring at RT for 2 hours, the reaction solution was concentrated *in vacuo.* The residue was purified by SCX column (300 mg, eluting with 2M ammonia in methanol) to give the title compound as a TFA 3.2mgs (64%). ¹H NMR (500 MHz, MeOH) δ ppm 7.64 (1 H, s), 7.47 (2 H, m), 7.31 (2 H, m), 7.06 (2 H, m), 6.71 (2 H, m), 3.63 (1 H, m), 2.71 (1 H, m), 2.15 (2 H, m),1.91 (2 H, m),1.28 -1.33 (4 H, t). LC/MS, *m*/*e* 366.27 (M+1). HPLC Rt, 2.18min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.

### EXAMPLE XII(1)

### N,N'-bis(4-trans-aminocyclohezyl)imidazo[1,2-b]pyridazine-6,8-diamine

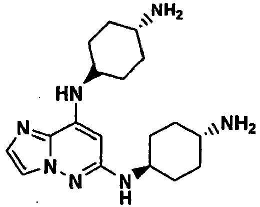

To 8-bromo-6-chloroimidazo[1,2-*b*]pyridazine (50 mg, 0.19 mmol) from Example 1, step *1b* was added trans-1,4-diaminocyclohexane (430 mg, 3.8 mmol). The mixture heated at 180 °C for 48h. The reaction vessel was cooled to rt. and diluted with water (10 mL) and extracted with DCM (3X10 mL). The organic extracts were combined, concentrated in vacuo and purified using preparative HPLC to provide the title compound (40 mg, 30%) as a TFA salt. ¹H NMR (400 MHz, MeOH) δ ppm 7.90 (1H, d, J=2 Hz), 7.81 (1H, d, J=2 Hz), 6.06 (1 H, s), 3.75 (1H, m), 3.50 (1H, m), 3.20 (2H, m), 2.28 (4H, M), 2.16 (4H, m), 1.70-1.48 (6H.m), 1.42 (2H, m). ). LC/MS, *m*/*e* 344 (M+1). HPLC Rt, 1.01 min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.

### EXAMPLE XIII(1)

### N⁶-(trans-4-aminocyclohexyl)-N⁸-(4-ethyloxyphenyl)-7-phenylimidazo[1,2-b]pyridazine-6,8-diamine

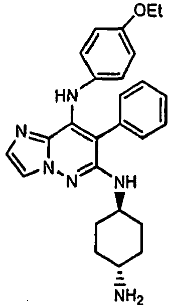

*(1a)* 3,6-dichloro-4-phenylpyridazine (J. Med. Chem. 2005, 48, 7089; 5.93 g, 26.3 mmol) was divided into 6 microwave tubes, and concentrated NH₄OH (7 mL) was added to each. After sealing, each was heated at 140 °C for 1 h. The microwave tubes was uncapped, and the precipitate was filtered and washed with cold water. The precipitates from all reactions were combined, and Et₂O (150 mL) was added to it. After stirring overnight, the remaining solid was filtered, rinsed with Et₂O and dried to give 6-chloro-5-phenylpyridazin-3-amine (3.06 g, 56 %)
*(1b)* To a suspension of 6-chloro-5-phenylpyridazin-3-amine (1.01 g, 4.9 mmol) from *1a* in methanol (25 mL) under nitrogen was added NaHCO₃ (1.09 g, 13.0 mmol). At 0 °C, bromine (0.55 M in methanol, 10 mL, 5.5 mmol) was added over 5 min. After 1 h, the cold bath was removed, and the reaction mixture was stirred to room temperature for 6 h. After concentrating *in vacuo,* the residue was taken up in CH₂Cl₂ and saturated aqueous Na₂S₂O₅, and the layers were separated. The organic layer was washed with saturated aqueous NaHCO₃ and brine, dried over Na₂SO₄, filtered, and concentrated *in vacuo* to give crude 4-bromo-6-chloro-5-phenylpyridazin-3-amine (1.28 g).
*(1c)* Chloroacetaldehyde (2.0 mL, 31.5 mmol) was added to a solution of crude 4-bromo-6-chloro-5-phenylpyridazin-3-amine (0.193 g, 0.676 mmol) from *1b* in EtOH (5.0 mL). The mixture was heated in a sealed tube at 118 °C for 5 h. After cooling to room temperature, the reaction mixture was concentrated *in vacuo* and suspended in acetone/Et₂O (1:1, 3 mL), filtered, washed with Et₂O to give 8-bromo-6-chloro-7-phenylimidazo[1,2-b]pyridazine HCl salt (0.147 g, > 94 % purity).
*(1d)* To a solution of 8-bromo-6-chloro-7-phenylimidazo[1,2-b]pyridazine HCl salt (0.0431 g, 0.125 mmol) from *1c* and 4-ethoxyaniline (0.31 M in THF, 0.40 mL) under nitrogen at 0 °C was added KOtBu (1 N in THF, 0.32 mL, 0.32 mmol). After 1 min, the cold bath was removed, and the reaction mixture was stirred to room temperature for 1 h. After concentrating *in vacuo,* the residue was taken up in CH₂Cl₂ and water, and the layers were separated. The aqueous layer was extracted with CH₂Cl₂ (2x). The organic layers were combined, dried over Na₂SO₄, filtered, and concentrated *in vacuo*. Trituration with hexanes provided 6-chloro-N-(4-ethoxyphenyl)-7-phenylimidazo[1,2-b]pyridazin-8-amine (0.0334 g).
*(1e)* 6-chloro-N-(4-ethoxyphenyl)-7-phenylimidazo[1,2-b]pyridazin-8-amine (0.0201 g, 0.058 mmol) from *1d* and (trans)-cyclohexane-1,4-diamine (0.1702 g, 1.49 mmol) were heated at 165 °C for 6 d. After cooling to room temperature, the mixture was taken up in CH₂Cl₂ and water, and the layers were separated. The aqueous layer was extracted with CH₂Cl₂ (2x). The organic layers were combined, dried over Na₂SO₄, filtered, and concentrated *in vacuo.* The residue was purified using preparative HPLC. The appropriate fraction was collected, and NaHCO₃ (solid) was added to it. It was concentrated *in vacuo* not to dryness, and extracted with CH₂Cl₂ (2x). The organic layers were combined, dried over Na₂SO₄, filtered, and concentrated *in vacuo* to give the above titled compound (1.5 mg, 4.0 % yield). LC/MS, m/e 443.40 (M+1). HPLC RT, 2.29 min. YMC ODSC18 column (4.6 x 50 mm): 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.

Compounds having the formula (Ia) were prepared according to procedures similar to Example XIII(1), where in R₁, R₂, R₃, X and Y have the values listed in Table 6 using the appropriate starting materials and substantially the same procedures as indicated.

**Table 6**

| Exp | Name | R₁ | R₂ | R₃ | **X** | Y | LC/MS m/z (M+1) |
|---|---|---|---|---|---|---|---|
| XIII(2) | *N⁶*-(*trans*-4-aminocyclohexyl)-*N³*-(phenyl)-7-phenylimidazo[1,2-*b*]pyridizine-6,8-diamine | H | H | Ph | 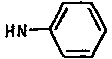 | 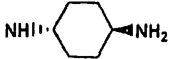 | 399 |
| XIII(3) | *N⁶*-(cis-4-aminocyclohexyl)-*N⁸*-(phenyl)-7-phenylimidazo[1,2-*b*]pyridazine-6,8-diamine | H | H | Ph | 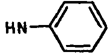 | 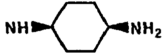 | 399 |
| XII(4) | *N⁶*-(*cis*-4-aminocyclohexyl)-*N⁸*-(4-ethoxyphenyl)-7-phenylimidazo[1,2-*b*]pyridazine-6,8-diamine | H | H | Ph | 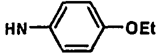 | 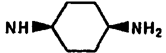 | 443 |

| | | | | | | | |
|---|---|---|---|---|---|---|---|
| *For substituents X and Y, substitution on the core (formula Ia) occurs at me available nitrogen atom | | | | | | | |

### EXAMPLE XIV(1)

### 4-((6-((trans-4-aminocyclohexyl)amino)imidazo[1,2-b]pyridazin-8-yl)amino)-N-(2-(4-pyridinyl)ethyl)benzamide

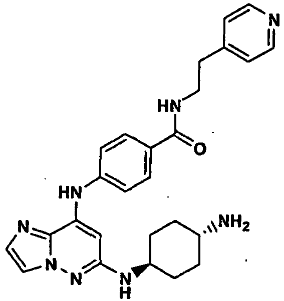

*(1a)* In a 250 ml round bottom flask, under a nitrogen atmosphere, was added 8-bromo-6-chloroimidazo[1,2-b]pyridazine (5.0 g, 18.6 mmol) from Example I(1), step *1b* tert-butyl 4-aminobenzoate (3.95 g, 20.5 mmol), and DMF (30 ml). The solution was cooled to 0 °C and 1.0M potassium *tert*-butoxide in THF (46 ml) was added drop-wise via syringe over 30 minutes. The reaction was allowed to stir at room temperature for 30 minutes and then warmed to 50 °C for 2 hrs and the concentrated in vacuo to remove THF. The resulting solution was taken up in ethyl acetate and washed with H₂O (3 x 300 ml) and then brine (1 x 50 ml). The organic layers were combined and dried over Na₂SO₄ and filtered. Following solvent evaporation, 6.0 g of crude product was obtained. Further purification was done via triteration with 3:1 diethyl ether/heptane to give 2.2 g of tert-butyl 4-(6-chloroimidazo[1,2-b]pyridazin-8-ylamino)benzoate as a brown solid after filtration.
*(1b)* In a 50 ml round bottom flask was added tert-butyl 4-(6-chloroimidazo[1,2-b]pyridazin-8-ylamino)benzoate (1.07 g, 3.1 mmol) from *1a* and 4M HCl in 1,4 Dioxane (8.0 ml, 31.0 mmol). The reaction was stirred at room temperature for 18 hrs and then concentrated in vacuo. The resulting 4-(6-chloroimidazo[1,2-b]pyridazin-8-ylamino)benzoic acid HCl salt (1.05 g) was used crude.
(*1c*) In a 50 ml round bottom flask added 4-(6-chloroimidazo[1,2-*b*]pyridazin-8-ylamino)benzoic acid HCl salt (0.53 g, 1.6 mmol) from *1b*, dichloromethane (10 ml) and DMF (20 ul). To this solution, neat oxalyl chloride (0.71 ml 8.2 mmol) was added dropwise. The solution was allowed to stir for 1 hr then concentrated in vacuo. This gave 0.5 g of 4-(6-chloroimidio[1,2-*b*]pyridazin-8-ylamino)benzoyl chloride as a yellowish solid.
*(1d)* To a one dram concave vial was added 4-(6-chloroimidazo[1,2-b]pyridazin-8-ylamino)benzoyl chloride (0.024g, 0.070 mmol) from *1c*, dichloromethane (0.7ml, 0.1M), 2-(pyridine-4-yl)ethanamine (0.017 ml, 0.13 mmol) and diisopropylethylamine. (0.012 ml, 0.18 mmol) The reaction was capped and allowed to stir at room temperature for 2 hours. The solvent was removed in vacuo and trans-1,4-cyclohexyldiamine was added. The reaction was sealed and allowed to stir at 165 °C for 18 hrs. Upon cooling, the sample was dissolved in a methanol (25%)/water (75%) mixture with 4 drops of trifluoroacetic acid. The solution was purified by HPLC (5-60% methanol gradient), which provided the TFA salt of the title compound as a brown solid 0.0.128g (21%). ¹H NMR (400 MHz, MeOH) δ ppm 8.75 (2 H, d, 6.6 Hz), 8.04 - 8.06 (2 H, dd, 6.5 Hz), 8.01 (1 H, d, 2 Hz), 7.88 - 7.90 (3 H, m), 7.43 (2 H, d, J=8.6 Hz), 6.74 (1 H, s), 3.81 -3.83 (3 H, m), 3.30 - 3.34 (2 H; m), 3.10 - 3.20 (1 H, m), 2.30 (2 H, m), 2.10 (2 H, m), 1.55 -1.58 (2 H, m), 1.35 - 1.45 (2 H, m,). LC/MS, *m*/*e* 471 (M+1). HPLC Rt, 1.39 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1 % TFA). Gradient, start %B = 0, final % B = 100, gradient time 4 min, hold at 100% B 1 min, flow rate 4 mL/min.

Compounds having the formula (Ia) were prepared according to procedures similar to Example XIV(1), where in R₁, R₂, R₃, X and Y have the values listed in Table 7 using the appropriate starting materials and substantially the same procedures as indicated.

**Table 7**

| Exp | Name | R₁ | R₂ | R₃ | **X** | Y | LC/MS m/z (M+1) |
|---|---|---|---|---|---|---|---|
| XIV(2) | 4-((6-((*trans*-4-aminocyclohexyl)amino)im idazo[1,2-*b*]pyridazin-8-yl)amino)-*N*-(2-furanylmethyl)benzamide | H | H | H | 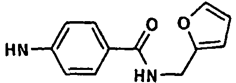 | 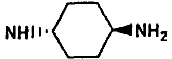 | 446 |
| XIV(3) | 4-((6-((*trans*-4-aminocyclohexyl)amino)im idazo[1,2-*b*]pyridazin-8-yl)amino)-*N*-(1*H*-imidazol-4-ylmethyl)benzamide | H | H | H | 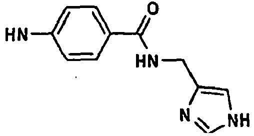 | 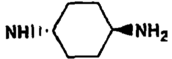 | 446 |
| XIV(4) | 4-((6-((*trans*-4-aminocyclohexyl)amino)im idazo[1,2-*b*]pyridazin-8-yl)amino)-*N*-(3-pyridinylmethyl)benzamide | H | H | H | 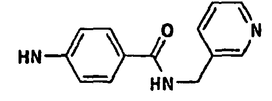 | 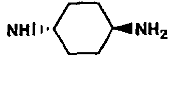 | 457 |
| XIV(5) | *N⁶*-(*trans*-4-aminocyclohexyl)-*N⁸*-(4-((4-phenyl-1-piperidinyl)carbonyl)pheny l)imidazo[1,2-*b*]pyridazine-6,8-diamine | H | H | H | 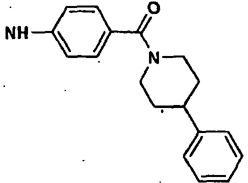 | 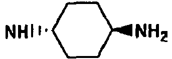 | 510 |
| XIV(6) | *N⁶*-(*trans*-4-aminocyclohexyl)-*N⁸*-(4(1-pyrrolidinylcarbonyl)pheny l)imidazo[1,2-*b*]pyridazine-6,8-diamine | H | H | H | 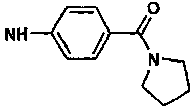 | 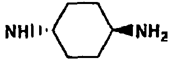 | 420 |
| XIV(7) | *N⁶-*(*trans*-4-aminocyclohexyl)-*N⁸*-(4-(1-piperidinylcarbonyl)phenyl )imidazo[1,2-*b*]pyridazine-6,8-diamine | H | H | H | 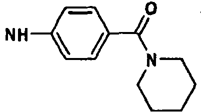 | 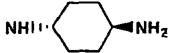 | 434 |
| XIV(8) | 4-((6-(*trans*-4-aminocyclohexyl)amino)im idazo[1,2-*b*]pyridazin-8-yl)amino)-*N*-(phenylmethyl)benzamide | H | H | H | 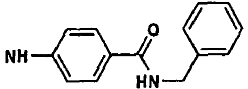 | 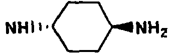 | 456 |
| XIV(9) | 4-((6-((*trans*-4-aminocyclohexyl)amino)im idazo[1,2-*b*]pyridazin-8-yl)amino)-*N*-(3-(methyloxy)phenyl)benzam ide | H | H | H | 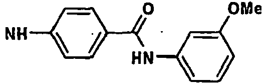 | 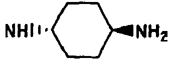 | 472 |
| XIV(10) | *N⁶*-(*trans*-4-aminocyclohexyl)-*N⁸*-(4-((3-phenyl-1-pyrrolidinyl)carbonyl)phen yl)imidazo[1,2-*b*]pyridazine-6,8-diamine | H | H | H | 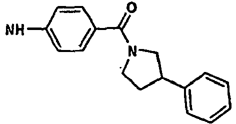 | 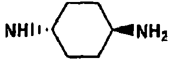 | 496 |
| XVI(11) | 3-((6-((*trans*-4-aminocyclohexyl)amino)im idazo[1,2-*b*]pyridazin-8-yl)amino)-*N*-phenylbenzamide | H | H | H | 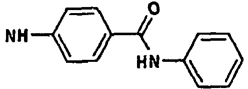 | 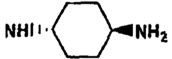 | 442 |
| XIV(12) | 4-((6-((*trans*-4-aminocyclohexyl)lamino)im idazo[1,2-*b*]pyridazin-8-yl)amino)-*N*-(2-hydroxyethyl)benzamide | H | H | H | 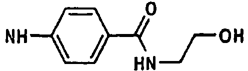 | 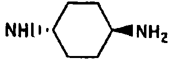 | 410 |
| XIV(13) | 4-((6-((*trans*-4-aminocyclohexyl)amino)im idazo[1,2-*b*]pyridazin-8-yl)amino)-*N*-(3-(2-oxo-1-pyrrolidinyl)propyl)benzam ide | H | H | H | 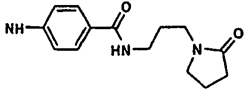 | 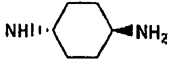 | 491 |
| XIV(14) | *N*⁶-(*trans-*4-aminocyclohexyl)-*N*⁸-(4-(4-morpholinylcarbonyl)phen yl)imidazo[1,2-*b*]pyridazine-6,8-diamine | H | H | H | 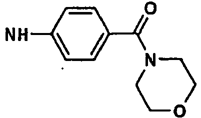 | 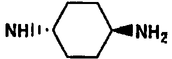 | 436 |
| XIV(15) | 4-((6-((*trans*-4-aminocyclohexyl)amino)im idazo[1,2-*b*]pyridazin-8-yl)amino)-*N*-methyl-*N*-(2-(2-pyridinyl)ethyl)benzamide | H | H | H | 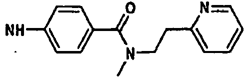 | 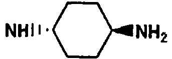 | 485 |
| XIV(16) | 4-((6-((*trans*-4-aminocyclohexyl)amino)im idazo[1,2-*b*]pyridazin-8-yl)amino)-*N,N*-diethylbenzamide | H | H | H | 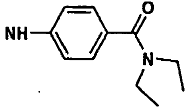 | 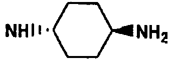 | 423 |
| XIV(17) | 4-((6-(*trans*-4-aminocyclohexyl)amino)im idazo[1,2-*b*]pyridazin-8-yl)amino)-*N*-cyclopropylbenzamide | H | H | H | 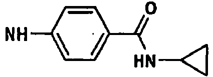 | 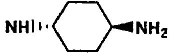 | 406 |
| XIV(18) | 4-((6-((*trans*-4-aminocyclohexyl)amino)im idazo[1,2-*b*]pyridazin-8-yl)amino)-*N*-(cyclohexylmethyl)benzami de | H | H | H | 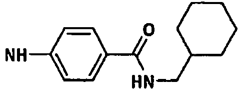 | 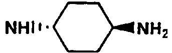 | 462 |
| XIV(19) | 4-((6-((*trans*-4-aminocyclohexyl)amino)im idazo[1-2-*b*]pyridazin-8 -yl)amino)-*N*-3-pyridinylbenzamide | H | H | H | 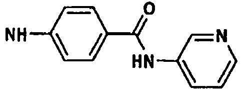 | 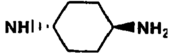 | 443 |
| XIV(20) | 4-((6-((*trans*-4-aminocyclohexyl)amino)im idazo[1,2-*b*]pyridazin-8-yl)amino)-*N*-(1-methyl-1*H*-Pyrazol-5-yl)benzamide | H | H | H | 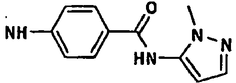 | 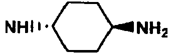 | 446 |

| | | | | | | | |
|---|---|---|---|---|---|---|---|
| *For substituents X and Y, substitution on the core (formula Ia) occurs at the available nitrogen atom | | | | | | | |

### EXAMPLE XV(1)

### 4-((6-((trans-4-aminocyclohexyl)amino)imidazo[1,2-b]pyridazin-8-yl)amino)-N-(1-(4-fluorophenyl)-2-oxo-1,2-dihydro-3-pyridinyl)benzamide

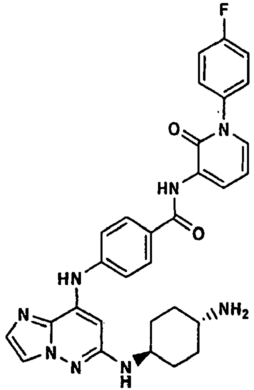

*(1a)* To a 2 dram vial was added 4-(6-chloroimidazo[1,2-*b*]pyridazin-8-ylamino)benzoic acid (0.050 g, 0.17 mmol, prepared as described in Example XIV, step 1b), 3-amino-1-(4-fluorophenyl)pyridin-2(1H)-one (0.053 g, 0.26) from *1a-1* and *1a-2* described below, EDCI (0.050 g, 0.26 mmol), HOBt (0.035 g, 0.26 mmol), TEA (0.07 ml, 0.51 mmol), DMF (0.8 ml) and CH₃CN (0.8 ml), the reaction was allowed to stir at 50 °C for 12 hrs. Upon cooling, the solvent was removed in vacuo and diluted with methanol (2 ml). The solution was purified by HPLC (20-100% methanol gradient), which yielded 0.0.12 g of 4-(6-chloroimidazo[1,2-b]pyridazin-8-ylamino)-N-(1-(4-fluorophenyl)-2-oxo-1,2-dihydropyridin-3-yl)benzamide.
*(1a-1)* To a solution of 2-hydroxy-3-nitropyridine (Aldrich, 3.0 mmol, 420 mg) in 1,4-dioxane (20 mL), were added 4-fluorophenyl boronic acid (Combi-block, 6.0 mmol, 840 mg), copper(II) acetate (Aldrich, 4.5 mmol, 815 mg) and pyridine (2 mL). The reaction was heated at 80°C for 20 h. After cooling to room temperature, 30 mL of cold water was added. The solid formed was collected by filtration, washed with ammonium hydroxide and water, and dried under vacuum to give 1-(4-fluorophenyl)-3-nitropyridin-2(1*H*)-one (610 mg, 87% yield) as a solid.
*(1a-2)* To a solution of 1-(4-fluorophenyl)-3-nitropyridin-2(1*H*)-one (610 mg, 2.6 mmol) from *1a-1* in THF (50 mL) and MeOH (50 mL), were added ammonium chloride (695 mg, 13.0 mmol, EMD) and Zn dust (850 mg, 13.0 mmol, Aldrich). The reaction mixture was stirred at room temperature for 3 h, diluted with 200 mL of EtOAc and filtered through a pad of Celite^{®}. The filtrate was concentrated *in vacuo* to give 3-Arnino-1-(4-fluorophenyl)pyridin-2(1*H*)-one (530 mg, 100% yield) as a brown solid.
*(1b)* To 4-(6-chloroimidazo[1,2-b]pyridazin-8-ylamino)-N-(1-(4-fluorophenyl)-2-oxo-1,2-dihydropyridin-3-yl)benzamide (0.011 g, 0.023 mmol) from *1a* was added trans-1,4-diaminocyclohexane (0.5 g, 57.0 mmol). The mixture was allowed to melt at 160 °C for 12 hours. The melt was then cooled, diluted with water and extracted with dichloromethane. The organic layer was then concentrated in vacuo to give 0.020 g of crude product. Purification was done via preparative HPLC providing 0.006 g of the title compound as aTFA salt. 1H NMR (400 MHz, *MeOD*) δ ppm 8.56 (1 H, dd, *J*=7.38, 1.78 Hz), 7.92 - 8.10 (3 H, m), 7.88 (1 H, d, *J*=2.03 Hz), 7.42 - 7.61 (3 H, m), 7.39 (1 H, dd, *J*=12, 1.53 Hz), 7.30 (2 H, t, *J*=8.65 Hz), 6.77 (1 H, s), 6.54 (1 H, t, *J*=7.12 Hz), 3.97 (1 H, s), 3.66 - 3.84 (1 H, m), 3.03 - 3.24 (1 H, m), 2.26 (2 H,, m), 2.00 - 2.20 (2 H, m), 1.44 - 1.68 (2 H, m), 1.22 - 1.46 (2H, m). LC/MS, *m*/*e* 533 (M+1). HPLC Rt, 2.14 min. YMC ODSC18 column (4.6 x 50 mm). 0%-100%B. solvent B: (90% MeOH, 10 % H₂O, 0.1% TFA). Solvent A: (10% MeOH, 90% H₂O, 0.1 % TFA). Gradient, start %B = 0, final % B = 100, gradient time 4 min, hold at 100% B 1 min, flow rate 4 mL/min.

Compounds having the formula (Ia) were prepared according to procedures similar to Example XV(1), where in R₁, R₂, R₃, X and Y have the values listed in Table 8 using the appropriate starting materials and substantially the same procedures as indicated.

**Table 8**

| **Exp** | **Name** | **R₁** | **R₂** | **R₃** | **X** | **Y** | **LC/MS m/z (M+1)** |
|---|---|---|---|---|---|---|---|
| XV(2) | 4-((6-(((*trans*-4-aminocyclohexyl)amino)imidazo[1,2 -*b*]pyridazin-yl)amino)-*N*-phenylbenzamide | **H** | **H** | **H** | 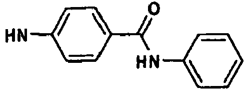 | 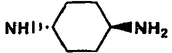 | 442 |
| XV(3) | 4-((6-((*trans*-4-aminocyclohexyl)amino)imidam[l,2 -*b*]pyridazin-8-yl)amino)-*N*-cyclohexylbenzamide | **H** | **H** | **H** | 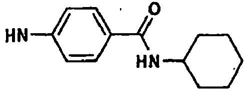 | 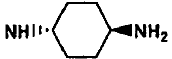 | 448 |
| *XV(4)* | 4-((6-((*trans*-4-aminocyclohexyl)amino)imidazo[1,2 -*b*]pyridazin-8-yl)amino)-*N*-(4-pyridinylmethyl)benzamide | **H** | **H** | **H** | 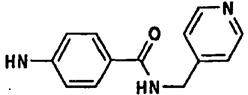 | 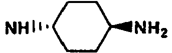 | 457 |

| | | | | | | | |
|---|---|---|---|---|---|---|---|
| *For substituents X and Y, substitution on the core (formula Ia) occurs at the available nitrogen atom | | | | | | | |

### EXAMPLE XVI(1)

### 1-(4-((6-((trans-4-aminocyclohexyl)amino)imidazo[1,2-b]pyridazin-8-yl)amino)phenyl)-3-phenylurea

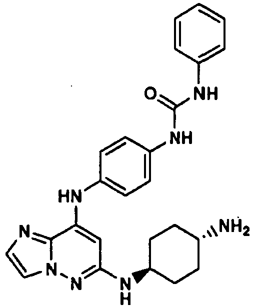

*(1a) p*-Aminoaniline (0.48 g, 4.4 mmol) and triethylamine (1.3 ml, 9.2 mmol) were added to 8-bromo-6-chloroimidazo[1,2-b]pyridazine HCl (1.0 g, 4.2 mmol) from Example I(1), step *1b* in EtOH (20 mL). The mixture was heated to 90 °C and stirred for 1 hr. The solution was then concentrated in vacuo to give crude N1-(6-chloroimidazo-[1,2-b]pyridazin-8-yl)benzene-1,4-diamine. Cold ethanol was then added and the crude solid rinsed 3x. Following filtration, collected 0.45 g of the desired material.
*(1b)* To N1-(6-chloroimidazo-(1,2-b]pyridazin-8-yl)benzene-1,4-diamine (0.2 g, 0.77 mmol) from *1a* was added trans-1,4-diaminocyclohexane (1.7 g, 15.0 mmol). The mixture was allowed to melt at 160 °C for 3 days. The melt was then cooled, diluted with water and extracted with dichloromethane. The organic layer was then concentrated in vacuo to give 0.4 g of crude N6-(trans-4-aminocyclohexyl)-N8-(4-aminophenyl)imidazo[1,2-b]pyridazine-6,8-diamine.
*(1c)* In a 200 ml round bottom flask charged with N⁶-(trans)-4-aminocyclohexyl)-N⁸-(4-aminophenyl)imidazo[1,2-b]pyridazine-6,8-diamine (0.39 g, 1.1 mmol) from *1b* and dichloromethane (10 ml) was added di-tert-butyl dicarbonate (0.25 g, 1.1 mmol) in dichloromethane (2 ml). The reaction was stirred at room temperature for 30 min, then concentrated and purified on silica gel (ethyl acetate/heptane, 20 min gradient: 15-100% ethyl acetate). tert-butyl (trans)-4-(8-(4-aminophenylamino)imidazo[1,2-b]pyridazin-6-ylamino)cyclohexylcarbamate (0.050 g) was obtained as a white solid.
*(1d)* In a 2 dram vial was added tert-butyl (trans)-4-(8-(4-aminophenylamino)imidazo[1,2-b]pyridazin-6-ylamino)cyclohexylcarbamate (0.04 g, 0.09 mmol) from *1c*, dichloroethane (1.0 ml) and phenylisocyanate (0.05 ml, 0.5 mmol). The reaction was allowed to stir for 2 hrs and then concentrated to dryness. Diethyl ether was added and the resulting suspension was stirred for 30 min. Upon filtration, tert-butyl (trans)-4-(8-(4-(3-phenylureido)phenylamino)imidazo[1,2-b]pyridazin-6-ylamino)cyclohexylcarbamate (0.035 g) was collected and used with no further purification.
*(1e)* In a 2 dram vial was added tert-butyl (trans)-4-(8-(4-(3-phenylureido)phenylamino)imidazo[1,2-b]pyridazin-6-ylamino)cyclohexylcarbamate (0.035 g, 0.06 mmol) from *1d* and 4.0M HCl in 1,4 Dioxane (2.0 ml). The reaction was stirred for 1 hr at 25°C, concentrated and then triterated with diethyl ether. The titled compound was filtered off as an HCl salt (0.018 g). 1H NMR (400 MHz, *DMSO-D6)* δ ppm 9.36 (1 H, s), 9.00 (1 H, s), 8.88 (1 H, s), 8.67 (1 H, s), 8.60 (1 H, s), 8.10 (1 H, s), 7.87 (2 H, d, *J*=5.09 Hz), 7.54 (1 H, d, *J*=9.16 Hz), 7.44 (2 H, t, *J*=7.63 Hz), 7.34 (1 H, s), 7.20 - 7.31 (2H, m), 6.85 - 7.04 (2 H, m), 6.27 (1 H, s), 3.38 (1 H, s), 3.02 (1 H, s), 2.04 (2 H, m), 1.95 (2 H,m), 1.31 - 1.53 (2 H, m), 1.11-1.29 (2 H, m). LC/MS, *m*/*e* 457 (M+1). HPLC Rt, 1.93 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B =100, gradient time 4 min, hold at 100% B 1 min, flow rate 4 mL/min.

Compounds having the formula (Ia) were prepared according to procedures similar to Example XVI(1), where in R₁, R₂, R₃, X and Y have the values listed in Table 9, using the appropriate starting materials and substantially the same procedures as indicated.

**Table 9**

| **Exp** | **Name** | **R₁** | **R₂** | **R₃** | **X** | **Y** | **LC/MS m/z (M+1)** |
|---|---|---|---|---|---|---|---|
| XVI(2) | 1-(4-((6-((*cis*-4-aminocyclohexyl)amino)-7-methylimidazo[1,2-*b*]pyridazin-8-y))amino)phenyl)-3-phenylurea | **H** | **H** | **Me** | 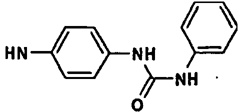 | 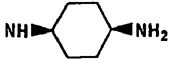 | 471 |
| XVI(3) | 1-(4-((6-((*trans*-4-aminocyclohexyl)amino)-7-methylimidazo[1,2-*b*]pyridazin-8-yl)amino)phenyl)-3-phenylurea | **H** | **H** | **Me** | 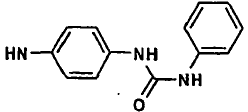 | 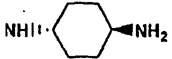 | 471 |

| | | | | | | | |
|---|---|---|---|---|---|---|---|
| *For substituents X and Y, substitution on the core (formula Ia) occurs at the available nitrogen atom | | | | | | | |

### EXAMPLE XVII(1)

### N-(4-((6-((trans-4-aminocyclohexyl)amino)imidazo[1,2-b]pyridazin-8-yl)amino)-phenyl)-1-(4-fluorophenyl)-2-oxo-1,2-dihydro-3-pyridinecarboxamide

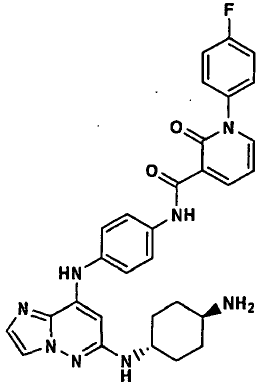

*(1a)* In a 2 dram reaction vial was added 8-bromo-6-chloroimidazo[1,2-b]pyridazine hydrochloric acid salt (0.5 g, 1.9 mmol) from Example I(1), step *1b*, 4-nitroaniline (0.27 g, 1.9 mmol), 1.0M potassium *tert-*butoxide in THF(7.6 ml, 7.6 mmol) and DMF (1.4 ml). Under N₂, the reaction was stirred for 16 hr at 50°C and the concentrated in vacuo. The resulting material was taken up in ethyl acetate and washed with H₂O (2 x 50 ml) and then brine (1x 20 ml). the organic layers were combined and dried over Na₂SO₄ and filtered. Following solvent evaporation, 0.42 g of crude 6-chloro-N-(4-nitrophenyl)imidazo[1,2-b]pyridazin-8-amine was obtained.
*(1b)* In a 2 dram reaction vial was added crude 6-chloro-N-(4-nitrophenyl)imidazo[1,2-b]pyridazin-8-amine (0.42 g, 1.4 mmol) from *1a* and trans-1,4-diaminocyclohexane (1.0 g, 8.0 mmol). The mixture was allowed to melt at 160 °C for 8 hrs. The melt was then cooled, diluted with water and extracted with dichloromethane. The organic layer was concentrated to provide crude N6-((trans)-4-aminocyclohexyl)-N8-(4-nitrophenyl)imidazo[1,2-b]pyridazine-6,8-diamine which was used without further purification.
*(1c)* In a 100 ml round bottom flask charged with N6-((trans)-4-aminocyclohexyl)-N8-(4-nitrophenyl)imidazo[1,2-b]pyridazine-6,8-diamine (0.31 g, 0.85 mmol) from from *1b* triethylamine (0.13 ml, 0.93 mmol) add dichloromethane (10 ml) was added di-tert-butyl dicarbonate (0.3 ml, 1.3 mmol). The reaction was stirred at 25°C for 0.5 hrs, then concentrated and purified on silica gel (ethyl acetate/heptane, 20 min gradiant :25-100% ethyl acetate) to give tert-butyl (trans)-4-(8-(4-nitrophenylamino)imidazo[1,2-b]pyridazin-6-ylamino)cyclohexylcarbamate (0.090 g) as a white solid.
*(1d)* In a 50ml round bottom flask was added tert-butyl (trans)-4-(8-(4-nitrophenylamino)imidazo[1,2-b]pyridazin-6-ylamino)cyclohexylcarbamate (0.090 g, 0.19 mmol) from *1c*, chloroform (2.0 ml), methanol (2.0 ml), ammonium chloride (0.12 g, 0.19 mmol) and zinc dust (013 g, 0.19 mmol). The reaction was allowed to stir for 30 min at room temperature. The reaction was then filtered through a plug of celite and rinsed with dichloromethane to afford 0.1 g of crude tert-butyl (trans)-4-(8-(4-aminophenylamino)imidazo[1,2-b]pyridazin-6-ylamino)cyclohexylcarbamate.
*(1e)* To a 2 dram vial was added crude tert-butyl (trans)-4-(8-(4-aminophenylamino)imidazo[1,2-b]pyridazin-6-ylamino)cyclohexylcarbamate (0.064 g, 0.15 mmol) from *1d* 1-(4-fluorophenyl)-2-oxo-1,2-dihydropyridine-3-carboxylic acid (0.051 g, 0.22 mmol) prepared as described in steps *1e-1,1e-2* below, EDCI (0.043 g, 0.22 mmol), HOBt (0.030 g, 0.22 mmol), TEA (0.06 ml, 0.45 mmol),and CH₃CN (1 ml). The reaction was allowed to stir around 25 °C for 16 hrs. The solvent was then removed in vacuo and the sample was diluted with MeOH (4 ml). The solution was purified by HPLC (20-100% methanol gradient), which yielded 0.025 g of tert-butyl (trans)-4-(8-(4-(5-(4-fluorophenyl)-6-oxocyclohexa-1,3-dienecarboxamido)phenylamino)imidazo[ 1,2-b]pyridazin-6-ylamino)cyclohexylcarbamate as a TFA salt
*(1e-1)* In a 200 ml round bottom flask added methyl 2-oxo-2H-pyran-3-carboxylate (5.45 g, 35.0 mmol), 4-floroaniline (3.35 ml, 35.0 mmol) and DMF (63 ml). The reaction was stirred for 3 hrs at room temperature. EDCI (9.4 g, 50.0 mmol) and DMAP (0.3 g, 2.0 mmol) were then added and the reaction was allowed to stir at room temperature overnight. The reaction was quenched with 50 ml of 1N HCl and ethyl acetate was added. The layers were separated and the resulting aqueous phase was extracted 2x with ethyl acetate. The combined organics were washed 2x with 10% lithium chloride solution and dried over Na₂SO₄. Following filtration of the solids and concentration in vacuo, the product was obtained as a yellow solid.
*(1e-2)* In a 200 ml round bottom flask added methyl 5-(4-fluorophenyl)-6-oxocyclohexa-1,3-dienecarboxylate (1.1 g, 4.3 mmol), THF (8 ml), MeOH (8 ml) and 1N NaOH solution (13 ml). The reaction was allowed to stir overnight at room temperature. Upon concentration of the volatiles, the basic solution was extracted 2x with diethyl ether. The aqueous phase was then acidified to pH 3 with 1N HCl amd subsequently extracted 2xwith dichloromethane. The organics were collected and washed with saturated NaCl and the layers separated. The organic solution was dried over Na₂SO₄, filtered and concentrated in vacuo. This resulted in 0.7 g of product, 5-(4-fluorophenyl)-6-oxocyclohexa-1,3-dienecarboxylic acid.
*(1f)* In a 50 ml round bottom flask added tert-butyl (trans)-4-(8-(4-(5-(4-fluorophenyl)-6-oxocyclohexa-1,3-dienecarboxamido)phenylamino)imidazo[1,2-b]pyridazin-6-ylamino)cyclohexylcarbamate (0.025 g, 0.04 mmol) from *1e* and 20% trifloroacetic acid in methylene chloride (4 ml). The reaction was allowed to stir at room temperature for 5 min. The reaction was then concentrated in vacuo and diluted with methanol and purified via HPLC (20-100% methanol gradient), which provided titled compound as a TFA salt (0.007 g). 1H NMR (400 MHz, *MeOD)* δ ppm 12.11 (1 H,.s), 3.68 (1 H, dd, *J*=7.38, 2.29 Hz), 7.87 - 8.13 (2 H, m), 7.71 - 7.88 (3 H, m), 7.41 - 7.63 (2 H, m), 7.33 (4 H, t, *J*=8.65 Hz), 6.65 - 6.82 (1 H, m), 6.43 (1 H, s), 3.54 - 3.88 (1 H, m), 2.96 - 3.17 (1 H, m), 2.23 (2 H, m), 2.08 (2 H, m), 1.441.68 (2 H, m), 1.17-1.43(2H,m). LC/MS, *m*/*e* 553 (M+1). HPLC Rt, 2.14 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.

Compounds having the formula (Ia) were prepared according to procedures similar to Example XVII(1), where in R₁, R₂, R₃, X and Y have the values listed in Table 10, using the appropriate starting materials and substantially the same procedures as indicated.

**Table 10**

| **Exp** | **Name** | **R₁** | **R₂** | **R₃** | **X** | **Y** | **LC/MS m/z M+1)** |
|---|---|---|---|---|---|---|---|
| XVII(2) | N-(4-((6-((*trans*-4-aminocyclohexyl)amino)i midazo[1,2-*b*]pyridazin-8-yl)amino)-2-ethylphenyl)-1-(4-fluorophenyl)-2-oxo-1,2-dihydro-3-pyridinecarboxamide | **H** | **H** | **H** | 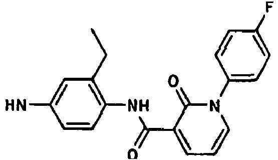 | 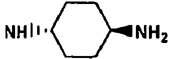 | 581 |

| | | | | | | | |
|---|---|---|---|---|---|---|---|
| *For substituent X and Y, substitution on the core (formula la) occurs at the available nitrogen atom | | | | | | | |

### EXAMPLE XVIII(1)

### N-(4-((6-((trans-4-aminocyclohexyl)amino)imidazo[1,2-b]pyridazin-8-yl)amino)phenyl)benzamide

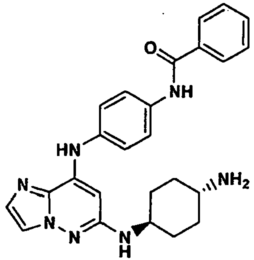

In a 2 dram reaction vial was added N-1-(6-chloroimidazo[1,2-b]pyridazin-8-yl)benzene-1,4-diamine (0.050 g, 0.19 mmol, prepared in Example XVI, step 1a), dichloromethane (1.0 ml) and triethylamine (0.040 ml, 0.29 mmol). To this solution benzoyl chloride (0.025 ml, 0.21 mmol) was added drop-wise. The reaction was stirred for 30 min at 25 °C and then concentrated in-vacuo. To this was added trans-1,4-diaminocyclohexane (0.5g, 23.0 mmol). The mixture was allowed to melt at 160 °C for 24 hrs. The melt was then cooled, diluted with water and extracted with dichloromethane. The organic layer was then concentrated in vacuo to give 0.036 g of crude product. The crude material was then purified by preparative HPLC to provide 0.018 g of the title compound as aTFA salt. 1H NMR (400 MHz, *MeOD*) δ ppm 7.94 (3 H, m), 7.76 - 7.88 (3 H, m), 7.45 - 7.67 (3 H, m), 7.36 (2 H, d, 8.8 Hz), 6.45 (1 H, s), 3.59 - 3.87 (1 H, m), 3.01 - 3.21 (1 H, m), 2.24 (2 H, m), 2.09 (2 H, m), 1.46 - 1.65 (2 H, m), 1.24 - 1.42 (2 H, m). LC/MS, *m*/*e* 442 (M+1). HPLC Rt, 1.80 min. YMC ODSC18 column (4.6 x 50 mm). 0%-100%B. Solvent B: (90% MeOH, 10% H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.

### EXAMPLE XIX(1)

### N-(4-((6-((trans-4-aminocyclohexyl)amino)imidazo[1,2-b]pyridazin-8-yl)amino)phenyl)acetamide

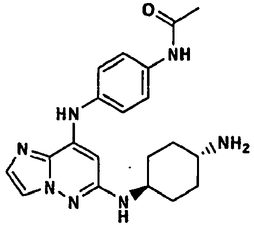

In a 2 dram reaction vial was added *N*-1-(6-chloroimidazo[1,2-b]pyridazin-8-yl)benzene-1,4-diamine (0.050 g, 0.19 mmol, prepared as described in Example XVI, step 1a), dichloromethane (1.0 ml) and triethylamine (0.040 ml, 0.29 mmol). To this solution acetic anhydride (0.022 ml, 0.23 mmol) was added drop-wise. The reaction was stirred for 30 min at 25°C and then concentrated in-vacuo. To this was added trans-1,4-diaminocyclohexane (0.5g, 4.3 mmol). The mixture was allowed to melt at 160°C for 24 hrs. The melt was then cooled, diluted with water and extracted with dichloromethane. The organic layer was then concentrated in vacuo to give 0.030 g of crude product The crude material was then purified by preparative HPLC. This gave 0.008 g of the title compound as aTFA salt. 1H NMR (400 MHz, *MeOD)* δ ppm 7.95 (1 H, d, *J*=2.03 Hz), 7.86 (1 H, d, *J*=2.03 Hz), 7.65 (2 H, d, *J*=8.65 Hz), 7.29 (2 H, d, *J*=9.16 Hz), 6.40 (1 H, s), 3.60 - 3.81 (1 H, m), 3.02 - 3.24 (1 H, m), 2.17 - 2.30 (2 H, m), 2.13 (3 H, s), 2.04 - 2.12 (2 H, m), 1.43 - 1.64 (2 H, m), 1.24 - 1.42 (2 H, m). LC/MS, *m*/*e* 380 (M+1). HPLC Rt, 1.62 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B =100, gradient time 4 min, hold at 100% B 1 min, flow rate 4 mL/min.

### EXAMPLE XX(1)

### 3-((6-((trans-4-aminocyclohexyl)amino)-7-methylimidazo[ 1,2-b]pyridazin-8-yl)amino)phenol

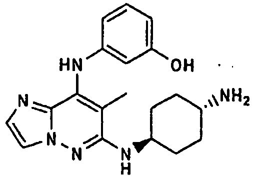

*(1a)* In a microwave vial was added 8-bromo-6-chloro-7-methylimidazo[1,2-b]pyridazine (0.070 g, 0.24 mmol, prepared as described in Example III, step 1c), NMP (1.4 ml), K₂CO₃ (0.17 g, 1.2 mmol) and 3-(benzyloxy)aniline (0.050 g, 0.24 mmol). The reaction was heated via microwave at 225 °C for 15 min. Upon cooling, the solution was diluted with MeOH (2 ml) and purified by preparative HPLC. This gave 0.011 g of 3-(6-chloro-7-methylimidazo[1,2-b]pyridazin-8-ylamino)phenol. *m*/*z* = 275. (Note: the benzyl ether was cleaved during the reaction)
*(1b)* In a 2 dram reaction vial added 3-(6-chloro-7-methylimidazo[1,2-b]pyridazin-8-ylamino)phenol (0.011 g, 0.028 mmol) from *1**a* and trans-1,4-diaminocyclohexane ( 1.0 g, 8.0 mmol). The mixture was allowed to melt at 160 °C for 24 hrs. The melt was then cooled, diluted with water and MeOH, and then purified by preparative HPLC to give 0.005 g of the title compound as aTFA salt. 1H NMR (400 MHz, *MeOD*) δ ppm 8.01 (1 H, s), 7.73 (1 H, s), 7.11 (1 H, t, *J*=8.14 Hz), 6.48 (1H, d, *J*=7.63 Hz), 6.28 (1H, s), 3.88 - 4.10 (1 H, m), 3.05 - 3.27 (1 H, m), 2.24 - 2.37 (2 H, m, *J*=8.65 Hz), 2.11 - 2.23 (5 H, m), 1.45 - 1.70 (4 H, m). LC/MS, *m*/*e* 353 (M+1). HPLC Rt, 1.20 min. YMC ODSC18 column (4.6 x 50 mm). 0%-100%B. Solvent B: (90% MeOH, 10 % H₂O, 0.1% TFA). Solvent A: (10% MeOH, 90 % H₂O,0.1% TFA). Gradient, start %B = 0, final % B =100, gradient time 4 min, hold at 100% B 1 min, flow rate 4 mL/min.

### EXAMPLE XM(1)

### N-(4-((6-chloroimidazo[1,2-b]pyridazin-8-yl)amino)phenyl)-1-(4-fluorophenyl)-2-oxo-1,2-dihydro-3-pyridinecarboxamide

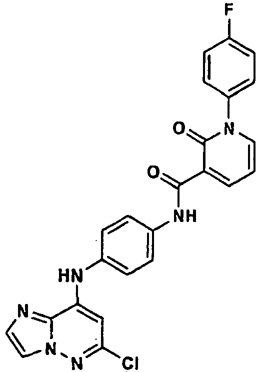

To a 2 dram vial was added N1-(6-chloroimidazo[1,2-b]pyridazin-8-yl)benzene-1,4-diamine (0.078 g, 0.3 mmol, prepared as described in Example XV1, step 1a), 1-(4-fluorophenyl)-2-oxo-1,2-dihydropyridine-3-carboxylic acid (0.10 g, 0.45 mmol, prepared as described in Example XVII, step 1e2), EDCI (0.086 g, 0.45 mmol), HOBt (0.061 g, 0.45 mmol), TEA (0.12 ml, 0.9 mmol), DMF (0.8 ml) and CH₃CN (1.5 ml). The reaction was allowed to stir around 50 °C for 12 hrs. Upon cooling, the solvent was removed in vacuo and diluted with methanol (2 ml). The solution was purified by HPLC (20-100% methanol gradient), which yielded 0.014 g of the title compound as a TFA salt. 1H NMR (400 MHz, *CHLOROFORM-D*) δ ppm 11.88 (1 H, s), 8.70 (1 H, dd, *J*=7.12,2.03 Hz), 7.71 - 7.91 (3 H, m), 7.67 (1 H, s), 7.50 - 7.63 (1 H, m), 7.10 - 7.43 (5 H, m), 6.64 (1 H, s), 6.56 (1 H, t, *J*=7.12 Hz). LC/MS, *m*/*e* 475 (M+1). HPLC Rt, 2.82 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1 % TFA). Gradient, start %B = 0, final % B = 100, gradient time 4 min, hold at 100% B1 min, flow rate 4 mL/min.

### EXAMPLE XXII(1)

### 1-(4-fluorophenyl)-N-(4-(imidazo[1,2-b]pyridazin-8-ylamino)phenyl)-2-oxo-3-piperidinecarboxamide

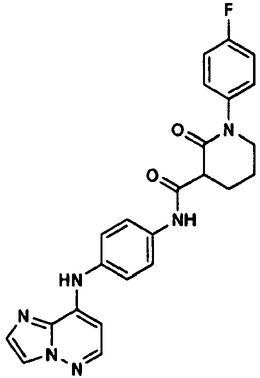

To a suspension of N-(4-(6-chloroimidazo[1,2-b]pyridazin-8-ylamino)phenyl)-1-(4-fluorophenyl)-2-oxo-1,2-dihydro-3-pyridinecarboxamide TFA salt (0.013g, 0.027 mmol) From Example XXI and EtOH (4 mL) in a 500 ml PARR bottle was added 10% Pd/C (20 mg) and 2 drops of triethylamine. The PARR bottle was then charged with H2 at 55 psi and allow to shake at room temperature for 12 hours. The reaction mixture was then filtered and the filtrate concentrated in vacuo. The residue was purified by preparative HPLC to furnish 0.008 g of the title compound as a TFA salt. LC/MS, *m*/*e* 445 (M+1). HPLC Rt, 1.81 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.

### EXAMPLE XXIII(1)

### 1-(4-fluorophenyl)-N-(4-(imidazo[ 1,2-b]pyridazin-8-ylamino)phenyl)-2-oxo-1,2-dihydro-3-pyridinecarboxamide

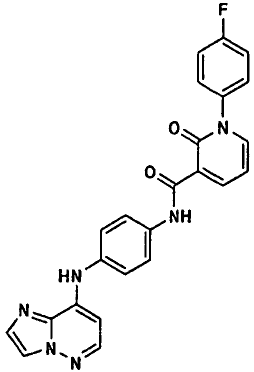

*(1a)* In a 100 ml round bottom flask charged with N1-(6-chloroimidazo[1,2-b]pyridazin-8-yl)benzene-1,4-diamine (0.5 g, 1.9 mmol, prepared as described in Example XVI, step 1a) and tetrahydrofuran (10 ml) was added di-tert-butyl dicarbonate (0.44 g, 1.9 mmol). The reaction was stirred at 50 °C for 5 hrs, then concentrated and purified on silica gel (ethyl acetate/heptane, 20 min gradient: 5-50% ethyl acetate) to give tert-butyl 4-(6-chloroimidazo[1,2-b]pyridazin-8-ylamino)phenylcarbamate (0.050 g) as a white solid.
*(1b)* To a mixture of tert-butyl 4-(6-chloroimidazo[1,2-b]pyridazin-8-ylamino)phenylcarbamate (0.1 g, 0.28 mmol) from *1a* and EtOH (4 mL) in a 500 ml PARR bottle was added 10% Pd/C (0.02 g) and 2 drops of triethyl amine. The PARR bottle was then charged with H₂ at 55 psi and allow to shake at room temperature for 12 hours. The reaction mixture was then filtered and the filtrate concentrated in vacuo to give 0.085 g of crude tert-butyl 4-(imidazo[1,2-b]pyridazin-8-ylamino)phenylcarbamate.
*(1c)* In a 2 dram vial was added tert-butyl 4-(imidazo[1,2-b]pyridazin-8-ylamino)phenylcarbamate. (0.085 g, 0.26 mmol) from *1b* and 4.0M HCl in 1,4 dioxane (5.0 ml). The reaction was stirred for 2 hr at 25 °C, concentrated. N¹-(imidazo[1,2-b]pyridazin-8-yl)benzene-1,4-diamine resulted as a di-HCl salt (0.085 g). The product was used with no further purification.
*(1d)* To a 2 dram vial was added N¹-(imidazo[1,2-b]pyridazin-8-yl)benzene-1,4-diamine di-HCl salt (0.080 g, 0.3 mmol) from *1c* 1-(4-ffuorophenyl)-2-oxo-1,2-dihydropyridine-3-carboxylic acid (0.13 g, 0.57 mmol), EDCI (0.11 g, 0.57 mmol), HOBt (0.077 g, 0.57 mmol), TEA (0.16 ml, 1.1 mmol),and CH₃CN (4 ml). The reaction was allowed to stir around 25 °C for 12 hrs. The solvent was then removed in vacuo and the sample was diluted with MeOH (4 ml). The solution was purified by HPLC (20-100% methanol gradient), which yielded 0.015 g of the title compound as a TFA salt. 1H NMR (400 MHz, *DMSO-D6)* δ ppm 11.96 (1 H, s), 9.47 (1 H, s), 8.59 (1 H, dd, *J*=7.38,2.29 Hz), 7.99 - 8.27 (3 H, m), 7.67 - 7.85 (3 H, m), 7.55 - 7.67 (2 H, m, *J*=9.16,5.09 Hz), 7.33 - 7.49 (3 H, m), 6.66 - 6.82 (1 H, m), 6.55 (1 H, d, *J*=6.10 Hz) LC/MS, *m*/*e* 441 (M+1). HPLC Rt, 2.64 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.

### EXAMPLE XXIV(1)

### 6-((trans-4-aminocyclohexyl)amino)-8-((4-(ethyloxy)phenyl)amino)imidazo[1,2-b]pyridazine-3-carbonitrile

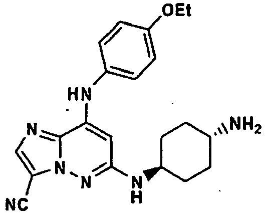

*(1a)* In a 200 ml round bottom flask was added 8-bromo-6-chloroimidazo[1,2-b]pyridazine hydrochloride (3.0 g, 11.2 mmol) from Example I(1), step *1b*, chloroform (55 ml) and NBS (3.0 g, 16.8 mmol). The reaction was heated at 80 °C for 1 hr, cooled to room temperature and the volatiles removed under reduced pressure. Ethyl acetate was added and the mixture was washed with Na₂CO₃ (2 x 100 ml), H₂O (2 x 100 ml) and then brine (1 x 25 ml). The organic layers were combined and dried over Na₂SO₄ and then concentrated. This gave 1.8 g of crude 3,8-dibromo-6-chloroimidazo[1,2-b]pyridazine.
*(1b) p*-Phenetidine (0.38 ml, 2.9 mmol) and triethylamine (0.8 ml, 5.8 mmol) were added to a mixture of 3,8-dibromo-6-chloroimidazo[1,2-b]pyridazine (0.83 g, 2.67 mmol) from *1a* in EtOH (10 mL). The mixture was heated to 80°C and stirred for 16 hrs. The solution was then concentrated in vacuo to give crude 3-bromo-6-chloro-N-(4-ethoxyphenyl)imidazo[ 1,2-b]pyridazin-8-amine.
*(1c)* In a 5 ml microwave vial was added 3-bromo-6-chloro-N-(4-ethoxyphenyl)imidazo[1,2-b]pyridazin-8-amine (0.3 g, 0.8 mmol) from *1b*, Pd(PPh₃)₄ (0.18 g, 0.16 mmol), Zn(CN)₂ (0.47 g, 4.0 mmol) and DMF (3 ml)). The reaction was heated via microwave for 25 min at 200 °C. Upon cooling, the reaction mixture was diluted with ethyl acetate and filtered through a plug of celite. The solvent was removed in vacuo and the resulting material was purified by silica gel chromatography (ethyl acetate/heptane, 25 min gradient :5-50% ethyl acetate) to give 0.11 g of 6-chloro-8-(4-ethoxyphenylamino)imidazo[1,2-b]pyridazine-3-carbonitrile.
*(1d)* In a 2 dram reaction vial was added 6-chloro-8-(4-ethoxyphenylamino)imidazo[1,2-b]pyridazine-3-carbonitrile (0.055 g, 0.017 mmol) from *1c* and trans-1,4-diaminocyclohexane (1.0 g, 8.0 mmol). The mixture was allowed to melt at 160 °C for 1.5 hrs. The melt was then cooled, diluted with water and extracted with dichloromethane. The organic layer was concentrated and then diluted with MeOH, and then purified by preparative HPLC to give 0.039 g of the title compound as a TFA salt. 1H NMR (500 MHz, *Solvent)* δ ppm 7.91 (1 H, s), 7.23 (2 H, d, *J*=8.80 Hz), 6.96 (2 H, d, *J*=8.80 Hz), 5.96 (1 H, s), 4.04 (4 H, q, *J*=6.78 Hz), 3.61-3.80(1H,m),2.99-319(1H,m),2.16-2.35(2H,m, *J*=11.55 Hz), 2.01-2.16 (2 H, m, *J*=12.65 Hz), 1.47 - 1.65 (2 H, m), 1.39 (3 H, t, *J*=7.1 Hz), 1.24 - 1.36 (2H, m). LC/MS, *m*/*e* 392 (M+1). HPLC Rt, 2.69 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B =100, gradient time 4 min, hold at 100% B 1 min, flow rate 4 mL/min.

Compounds having the formula (Ia) were prepared according to procedures similar to Example XXIV(1), where in R₁, R₂, R₃, X and Y have the values listed in Table 11, using the appropriate starting materials and substantially the same procedures as indicated.

**Table 11**

| **Exp** | **Name** | **R₁** | **R₂** | **R₃** | X | **Y** | **LC/MS m/z (M+1** |
|---|---|---|---|---|---|---|---|
| XXIV(2) | 6-((*trans*-4-aminocylohexyl)amino-8-(phenylamino)imidazo[1,2-*b*]pyridazine-3-carbonitrile | **H** | **CN** | **H** | 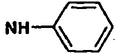 | 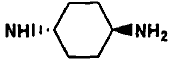 | 348 |

| | | | | | | | |
|---|---|---|---|---|---|---|---|
| *For substitute X and Y, substitution on the core (formula la) occurs at me available nitrogen atom | | | | | | | |

### EXAMPLE XXV(1)

### 6-((4-trans-aminocyclohexyl)amino)-7-methyl-8-(phenylamino)imidazo[1,2-b]pyridazine-3-carbonitrile

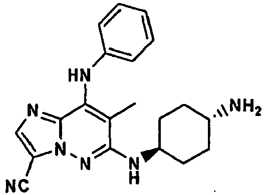

*(1a)* In a 200 ml round bottom flask was added 8-bromo-6-chloro-7-methylimidazo[1,2-b]pyridazine hydrochloride (0.52 g, 1.8 mmol) from Example III, step 1c, chloroform (10 ml) and NBS (0.5 g, 2:7 mmol). The reaction was heated at 80°C for 1 hr, cooled to room temperature and the volatiles removed under reduced pressure. Ethyl acetate was added and the mixture was washed with Na₂CO₃ (2 x 100 ml), H₂O (2 x 100 ml) and then brine (1 x 25 ml). The organic layers were combined and dried over Na₂SO₄ and then concentrated. This gave 1.8 g of crude product that was then dry loaded onto silica gel and purified via column chromatography using 30% ethyl acetate as the mobile phase. This resulted in 0.14 g of pure 3,8-dibromo-6-chloro-7-methylimidazo[1,2-b]pyridazine.
*(1b)* In a 2 dram reaction vial was added 3,8-dibromo-6-chloro-7-methylimidazo[1,2-b]pyridazine (0.14 g, 0.43 mmol) from *1a*, 1.0M potassium tert-butoxide in THF (1.0 ml) and THF (1.4 ml). Under N₂, the reaction was stirred for 1 hr at room temperature and the concentrated in vacuo. The resulting material was taken up in ethyl acetate and washed with H₂O (2 x 50 ml) and then brine (1 x 20 ml). the organic layers were combined and dried over Na₂SO₄ and filtered. Following solvent evaporation, 0.14 g of crude product was obtained. Further purification was done via silica gel chromatography (ethyl acetate/heptane, 25 min gradient :5-50% ethyl acetate) to give 0.053 g of 3-bromo-6-chloro-7-methyl-N-phenylimidazo[1,2-b]pyridazin-8-amine.
(*1c*) In a 5 ml microwave vial was added 3-bromo-6-chloro-7-methyl-N-phenylimidazo[1,2-b]pyridazin-8-amine (0.053 g, 0.16 mmol) from *1a*, Pd(PPh₃)₄ (0.036 g, 0.032 mmol), Zn(CN)₂ (0.088 g, 0.78 mmol) and DMF (3 ml). The reaction was heated via microwave for 30 min at 180°C. Upon cooling, the reaction mixture was diluted with ethyl acetate and filtered through a plug of celite. The solvent was removed in vacuo and the resulting material was purified by preparative HPLC to give 0.013 g of 3-bromo-6-chloro-7-methyl-N-phenylimidazo[1,2-b]pyridazin-8-amine. *(1d)* In a 2 dram reaction vial was added 3-bromo-6-chloro-7-methyl-N-phenylimidazo[1,2-b]pyridazin-8-amine (0.013 g, 0.045 mmol) from *1b* and trans-1,4-diaminocyclohexane (1.0 g, 8.0 mmol). The mixture was allowed to melt at 160 °C for 45 min. The melt was then cooled, diluted with water and extracted with dichloromethane. The organic layer was concentrated and then diluted with MeOH and then purified by preparative HPLC to give the title compound as a TFA salt. 1H NMR (400 MHz, *MeOD)* δ ppm 7.99 (1 H, s), 7.19 - 7.33 (2 H, m), 6.97 (1 H, t, *J*=7.38 Hz), 6.87 (2 H, d, *J*=7.63 Hz), 3.88 - 4.02 (1 H, m), 3.07 - 3.25 (1 H, m), 2.25 - 2.40 (2 H, m, *J*=12.21 Hz), 2.07 - 2.22 (2 H, m, *J*=12.21 Hz), 1.94 (3 H, s), 1.41 - 1.72 (4 H, m). LC/MS, *mle* 362 (M+1). HPLC Rt, 2.58 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B =100, gradient time 4 min, hold at 100% B 1 min, flow rate 4 mL/min.

Compounds having the formula (Ia) were prepared according to procedures similar to Example XXV(1), where in R₁, R₂, R₃, X and Y have the values listed in Table 12 using the appropriate starting materials and substantially the same procedures as indicated.

**Table 12**

| **Exp** | **Name** | **R₁** | **R₂** | **R₃** | X | **Y** | **LC/MS m/z (M+1)** |
|---|---|---|---|---|---|---|---|
| XXV(2) | 6-((trans)-4-aminocyclohexylamino)-7-ethyl-8-(phenylamino)imidazo[1,2-b]pyridazine-3-carbonitrile | H | CN | Et | 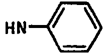 | 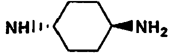 | 376 |
| XXV(3) | 6-((*trans*-4-aminocyclohexyl)amino)-8-anilino-7-isopropylimidazo[1,2-*b*]pyridazine-3-cabonitrile | H | CN | i-Pr | 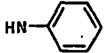 | 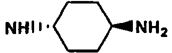 | 390 |

| | | | | | | | |
|---|---|---|---|---|---|---|---|
| *For substituents X and Y, substitution on the core (formula Ia) occurs at the available nitrogen atom | | | | | | | |

### EXAMPLE XXVI(1)

### N⁶-(trans-4-aminocyclohexyl)-N⁸-(4-(ethyloxy)phenyl)-3-fluoroimidazo[1,2-b]pyridazine-6,8-diamine

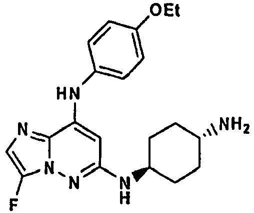

(1a) In a 20 ml reaction vial was added 8-bromo-6-chloroimidazo[1,2-b]pyridazine hydrochloride salt (0.33 g, 1.4 mmol) from Example I(1), step *1b* CH₃CN (7.0 ml) and selectflor (0.5 g, 1.4 mmol). The reaction was stirred at 50 C for 6 hrs, then concentrated to dryness. Purification was done by silica gel chromatography (ethyl acetate/heptane, 25 min gradiant :5-50% ethyl acetate) to give 0.085 g of 8-bromo-6-chloro-3-fluoroimidazo[1,2-b]pyridazine..
(*1b*) *p*-Phenetidine (0.044 ml, 0.34 mmol) and triethylamine (0.1 ml, 0.75 mmol) were added to a mixture of 8-bromo-6-chloro-3-fluoroimidazo[1,2-b]pyridazine (0.085 g, 0.34 mmol) from *1a* in EtOH (1.7 mL). The mixture was heated to 80 °C and stirred for 16 hors. The solution was then concentrated in vacuo to give crude 6-chloro-N-(4-ethoxyphenyl)-3-fluoroimidazo[1,2-b]pyridazin-8-amine.
(*1c*) In a 2 dram reaction vial was added 6-chloro-N-(4-ethoxyphenyl)-3-fluoroimidazo[1,2-b]pyridazin-8-amine (0.080 g, 0.26 mmol) from *1b* and trans-1,4-diaminocyclohexane (1.0 g, 8.0 mmol). The mixture was allowed to melt at 160 °C for 5 hrs. The melt was then cooled, diluted with water and extracted with dichloromethane. The organic layer was concentrated and then diluted with MeOH, and then purified by preparative HPLC to give the titled compound as a TFA salt. 1H NMR (500 MHz, *Solvent*) δ ppm 7.32 (1 H, d, *J*=6.05 Hz), 7.24 (2 H, d, *J*=8.80 Hz), 6.98 (2 H, d, *J*=8.80 Hz), 5.99 (1 H, s), 4.05 (2 H, q, *J*=7.15 Hz), 3.66 - 3.79 (1 H, m), 3.04 - 3.18 (1 H, m), 2.16 - 2.28 (2 H, m, *J*=11.55 Hz), 1.99 - 2.12 (2 H, m, *J*=12.10 Hz), 1.46 - 1.62 (2 H, m), 1.39 (3 H, t, *J*=6.87 Hz), 1.25 - 1.36 (2 H, m). LC/MS, *m*/*e* 385 (M+1). HPLC Rt, 2.25 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.

### EXAMPLE XXVII(1)

### N⁶-(trans-4-aminocyclohexyl)-N⁸-(4-(ethyloxy)phenyl)-3-methylimidazo[1,2-b]pyridazine-6,8-diamine

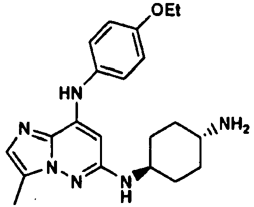

*(1a)* To a mixture of crude 4-bromo-6-chloro-pyridazin-3-amine (0.5 g, 2.3 mmol) from Example I(1), step *1a*, 2-chloro-1,1-dimethoxypropane (1.6 ml), EtOH (5 ml) and H₂O (2 ml) was added 4 drops of 35% HBr in acetic acid. The reaction was stirred at 100 °C for 16 hrs. The reaction was then concentrated to dryness and triterated from diethyl ether. Upon filtration a mixture of 8-bromo-6-chloro-3-methylimidazo[1,2-b]pyridazine and 6,8-dichloro-3-methylimidazo[1,2-b]pyridazine were obtained as HCl salts.
(1b) *p*-Phenetidine (0.068 g, 0.5 mmol) and triethylamine (0.15 ml, 1.1 mmol) were added to a mixture of 8-bromo-6-chloro-3-methylimidazo[1,2-b]pyridazine and 6,8-dichloro-3-methylimidazo[1,2-b]pyridazine (0.011 g, 0.5 mmol) both from *1a* in EtOH (10 mL). The mixture was heated to 90 °C and stirred for 30 hrs. The solution was then concentrated in vacuo to give crude 6-chloro-N-(4-ethoxyphenyl)-3-methylimidazo[1,2-b]pyridazin-8-amine.
*(1c)* In a 2 dram reaction vial was added crude 6-chloro-N-(4-ethoxyphenyl)-3-methylimidazo[1,2-b]pyridazin-8-amine (0.15 g, 0.5 mmol) from *1b* and trans-1,4-diaminocyclohexane (1.0 g, 8.0 mmol). The mixture was allowed to melt at 160 °C for 48 hrs hrs. The melt was then cooled, diluted with water and extracted with dichloromethane. The organic layer was concentrated and then diluted with MeOH and then purified by preparative HPLC to give the title compound as a TEA salt. 1H NMR (400 MHz, *MeOD*) δ ppm 7.62 (1 H, s), 7.25 (2 H, d, *J*=9.16 Hz), 6.99 (2 H, d, *J*=9.16 Hz), 6.21 (1 H, s), 4.05 (2 H, q, *J*=7.12 Hz), 3.64 - 3.89 (1 H, m), 3.00 - 3.22 (1 H, m), 2.48 (3 H, s), 2.17 - 2.38 (2 H, m, *J*=11.19 Hz), 1.99 - 2.18 (2 H, m, *J*=12.21 Hz), 1.45 - 1.71 (2 H, m), 1.22 - 1.46 (5 H, m). LC/MS, *m*/*e* 381 (M+1). HPLC Rt, 1.91 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min

### Example XXVIII(1)

### N⁶-(trans-4-aminocyclohexyl)-N⁸-(4-(ethyloxy)phenyl)-2,3-dimethylimidazo[1,2-b] pyridazine-6,8-diamine

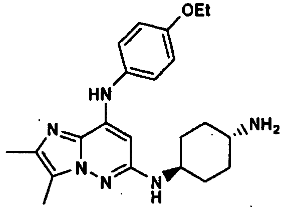

(*1a*) A mixture of 4-bromo-6-chloro-pyridazin-3-amine (0.1 g, 0.48 mmol) from Example I(1), step *1a*, 3-chlorobutan-2-one (0.42 g, 3.3 mmol) and EtOH (1 ml) was stirred at 90°C for 48 hrs. The reaction was then concentrated to dryness and triterated from diethyl ether. Upon filtration, an oily material resulted. The frit containing the oily solid was rinsed with methanol and the material collected as a mixture of 8-bromo-6-chloro-2,3-dimethylimidazo[1,2-b]pyridazine and 6,8-dichloro-2,3-dimethylimidazo[1,2-b]pyridazine (HCl salt)..
(*1b*) *p*-Phenetidine (0.04 g, 0.29 mmol) and potassium carbonate (0.12 g, 0.87 mmol) were added to a mixture of 8-bromo-6-chloro-2,3-dimethylimidazo[1,2-b]pyridazine and 6,8-dichloro-2,3-dimethylimidazo[1,2-b]pyridazine (HCl salt) (0.063 g, 0.29 mmol) both from *1a,* in EtOH (1.0 mL). The mixture was heated to 90 °C and stirred for 48 hrs. The solution was then concentrated in vacuo and purified by preparative HPLC (20-100% Methanol/Water gradient). This gave 0.013 g of 6-chloro-N-(4-ethoxyphenyl)-3-methylimidazo[1,2-b]pyridazin-8-amine.
(*1c*) In a 2 dram reaction vial was added crude 6-chloro-N-(4-ethoxyphenyl)-2,3-dimethylimidazo[1,2-b]pyridazin-8-amine (0.013 g, 0.04 mmol) from *1b* and trans-1,4-diaminocyclohexane (1.0 g, 8.0 mmol). The mixture was allowed to melt at 160 °C for 4 days. The melt was then cooled, diluted with water and extracted with dichloromethane. The organic layer was concentrated and then diluted with MeOH and then purified by preparative HPLC. This gave 0.005 g of the titled compound as a TFA salt. 1H NMR (500 MHz, MeOD) δ ppm 7.24 (2 H, d, *J*=8.25 Hz), 7.00 (2 H, d, *J*=8.80 Hz), 6.17 (1 H, s), 4.05 (2 H, q, *J*=6.96 Hz), 3.62 - 3.83 (1 H, m), 3.02 - 3.21 (1 H, m), 2.47 (3 H, s), 2.42 (3 H, s), 2.18 - 2.29 (2 H, m, *J*=11.55 Hz), 2.03 - 2.14 (2 H, m, *J*=12.65 Hz), 1.45 - 1.62 (2 H, m), 1.39 (3 H, t, *J*=6.87 Hz), 1.26 - 1.36 (2 H, m). LC/MS, *m*/*e* 395 (M+1). HPLC Rt, 2.49 min. YMC ODSC18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B=100, gradient time 4 min, hold at 100% B 1 min, flow rate 4 mL/min.

### Example XXIX(1)

### N-(6-((trans-4-aminocyclohexyl)amino)imidazo[1,2-b]pyridazin-8-yl)benzenesulfonamide

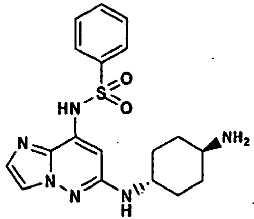

*(1a)* To a 16 X 100 mm tube was added benzenesulfonamide (58 mg, 0.37 mmol), tris(dibenzylideneacetone)dipalladium (0) (2 mg, 0.0022 mmol), 4,5-bis(diphenylphosphino)-9,9-dimethylxanthene (4 mg 0.0077 mmol) and cesium carbonate (240 mg, 1.25 mmol). The tube was evacuated and back filled with nitrogen. 8-Bromo-6-chloroimidazo[1,2-b]pyridazine (120 mg, 0.4466 mmol) Example I(1), step *1b* and 1,4-dioxane (1.0 ml) was then added. The mixture was allowed to heat at 100 C for 16 hours. The solution was then diluted with dichloromethane, filtered and concentrated in vacuo to afford crude N-(6-chloroimidazo[1,2-b]pyridazin-8-yl)benzenesulfonamide 100 mg (73%).
(*1b*) To N-(6-chloroimidazo[1,2-b]pyridazin-8-yl)benzenesulfonamide (100 mg, 0.325 mmol) from *1a* was added trans-1,4-diaminocyclohexane (1000 mg, 8.77 mmol). The mixture was allowed to melt at 165 C for 3 days. The melt was then cooled, water was added followed by extraction with dichloromethane. The organic layer was then concentrated in vacuo and purified by preparative HPLC. This gave 4.5 mg (2%) of the title compound as a di-TFA salt. ¹H NMR (500 MHz, MeOH-D₃) δ ppm 7.97 (2 H, d, J=5 Hz), 7.85 (1 H, s), 7.71 (1 H, s), 7.63 (1 H, m), 7.56 (2 H, m), 6.63 (1H, s), 3.64 (1 H, m), 3.12 (1 H, m), 2.23-2.04 (4 H, m), 1.51 (2 H, m), 1.34 (2 H, m). LC/MS *m*/*e* 387 (MH+). HPLC, 1.600 min. Waters Sunfire C18 4.6 x 50. 0%-100%B. B: 90% MeOH, 10 % H₂O, 0.1% TFA. A: 10% MeOH, 90 % H₂O, 0.1% TFA.

### Example XXXI(1)

### 6-((trans-4-aminocyclohexyl)oxy)-8-anilinoimidazo[1,2-b]pyridazine-3-carbonitrile

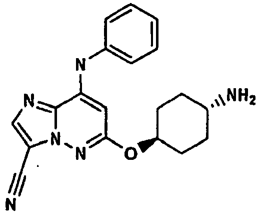

*(1a)* N-(4-Methoxybenzyl)aniline (215 mg, 1 mmol) was dissolved in dry DMF (4 mL), placed under a nitrogen atmosphere, and cooled to 0 °C in an ice bath. Potassium t-butoxide (1 mL, 1 mmol, 1M THF solution) was added and the mixture was allowed to stir for 10 min at 0 °C and room temperature for 30 min. The resulting mixture was cool to 0 °C. Solid 3,8-dibromo-6-chloroimidazo[1,2-b]pyridazine (310 mg, 1 mmol) from Example XXIV, step *1a* was added. The mixture was allowed to stir for 30 min at 0 °C and overnight at room temperature. The mixture was diluted with ethyl acetate (100 mL), washed sequentially with 10% LiCl, water and brine, and dried over anhydrous magnesium sulfate. The solvent was removed in vacuo and the resulting material was purified by silica gel chromatography (ethyl acetate/heptane, 25 min gradient :5-50% ethyl acetate) to provide 0.27 g of 3-bromo-6-chloro-N-(4-methoxybenzyl)-N-phenylimidazo[1,2-b]pyridazin-8-amine.
*(1b)* A microwave vial was charged with 3-bromo-6-chloro-N-(4-methoxybenzyl)-N-phenylimidazo[1,2-b]pyridazin-8-amine (220 mg, 0.49 mmol) from step 1a, zinc cyanide (34.8 mg, 0.3 mmol), Tris(dibenzylidenaceton)dipalladium(0) (22.7 mg, 0.025 mmol), 1,1'-Bis(diphenylphosphino)ferrocene (19.8 mg, 0.036 mmol) and DMF (2mL). The resulting mixture was heated in a microwave for 15 min at 150 °C. The solution was cooled, diluted with ethyl acetate, washed with saturated LiCl solution, dried over anhydrous magnesium sulfate and concentrated in vacuo. The resulting residue was purified silica gel chromatography (5% ethyl acetate/heptane) to provide 91 mg of 6-chloro-3-cyano-N-(4-methoxybenzyl)-N-phenylimidazo[1,2-b]pyridazin-8-amine
(*1c*) A mixture of 6-chloro-3-cyano-N-(4-methoxybenzyl)-N-phenylimidazo[1,2-b]pyridazin-8-amine (20 mg, 0.05 mmol) from *1b*, tert-butyl (trans)-4-hydroxycyclohexylcarbamate (22 mg, 0.1 mmol), palladium(II) acetate (1.1 mg, 0.005 mmol), 1,2,3,4,5-Pentaphenyl-1'-(di-tert-butylphosphino)ferrocene (7.5, 0.01 mmol) and cesium carbonate (100 mg) was suspended in anhydrous dioxane (1 mL) in a Teflon lined septum capped vial. The vessel was purged with argon and heated at 105 °C for 48h. LCMS shows that product (m/z, M+1, 569.4) is present (∼25% conversion) along with starting materials. The reaction is cooled and filtered thru a plug of celite and the celite pad rinsed with ∼5 ml of ethyl acetate. The solvents are removed in vacuo and the resulting oil suspended in 1 mL of TFA and heated at 50 °C of 2h. The reaction showed complete removal of the P-methoxybenzyl protecting group. TFA is removed under a stream of air and the resulting mixture taken up in MeOH (2 mL) and purified by preparative HPLC to yield 2.2 mg of the titled compound as a TFA salt. MS *m*/*e* 349 (M+1); 1H NMR (MeOH, δ) 8.1 (1H, s), 7.48 (2H, t, J=8Hz), 7.39 (2H, d, J=8 Hz), 7.27 (1H, t, J=8 Hz), 6.21 (1H, s), 5.00 1H, m), 3.21 (1H, m), 2.38 (2H, m), 2.15, 2H, m), 1.62 (4H, m).

### Example XXXII(1)

### 6-((trans-4-aminocyclohexyl)amino)-8-anilinoimidazo[1,2-b]pyridazine-3-carboxamide

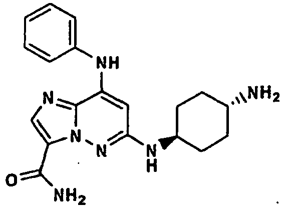

(*1a*)A mixture of 6-chloro-3-cyano-N-(4-methoxybenzyl)-N-phenylimidazo[1,2-b]pyridazin-8-amine (25 mgs, mmol) from Example XXXI(1) step 1b, 6N NaOH (0.1 mL), MeOH (0.1 mL) and dioxane (1 mL) in a vial was heated to 100 °C for 16 h. The mixture was cooled to room temperature and concentrated in vacuo. Upon addition of 1M HCl a precipitate forms. The precipitate was collected by filtration and dried to provide 35 mgs of crude 6-chloro-8-((4-methoxybenzyl)(phenyl)amino)imidazo[1,2-b]pyridazine-3-carboxamide.
*(1b)* To a vial containing crude 6-chloro-8-((4-methoxybenzyl)(phenyl)amino)imidazo[1,2-b]pyridazine-3-carboxamide 35 mgs from *1a* was added trans-1,4-cyclohexyldiamine (250 mgs, mmol). The resulting mixture was heated to 160 °C for 4h. The mixture was cooled to room temperature and diluted with water causing a precipitate to form. The precipitate was collected, washed with water and dried to give crude 6-((trans)-4-aminocyclohexylamino)-8-((4-methoxybenzyl)(phenyl)amino)imidazo[1,2-b]pyridazine-3-carboxamide.
(*1c*) Crude 6-((trans)-4-aminocyclohexylamino)-8-((4-methoxybenzyl)(phenyl)amino)imidazo[1,2-b]pyridazine-3-carboxamide from *1b* was suspended in trifluoracetic acid (1mL) and heated to 50 °C for 2h. The mixture was cooled to room temperature and the trifluoractic acid was evaporated under a stream of nitrogen. The residue was suspended in methanol, filteedr to remove solids and purified by prep HPLC to provide 2.1 mg of the titled compound as a TFA salt. MS *m*/*e* 366 (M+1); 1H NMR (MeOH, δ) 8.1 (1H, s), 7.46 (2H, t, J=8Hz), 7.39 (2H, d, J=8 Hz), 7.23 (1H, t, J=8 Hz), 6.31 (1H, s), 3.62 (1H, m), 3.17 (1H, m), 2.30 (2H, m), 2.13, (2H, m), 1.55 (2H, m), 1.42 (2H, m).

### EXAMPLE XXXIII(1)

### N⁶-(trans-4-aminocyclohexyl)-7-ethyl-N⁸-phenylimidazo[1,2-b]pyridazine-6,8-diamine

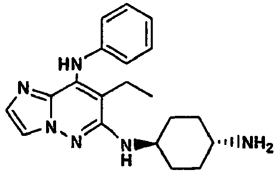

*(1a)* To a suspension of 3,6-dichloropyridazine (11.25g, 0.076 mol, 1.0 eq), silver nitrate (6.41g, 0.038 mol, 0.5 eq), propionic acid (8.39 g, 0.113 mol, 1.5 eq) in water (125 mL) at 50°C was added a solution of sulfuric acid (11.54 mL, 0.227 ml. 3.0 eq) in water (125 mL). The solution was heated to 60°C and then a solution of ammonium persulfate (51.7 g, 0.227 mol, 3.0 eq) was added in slowly in 20 minutes. The solution was then heated to 75°C for 30 minutes. The reaction solution was poured into ice water and adjusted to pH 7 with 30% ammonium hydroxide solution. The product *1* was extracted with dichloromethane (3x), and the extracts washed with water, brine, dried with sodium sulfate and concentrated in vacuo. The resulting residue was purified using an ISCO chromatography system (120 g silica cartridge, 5% ethyl acetate in heptane) to provide the compound 3,6-dichloro-4-ethylpyridazine (7.3 g, 54% yield). LC/MS, *m*/*e* 177.15 (M+1). HPLC Rt, 2.03 min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.
(*1b*) 3, 6-Dichloro-4-ethylpyridazine (3.5 g, 0.020 mol) from *1a* was suspended in aqueous 28% NH₄OH (12 mL) in a sealed microwave tube and heated at 145 °C for 1h. The reaction solution was cooled and then heated once more at 145°C for 1 h. The microwave tube was uncapped and allowed to stir at room temperature for 30 min and in an ice bath for 30 min. The solid that crashed out was filtered and then washed with ice water and dried to give 3.5 g of a mixture of the desired 6-chloro-5-ethylpyridazin-3-amine and the 6-chloro-4-ethylpyridazin-3-amine regioisomer LC/MS, *m*/*e* 158.19 (M+1). HPLC Rt, 0.78 min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.
(*1c*) The mixture of 6-Chloro-5-ethylpyridazin-3-amine and chloro-4-ethylpyridazin-3-amine (3.50 g, 0.022 mol) from *1b* and NaHCO₃ (3.73 g, 0.044mol, 2eq) were suspended in MeOH (20 mL) and treated with Br₂ (1.25 mL, 0.024 mol). The mixture was stirred at room temperature for 24h, then filtered. The filtrate was condensed *in vacuo.* The resulting residue was resuspended in EtOAc (100 mL) and washed sequentially with sat. aqueous NaHCO₃ solution (2X20 mL) and aqueous NaCl solution (1X 20 mL). The solution was dried over sodium sulfate. The solvent was removed *in vacuo* to give crude 4-bromo-6-chloro-5-ethylpyridazin-3-amine. LC/MS, *m*/*e* 236 (M+1). HPLC Rt, 2.15 min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B =100, gradient time 4 min, hold at 100% B 1 min, flow rate 4 mL/min.
*(1d)* Chloroacetaldehyde (17.26 ml, 0.111 mol, 50% in H₂O) was added to a solution of crude 4-bromo-6-chloro-5-ethylpyridazin-3-amine (5.23 g, 0.022 mol) from *1c* in EtOH (30 mL). The mixture was heated in a sealed vial at 50 °C for 24h. Solvent was removed in vacuo and the solid was resuspended in acetone/Et₂O (1/1, 5 mL), filtered, and then washed with Et₂O to give 8-bromo-6-chloro-7-ethylimidazo[1,2-*b*]pyridazine as an HCl salt (3.02 g, >80% pure). LC/MS, *m*/*e* 260 (M+1). HPLC Rt, 2.68 min. Waters Sunfire C18 column (4.6 x 50 mm). 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.
*(1e)* A mixture of 8-bromo-6-chloro-7-ethylimidazo[1,2-b]pyridazine (60 mg, 0.2 mmol, 1 eq) from *1d*, aniline (20.3 µL, 0.22 mmol, 1.1 eq) and potassium *tert-*butoxide (0.51 mL, 0.51 mmol, 2.5 eq) were suspended in DMF (3 mL) and stirred at RT for 1h. The mixture was quenched with ethyl acetate and washed with lithium chloride saturated solution, dried with sodium sulfate and concentrated *in vacuo.* The residue was purified using an ISCO chromatography system (4 g silica cartridge, 5% ethyl acetate in heptane) to provide 6-chloro-*N*-phenyl-7-ethylimidazo[1,2-b]pyridazin-8-amine (22 mg). LC/MS, *mle* 273.14 (M+1). HPLC Rt, 2.24 min. Waters Sunfire C18 column (4.6 x 50 mm), 0%-100%B. Solvent B: (90% MeOH, 10 % H₂O, 0.1% TFA). Solvent A: (10% MeOH, 90 % H₂O, 0.1% TFA). Gradient, start %B = 0, final % B = 100, gradient time 4 min, hold at 100% B 1 min, flow rate 4 mL/min.
*(1f)* 6-chloro-*N*-phenyl-7-ethylimidazo[1,2-*b*]pyridazin-8-amine (22 mg, 0.08 mmol) from *1e* and trans-1,4-diaminohexane (230 mg) were combined and heated at 165 °C for 4 days. The mixture was cooled to room temperature, then diluted with methanol. The resulting residue was purified using preparative HPLC to give the above titled compound as a TFA salt (18.9 mg, 66%). ¹H NMR (500 MHz, CD₃OD) δ 7.95 (d, 1H), 7.62 (d, 1H), 7.27 (t, J=7.4 Hz, 2H), 7.0 (t, J=7.4 Hz, 1H), 6.85 (d, J= 8.2 Hz, 2H), 4.0 (m, 1H), 3.20 (m, 1H), 2.79 (m, 2H), 2.25 (m, 2H), 2.14 (m, 2H), 1.58 (m, 4H), 1.13 (t, *J*=7.2 Hz, 3H). LC/MS, *m*/*e* 351 (M+1). HPLC Rt, 1.67 min. Waters Sunfire C18 column (4.6 x 50 nun). 0%-100%B. Solvent B: (90% MeOH, 10 % H₂O, 0.1% TFA). Solvent A: (10% MeOH, 90 % H₂O, 0.1% TFA). Gradient, start %B = 0, final % B =100, gradient time 4 min, hold at 100% B 1 min, flow rate 4 mL/min.

Compounds having the formula (Ia) were prepared according to procedures similar to Example XXXIII(1), where in R₁, R₂, R₃, X and Y have the values listed in Table 13, using the appropriate starting materials and substantially the same procedures as indicated.

**Table 13**

| **Exp** | **Name** | **R₁** | **R₂** | **R₃** | **X** | **Y** | **LC/MS m/z (M+1)** |
|---|---|---|---|---|---|---|---|
| XXXIII(2) | *N⁶*-(*trans*-4-aminocyclohexyl)-7-ethyl-*N⁸*-[4-(ethyloxy)phenyl]imidazo[1,2-*b*]pyridazine-6,8-diamine | **H** | **H** | **i-Pr** | 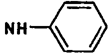 | 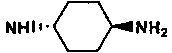 | 365 |

| | | | | | | | |
|---|---|---|---|---|---|---|---|
| *For substituents X and Y, substitution on the core (formula **Ia)** occurs at the available nitrogen atom | | | | | | | |

## Claims

1. A compound according to formula (I), 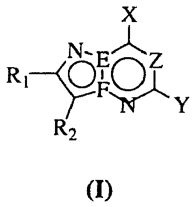 or an enantiomer, diastereomer, or a pharmaceutically-acceptable salt, thereof, wherein:
E is C; F is N;
X is NR₄R₅;
Z is CR₃;
Y is selected from hydrogen, halogen, nitro, cyano, SR₈, S(O)*ₚ*R₈, OR₈, NR₆R₇, CO₂R₈, C(=O)R₈, O-C(=O)R₈, C(=O)NR₈R₉, cycloalkyl, cycloalkenyl, cycloalkynyl, heterocyclo, aryl, and heteroaryl, provided that if Y is hydrogen then R₄ is phenyl substituted with a carboxamido group;
R₁ and R₂ are independently selected from (i) hydrogen, alkyl, halogen, nitro, cyano, SR₁₀, OR₁₀, NR₁₀R₁₁, NR₁₀C(=O)R₁₁, CO₂R₁₀, C(=O)R₁₀, -O-C(=O)R₁₀, C(=O)NR₁₀R₁₁ ;
R₃ is selected from hydrogen, halogen, alkyl, substituted alkyl, alkenyl, substituted alkenyl alkynyl, substituted alkynyl, nitro, cyano, SR₁₃, OR₁₃, NR₁₃R₁₄, NR₁₃C(=O)R₁₄, CO₂R₁₃, C(=O)R₁₃, -O-C(=O)R₁₃, -C(=O)NR₁₃R₁₄, cycloalkyl, heterocyclo, aryl, and heteroaryl;
R₄, R₅, R₆, and R₇ are independently selected from hydrogen, alkyl, substituted alkyl, alkenyl, substituted alkenyl, alkynyl, substituted alkynyl, OR₁₅, SR₁₅, C(=O)R₁₅, CO₂R₁₅, C(=O)NR₁₅R₁₆, C(W)OR₁₆, S(O)ₚR₁₇, SO₂NR₁₅R₁₆, cycloalkyl, heterocyclo, aryl, and heteroaryl; or (ii) R₄ is taken together with R₅ and the nitrogen atom to which they are both attached and/or R₆ is taken together with R₇ and the nitrogen atom to which they are both attached to form a heteroaryl or heterocyclo;
R₈, R₉, R₁₀, R₁₁, R₁₃, R₁₄, R₁₅, and R₁₆ at each occurrence are independently selected from (i) hydrogen, alkyl, substituted alkyl, alkenyl, substituted alkenyl, alkynyl, substituted alkynyl, cycloalkyl, aryl, heteroaryl, and heterocyclo; or (ii) together with the nitrogen atom to which they are attached, R₈ is taken together with R₉, and/or R₁₀ is taken together with R₁₁, and/or R₁₃ is taken together with R₁₄, and/or R₁₅ is taken together with R₁₆ to form a heteroaryl or heterocyclo;
R₁₇ is selected from alkyl, substituted alkyl, alkenyl, substituted alkenyl, alkynyl, substituted alkynyl, cycloalkyl, aryl, heteroaryl, and heterocyclo;
W at each occurrence is O, S, N, CN, or NH; and
p is 1 or 2,
with the following provisos:
(1) if X is NH(Me), N(Me)₂, NH(unsubstituted phenyl), or NHNH₂, then Y is other than hydrogen or halogen; and
(2) the following compounds are excluded:

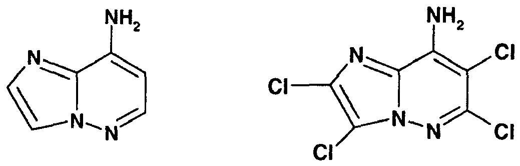

2. A compound according to claim 1 having formula (Ia) 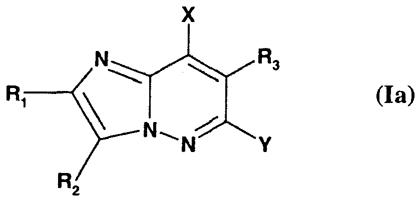 or an enantiomer, diastereomer, or a pharmaceutically-acceptable salt, thereof.

3. A compound according to claim 2, or an enantiomer, diastereomer, or a pharmaceutically-acceptable salt, thereof in which:
R₄ is -AM;
R₅ is hydrogen or C₁₋₄alkyl;
or R₄ and R₅ together with the nitrogen atom to which they are attached form a 5-, 6- or 7-membered monocyclic heteroaryl or heterocyclo ring, or a 7- to 11-membered bicyclic heteroaryl or heterocyclo ring, each ring optionally substituted with one to three groups, T₁, T₂; and/or T₃;
A is a bond, C₁₋₃alkylene, C₂₋₄alkenylene, C₂₋₄alkynylene, -C(O)-, or -SO₂-;
M is (i) hydrogen, alkyl, alkoxy, or alkenyl; or (ii) cycloalkyl, heterocyclo, aryl, or heteroaryl, each group optionally substituted by one to three groups, T₁, T₂, and/or T₃; T₁, T₂, and T₃ are independently selected from (i) halogen, alkyl, substituted alkyl, alkenyl, substituted alkenyl, alkynyl, substituted alkynyl, nitro, cyano, SO₃H SR₁₉, S(O)*ₚ*R₂₁, S(O)*ₚ*NR₁₉R₂₀, NR₁₉S(O)*ₚ*R₂₁, OR₁₉, NR₁₉R₂₀, NR₁₉C(=O)₂₀, NR₁₉C(=O)NR₁₉R₂₀, CO₂R₁₉, C(=O)R₁₉, -O-C(=O)R₁₉, -C(=O)NR₁₉R₂₀, cycloalkyl, heterocyclo, aryl, and heteroaryl, wherein *p* is one or 2; and/or (ii) two groups, T₁ and T₂, located on adjacent ring atoms are taken together with the ring atoms to which they are attached to form a fused cycloalkyl, aryl, heteroaryl, or heterocyclo;
R₁₉, R₂₀, and R₂₁ at each occurrence, are selected independently from (i) hydrogen, alkyl, substituted alkyl, alkenyl, substituted alkenyl, cycloalkyl, aryl, heteroaryl, and heterocyclo; or (ii) R₁₉ and R₂₀ together with the nitrogen atom to which they are both attached form a heteroaryl or heterocyclo; and
R₂₁ at each occurrence, is selected from alkyl, substituted alkyl, alkenyl, substituted alkenyl, alkynyl, substituted alkynyl, cycloalkyl, aryl, heteroaryl, and heterocyclo.

4. A compound according to claim 2, or an enantiomer, diastereomer, or a pharmaceutically-acceptable salt, thereof in which:
Y is hydrogen, halogen, OR₈, or NR₆R₇;
R₆ is selected from hydrogen or C₁₋₄alkyl optionally substituted by one to three groups selected from halogen, C₁₋₄alkyl, nitro, cyano, amino, C₁₋₄alkoxy, and OH;
R₇ and R₈ are independently selected from alkyl, cycloalkyl, heterocyclo, aryl, and heteroaryl, each group of which is optionally substituted by one to three groups, T₄, T₅, and/or T₆ ;
or R₆ and R₇ together with the nitrogen atom to which they are attached form a heteroaryl or heterocyclo ring, each ring is optionally substituted by one to three groups, T₄, T₅, and/or T₆;
T₄, T₅ and T₆ are independently selected from
(i) halogen, alkyl, substituted alkyl, alkenyl, substituted alkenyl, alkynyl, substituted alkynyl, nitro, cyano, SR₁₉, OR₁₉, NR₁₉R₂₀, NR₁₉C(=O)R₂₀, CO₂R₁₉, C(=O)R₁₉, -O-C(=O)R₁₉, -C(=O)NR₁₉R₂₀, cycloalkyl, heterocyclo, aryl, and heteroaryl; and/or (ii) two groups, T₄ and T₅, substituted on adjacent ring atoms are taken together with the ring atoms to which they are attached to form a fused cyclalkyl, heterocyclo, aryl, or heteroaryl; and R₁₉ and R₂₀, at each occurrence are selected independently from (i) hydrogen, alkyl, substituted alkyl, alkenyl, substituted alkenyl, cycloalkyl, aryl, heteroaryl, and heterocyclo; or
(ii) R₁₉ with R₂₀ together with the nitrogen atom to which they are both attached combine to form a heteroaryl or heterocyclo.

5. A compound of formula (Ia) : 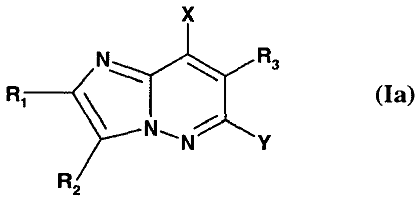 or an enantiomer, diastereomer, or a pharmaceutically-acceptable salt, thereof, wherein:
X is NR₄R₅;
Y is hydrogen, halogen, OR₈, or NR₆R₇;
R₁ and R₂ are independently selected from (i) hydrogen, alkyl, halogen, nitro, cyano, SR₁₀, OR₁₀, NR₁₀R₁₁, NR₁₀C(=O)R₁₁, CO₂R₁₀, C(=O)R₁₀, -O-C(=O)R₁₀, C(=O)NR₁₀R₁₁;
R₃ is selected from hydrogen, halogen, alkyl, substituted alkyl, alkenyl, substituted alkenyl, alkynyl, substituted alkynyl, nitro, cyano, SR₁₃, OR₁₃, NR₁₃R₁₄, NR₁₃C(=O)R₁₄, CO₂R₁₃, C(=O)R₁₃, -O-C(=O)R₁₃, -C(=O)NR₁₃R₁₄, cycloalkyl, heterocyclo, aryl, and heteroaryl;
R₄ is -AM;
R₅ is hydrogen or C₁₋₄alkyl;
or R₄ and R₅ together with the nitrogen atom to which they are attached form a 5-, 6- or 7-membered monocyclic heteroaryl or heterocyclo ring, or a 7- to 11-membered bicyclic heteroaryl or heterocyclo ring, each ring optionally substituted with one to three groups, T₁, T₂; and/or T₃;
A is a bond, C₁₋₃alkylene, C₂₋₄alkenylene, C₂₋₄alkynylene, -C(O)-, or -SO₂-; M is (i) hydrogen, NR₁₅R₁₆, alkyl, alkoxy, or alkenyl; or (ii) cycloalkyl, heterocyclo, aryl, or heteroaryl, each ring optionally substituted by one to three groups, T₁, T₂, and/or T₃;
R₆ is selected from hydrogen or C₁₋₄alkyl optionally substituted by one to three groups selected from halogen, C₁₋₄alkyl, nitro, cyano, amino, C₁-₄alkoxy, and OH;
R₇ is selected from alkyl, cycloalkyl, heterocyclo, aryl, and heteroaryl, each group of which is optionally substituted by one to three groups, T₄, T₅, and/or T₆ ;
or R₆ and R₇ together with the nitrogen atom to which they are attached form a heteroaryl or heterocyclo ring, each ring is optionally substituted by one to three groups, T₄, T₅, and/or T₆;
**R₈ is selected from alkyl, cycloalkyl, heterocyclo, aryl, and heteroaryl, each group of which is optionally substituted by one to three groups, T_{4,} T₅, and/or T₆ ;**
R₁₀, R₁₁, R₁₃, and R₁₄ at each occurrence are independently selected from (i) hydrogen, C₁-₄alkyl, and substituted C₁₋₄alkyl; or (ii) R₁₀ and R₁₁ together with the nitrogen atom they are both attached, and/or R₁₃ and R₁₄ together with the nitrogen atom they are both attached combine to form an optionally substituted 5-, 6-, or 7-membered heteroaryl or heterocyclo;
R₁₅ and R₁₆ are independently selected from (i) hydrogen, alkyl, substituted alkyl, alkenyl, substituted alkenyl, alkynyl, substituted alkynyl, cycloalkyl, aryl, heteroaryl, and heterocyclo; or (ii) together with the nitrogen atom to which they are attached R₁₅ is taken together with R₁₆ to form a heteroaryl or heterocyclo;
T₁, T₂, and T₃ are independently selected from (i) halogen, alkyl, substituted alkyl, alkenyl, substituted alkenyl, alkynyl, substituted alkynyl, nitro, cyano, SO₃H SR₁₉, S(O)*ₚ*R₂₁, S(O)*ₚ*NR₁₉R₂₀, NR₁₉S(O)*ₚ*R₂₁ , OR₁₉, NR₁₉R₂₀, NR₁₉C(=O)₂₀, NR₁₉C(=O)NR₁₉R₂₀, CO₂R₁₉, C(=O)R₁₉, -O-C(=O)R₁₉, -C(=O)NR₁₉R₂₀, cycloalkyl, heterocyclo, aryl, and heteroaryl, wherein *p* is one or 2; and/or (ii) two groups, T₁ and T₂, located on adjacent ring atoms are taken together with the ring atoms to which they are attached to form a fused cycloalkyl, aryl, heteroaryl, or heterocyclo;
T₄, T₅ and T₆ are independently selected from (i) halogen, alkyl, substituted alkyl, alkenyl, substituted alkenyl, alkynyl, substituted alkynyl, nitro, cyano, SR₁₉, OR₁₉, NR₁₉R₂₀, NR₁₉C(=O)R₂₀, CO₂R₁₉, C(=O)R₁₉, -O-C(=O)R₁₉, -C(=O)NR₁₉R₂₀, cycloalkyl, heterocyclo, aryl, and heteroaryl; and/or (ii) two groups, T₄ and T₅, substituted on adjacent ring atoms are taken together with the ring atoms to which they are attached to form a fused cyclalkyl, heterocyclo, aryl, or heteroaryl; and
R₁₉ and R₂₀ at each occurrence are selected independently from (i) hydrogen, alkyl, substituted alkyl, alkenyl, substituted alkenyl, cycloalkyl, aryl, heteroaryl, and heterocyclo; or (ii) R₁₉ and R₂₀ together with the nitrogen atom to which they are both attached form a heteroaryl or heterocyclo ring; and
R₂₁ at each occurrence, is selected from alkyl, substituted alkyl, alkenyl, substituted alkenyl, alkynyl, substituted alkynyl, cycloalkyl, aryl, heteroaryl, and heterocyclo;
with the following provisos:
(1) if X is NH(Me), N(Me)₂, NH(unsubstituted phenyl), or NHNH₂, then Y is other than hydrogen or halogen; and
(2) the following compounds are excluded:

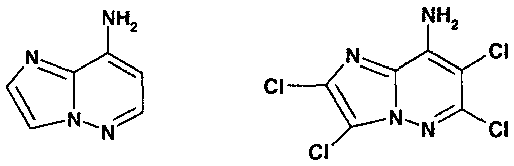

6. A compound according to claim 5, or an enantiomer, diastereomer, or a pharmaceutically-acceptable salt, thereof in which:
R₄ is -AM;
A is a bond, -C(O)-, or -S(O)₂-, or C₁₋₃alkylene;
M is (i) hydrogen, -NH(aryl), C₁₋₆alkyl, C₂₋₄alkenyl, or -OC₁₋₄alkyl or (ii) C₃₋₆cycloalkyl, phenyl, fluorenyl, 1-naphthyl, or 2-naphthyl, each group optionally substituted by one to three groups, T₁, T₂, and/or T₃; or (iii) a 5-, 6- or 7-membered monocyclic or a 7- to 11-membered bicyclic heteroaryl or heterocyclo ring, each ring optionally substituted by one to three groups, T₁, T₂, and/or T₃; and
T₁, T₂, and T₃ are independently selected from (i) C₁₋₄alkyl, substituted C₁₋₄alkyl, C₁₋₄alkyloxy, substitutedC₁₋₄alkyloxy, C₁₋₄alkylthio, phenoxy, -NR₁₉R₂₀, halogen, hydroxy, cyano, SO₃H, COOH, -C(O)(R₁₉), C(O)NR₁₉R₂₀, NR₁₉C(O)R₂₀, S(O)₂R₂₁, S(O)₂NR₁₉R₂₀ and NR₁₉(C(O)NR₁₉R₂₀; and/or (ii) phenyl, cyclopropyl, cyclohexyl, tetrazolyl, imidazolyl, pyrazolyl, triazolyl, thiazolyl, furyl, and morpholinyl, each group of which is optionally substituted as valence allows from one to three groups, R₂₂, R₂₃ and/or R₂₄; and/or (iii) two groups, T₁ and T₂, substituted on adjacent ring atoms are taken together with the ring atoms to which they are attached to form, a fused five- to seven-membered cycloalkyl, a fused phenyl or a fused 5- or 6-membered heterocyclo or heteroaryl, each group of which is optionally substituted as valence allows from one to three groups, R₂₂, R₂₃ and/or R₂₄; and
R₁₉ and R₂₀ at each occurrence are selected independently from (i) hydrogen, -(CH₂)ᵥOH, and C₁₋₄alkyl; or (ii) -(CH₂)ᵥcyclohexyl, -(CH₂)ᵥphenyl, -(CH₂)ᵥmorpholinyl, - (CH₂)ᵥpyridyl, -(CH₂)ᵥpyrazolyl, -(CH₂)ᵥcyclopropyl, -(CH₂)ᵥpyrrolidinyl, - (CH₂)ᵥpiperidinyl, -(CH₂)ᵥfuryl, -(CH₂)ᵥimidazolyl, -(CH₂)ᵥpyrimidinyl, - (CH₂)ᵥpiperazinyl, and -(CH₂)ᵥ pyradizinyl, each group of which is optionally substituted as valence allows from one to three groups, R₂₂, R₂₃ and/or R₂₄;or R₁₉ and R₂₀ are taken together with the nitrogen atom to which they are both attached to form a pyrrolindyl, morpholinyl, piperidinyl, pyradazinyl, or piperazinyl, each group of which is optionally substituted as valence allows from one to three groups, R₂₂, R₂₃ and/or R₂₄;
R₂₁ at each occurrence is selected from (i) -(CH₂)ᵥOH, and C₁₋₄alkyl; or (ii) - (CH₂)ᵥcyclohexyl, -(CH₂)ᵥphenyl, -(CH₂)ᵥmorpholinyl, -(CH₂)ᵥpyridyl, - (CH₂)ᵥpyrazolyl, -(CH₂)ᵥcyclopropyl, -(CH₂)ᵥpyrrolidinyl, -(CH₂)ᵥpiperidinyl, - (CH₂)ᵥfuryl, -(CH₂)ᵥimidazolyl, -(CH₂)ᵥpyrimidinyl, -(CH₂)ᵥpiperazinyl, and - (CH₂)ᵥ pyradizinyl, each group of which is optionally substituted as valence allows from one to three groups, R₂₂, R₂₃ and/or R₂₄;R₂₂, R₂₃, and R₂₄ at each occurrence, are selected independently from (C₁₋₄)alkyl, (C₂₋₄)alkenyl, halogen, hydroxy, cyano, nitro, CF₃, =O, O(C₁₋₄alkyl), OCF₃, C(=O)H, C(=O)(C₁₋₄alkyl), CO₂H, CO₂(C₁₋₄alkyl), NHCO₂(C₁₋₄alkyl), -S(C₁₋₄alkyl), -NH₂, NH(C₁₋₄alkyl), N(C₁₋₄alkyl)₂, N(C₁₋₄alkyl)₃⁺, SO₂(C₁₋₄alkyl), C(=O)(C₁₋₄alkylene)NH₂, C(=O)(C₁₋₄alkylene)NH(alkyl), C(=O)(C₁₋₄alkylene)N(C₁₋₄alkyl)₂, and optionally substituted phenyl; and v is 0, 1, 2, or 3.

7. A compound according to claim 6, or an enantiomer, diastereomer, or a pharmaceutically-acceptable salt, thereof, wherein NR₄R₅ is selected from the following: 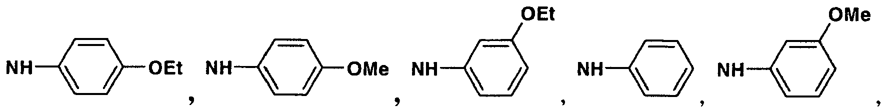 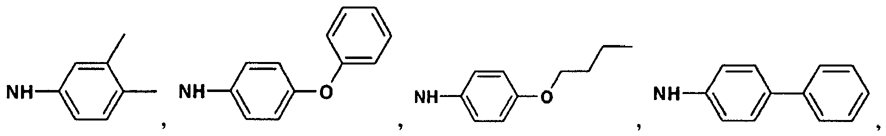 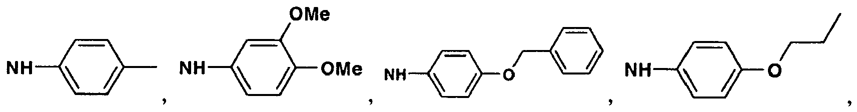 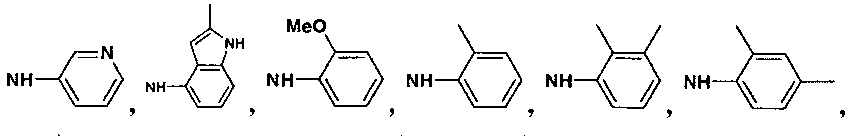 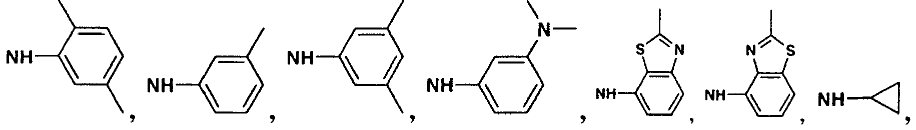 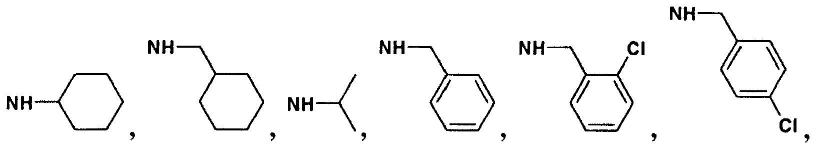 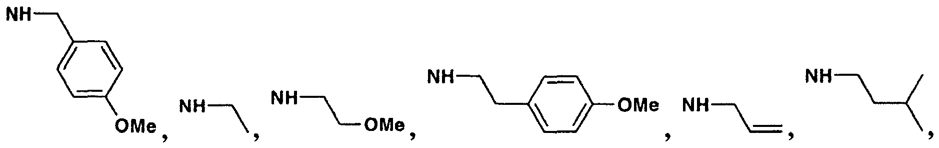 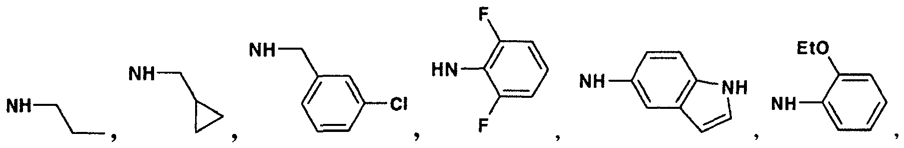 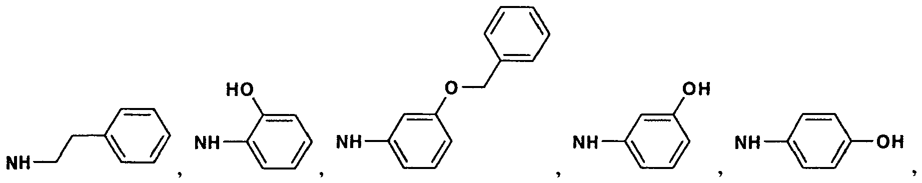 NH₂,

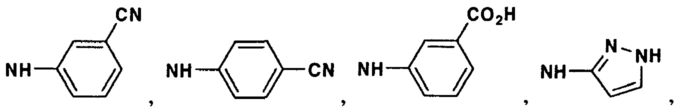

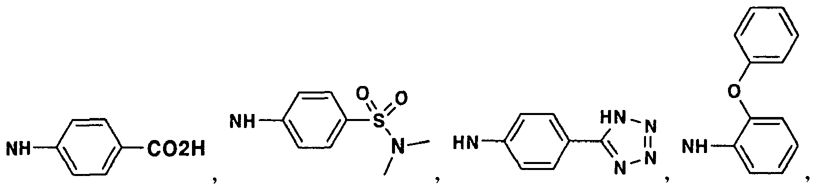

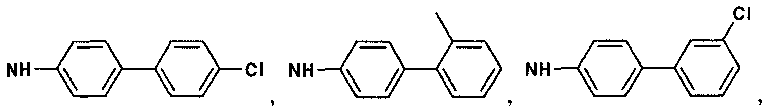

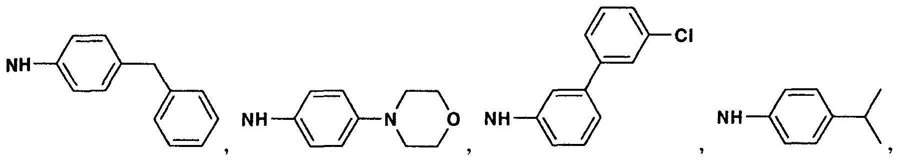

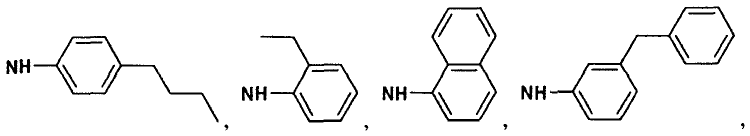

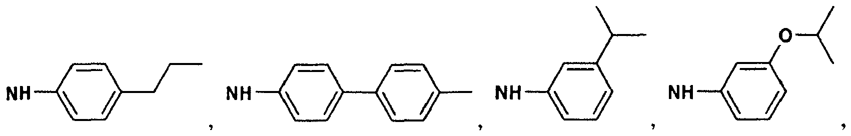

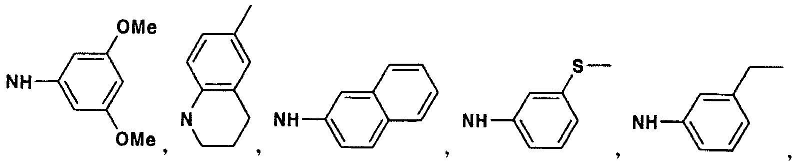

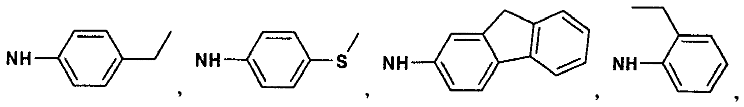

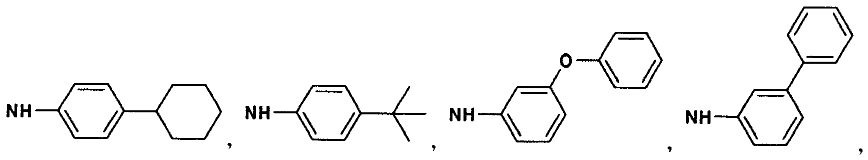

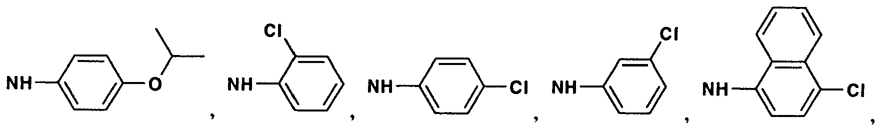

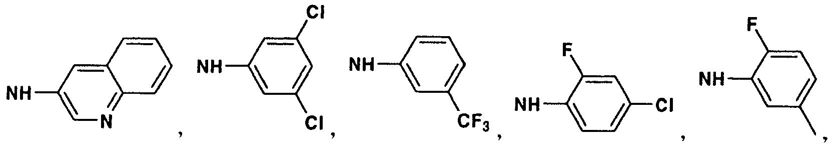

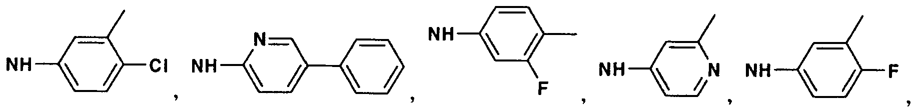

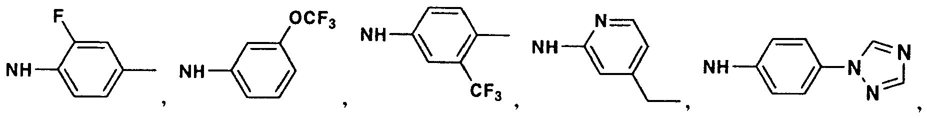

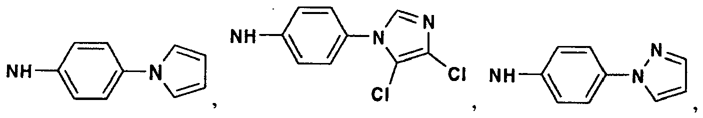

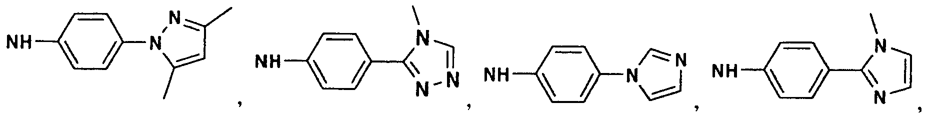

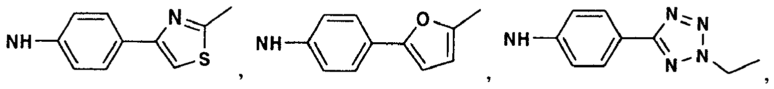

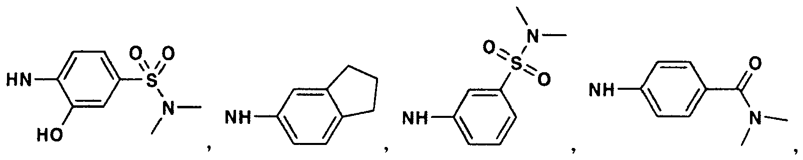

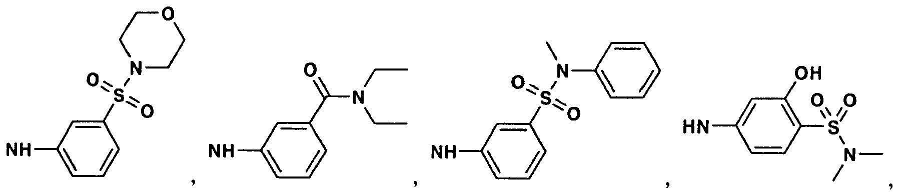

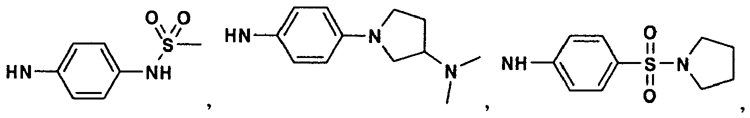

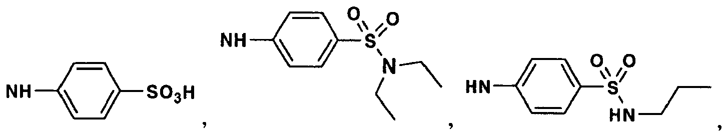

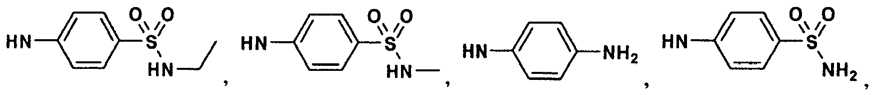

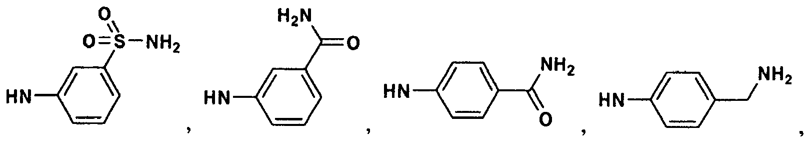

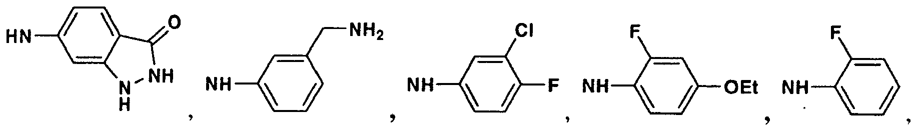

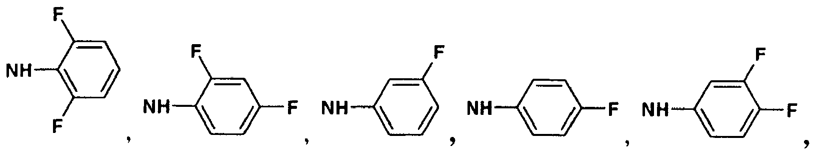

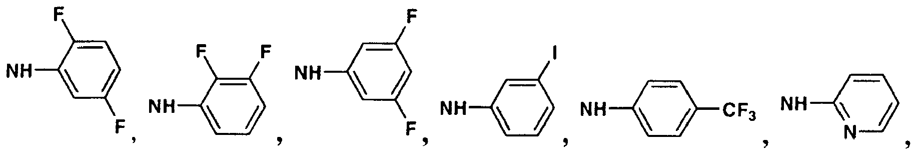

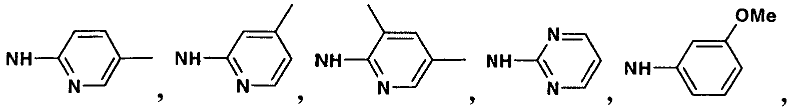

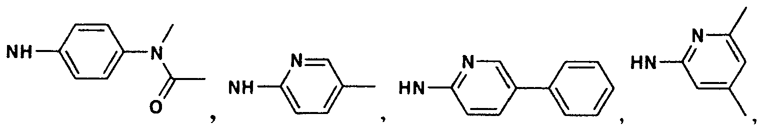

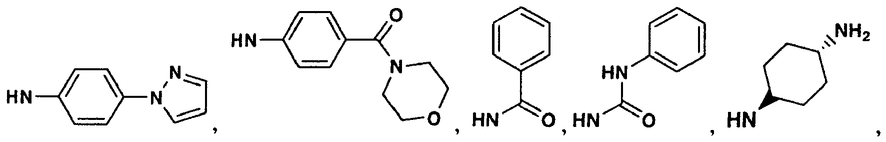

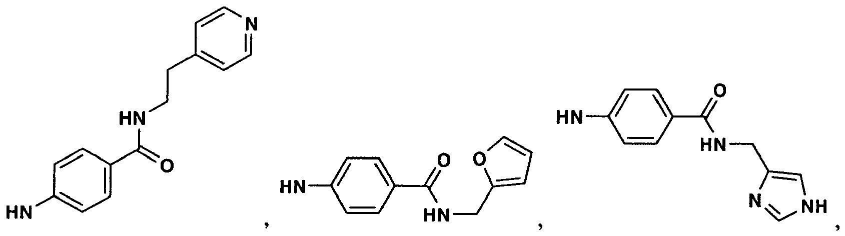

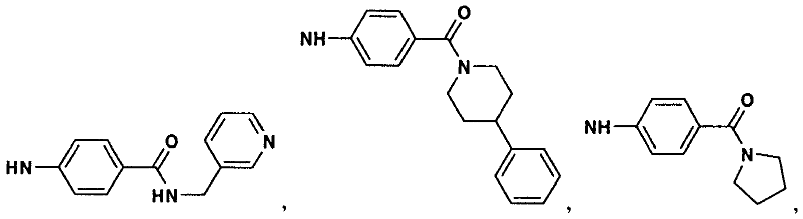

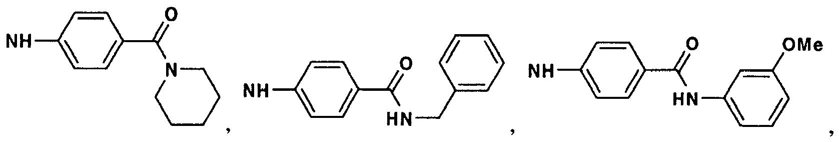

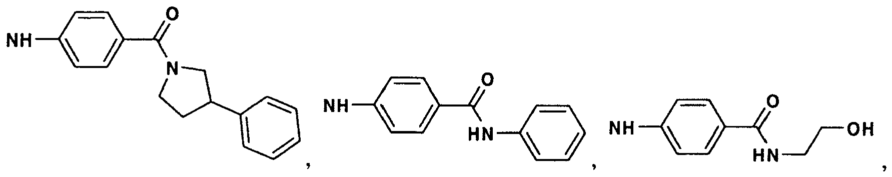

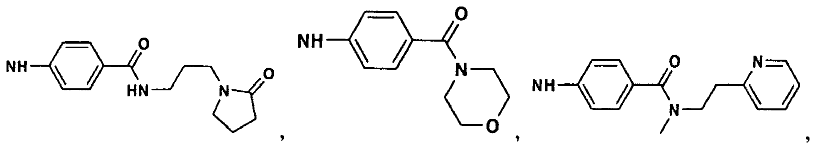

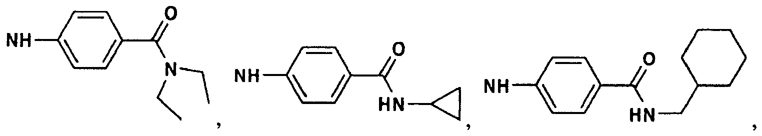

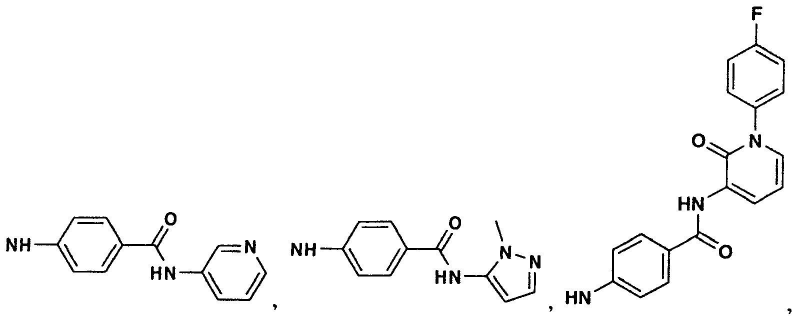

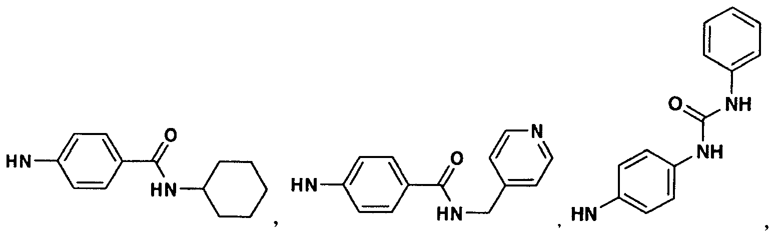

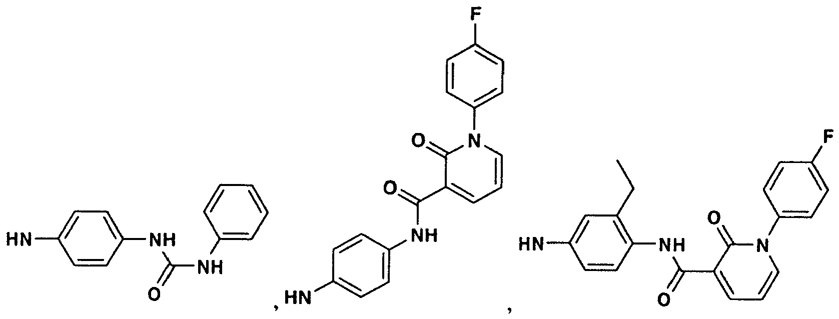

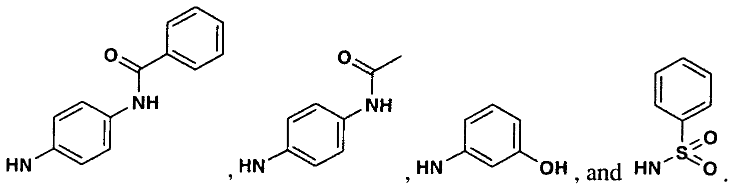

8. A compound according to claim 5, or an enantiomer, diastereomer, or a pharmaceutically-acceptable salt, thereof in which:
Y is NR₆R₇;
R₆ is selected from hydrogen or C₁₋₄alkyl;
R₇ is selected from C₁₋₄alkyl, cyclopentyl, cyclohexyl, bicyclo[2.2.2]octyl, pyrrolidinyl, and piperidinyl, each group of which is optionally substituted by one to three groups, T₄, T₅, and/or T₆;
or R₆ and R₇ together with the nitrogen atom to which they are attached form piperazinyl, piperidinyl, pyrrolidinyl, or diazepanyl, each group of which is optionally substituted by one to three groups, T₄, T₅, and/or T₆; and
T₄, T₅, and T₆ are independently selected from (i) C₁₋₄alkyl, OH, NH₂, NH(C₁₋₄alkyl), furyl, and N(C₁₋₄alkyl)₂, and NH(pyrimidinyl) wherein the pyrimidinyl is substituted by halogen; or (ii) C₁₋₄alkyl substituted by cyclohexyl or OH, wherein the cyclohexyl is substituted by NH₂.

9. A compound according to claim 8, or an enantiomer, diastereomer, or a pharmaceutically-acceptable salt, thereof wherein NR₆R₇ is selected from the following: NH-(CH₂)₂-NH₂, NH-(CH₂)₄-NH₂,

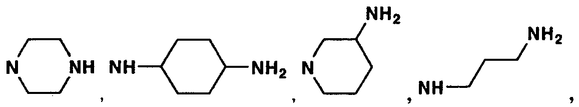

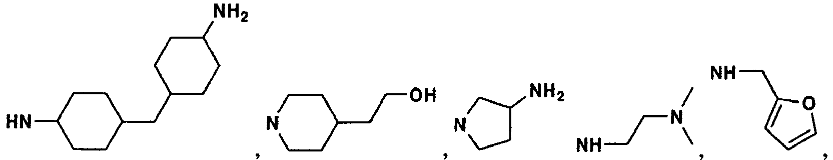

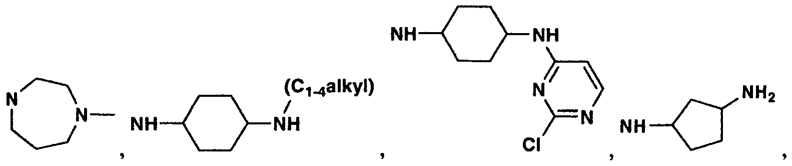

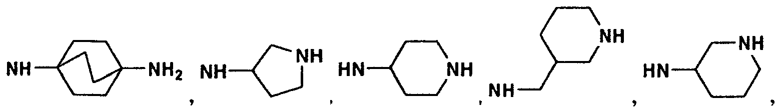

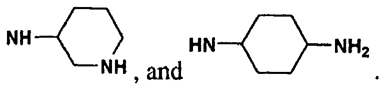

10. A compound according to claim 8, or an enantiomer, diastereomer, or a pharmaceutically-acceptable salt, thereof wherein NR₆R₇ is selected from: 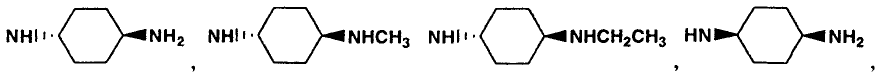 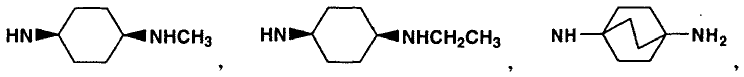 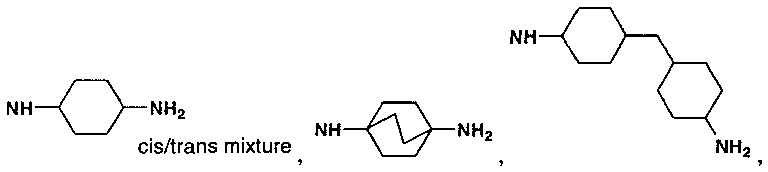 and

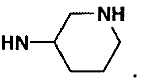

11. A compound according to claim 5, or an enantiomer, diastereomer, or a pharmaceutically-acceptable salt, thereof wherein R₅ is hydrogen and R₄ is selected from phenyl, pyridyl, pyrimidinyl, cyclohexyl, and piperidinyl ring, each ring optionally substituted by one to two groups, T₁, and/or T₂.

12. A compound according to claim 1 or 5, or an enantiomer, diastereomer, or a pharmaceutically-acceptable salt, thereof, in which:
R₁ and R₂ are independently selected from hydrogen, halogen, OR₁₀, cyano, C₁₋₄alkyl, CO₂R₁₀, and C(O)NR₁₀R₁₁;
R₃ is selected from (i) hydrogen, halogen, nitro, cyano, OR₁₀, NR₁₀R₁₁, CO₂R₁₀, and C(=O)R₁₀, (ii) C₁₋₄alkyl, substituted C₁₋₄alkyl, cycloalkyl, aryl, and heteroaryl; and
R₁₀ and R₁₁ are independently selected from (i) hydrogen, C₁₋₄alkyl, and substituted C₁₋₄alkyl; or (ii) R₁₀ and R₁₁ together with the nitrogen atom they are both attached combine to form an optionally substituted 5-, 6-, or 7-membered heteroaryl or heterocyclo.

13. A compound according to claim 1 or 5 wherein the compound has the formula (Ia), 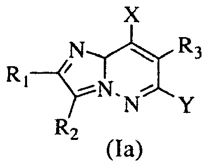 or an enantiomer, diastereomer, or a pharmaceutically-acceptable salt, thereof.

14. A compound selected from the following:
(i)
*N*⁶-(*trans*-4-aminocyclohexyl)-N⁸-[4-(ethyloxy)phenyl]imidazo[1,2-*b*]pyridazine-6,8-diamine,
*N*⁶-(2-aminoethyl)-*N*⁸-(4-(ethyloxy)phenyl)imidazo[1,2-*b*]pyridazine-6,8-diamine;
*N*⁶-(4-aminobutyl)-*N*⁸-(4-(ethyloxy)phenyl)imidazo[1,2-*b*]pyridazine-6,8-diamine;
7-chloro-*N*-(4-(ethyloxy)phenyl)-6-(1-piperazinyl)imidazo[1,2-*b*]pyridazin-8-amine;
*N*⁶-(trans-4-aminocyclohexyl)-*N*⁸-(4-(methyloxy)phenyl)imidazo[1,2-*b*]pyridazine-6,8-diamine;
*N*⁶-(*trans*-4-aminocyclohexyl)-*N*⁸-(3-(ethyloxy)phenyl)imidazo[1,2-*b*]pyridazine-6,8-diamine;
*N*⁶-(*trans*-4-aminocyclohexyl)-*N*⁸-phenylimidazo[1,2-*b*]pyridazine-6,8-diamine;
*N*⁶-(*trans*-4-aminocyclohexyl)-*N*⁸-(3-(methyloxy)phenyl)imidazo[1,2-*b*]pyridazine-6,8-diamine;
*N*⁶-(*trans*-4-aminocyclohexyl)-*N*⁸-(3,4-dimethylphenyl)imidazo[1,2-*b*]pyridazine-6,8-diamine;
*N*⁶-(*trans*-4-aminocyclohexyl)-*N*⁸-(4-(phenyloxy)phenyl)imidazo[1,2-*b*]pyridazine-6,8-diamine;
*N*⁶-(*trans*-4-aminocyclohexyl)-*N*⁸-(4-(butyloxy)phenyl)imidazo[1,2-*b*]pyridazine-6,8-diamine;
*N*⁶-(*trans*-4-aminocyclohexyl)-*N*⁸-4-biphenylylimidazo[1,2-*b*]pyridazine-6,8-diamine;
N⁶-(trans-4-aminocyclohexyl)-*N*⁸-(4-methylphenyl)imidazo[1,2-*b*]pyridazine-6,8-diamine;
*N*⁶-(*trans*-4-aminocyclohexyl)-*N*⁸-(3,4-bis(methyloxy)phenyl)imidazo[1,2-*b*]pyridazine-6,8-diamine;
*N*⁶-(*trans*-4-aminocyclohexyl)-*N*⁸-(4-((phenylmethyl)oxy)phenyl)imidazo[1,2-*b*]pyridazine-6,8-diamine;
*N*⁶-(*trans*-4-aminocyclohexyl)-*N*⁸-(4-(propyloxy)phenyl)imidazo[1,2-*b*]pyridazine-6,8-diamine;
*N*⁶-(*trans*-4-aminocyclohexyl)-*N*⁸-pyridin-3-ylimidazo[1,2-*b*]pyridazine-6,8-diamine;
*N*⁶-(*trans*-4-aminocyclohexyl)-*N*⁸-(2-methyl-1*H*-indol-5-yl)imidazo[1,2-*b*]pyridazine-6,8-diamine;;
*N*⁶-(*trans*-4-aminocyclohexyl)-*N*⁸-methyl-*N*⁸-phenylimidazo[1,2-*b*]pyridazine-6,8-diamine; ;
*N*⁶-(*trans*-4-aminocyclohexyl)-*N*⁸-[2-(methyloxy)phenyllimidazo[1,2-*b*]pyridazine-6,8-diamine;
*N*⁶-(*trans*-4-aminocyclohexyl)-*N*⁸-(2-methylphenyl)imidazo[1,2-*b*]pyridazine-6,8-diamine;
*N*⁶-(*trans*-4-aminocyclohexyl)-*N*⁸-(2,3-dimethylphenyl)imidazo[1,2-*b*]pyridazine-6,8-diamine;
*N*⁶-(*trans*-4-aminocyclohexyl)-*N*⁸-(2,4-dimethylphenyl)imidazo[1,2-*b*]pyridazine-6,8-diamine;
*N*⁶-(*trans*-4-aminocyclohexyl)-*N*⁸-(2,5-dimethylphenyl)imidazo[1,2-*b*]pyridazine-6,8-diamine;
*N*⁶-(*trans*-4-aminocyclohexyl)-*N*⁸-(3-methylphenyl)imidazo[1,2-*b*]pyridazine-6,8-diamine;
*N*⁶-(*trans*-4-aminocyclohexyl)-*N*⁸-(3,5-dimethylphenyl)imidazo[1,2-*b*]pyridazine-6,8-diamine;
*N*⁶-(*trans*-4-aminocyclohexyl)-*N*⁸-[3-(dimethylamino)phenyl] imidazo[1,2-*b*]pyridazine-6,8-diamine;
*N*⁶-(*trans*-4-aminocyclohexyl)-*N*⁸-(2-methyl-1,3-benzothiazol-6-yl)imidazo[1,2-*b*]pyridazine-6,8-diamine;
*N*⁶-(*trans*-4-aminocyclohexyl)-*N*⁸-(2-methyl-1,3-benzothiazol-5-yl)imidazo[1,2-*b*]pyridazine-6,8-diamine;
*N*⁶-(*trans*-4-aminocyclohexyl)-*N*⁸-cyclopropylimidazo[1,2-*b*]pyridazine-6,8-diamine;
*N*⁶-(*trans*-4-aminocyclohexyl)-*N*⁸-cyclohexylimidazo[1,2-*b*]pyridazine-6,8-diamine;
*N*⁶-(*trans*-4-aminocyclohexyl)-*N*⁸-(cyclohexylmethyl)imidazo[1,2-*b*]pyridazine-6,8-diamine;
*N*⁶-(*trans*-4-aminocyclohexyl)-*N*⁸-(1-methylethyl)imidazo[1,2-*b*]pyridazine-6,8-diamine;
*N*⁶-(*trans*-4-aminocyclohexyl)-*N*⁸-(phenylmethyl)imidazo[1,2-*b*]pyridazine-6,8-diamine;
*N*⁶-(*trans*-4-aminocyclohexyl)-*N*⁸-[(2-chlorophenyl)methyl]imidazo[1,2-*b*]pyridazine-6,8-diamine;
*N*⁶-(*trans*-4-aminocyclohexyl)-*N*⁸-((4-chlorophenyl)methyl)imidazo[1,2-*b*]pyridazine-6,8-diamine;
*N*⁶-(*trans*-4-aminocyclohexyl)-*N*⁸-((4-(methyloxy)phenyl)methyl)imidazo[1,2-*b*]pyridazine-6,8-diamine;
*N*⁶-(*trans*-4-aminocyclohexyl)-*N*⁸-ethylimidazo[1,2-*b*]pyridazine-6,8-diamine;
*N*⁶-(*trans*-4-aminocyclohexyl)-*N*⁸-(2-(methyloxy)ethyl)imidazo[1,2*-b*]pyridazine-6,8-diamine;
*N*⁶-(*trans*-4-aminocyclohexyl)-*N*⁸-(2-(4-(methyloxy)phenyl)ethyl)imidazo[1,2-*b*]pyridazine-6,8-diamine;
*N*⁶-(*trans*-4-aminocyclohexyl)-*N*⁸-2-propen-1-ylimidazo[1,2-*b*]pyridazine-6,8-diamine;
*N*⁶-(trans-4-aminocyclohexyl)-*N*⁸-(3-methylbutyl)imidazo[1,2-*b*]pyridazine-6,8-diamine;
*N*⁶-(*trans*-4-aminocyclohexyl)-*N*⁸-propylimidazo[1,2-*b*]pyridazine-6,8-diamine;
*N*⁶-(*trans*-4-aminocyclohexyl)-*N*⁸-(cyclopropylmethyl)imidazo[1,2-*b*]pyridazine-6,8-diamine;
*N*⁶-(*trans*-4-aminocyclohexyl)-*N*⁸-((3-chlorophenyl)methyl)imidazo[1,2-*b*]pyridazine-6,8-diamine;
6-(3-amino-1-piperidinyl)-*N*-(4-(ethyloxy)phenyl)imidazo[1,2-*b*]pyridazin-8-amine
*N*⁶-(3-aminopropyl)-*N*⁸-(4-(ethyloxy)phenyl)imidazo[1,2*-b*]pyridazine-6,8-diamine;
*N*⁶-(*trans*-4-aminocyclohexyl)-*N*⁸-(2,6-difluorophenyl)imidazo[1,2-*b*]pyridazine-6,8-diamine;
*N*⁶-(4-((4-aminocyclohexyl)methyl)cyclohexyl)-*N*⁸-(4-(ethyloxy)phenyl)imidazo[1,2-*b*]pyridazine-6,8-diamine;
2-(1-(8-((4-(ethyloxy)phenyl)amino)imidazo[1,2-*b*]pyridazin-6-yl)-4-piperidinyl)ethanol ;
*N*⁶-(*trans*-4-aminocyclohexyl)-*N*⁸-1*H*-indol-5-ylimidazo[1,2-*b*]pyridazine-6,8-diamine;
*N*⁶-(*trans*-4-aminocyclohexyl)-*N*⁸-(2-(ethyloxy)phenyl)imidazo[1,2-*b*]pyridazine-6,8-diamine;
6-(3-amino-1-pyrrolidinyl)-*N*-(4-(ethyloxy)phenyl)imidazo[1,2-*b*]pyridazin-8-amine
*N*⁶-(*trans*-4-aminocyclohexyl)-N⁸-(2-phenylethyl)imidazo[1,2-*b*]pyridazine-6,8-diamine;
*N*-(4-(ethyloxy)phenyl)-6-(1-piperazinyl)imidazo[1,2-*b*]pyridazin-8-amine
*N*⁶-(2-(dimethylamino)ethyl)-*N*⁸-(4-(ethyloxy)phenyl)imidazo[1,2-*b*]pyridazine-6,8-diamine;
*N*⁸-(4-(ethyloxy)phenyl)-*N*⁶-(2-furanylmethyl)imidazo[1,2-*b*]pyridazine-6,8-diamine;
*N*-(4-(ethyloxy)phenyl)-6-(4-methyl-1,4-diazepan-1-yl)imidazo[1,2-*b*]pyridazin-8-amine ;
2-((6-((*trans*-4-aminocyclohexyl)amino)imidazo[1,2-*b*]pyridazin-8-yl)amino)phenol ;
*N*⁶-(trans-4-aminocyclohexyl)-*N*⁸-(3-((phenylmethyl)oxy)phenyl)imidazo[1,2-*b*]pyridazine-6,8-diamine;
3-((6-((*trans*-4-aminocyclohexyl)amino)imidazo[1,2*-b*]pyridazin-8-yl)amino)phenol ;
4-((6-((*trans*-4-aminocyclohexyl)amino)imidazo[1,2-*b*]pyridazin-8-yl)amino)phenol ;
*N*⁶-(*trans*-4-aminocyclohexyl)imidazo[1,2-*b*]pyridazine-6,8-diamine;
3-((6-((*trans*-4-aminocyclohexyl)amino)imidazo[1,2-*b*]pyridazin-8-yl)amino)benzonitrile ;
4-((6-((*trans*-4-aminocyclohexyl)amino)imidazo[1,2-*b*]pyridazin-8-yl)amino)benzonitrile ;
3-((6-((*trans*-4-aminocyclohexyl)amino)imidazo[1,2-*b*]pyridazin-8-yl)amino)benzoic acid ;
*N*⁶-(*trans*-4-aminocyclohexyl)-*N*⁸-1*H*-pyrazol-3-ylimidazo[1,2-*b*]pyridazine-6,8-diamine;
4-((6-((*trans*-4-aminocyclohexyl)amino)imidazo[1,2-*b*]pyridazin-8-yl)amino)benzoic acid ;
4-((6-((*trans*-4-aminocyclohexyl)amino)imidazo[1,2-*b*]pyridazin-8-yl)amino)-*N,N-*dimethylbenzenesulfonamide ;
*N*⁶-(*trans*-4-aminocyclohexyl)-N⁸-(4-(1*H*-tetrazol-5-yl)phenyl)imidazo[1,2-*b*]pyridazine-6,8-diamine; ;
*N*⁶-(*trans*-4-(ethylamino)cyclohexyl)-*N*⁸-phenylimidazo[1,2-*b*]pyridazine-6,8-diamine;
*N*⁶-(*trans*-4-(methylamino)cyclohexyl)-*N*⁸-phenylimidazo[1,2-*b*]pyridazine-6,8-diamine;
*N*⁶-(*trans*-4-aminocyclohexyl)-*N*⁸-(2-(phenyloxy)phenyl)imidazo[1,2-*b*]pyridazine-6,8-diamine;
*N*⁶-(*trans*-4-aminocyclohexyl)-*N*⁸-(4'-chloro-4-biphenylyl)imidazo[1,2-*b*]pyridazine-6,8-diamine;
*N*⁶-(*trans*-4-aminocyclohexyl)-*N*⁸-(2'-methyl-4-biphenylyl)imidazo[1,2-*b*]pyridazine-6,8-diamine;
*N*⁶-(*trans*-4-aminocyclohexyl)*-N*⁸-(3'-chloro-4-biphenylyl)imidazo[1,2-*b*]pyridazine-6,8-diamine;
*N*⁶-(*trans*-4-aminocyclohexyl)-*N*⁸-(4-(phenylmethyl)phenyl)imidazo[1,2-*b*]pyridazine-6,8-diamine;
*N*⁶-(*trans*-4-aminocyclohexyl)-*N*⁸-(4-(4-morpholinyl)phenyl)imidazo[1,2-*b*]pyridazine-6,8-diamine;
*N*⁶-(*trans*-4-aminocyclohexyl)-*N*⁸-(3'-chloro-3-biphenylyl)imidazo[1,2-*b*]pyridazine-6,8-diamine;
*N*⁶-(*trans*-4-aminocyclohexyl)-*N*⁸-(4-(1-methylethyl)phenyl)imidazo[1,2-*b*]pyridazine-6,8-diamine;
*N*⁶-(*trans*-4-aminocyclohexyl)-*N*⁸-(4-butylphenyl)imidazo[1,2-*b*]pyridazine-6,8-diamine;
*N*⁶-(*trans*-4-aminocyclohexyl)-*N*⁸-(5,6,7,8-tetrahydro-1-naphthalenyl)imidazo[1,2-*b*]pyridazine-6,8-diamine;
*N*⁶-(*trans*-4-aminocyclohexyl)-*N*⁸-1-naphthalenylimidazo[1,2-*b*]pyridazine-6,8-diamine;
*N*⁶-(*trans*-4-aminocyclohexyl)-*N*⁸-(3-(phenylmethyl)phenyl)imidazo[1,2-*b*]pyridazine-6,8-diamine;
*N*⁶-(*trans*-4-aminocyclohexyl)-*N*⁸-(4-propylphenyl)imidazo[1,2-*b*]pyridazine-6,8-diamine;
*N*⁶-(trans-4-aminocyclohexyl)-*N*⁸-(4'-methyl-4-biphenylyl)imidazo[1,2-*b*]pyridazine-6,8-diamine;
*N*⁶-(trans-4-aminocyclohexyl)-*N*⁸-(3-(1-methylethyl)phenyl)imidazo[1,2-*b*]pyridazine-6,8-diamine;
*N*⁶-(trans-4-aminocyclohexyl)-*N*⁸-(3-((1-methylethyl)oxy)phenyl)imidazo[1,2-*b*]pyridazine-6,8-diamine;
*N*⁶-(*trans*-4-aminocyclohexyl)-*N*⁸-(3,5-bis(methyloxy)phenyl)imidazo[1,2-*b*]pyridazine-6,8-diamine;
*trans*-*N*-(8-(6-methyl-3,4-dihydro-1(2*H*)-quinolinyl)imidazo[1,2-*b*]pyridazin-6-yl)-1,4-cyclohexanediamine;
*N*⁶-(*trans*-4-aminocyclohexyl)-*N*⁸-2-naphthalenylimidazo[1,2-*b*]pyridazine-6,8-diamine;
*N*⁶-(*trans*-4-aminocyclohexyl)-*N*⁸-(3-(methylsulfanyl)phenyl)imidazo[1,2-b]pyridazine-6,8-diamine;
*N*⁶-(*trans*-4-aminocyclohexyl)-*N*⁸-(3-ethylphenyl)imidazo[1,2-*b*]pyridazine-6,8-diamine;
*N*⁶-(*trans*-4-aminocyclohexyl)-*N*⁸-(4-ethylphenyl)imidazo[1,2-*b*]pyridazine-6,8-diamine;
*N*⁶-(*trans*-4-aminocyclohexyl)-*N*⁸-(4-(methylsulfanyl)phenyl)imidazo[1,2-*b*]pyridazine-6,8-diamine;
*N*⁶-(*trans*-4-aminocyclohexyl)-*N*⁸-9*H*-fluoren-2-ylimidazo[1,2-*b*]pyridazine-6,8-diamine;
*N*⁶-(*trans*-4-aminocyclohexyl)-*N*⁸-(2-ethylphenyl)imidazo[1,2-*b*]pyridazine-6,8-diamine;
*N*⁶-(*trans*-4-aminocyclohexyl)-*N*⁸-(4-cyclohexylphenyl)imidazo[1,2-*b*]pyridazine-6,8-diamine;
*N*⁶-(*trans*-4-aminocyclohexyl)-*N*⁸-(4-(1,1-dimethylethyl)phenyl)imidazo[1,2-*b*]pyridazine-6,8-diamine;
*N*⁶-(*trans*-4-aminocyclohexyl)-*N*⁸-(3-(phenyloxy)phenyl)imidazo[1,2-*b*]pyridazine-6,8-diamine;
*N*⁶-(*trans*-4-aminocyclohexyl)-*N*⁸-3-biphenylylimidazo[1,2-*b*]pyridazine-6,8-diamine;
*N*⁶-(*trans*-4-aminocyclohexyl)-*N*⁸-(4-((1-methylethyl)oxy)phenyl)imidazo[1,2-*b*]pyridazine-6,8-diamine;
*N*⁶-(*trans*-4-aminocyclohexyl)-*N*⁸-(2-chlorophenyl)imidazo[1,2-*b*]pyridazine-6,8-diamine;
*N*⁶-(*trans*-4-aminocyclohexyl)-*N*⁸-(4-chlorophenyl)imidazo[1,2-*b*]pyridazine-6,8-diamine;
*N*⁶-(*trans*-4-aminocyclohexyl)-*N*⁸-(3-chlorophenyl)imidazo[1,2-*b*]pyridazine-6,8-diamine;
*N*⁶-(*trans*-4-aminocyclohexyl)-*N*⁸-(4-chloro-1-naphthalenyl)imidazo[1,2-*b*]pyridazine-6,8-diamine;
*N*⁶-(*trans*-4-aminocyclohexyl)-*N*⁸-3-quinolinylimidazo[1,2-*b*]pyridazine-6,8-diamine;
*N*⁶-(*trans*-4-aminocyclohexyl)-*N*⁸-(3,5-dichlorophenyl)imidazo[1,2-*b*]pyridazine-6,8-diamine;
*N*⁶-(*trans*-4-aminocyclohexyl)-*N*⁸-(3-(trifluoromethyl)phenyl)imidazo[1,2-*b*]pyridazine-6,8-diamine;
*N*⁶-(*trans*-4-aminocyclohexyl)-*N*⁸-(4-chloro-2-fluorophenyl)imidazo[1,2-*b*]pyridazine-6,8-diamine;
*N*⁶-(*trans*-4-aminocyclohexyl)-*N*⁸-(2-fluoro-5-methylphenyl)imidazo[1,2-*b*]pyridazine-6,8-diamine;
*N*⁶-(*trans*-4-aminocyclohexyl)-*N*⁸-(4-chloro-3-methylphenyl)imidazo[1,2-*b*]pyridazine-6,8-diamine;
*N*⁶-(*trans*-4-aminocyclohexyl)-*N*⁸-(5-phenyl-2-pyridinyl)imidazo[1,2-*b*]pyridazine-6,8-diamine;
*N*⁶-(*trans*-4-aminocyclohexyl)-*N*⁸-(3-fluoro-4-methylphenyl)imidazo[1,2-*b*]pyridazine-6,8-diamine;
*N*⁶-(*trans*-4-aminocyclohexyl)-*N*⁸-(2-methyl-4-pyridinyl)imidazo[1,2-*b*]pyridazine-6,8-diamine;
*N*⁶-(*trans*-4-aminocyclohexyl)-*N*⁸-(4-fluoro-3-methylphenyl)imidazo[1,2-*b*]pyridazine-6,8-diamine;
*N*⁶-(*trans*-4-aminocyclohexyl)-*N*⁸-(2-fluoro-4-methylphenyl)imidazo[1,2-*b*]pyridazine-6,8-diamine;
*N*⁶-(*trans*-4-aminocyclohexyl)-*N*⁸-(3-((trifluoromethyl)oxy)phenyl)imidazo[1,2-*b*]pyridazine-6,8-diamine;
*N*⁶-(*trans*-4-aminocyclohexyl)-*N*⁸-(4-methyl-3-(trifluoromethyl)phenyl)imidazo[1,2-*b*]pyridazine-6,8-diamine;
*N*⁶-(*trans*-4-aminocyclohexyl)-*N*⁸-(4-ethyl-2-pyridinyl)imidazo[1,2-*b*]pyridazine-6,8-diamine;
*N*⁶-(*trans*-4-aminocyclohexyl)-*N*⁸-(4-(1*H*-1,2,4-triazol-1-yl)phenyl)imidazo[1,2-*b*]pyridazine-6,8-diamine;
*N*⁶-(*trans*-4-aminocyclohexyl)-N⁸-(4-(1H-pyrrol-1-yl)phenyl)imidazo[1,2-*b*]pyridazine-6,8-diamine;
*N*⁶-(*trans*-4-aminocyclohexyl)-*N*⁸-(4-(4,5-dichloro-1*H*-imidazol-1-yl)phenyl)imidazo[1,2-*b*]pyridazine-6,8-diamine;
*N*⁶-(*trans*-4-aminocyclohexyl)-*N*⁸-(4-(1*H*-pyrazol-1-yl)phenyl)imidazo[1,2-*b*]pyridazine-6,8-diamine;
*N*⁶-(*trans*-4-aminocyclohexyl)-*N*⁸-(4-(3,5-dimethyl-1*H*-pyrazol-1-yl)phenyl)imidazo[1,2-*b*]pyridazine-6,8-diamine;
*N*⁶-(*trans*-4-aminocyclohexyl)-*N*⁸-(4-(4-methyl-4*H*-1,2,4-triazol-3-yl)phenyl)imidazo[1,2-*b*]pyridazine-6,8-diamine;
*N*⁶-(*trans*-4-aminocyclohexyl)-*N*⁸-(4-(1*H*-imidazol-1-yl)phenyl)imidazo[1,2-*b*]pyridazine-6,8-diamine;
*N*⁶-(*trans*-4-aminocyclohexyl)-*N*⁸-(4-(1-methyl-1*H*-imidazol-2-yl)phenyl)imidazo[1,2-*b*]pyridazine-6,8-diamine;
*N*⁶-(*trans*-4-aminocyclohexyl)-*N*⁸-(4-(2-methyl-1,3-thiazol-4-yl)phenyl)imidazo[1,2-*b*]pyridazine-6,8-diamine;
*N*⁶-(*trans*-4-aminocyclohexyl)*-*N⁸-(4-(5-methyl-2-furanyl)phenyl)imidazo[1,2-*b*]pyridazine-6,8-diamine;
*N*⁶-(*trans*-4-aminocyclohexyl)-*N*⁸-(4-(2-ethyl-2*H*-tetrazol-5-yl)phenyl)imidazo[1,2-*b*]pyridazine-6,8-diamine;
4-((6-((*trans*-4-aminocyclohexyl)amino)imidazo[1,2-*b*]pyridazin-8-yl)amino)-3-hydroxy-*N,N*-dimethylbenzenesulfonamide ;
*N*⁶-(*trans*-4-aminocyclohexyl)-*N*⁸-(2,3-dihydro-1*H*-inden-5-yl)imidazo[1,2-*b*]pyridazine-6,8-diamine;
3-((6-((*trans*-4-aminocyclohexyl)amino)imidazo[1,2-*b*]pyridazin-8-yl)amino)*-N,N-*dimethylbenzenesulfonamide ;
4-((6-((*trans*-4-aminocyclohexyl)amino)imidazo[1,2-*b*]pyridazin-8-yl)amino)-*N,N-*dimethylbenzamide ;
*N*⁶-(*trans*-4-((2-chloro-4-pyrimidinyl)amino)cyclohexyl)-*N*⁸-phenylimidazo[1,2-*b*]pyridazine-6,8-diamine;
*N*⁶-(3-aminocyclopentyl)-*N*⁸-phenylimidazo[1,2-*b*]pyridazine-6,8-diamine;
*N*⁶-(*trans*-4-aminocyclohexyl)-*N*⁸-(3-(4-morpholinylsulfonyl)phenyl)imidazo[1,2-*b*]pyridazine-6,8-diamine;
3-((6-((*trans*-4-aminocyclohexyl)amino)imidazo[1,2-*b*]pyridazin-8-yl)amino)-*N,N-*diethylbenzamide
3-((6-((*trans*-4-aminocyclohexyl)amino)imidazo[1,2-*b*]pyridazin-8-yl)amino)-*N-*methyl-*N*-phenylbenzenesulfonamide
4-((6-((*trans*-4-aminocyclohexyl)amino)imidazo[1,2-*b*]pyridazin-8-yl)amino)-2-hydroxy-*N,N*-dimethylbenzenesulfonamide
*N*⁶-(4-aminobicyclo[2.2.2]oct-1-yl)-N⁸-phenylimidazo[1,2-*b*]pyridazine-6,8-diamine;
*N*-(4-((6-((*trans*-4-aminocyclohexyl)amino)imidazo[1,2-*b*]pyridazin-8-yl)amino)phenyl)methanesulfonamide;
*N*⁶-(*trans*-4-aminocyclohexyl)-*N*⁸-(4-(3-(dimethylamino)-1-pyrrolidinyl)phenyl)imidazo[1,2-*b*]pyridazine-6,8-diamine;
*N*⁶-(*trans*-4-aminocyclohexyl)-*N*⁸-(4-(1-pyrrolidinylsulfonyl)phenyl)imidazo[1,2-*b*]pyridazine-6,8-diamine;
4-((6-((*trans*-4-aminocyclohexyl)amino)imidazo[1,2*-b*]pyridazin-8-yl)amino)benzenesulfonic acid;
4-((6-((*trans*-4-aminocyclohexyl)amino)imidazo[1,2-*b*]pyridazin-8-yl)amino)-*N,N-*diethylbenzenesulfonamide;
4-((6-((*trans*-4-aminocyclohexyl)amino)imidazo[1,2-*b*]pyridazin-8-yl)amino)-*N-*propylbenzenesulfonamide;
4-((6-((*trans*-4-aminocyclohexyl)amino)imidazo[1,2-*b*]pyridazin-8-yl)amino)-*N-*ethylbenzenesulfonamide;
4-((6-((*trans*-4-aminocyclohexyl)amino)imidazo[1,2*-b*]pyridazin-8-yl)amino)-*N-*methylbenzenesulfonamide;
N⁶-(*trans*-4-aminocyclohexyl)-*N*⁸-(4-aminophenyl)imidazo[1,2-*b*]pyridazine-6,8-diamine;
4-((6-((*trans*-4-aminocyclohexyl)amino)imidazo[1,2-*b*]pyridazin-8-yl)amino)benzenesulfonamide ;
3-((6-((*trans*-4-aminocyclohexyl)amino)imidazo[1,2-*b*]pyridazin-8-yl)amino)benzenesulfonamide ;
3-((6-((*trans*-4-aminocyclohexyl)amino)imidazo[1,2-*b*]pyridazin-8-yl)amino)benzamide ;
4-((6-((*trans*-4-aminocyclohexyl)amino)imidazo[1,2-*b*]pyridazin-8-yl)amino)benzamide ;
*N*⁶-(*trans*-4-aminocyclohexyl)-*N*⁸-(4-(aminomethyl)phenyl)imidazo[1,2-*b*]pyridazine-6,8-diamine;
6-((6-((*trans*-4-aminocyclohexyl)amino)imidazo[1,2-*b*]pyridazin-8-yl)amino)-1,2-dihydro-3*H*-indazol-3-one ;
*N*⁶-(*trans*-4-aminocyclohexyl)-*N*⁸-(3-(aminomethyl)phenyl)imidazo[1,2-*b*]pyridazine-6,8-diamine;
*N*⁶-(*trans*-4-aminocyclohexyl)-*N*⁸-(2,6-difluorophenyl)imidazo[1,2-*b*]pyridazine-6,8-diamine;
*N*⁶-(*trans*-4-aminocyclohexyl)-7-chloro-*N*⁸-[4-(ethyloxy)phenyl]imidazo[1,2-*b*]pyridazine-6,8-diamine;
*N*⁶-(3-aminopropyl)-*N*⁸-[4-(ethyloxy)phenyl]imidazo[1,2-*b*]pyridazine-6,8-diamine;
*N*⁶-(*trans*-4-aminocyclohexyl)-*N*⁸-(2-fluorophenyl)imidazo[1,2-*b*]pyridazine-6,8-diamine;
*N*⁶-(*trans*-4-aminocyclohexyl)-*N*⁸-(2,6-difluorophenyl)imidazo[1,2-*b*]pyridazine-6,8-diamine;
*N*⁶-(*trans*-4-aminocyclohexyl)-*N*⁸-(2,4-difluorophenyl)imidazo[1,2-*b*]pyridazine-6,8-diamine;
*N*⁶-(*trans*-4-aminocyclohexyl)-*N*⁸-(3-fluorophenyl)imidazo[1,2-*b*]pyridazine-6,8-diamine;
*N*⁶-(*trans*-4-aminocyclohexyl)-*N*⁸-(4-fluorophenyl)imidazo[1,2-*b*]pyridazine-6,8-diamine;
*N*⁶-(*trans*-4-aminocyclohexyl)-*N*⁸-(3,4-difluorophenyl)imidazo[1,2-*b*]pyridazine-6,8-diamine;
*N*⁶-(*trans*-4-aminocyclohexyl)-*N*⁸-(2,5-difluorophenyl)imidazo[1,2-*b*]pyridazine-6,8-diamine;
*N*⁶-(*trans*-4-aminocyclohexyl)-*N*⁸-(2,3-difluorophenyl)imidazo[1,2-*b*]pyridazine-6,8-diamine;
*N*⁶-(*trans*-4-aminocyclohexyl)-*N*⁸-(3,5-difluorophenyl)imidazo[1,2-*b*]pyridazine-6,8-diamine;
*N*⁶-(*trans*-4-aminocyclohexyl)-*N*⁸-(3-iodophenyl)imidazo[1,2-*b*]pyridazine-6,8-diamine;
*N*⁶-(*trans*-4-aminocyclohexyl)-*N*⁸-[4-(trifluoromethyl)phenyl]imidazo[1,2-*b*]pyridazine-6,8-diamine;
*N*⁶-(*trans*-4-aminocyclohexyl)-*N*⁸-pyridin-2-ylimidazo[1,2-*b*]pyridazine-6,8-diamine;
*N*⁶-(*trans*-4-aminocyclohexyl)-*N*⁸*-*(4-methylpyridin-2-yl)imidazo[1,2-*b*]pyridazine-6,8-diamine;
*N*⁶-(*trans*-4-aminocyclohexyl)-*N*⁸-(5-methylpyridin-2-yl)imidazo[1,2-*b*]pyridazine-6,8-diamine;
*N*⁶-(*trans*-4-aminocyclohexyl)-*N*⁸-(4,6-dimethylpyridin-2-yl)imidazo[1,2-*b*]pyridazine-6,8-diamine;
*N*⁶-(*trans*-4-aminocyclohexyl)-*N*⁸-pyrimidin-2-ylimidazo[1,2-*b*]pyridazine-6,8-diamine;
*N*⁶-(*trans*-4-aminocyclohexyl)-*N*⁸-[4-(ethyloxy)phenyl]-7-methylimidazo[1,2-*b*]pyridazine-6,8-diamine;
*N*⁶-(*trans*-4-aminocyclohexyl)-7-ethyl-*N*⁸-[4-(ethyloxy)phenyl]imidazo[1,2-*b*]pyridazine-6,8-diamine;
*N*⁶-(*trans*-4-aminocyclohexyl)-7-methyl-*N*⁸-phenylimidazo[1,2-*b*]pyridazine-6,8-diamine;
*N*⁶-(*trans*-4-aminocyclohexyl)-*N*⁸-(3,4-dimethylphenyl)-7-methylimidazo[1,2-*b*]pyridazine-6,8-diamine;
*N*⁶-(*trans*-4-aminocyclohexyl)-7-methyl-*N*⁸-(4-methylphenyl)imidazo[1,2-*b*]pyridazine-6,8-diamine;
*N*⁶-(*trans*-4-aminocyclohexyl)-7-methyl-*N*⁸-[3-(methyloxy)phenyl]imidazo[1,2-*b*]pyridazine-6,8-diamine;
*N*⁶-(*trans*-4-aminocyclohexyl)-*N*⁸-biphenyl-4-yl-7-methylimidazo[1,2-*b*]pyridazine-6,8-diamine;
*N*⁶-(*trans*-4-aminocyclohexyl)-7-methyl-*N*⁸-[4-(propyloxy)phenyl]imidazo[1,2-*b*]pyridazine-6,8-diamine;
4-((6-((*trans*-4-aminocyclohexyl)amino)-7-methylimidazo[1,2-*b*]pyridazin-8-yl)amino)benzoic acid;
4-((6-((4-aminocyclohexyl)amino)-7-methylimidazo[1,2-*b*]pyridazin-8-yl)amino)-*N,N-*dimethylbenzenesulfonamide;
*N*-(4-((6-((*trans*-4-aminocyclohexyl)amino)-7-methylimidazo[1,2-*b*]pyridazin-8-yl)amino)phenyl)-*N*-methylacetamide;
*N*⁶-(*trans*-4-aminocyclohexyl)-7-ethyl-*N*⁸-phenylimidazo[1,2-*b*]pyridazine-6,8-diamine;
*N*⁶-(*trans*-4-aminocyclohexyl)-7-chloro-*N*⁸-[4-(ethyloxy)phenyl]imidazo[1,2-*b*]pyridazine-6,8-diamine;
*N*⁸-[4-(ethyloxy)phenyl]-*N*⁶-piperidin-3-ylimidazo[1,2-*b*]pyridazine-6,8-diamine;
*N*⁸-[4-(ethyloxy)phenyl]-*N*⁶-pyrrolidin-3-ylimidazo[1,2-*b*]pyridazine-6,8-diamine;
*N*⁸-[4-(ethyloxy)phenyl]-*N*⁶-piperidin-4-ylimidazo[1,2-*b*]pyridazine-6,8-diamine;
6-(3-amino-1-piperidinyl)-*N*-(4-(ethyloxy)phenyl)imidazo[1,2-*b*]pyridazin-8-amine;
*N*⁸-(4-(ethyloxy)phenyl)-7-methyl-*N*⁶-3-piperidinylimidazo[1,2-*b*]pyridazine-6,8-diamine;
*N*⁸-phenyl-*N*⁶-3-piperidinylimidazo[1,2-*b*]pyridazine-6,8-diamine;
6-[(3*S*)-3-aminopyrrolidin-1-yl]-*N*-[4-(ethyloxy)phenyl]imidazo[1,2-*b*]pyridazin-8-amine;
*N*⁶-(*cis*-4-aminocyclohexyl)-*N*⁸-[4-(ethyloxy)phenyl]-7-methylimidazo[1,2-*b*]pyridazine-6,8-diamine;
*N*⁶-(*cis*-4-aminocyclohexyl)-*N*⁸-(5-methyl-2-pyridinyl)imidazo[1,2-*b*]pyridazine-6,8-diamine;
*N*⁶-(*cis*-4-aminocyclohexyl)-*N*⁸-phenylimidazo[1,2-*b*]pyridazine-6,8-diamine;
*N*⁶-(*cis*-4-aminocyclohexyl)-7-methyl-*N*⁸-phenylimidazo[1,2-*b*]pyridazine-6,8-diamine;
*N*⁶-(*cis*-4-aminocyclohexyl)-*N*⁸-(4-(ethyloxy)phenyl)imidazo[1,2-*b*] pyridazine-6,8-diamine;
*N*⁶-(*cis*-4-aminocyclohexyl)-*N*⁸-(3,4-dimethylphenyl)imidazo[1,2-*b*]pyridazine-6,8-diamine;
*N*⁶-(*cis*-4-aminocyclohexyl)-*N*⁸-(5-phenyl-2-pyridinyl)imidazo[1,2-*b*]pyridazine-6,8-diamine;
4-((6-((*cis*-4-aminocyclohexyl)amino)imidazo[1,2-*b*]pyridazin-8-yl)amino)-*N,N-*dimethylbenzenesulfonamide;
*N*⁶-(*cis*-4-aminocyclohexyl)-*N*⁸-(4-methylphenyl)imidazo[1,2-*b*]pyridazine-6,8-diamine;
*N*⁶-(*cis*-4-aminocyclohexyl)-*N*⁸-(4,6-dimethyl-2-pyridinyl)imidazo[1,2-*b*]pyridazine-6,8-diamine;
*N*⁶-(*cis*-4-aminocyclohexyl)-*N*⁸-(4-(1*H*-pyrazol-1-yl)phenyl)imidazo[1,2-*b*]pyridazine-6,8-diamine;
*N*⁶-(*cis*-4-aminocyclohexyl)-*N*⁸-(4-(4-morpholinylcarbonyl)phenyl)imidazo[1,2-*b*]pyridazine-6,8-diamine;
*N*⁶-(*trans*-4-aminocyclohexyl)-7-methyl-*N*⁸-(2-methylphenyl)imidazo[1,2-*b*]pyridazine-6,8-diamine;
*N*⁶-(*trans*-4-aminocyclohexyl)-*N*⁸-(2-fluorophenyl)-7-methylimidazo[1,2-*b*]pyridazine-6,8-diamine;
*N*⁶-(*cis*-4-aminocyclohexyl)-7-methyl-*N*⁸-(2-methylphenyl)imidazo[1,2-*b*]pyridazine-6,8-diamine;
*N*⁶-(*cis*-4-aminocyclohexyl)-*N*⁸-(2-fluorophenyl)-7-methylimidazo[1,2-*b*]pyridazine-6,8-diamine;
*N*-(6-((*trans*-4-aminocyclohexyl)amino)imidazo[1,2-*b*]pyridazin-8-yl)benzamide;
1-(6-((trans-4-aminocyclohexyl)amino)imidazo[1,2-*b*]pyridazin-8-yl)-3-phenylurea;
*N,N'*-bis(4-trans-aminocyclohexyl)imidazo[1,2-*b*]pyridazine-6,8-diamine;
*N*⁶-(*trans*-4-aminocyclohexyl)-*N*⁸-(4-ethyloxyphenyl)-7-phenylimidazo[1,2-*b*]pyridazine-6,8-diamine;
*N*⁶-(*trans*-4-aminocyclohexyl)-*N*⁸-(phenyl)-7-phenylimidazo[1,2-*b*]pyridazine-6,8-diamine;
*N*⁶-(*cis*-4-aminocyclohexyl)-*N*⁸-(phenyl)-7-phenylimidazo[1,2-*b*]pyridazine-6,8-diamine;
*N*⁶-(*cis*-4-aminocyclohexyl)-*N*⁸-(4-ethoxyphenyl)-7-phenylimidazo[1,2-*b*]pyridazine-6,8-diamine;
4-((6-((*trans*-4-aminocyclohexyl)amino)imidazo[1,2-*b*]pyridazin-8-yl)amino)-*N*-(2-(4-pyridinyl)ethyl)benzamide
4-((6-((*trans*-4-aminocyclohexyl)amino)imidazo[1,2-*b*]pyridazin-8-yl)amino)-*N*-(2-furanylmethyl)benzamide;
4-((6-((*trans*-4-aminocyclohexyl)amino)imidazo[1,2-*b*]pyridazin-8-yl)amino)-*N*-(1*H-*imidazol-4-ylmethyl)benzamide;
4-((6-((*trans*-4-aminocyclohexyl)amino)imidazo[1,2-*b*]pyridazin-8-yl)amino)-*N*-(3-pyridinylmethyl)benzamide;
*N*⁶-(*trans-4-amin*ocyclohexyl)-*N*⁸-(4-((4-phenyl-1-piperidinyl)carbonyl)phenyl)imidazo[1,2-*b*]pyridazine-6,8-diamine;
*N*⁶-(*trans*-4-aminocyclohexyl)-*N*⁸-(4-(1-pyrrolidinylcarbonyl)phenyl)imidazo[1,2-*b*]pyridazine-6,8-diamine;
*N*⁶-(*trans*-4-aminocyclohexyl)-N⁸-(4-(1-piperidinylcarbonyl)phenyl)imidazo[1,2-*b*]pyridazine-6,8-diamine;
4-((6-((*trans*-4-aminocyclohexyl)amino)imidazo[1,2-*b*]pyridazin-8-yl)amino)-*N-*(phenylmethyl)benzamide;
4-((6-((*trans*-4-aminocyclohexyl)amino)imidazo[1,2*-b*]pyridazin-8-yl)amino)-*N*-(3-(methyloxy)phenyl)benzamide;
*N*⁶-(*trans*-4-aminocyclohexyl)-N⁸-(4-((3-phenyl-1 - pyrrolidinyl)carbonyl)phenyl)imidazo[1,2-*b*]pyridazine-6,8-diamine;
3-((6-((*trans*-4-aminocyclohexyl)amino)imidazo[1,2-*b*]pyridazin-8-yl)amino)-*N-*phenylbenzamide;
4-((6-((*trans*-4-aminocyclohexyl)amino)imidazo[1,2-b]pyridazin-8-yl)amino)-*N*-(2-hydroxyethyl)benzamide;
4-((6-((*trans*-4-aminocyclohexyl)amino)imidazo[1,2-b]pyridazin-8-yl)amino)-*N*-(3-(2-oxo-1-pyrrolidinyl)propyl)benzamide;
*N*⁶-(*trans*-4-aminocyclohexyl)-N⁸-(4-(4-morpholinylcarbonyl)phenyl)imidazo[1,2-*b*]pyridazine-6,8-diamine;
4-((6-((*trans*-4-aminocyclohexyl)amino)imidazo[1,2-*b*]pyridazin-8-yl)amino)-*N-*methyl-*N*-(2-(2-pyridinyl)ethyl)benzamide;
4-((6-((*trans*-4-aminocyclohexyl)amino)imidazo[1,2-*b*]pyridazin-8-yl)amino)-*N,N-*diethylbenzamide;
4-((6-((*trans*-4-aminocyclohexyl)amino)imidazo[1,2-*b*]pyridazin-8-yl)amino)-*N-*cyclopropylbenzamide;
4-((6-((*trans*-4-aminocyclohexyl)amino)imidazo[1,2-*b*]pyridazin-8-yl)amino)-*N-*(cyclohexylmethyl)benzamide;
4-((6-((*trans*-4-aminocyclohexyl)amino)imidazo[1,2-*b*]pyridazin-8-yl)amino)-*N*-3-pyridinylbenzamide;
4-((6-((*trans*-4-aminocyclohexyl)amino)imidazo[1,2-*b*]pyridazin-8-yl)amino)-*N*-(1-methyl-1*H*-pyrazol-5-yl)benzamide;
4-((6-((*trans*-4-aminocyclohexyl)amino)imidazo[1,2-*b*]pyridazin-8-yl)amino)-*N*-(1-(4-fluorophenyl)-2-oxo-1,2-dihydro-3-pyridinyl)benzamide;
4-((6-((*trans*-4-aminocyclohexyl)amino)imidazo[1,2-*b*]pyridazin-8-yl)amino)-*N-*phenylbenzamide;
4-((6-((*trans*-4-aminocyclohexyl)amino)imidazo[1,2-*b*]pyridazin-8-yl)amino)-*N-*cyclohexylbenzamide;
4-((6-((*trans*-4-aminocyclohexyl)amino)imidazo[1,2-*b*]pyridazin-8-yl)amino)-N-(4-pyridinylmethyl)benzamide;
1-(4-((6-((*trans*-4-aminocyclohexyl)amino)imidazo[1,2-*b*]pyridazin-8-yl)amino)phenyl)-3-phenylurea;
1-(4-((6-((*cis*-4-aminocyclohexyl)amino)-7-methylimidazo[1,2-*b*]pyridazin-8-yl)amino)phenyl)-3-phenylurea;
1-(4-((6-((*trans*-4-aminocyclohexyl)amino)-7-methylimidazo[1,2-*b*]pyridazin-8-yl)amino)phenyl)-3-phenylurea;
*N*-(4-((6-((*trans*-4-aminocyclohexyl)amino)imidazo[1,2-*b*]pyridazin-8-yl)amino)phenyl)-1-(4-fluorophenyl)-2-oxo-1,2-dihydro-3-pyridinecarboxamide;
*N*-(4-((6-((*trans*-4-aminocyclohexyl)amino)imidazo[1,2-*b*]pyridazin-8-yl)amino)-phenyl)-1-(4-fluorophenyl)-2-oxo-1,2-dihydro-3-pyridinecarboxamide;
*N*-(4-((6-((trans-4-aminocyclohexyl)amino)imidazo[1,2-*b*]pyridazin-8-yl)amino)-2-ethylphenyl)-1-(4-fluorophenyl)-2-oxo-1,2-dihydro-3-pyridinecarboxamide;
*N*-(4-((6-((*trans*-4-aminocyclohexyl)amino)imidazo[1,2-*b*]pyridazin-8-yl)amino)phenyl)benzamide;
*N*-(4-((6-((*trans*-4-aminocyclohexyl)amino)imidazo[1,2-*b*]pyridazin-8-yl)amino)phenyl)acetamide;
3-((6-((*trans*-4-aminocyclohexyl)amino)-7-methylimidazo[1,2-*b*]pyridazin-8-yl)amino)phenol;
*N*-(4-((6-chloroimidazo[1,2-*b*]pyridazin-8-yl)amino)phenyl)-1-(4-fluorophenyl)-2-oxo-1,2-dihydro-3-pyridinecarboxamide;
1-(4-fluorophenyl)-*N*-(4-(imidazo[1,2-*b*]pyridazin-8-ytamino)phenyl)-2-oxo-3-piperidinecarboxamide;
1-(4-fluorophenyl)-*N*-(4-(imidazo[1,2-*b*]pyridazin-8-ylamino)phenyl)-2-oxo-1,2-dihydro-3-pyridinecarboxamide;
6-((*trans*-4-aminocyclohexyl)amino)-8-((4-(ethyloxy)phenyl)amino)imidazo[1,2-*b*]pyridazine-3-carbonitrile;
6-((*trans*-4-aminocyclohexyl)amino)-8-(phenylamino)imidazo[1,2-*b*]pyridazine-3 - carbonitrile ;
6-((4-*trans*-aminocyclohexyl)amino)-7-methyl-8-(phenylamino)imidazo[1,2-*b*]pyridazine-3-carbonitrile;
6-((*trans*)-4-aminocyclohexylamino)-7-ethyl-8-(phenylamino)imidazo[ 1,2-*b*]pyridazine-3-carbonitrile;
6-((*trans*-4-aminocyclohexyl)amino)-8-anilino-7-isopropylimidazo[1,2-*b*]pyridazine-3-carbonitrile ;
*N*⁶-(*trans*-4-aminocyclohexyl)-*N*⁸-(4-(ethyloxy)phenyl)-3-fluoroimidazo[1,2-*b*]pyridazine-6,8-diamine;
*N*⁶-(*trans*-4-aminocyclohexyl)-*N*⁸-(4-(ethyloxy)phenyl)-3-methylimidazo[1,2-*b*]pyridazine-6,8-diamine;
*N*⁶-(*trans*-4-aminocyclohexyl)-*N*⁸-(4-(ethyloxy)phenyl)-2,3-dimethylimidazo[1,2-*b*]pyridazine-6,8-diamine;
*N*-(6-((*trans*-4-aminocyclohexyl)amino)imidazo[1,2-*b*]pyridazin-8-yl)benzenesulfonamide;
6-((*trans*-4-aminocyclohexyl)oxy)-8-anilinoimidazo[1,2-*b*]pyridazine-3-carbonitrile;
6-((*trans*-4-aminocyclohexyl)amino)-8-anilinoimidazo[1,2-*b*]pyridazine-3-carboxamide ;
*N*⁶-(*trans*-4-aminocyclohexyl)-7-ethyl-*N*⁸-phenylimidazo[1,2-*b*]pyridazine-6,8-diamine; and
*N*⁶-(*trans*-4-aminocyclohexyl)-7-ethyl-*N*⁸-[4-(ethyloxy)phenyl]imidazo[1,2-*b*]pyridazine-6,8-diamine;
ii) or an enantiomer, diastereomer, or a pharmaceutically-acceptable salt of (i), thereof.

15. A pharmaceutical composition comprising one or more compounds according claim 1 or 14 and a pharmaceutically acceptable carrier or diluent.

16. A compound according to claim 1 or 14 for use in the treatment, in a mammal, of pancreatitis (acute or chronic), asthma, allergies, adult respiratory distress syndrome, chronic obstructive pulmonary disease, glomerulonephritis, rheumatoid arthritis, systemic lupus erythematosis, scleroderma, chronic thyroiditis, Grave's disease, autoimmune gastritis, diabetes, autoimmune hemolytic anemia, autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, graft vs. host disease, inflammatory reaction induced by endotoxin, tuberculosis, atherosclerosis, muscle degeneration, cachexia, psoriatic arthritis, Reiter's syndrome, gout, traumatic arthritis, rubella arthritis, acute synovitis, pancreatic β-cell disease; diseases **characterized by** massive neutrophil infiltration; rheumatoid spondylitis, gouty arthritis and other arthritic conditions, cerebral malaria, chronic pulmonary inflammatory disease, silicosis, pulmonary sarcoisosis, bone resorption disease, allograft rejections, fever and myalgias due to infection, cachexia secondary to infection, meloid formation, scar tissue formation, ulcerative colitis, pyresis, influenza, osteoporosis, osteoarthritis, acute myelogenous leukemia, chronic myelogenous leukemia, metastatic melanoma, Kaposi's sarcoma, multiple myeloma, sepsis, septic shock, and Shigellosis; Alzheimer's disease, Parkinson's disease, cerebral ischemias or neurodegenerative disease caused by traumatic injury; angiogenic disorders including solid tumors, ocular neovasculization, and infantile haemangiomas; acute hepatitis infection (including hepatitis A, hepatitis B and hepatitis C), HIV infection and CMV retinitis, AIDS, ARC or malignancy, and herpes; stroke, myocardial ischemia, ischemia in stroke heart attacks, organ hyposia, vascular hyperplasia, cardiac and renal reperfusion injury, thrombosis, cardiac hypertrophy, thrombin-induced platelet aggregation, endotoxemia and/or toxic shock syndrome, conditions associated with prostaglandin endoperoxidase synthase-2, edema, analgesia, neuromuscular pain, headache, pain caused by cancer, dental pain, arthritis pain, equine infectious anemia virus; feline immunodeficiency virus, bovine immunodeficiency virus, canine immunodeficiency virus, and pemphigus vulgaris.

17. A compound for use in the treatment according to claim 16 wherein the condition is selected from Crohns and ulcerative colitis, allograft rejection, rheumatoid arthritis, psoriasis, ankylosing spondylitis, psoriatic arthritis, and pemphigus vulgaris.

18. A compound for use in the treatment according to claim 16 wherein the condition is selected from ischemia reperfusion injury, including cerebral ischemia reperfusions injury arising from stroke and cardiac ischemia reperfusion injury arising from myocardial infarction.

19. A compound for use in the treatment according to claim 16 wherein the condition is multiple myeloma.

## Patentansprüche

1. Verbindung gemäß Formel (I), 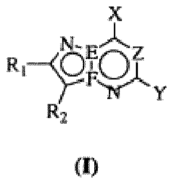 oder ein Enantiomer, Diastereomer oder ein pharmazeutisch annehmbares Salz davon, wobei:
E C ist; F N ist;
X NR₄R₅ ist;
Z CR₃ ist;
Y aus Wasserstoff, Halogen, Nitro, Cyano, SR₈, S(O)pR₈, OR₈, NR₆R₇, CO₂R₈, C(=O)R₈, O-C(=O)R₈, C(=O)NR₈R₉, Cycloalkyl, Cycloalkenyl, Cycloalkynyl, Heterocyclo, Aryl und Heteroaryl ausgewählt ist, vorausgesetzt dass, wenn Y Wasserstoff ist, R₄ Phenyl ist, substituiert mit einer Carboxamidogruppe;
R₁ und R₂ unabhängig aus (i) Wasserstoff, Alkyl, Halogen, Nitro, Cyano, SR₁₀, OR₁₀, NR₁₀R₁₁, NR₁₀C(=O)R₁₁, CO₂R₁₀, C(=O)R₁₀, -O-C(=O)R₁₀, C(=O)NR₁₀R₁₁ ausgewählt sind;
R₃ aus Wasserstoff, Halogen, Alkyl, substituiertem Alkyl, Alkenyl, substituiertem Alkenyl, Alkynyl, substituiertem Alkynyl, Nitro, Cyano, SR₁₃, OR₁₃, NR₁₃R₁₄, NR₁₃C(=O)R₁₄, CO₂R₁₃, C(=O)R₁₃, -O-C(=O)R₁₃, -C(=O)NR₁₃R₁₄, Cycloalkyl, Heterocyclo, Aryl und Heteroaryl ausgewählt ist;
R₄, R₅, R₆, und R₇ unabhängig aus Wasserstoff, Alkyl, substituiertem Alkyl, Alkenyl, substituiertem Alkenyl, Alkynyl, substituiertem Alkynyl, OR₁₅, SR₁₅, C(=O)R₁₅, CO₂R₁₅, C(=O)NR₁₅R₁₆, C(W)OR₁₆, S(O)ₚR₁₇, SO₂NR₁₅R₁₆, Cycloalkyl, Heterocyclo, Aryl und Heteroaryl ausgewählt sind; oder (ii) R₄ zusammen mit R₅ und dem Stickstoffatom, an das sie beide angefügt sind, genommen wird und/oder R₆ zusammen mit R₇ und dem Stickstoffatom, an das sie beide angefügt sind, genommen wird, um ein Heteroaryl oder Heterocyclo zu bilden;
R₈, R₉, R₁₀, R₁₁, R₁₃, R₁₄, R₁₅ und R₁₆ bei jedem Vorkommen unabhängig aus (i) Wasserstoff, Alkyl, substituiertem Alkyl, Alkenyl, substituiertem Alkenyl, Alkynyl, substituiertem Alkynyl, Cycloalkyl, Aryl, Heteroaryl und Heterocyclo ausgewählt sind; oder (ii) zusammen mit dem Stickstoffatom, an das sie angefügt sind, R₈ zusammen mit R₉ genommen wird und/oder R₁₀ zusammen mit R₁₁ genommen wird und/oder R₁₃ zusammen mit R₁₄ genommen wird und/oder R₁₅ zusammen mit R₁₆ genommen wird, um ein Heteroaryl oder Heterocyclo zu bilden;
R₁₇ aus Alkyl, substituiertem Alkyl, Alkenyl, substituiertem Alkenyl, Alkynyl, substituiertem Alkynyl, Cycloalkyl, Aryl, Heteroaryl und Heterocyclo ausgewählt ist;
W bei jedem Vorkommen O, S, N, CN oder NH ist; und
p 1 oder 2 ist,
mit den folgenden Bedingungen:
(1) wenn X NH(Me), NH(Me)₂, NH(nicht substituiertem Phenyl) oder NHNH₂ ist, dann Y anders als Wasserstoff oder Halogen ist; und
(2) die folgenden Verbindungen ausgeschlossen sind:

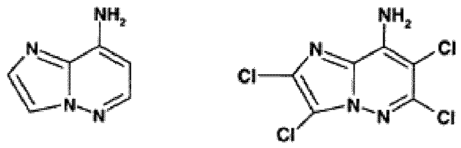

2. Verbindung nach Anspruch 1 mit Formel (Ia) 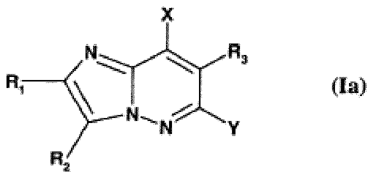 oder ein Enantiomer, Diastereomer oder ein pharmazeutisch annehmbares Salz davon.

3. Verbindung nach Anspruch 2 oder ein Enantiomer, Diastereomer oder ein pharmazeutisch annehmbares Salz davon, wobei:
R₄ -AM ist;
R₅ Wasserstoff oder C₁₋₄Alkyl ist;
oder R₄ und R₅ zusammen mit dem Stickstoffatom, an das sie angefügt sind, einen 5-, 6- oder 7-gliedrigen monocyclischen Heteroaryl- oder Heterocycloring oder einen 7-bis 11-gliedrigen bicyclischen Heteroaryl- oder Heterocycloring bilden, wobei jeder Ring optional mit einer bis drei Gruppen, T₁, T₂ und/oder T₃, substituiert ist;
A eine Bindung, C₁₋₃Alkylen, C₂₋₄Alkenylen, C₂₋₄Alkynylen, -C(O)- oder -SO₂-ist;
M (i) Wasserstoff, Alkyl, Alkoxy oder Alkenyl; oder (ii) Cycloalkyl, Heterocyclo, Aryl oder Heteroaryl ist, wobei jede Gruppe optional durch eine bis drei Gruppen, T₁, T₂ und/oder T₃, substituiert ist;
T₁, T₂ und T₃ unabhängig aus (i) Halogen, Alkyl, substituiertem Alkyl, Alkenyl, substituiertem Alkenyl, Alkynyl, substituiertem Alkynyl, Nitro, Cyano, SO₃H SR₁₉, S(O)*ₚ*R₂₁, S(O)*ₚ*NR₁₉R₂₀, NR₁₉S(O)*ₚ*R₂₁, OR₁₉, NR₁₉R₂₀, NR₁₉C(=O)₂₀, NR₁₉C(=O)NR₁₉R₂₀, CO₂R₁₉, C(=O)R₁₉, -O-C(=O)R₁₉, -C(=O)NR₁₉R₂₀, Cycloalkyl, Heterocyclo, Aryl und Heteroaryl ausgewählt sind, wobei *p* eins oder 2 ist; und/oder (ii) zwei Gruppen, T₁ und T₂, die sich an angrenzenden Ringatomen befinden, zusammen mit den Ringatomen, an die sie angefügt sind, genommen werden, um ein anelliertes Cycloalkyl, Aryl, Heteroaryl oder Heterocyclo zu bilden;
R₁₉, R₂₀ und R₂₁ bei jedem Vorkommen unabhängig aus (i) Wasserstoff, Alkyl, substituiertem Alkyl, Alkenyl, substituiertem Alkenyl, Cycloalkyl, Aryl, Heteroaryl und Heterocyclo ausgewählt sind; oder (ii) R₁₉ und R₂₀ zusammen mit dem Stickstoffatom, an das sie beide angefügt sind, ein Heteroaryl oder Heterocyclo bilden; und
R₂₁ bei jedem Vorkommen aus Alkyl, substituiertem Alkyl, Alkenyl, substituiertem Alkenyl, Alkynyl, substituiertem Alkynyl, Cycloalkyl, Aryl, Heteroaryl und Heterocyclo ausgewählt ist.

4. Verbindung nach Anspruch 2 oder ein Enantiomer, Diastereomer oder ein pharmazeutisch annehmbares Salz davon, wobei:
Y Wasserstoff, Halogen, OR₈ oder NR₆R₇ ist;
R₆ aus Wasserstoff oder C₁₋₄Alkyl ausgewählt ist, optional substituiert durch eine bis drei Gruppen, ausgewählt aus Halogen, C₁₋₄Alkyl, Nitro, Cyano, Amino, C₁₋₄Alkoxy und OH;
R₇ und R₈ unabhängig aus Alkyl, Cycloalkyl, Heterocyclo, Aryl und Heteroaryl ausgewählt sind, von denen jede Gruppe optional durch eine bis drei Gruppen, T₄, T₅ und/oder T₆, substituiert ist;
oder R₆ und R₇ zusammen mit dem Stickstoffatom, an das sie angefügt sind, einen Heteroaryl- oder Heterocycloring bilden, wobei jeder Ring optional durch eine bis drei Gruppen, T₄, T₅ und/oder T₆, substituiert ist;
T₄, T₅ und T₆ unabhängig aus (i) Halogen, Alkyl, substituiertem Alkyl, Alkenyl,
substituiertem Alkenyl, Alkynyl, substituiertem Alkynyl, Nitro, Cyano, SR₁₉, OR₁₉, NR₁₉R₂₀, NR₁₉C(=O)R₂₀, CO₂R₁₉, C(=O)R₁₉, -O-C(=O)R₁₉, -C(=O)NR₁₉R₂₀, Cycloalkyl, Heterocyclo, Aryl und Heteroaryl ausgewählt sind; und/oder (ii) zwei Gruppen, T₄ und T₅, die an angrenzenden Ringatomen substituiert sind, zusammen mit den Ringatomen, an die sie angefügt sind, genommen werden, um ein anelliertes Cyclalkyl, Heterocyclo, Aryl, oder Heteroaryl zu bilden; und R₁₉ und R₂₀ bei jedem Vorkommen unabhängig aus (i) Wasserstoff, Alkyl, substituiertem Alkyl, Alkenyl, substituiertem Alkenyl, Cycloalkyl, Aryl, Heteroaryl und Heterocyclo ausgewählt sind; oder (ii) R₁₉ mit R₂₀ zusammen mit dem Stickstoffatom, an das sie beide angefügt sind, kombiniert werden, um ein Heteroaryl oder Heterocyclo zu bilden.

5. Verbindung der Formel (Ia) 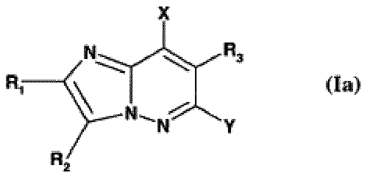 oder ein Enantiomer, Diastereomer oder ein pharmazeutisch annehmbares Salz davon, wobei:
X NR₄R₅ ist;
Y Wasserstoff, Halogen, OR₈ oder NR₆R₇ ist;
R₁ und R₂ unabhängig aus (i) Wasserstoff, Alkyl, Halogen, Nitro, Cyano, SR₁₀, OR₁₀, NR₁₀R₁₁, NR₁₀C(=O)R₁₁, CO₂R₁₀, C(=O)R₁₀, -O-C(=O)R₁₀, C(=O)NR₁₀R₁₁ ausgewählt sind;
R₃ aus Wasserstoff, Halogen, Alkyl, substituiertem Alkyl, Alkenyl, substituiertem Alkenyl, Alkynyl, substituiertem Alkynyl, Nitro, Cyano, SR₁₃, OR₁₃, NR₁₃R₁₄, NR₁₃C(=O)R₁₄, CO₂R₁₃, C(=O)R₁₃, -O-C(=O)R₁₃, -C(=O)NR₁₃R₁₄, Cycloalkyl, Heterocyclo, Aryl und Heteroaryl ausgewählt ist;
R₄ -AM ist;
R₅ Wasserstoff oder C₁₋₄Alkyl ist;
oder R₄ und R₅ zusammen mit dem Stickstoffatom, an das sie angefügt sind, einen 5-, 6- oder 7-gliedrigen monocyclischen Heteroaryl- oder Heterocycloring oder einen 7-bis 11-gliedrigen bicyclischen Heteroaryl- oder Heterocycloring bilden, wobei jeder Ring optional mit einer bis drei Gruppen, T₁, T₂ und/oder T₃, substituiert ist;
A eine Bindung, C₁₋₃Alkylen, C₂₋₄Alkenylen, C₂₋₄Alkynylen, -C(O)- oder -SO₂-ist; M (i) Wasserstoff, NR₁₅R₁₆, Alkyl, Alkoxy oder Alkenyl; oder (ii) Cycloalkyl, Heterocyclo, Aryl oder Heteroaryl ist, wobei jeder Ring optional durch eine bis drei Gruppen, T₁, T₂ und/oder T₃, substituiert ist;
R₆ aus Wasserstoff oder C₁₋₄Alkyl ausgewählt ist, optional substituiert durch eine bis drei Gruppen, ausgewählt aus Halogen, C₁₋₄Alkyl, Nitro, Cyano, Amino, C₁₋₄Alkoxy und OH;
R₇ aus Alkyl, Cycloalkyl, Heterocyclo, Aryl und Heteroaryl ausgewählt ist, von denen jede Gruppe optional durch eine bis drei Gruppen, T₄, T₅ und/oder T₆, substituiert ist;
oder R₆ und R₇ zusammen mit dem Stickstoffatom, an das sie angefügt sind, einen Heteroaryl- oder Heterocycloring bilden, wobei jeder Ring optional durch eine bis drei Gruppen, T₄, T₅ und/oder T₆, substituiert ist;
R₈ aus Alkyl, Cycloalkyl, Heterocyclo, Aryl und Heteroaryl ausgewählt ist, von denen jede Gruppe optional durch eine bis drei Gruppen, T₄, T₅ und/oder T₆, substituiert ist;
R₁₀, R₁₁, R₁₃ und R₁₄ bei jedem Vorkommen unabhängig aus (i) Wasserstoff, C₁₋₄Alkyl und substituiertem C₁₋₄Alkyl ausgewählt sind; oder (ii) R₁₀ und R₁₁ zusammen mit dem Stickstoffatom, an das sie beide angefügt sind, und/oder R₁₃ und R₁₄ zusammen mit dem Stickstoffatom, an das sie beide angefügt sind, kombiniert werden, um ein optional substituiertes 5-, 6- oder 7-gliedriges Heteroaryl oder Heterocyclo zu bilden;
R₁₅ und R₁₆ unabhängig aus (i) Wasserstoff, Alkyl, substituiertem Alkyl, Alkenyl, substituiertem Alkenyl, Alkynyl, substituiertem Alkynyl, Cycloalkyl, Aryl, Heteroaryl und Heterocyclo ausgewählt sind; oder (ii) zusammen mit dem Stickstoffatom, an das sie angefügt sind, R₁₅ zusammen mit R₁₆ genommen wird, um ein Heteroaryl oder Heterocyclo zu bilden;
T₁, T₂ und T₃ unabhängig aus (i) Halogen, Alkyl, substituiertem Alkyl, Alkenyl, substituiertem Alkenyl, Alkynyl, substituiertem Alkynyl, Nitro, Cyano, SO₃H SR₁₉, S(O)*ₚ*R₂₁, S(O)*ₚ*NR₁₉R₂₀, NR₁₉S(O)*ₚ*R₂₁, OR₁₉, NR₁₉R₂₀, NR₁₉C(=O)₂₀, NR₁₉C(=O)NR₁₉R₂₀, CO₂R₁₉, C(=O)R₁₉, -O-C(=O)R₁₉, -C(=O)NR₁₉R₂₀, Cycloalkyl, Heterocyclo, Aryl und Heteroaryl ausgewählt sind, wobei *p* eins oder 2 ist; und/oder (ii) zwei Gruppen, T₁ und T₂, die sich an angrenzenden Ringatomen befinden, zusammen mit den Ringatomen, an die sie angefügt sind, genommen werden, um ein anelliertes Cycloalkyl, Aryl, Heteroaryl oder Heterocyclo zu bilden;
T₄, T₅ und T₆ unabhängig aus (i) Halogen, Alkyl, substituiertem Alkyl, Alkenyl, substituiertem Alkenyl, Alkynyl, substituiertem Alkynyl, Nitro, Cyano, SR₁₉, OR₁₉, NR₁₉R₂₀, NR₁₉C(=O)R₂₀, CO₂R₁₉, C(=O)R₁₉, -O-C(=O)R₁₉, -C(=O)NR₁₉R₂₀, Cycloalkyl, Heterocyclo, Aryl und Heteroaryl ausgewählt sind; und/oder (ii) zwei Gruppen, T₄ und T₅, substituiert an angrenzenden Ringatomen, zusammen mit den Ringatomen, an die sie angefügt sind, genommen werden, um ein anelliertes Cycloalkyl, Heterocyclo, Aryl oder Heteroaryl zu bilden; und
R₁₉ und R₂₀ bei jedem Vorkommen unabhängig aus (i) Wasserstoff, Alkyl, substituiertem Alkyl, Alkenyl, substituiertem Alkenyl, Cycloalkyl, Aryl, Heteroaryl und Heterocyclo ausgewählt sind; oder (ii) R₁₉ und R₂₀ zusammen mit dem Stickstoffatom, an das sie beide angefügt sind, einen Heteroaryl- oder Heterocycloring bilden; und
R₂₁ bei jedem Vorkommen aus Alkyl, substituiertem Alkyl, Alkenyl, substituiertem Alkenyl, Alkynyl, substituiertem Alkynyl, Cycloalkyl, Aryl, Heteroaryl und Heterocyclo ausgewählt ist;
mit den folgenden Bedingungen:
(1) wenn X NH(Me), NH(Me)₂, NH(nicht substituiertes Phenyl) oder NHNH₂ ist, dann Y anders als Wasserstoff oder Halogen ist; und
(2) die folgenden Verbindungen ausgeschlossen sind:

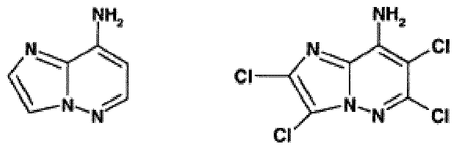

6. Verbindung nach Anspruch 5 oder ein Enantiomer, Diastereomer oder ein pharmazeutisch annehmbares Salz davon, wobei:
R₄ -AM ist;
A eine Bindung, -C(O)-, or -S(O)₂- oder C₁₋₃Alkylen ist;
M (i) Wasserstoff, -NH(Aryl), C₁₋₆Alkyl, C₂₋₄Alkenyl oder -OC₁₋₄Alkyl oder (ii) C₃₋₆Cycloalkyl, Phenyl, Fluorenyl, 1-Naphthyl oder 2-Naphthyl ist, wobei jede Gruppe optional durch eine bis drei Gruppen, T₁, T₂ und/oder T₃, substituiert ist; oder (iii) ein 5-, 6- oder 7-gliedriger monocyclischer oder ein 7- bis 11-gliedriger bicyclischer Heteroaryl- oder Heterocycloring ist, wobei jeder Ring optional durch eine bis drei Gruppen, T₁, T₂ und/oder T₃, substituiert ist; und
T₁, T₂ und T₃ unabhängig ausgewählt sind aus (i) C₁₋₄Alkyl, substituiertem C₁₋₄Alkyl, C₁₋₄Alkyloxy, substituiertem C₁₋₄Alkyloxy, C₁₋₄Alkylthio, Phenoxy, -NR₁₉R₂₀, Halogen, Hydroxy, Cyano, SO₃H, COOH, -C(O)(R₁₉), C(O)NR₁₉R₂₀, NR₁₉C(O)R₂₀, S(O)₂R₂₁, S(O)₂NR₁₉R₂₀ und NR₁₉(C(O)NR₁₉R₂₀; und/oder (ii) Phenyl, Cyclopropyl, Cyclohexyl, Tetrazolyl, Imidazolyl, Pyrazolyl, Triazolyl, Thiazolyl, Furyl und Morpholinyl, wobei jede Gruppe optional, wie die Valenz gestattet, von einer bis drei Gruppen, R₂₂, R₂₃ und/oder R₂₄, substituiert ist; und/oder (iii) zwei Gruppen, T₁ und T₂, die an angrenzenden Ringatomen substituiert sind, zusammen mit den Ringatomen, an die sie angefügt sind, genommen werden, um ein anelliertes fünf- bis siebengliedriges Cycloalkyl, ein anelliertes Phenyl oder ein anelliertes 5- oder 6-gliedriges Heterocyclo oder Heteroaryl zu bilden, von denen jede Gruppe optional, wie es die Valenz gestattet, von einer bis drei Gruppen, R₂₂, R₂₃ und/oder R₂₄, substituiert ist; und
R₁₉ und R₂₀ bei jedem Vorkommen unabhängig ausgewählt sind aus (i) Wasserstoff, -(CH₂)ᵥOH und C₁₋₄Alkyl; oder (ii) -(CH₂)ᵥCyclohexyl, -(CH₂)ᵥPhenyl, - (CH₂)ᵥMorpholinyl, -(CH₂)ᵥPyridyl, -(CH₂)ᵥPyrazolyl, -(CH₂)ᵥCyclopropyl, - (CH₂)ᵥPyrrolidinyl, -(CH₂)ᵥPiperidinyl, -(CH₂)ᵥFuryl, -(CH₂)ᵥImidazolyl, - (CH₂)ᵥPyrimidinyl, -(CH₂)ᵥPiperazinyl und -(CH₂)ᵥPyradizinyl, von denen jede Gruppe, optional, wie es die Valenz gestattet, von einer bis drei Gruppen, R₂₂, R₂₃ und/oder R₂₄, substituiert ist; oder R₁₉ und R₂₀ zusammen mit dem Stickstoffatom, an das sie beide angefügt sind, genommen werden, um ein Pyrrolindyl, Morpholinyl, Piperidinyl, Pyradazinyl oder Piperazinyl zu bilden, von denen jede Gruppe, optional, wie es die Valenz gestattet, von einer bis drei Gruppen, R₂₂, R₂₃ und/oder R₂₄, substituiert ist;
R₂₁ bei jedem Vorkommen ausgewählt ist aus (i) -(CH₂)ᵥOH und C₁₋₄Alkyl; oder (ii) -(CH₂)ᵥCyclohexyl, -(CH₂)ᵥPhenyl, -(CH₂)ᵥMorpholinyl, -(CH₂)ᵥPyridyl, - (CH₂)ᵥPyrazolyl, -(CH₂)ᵥCyclopropyl, -(CH₂)ᵥPyrrolidinyl, -(CH₂)ᵥPiperidinyl, - (CH₂)ᵥFuryl, -(CH₂)ᵥImidazolyl, -(CH₂)ᵥPyrimidinyl, -(CH₂)ᵥPiperazinyl und - (CH₂)ᵥPyradizinyl, von denen jede Gruppe, optional, wie es die Valenz gestattet, von einer bis drei Gruppen, R₂₂, R₂₃ und/oder R₂₄, substituiert ist; R₂₂, R₂₃ und R₂₄ bei jedem Vorkommen unabhängig ausgewählt sind aus (C₁₋₄)Alkyl, (C₂₋₄)Alkenyl, Halogen, Hydroxy, Cyano, Nitro, CF₃, =O, O(C₁₋₄Alkyl), OCF₃, C(=O)H, C(=O)(C₁₋₄Alkyl), CO₂H, CO₂(C₁₋₄Alkyl), NHCO₂(C₁₋₄Alkyl), -S(C₁₋₄Alkyl), -NH₂, NH(C₁₋₄Alkyl), N(C₁₋₄Alkyl)₂, N(C₁₋₄Alkyl)₃⁺, SO₂(C₁₋₄Alkyl), C(=O)(C₁₋₄Alkylen)NH₂, C(=O)(C₁-₄Alkylen)NH(Alkyl), C(=O)(C₁₋₄Alkylen)N(C₁₋₄Alkyl)₂ und optional substituiertem Phenyl; und v 0, 1, 2 oder 3 ist.

7. Verbindung nach Anspruch 6 oder ein Enantiomer, Diastereomer oder ein pharmazeutisch annehmbares Salz davon, wobei NR₄R₅ aus dem Folgenden ausgewählt ist: 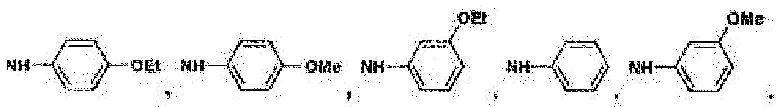 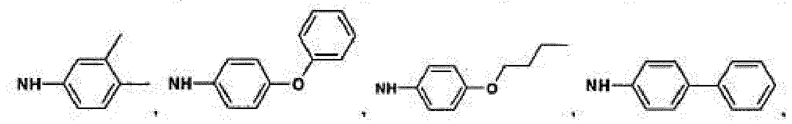 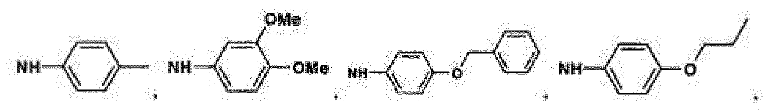 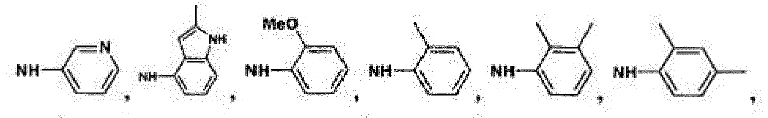 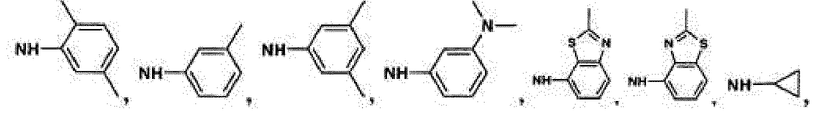 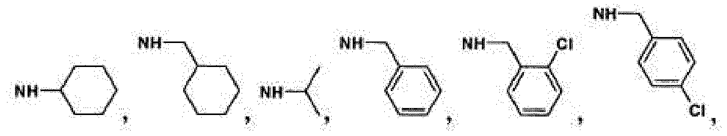 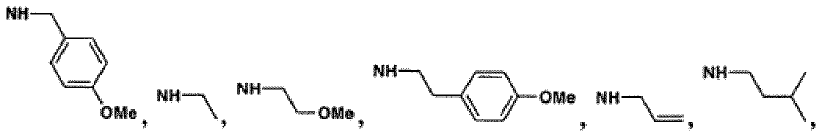 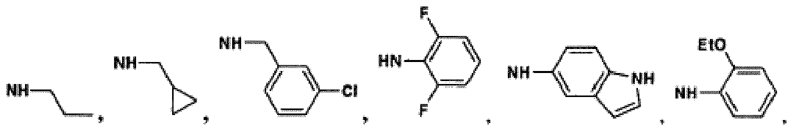 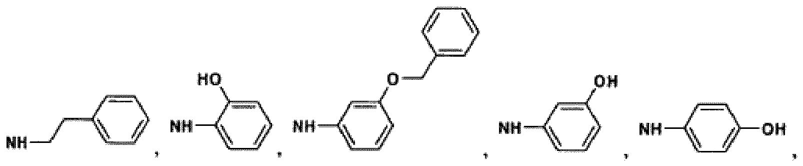 NH₂.

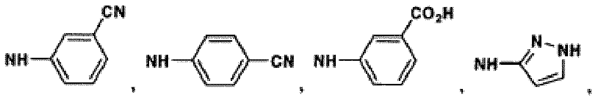

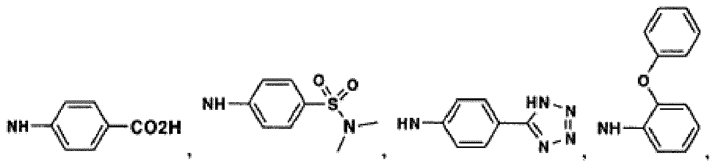

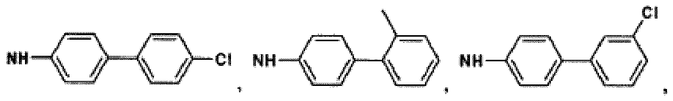

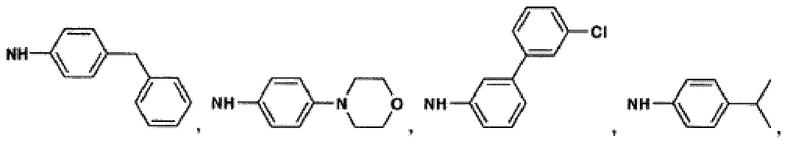

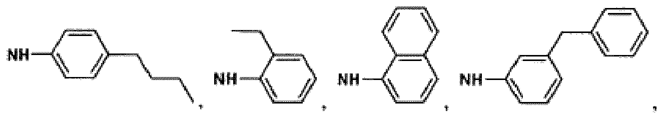

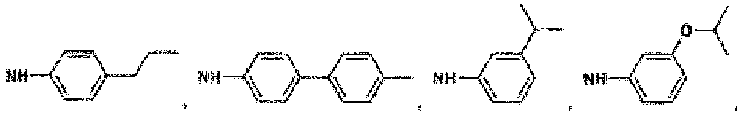

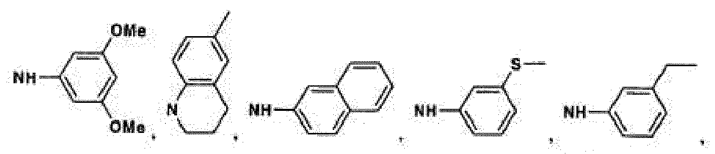

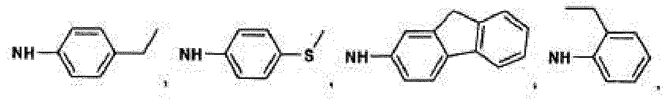

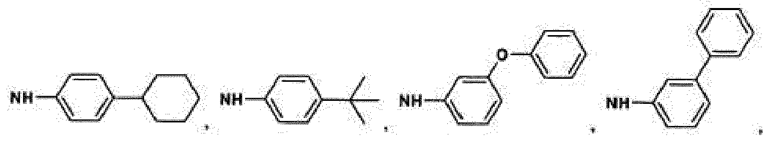

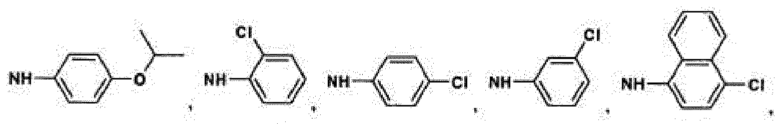

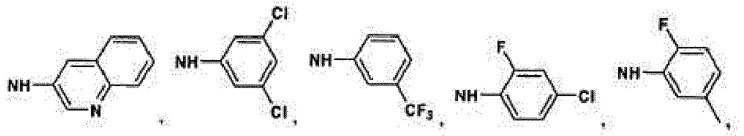

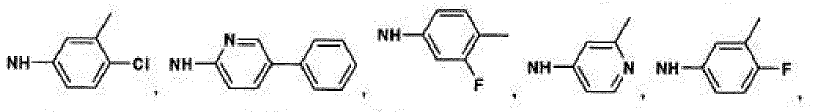

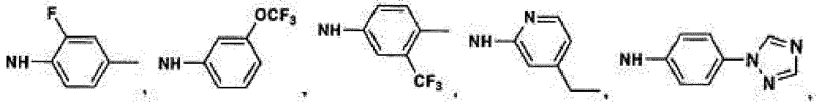

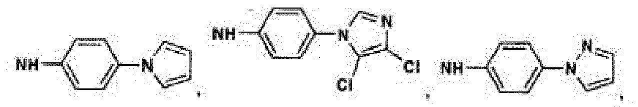

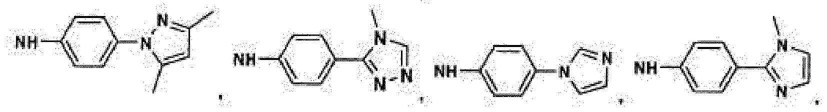

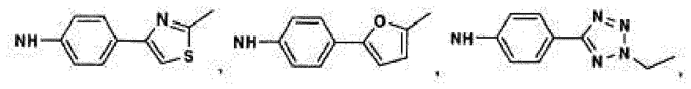

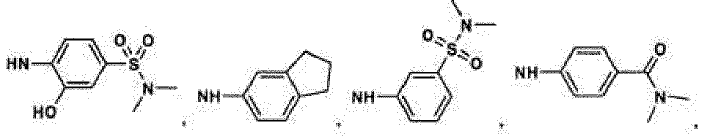

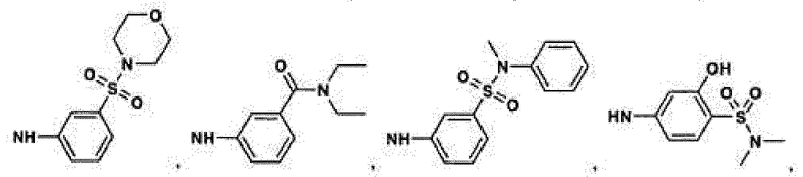

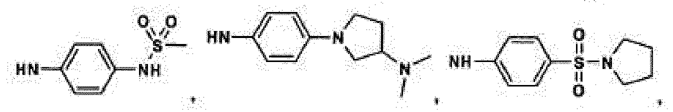

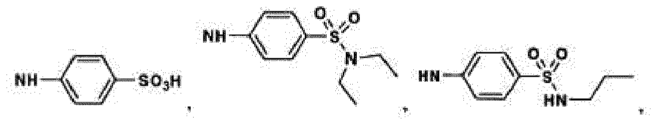

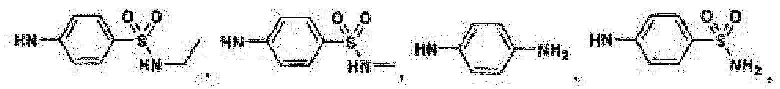

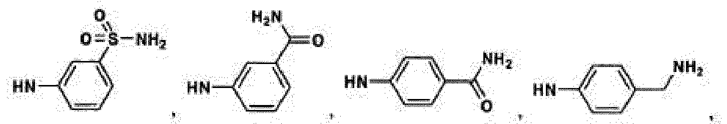

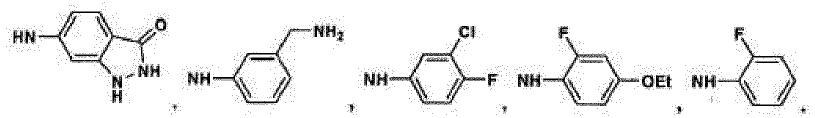

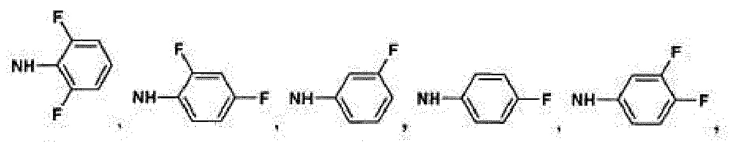

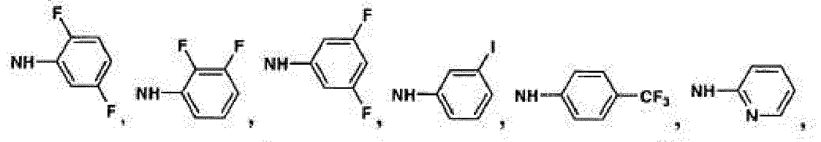

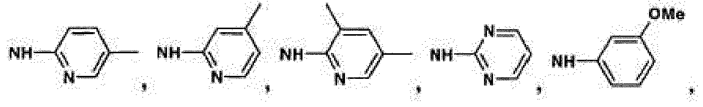

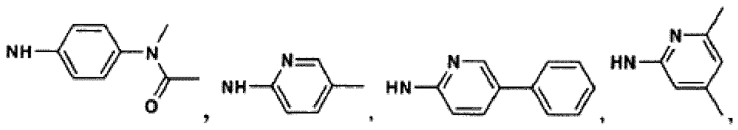

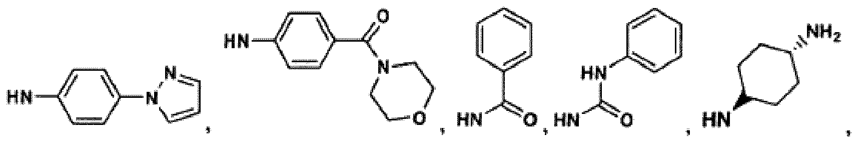

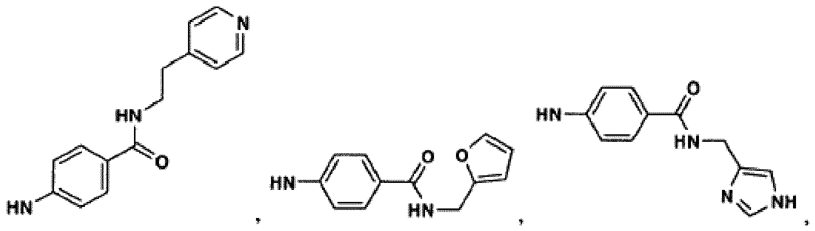

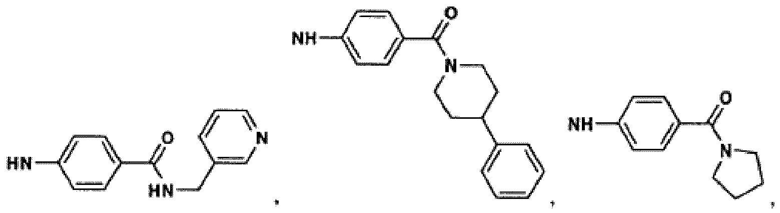

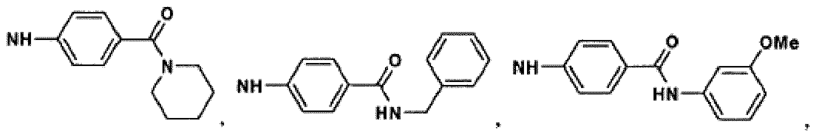

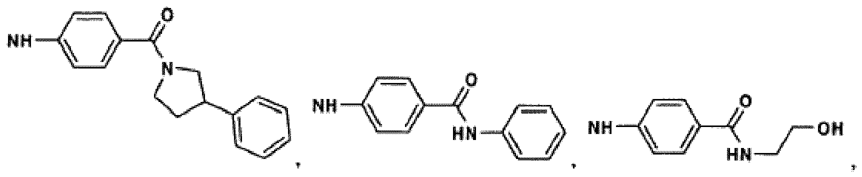

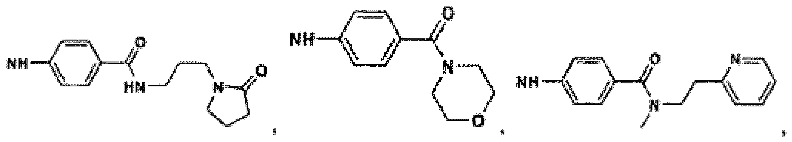

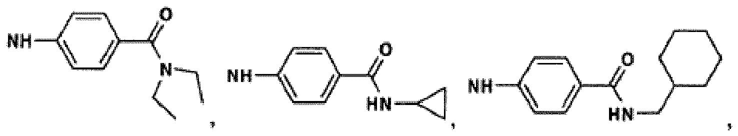

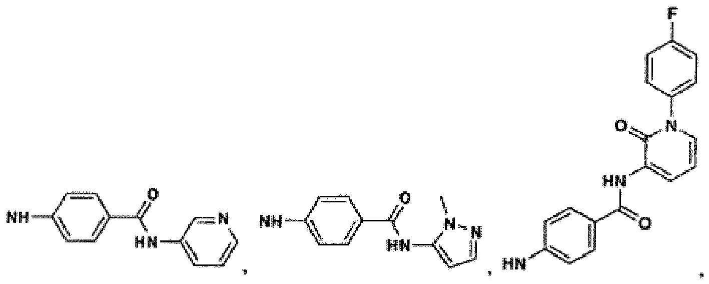

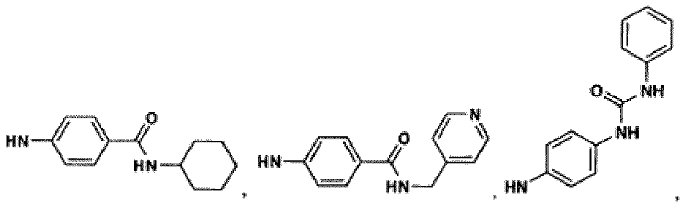

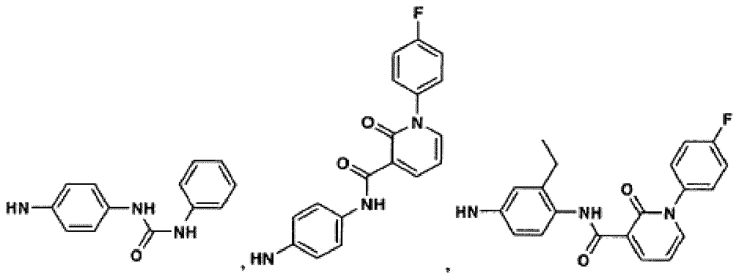

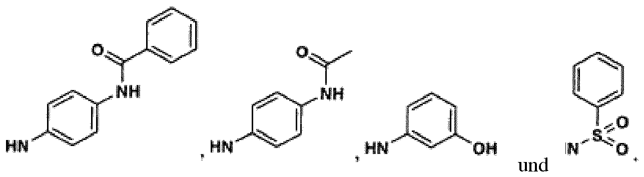

8. Verbindung nach Anspruch 5 oder ein Enantiomer, Diastereomer oder ein pharmazeutisch annehmbares Salz davon, wobei:
Y NR₆R₇ ist;
R₆ aus Wasserstoff oder C₁₋₄Alkyl ausgewählt ist;
R₇ aus C₁₋₄Alkyl, Cyclopentyl, Cyclohexyl, Bicyclo[2.2.2]octyl, Pyrrolidinyl und Piperidinyl ausgewählt ist, von denen jede Gruppe optional durch eine bis drei Gruppen, T₄, T₅ und/oder T₆, substituiert ist;
oder R₆ und R₇ zusammen mit dem Stickstoffatom, an das sie angefügt sind, Piperazinyl, Piperidinyl, Pyrrolidinyl oder Diazepanyl bilden, von denen jede Gruppe optional durch eine bis drei Gruppen, T₄, T₅ und/oder T₆, substituiert ist; und
T₄, T₅ und T₆ unabhängig ausgewählt sind aus (i) C₁₋₄Alkyl, OH, NH₂, NH(C₁₋₄Alkyl), Furyl und N(C₁₋₄Alkyl)₂ und NH(Pyrimidinyl), wobei das Pyrimidinyl durch Halogen substituiert ist; oder (ii) C₁₋₄Alkyl, substituiert durch Cyclohexyl oder OH, wobei das Cyclohexyl durch NH₂ substituiert ist.

9. Verbindung nach Anspruch 8 oder ein Enantiomer, Diastereomer oder ein pharmazeutisch annehmbares Salz davon, wobei NR₆R₇ aus dem Folgenden ausgewählt ist:
NH-(CH₂)₂-NH₃, NH-(CH₂)₄-NH₂.

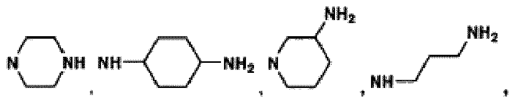

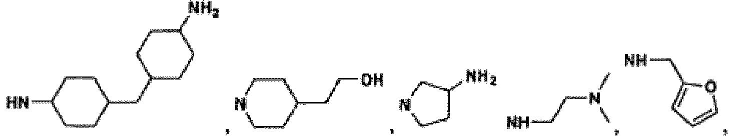

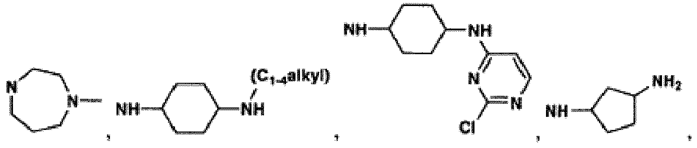

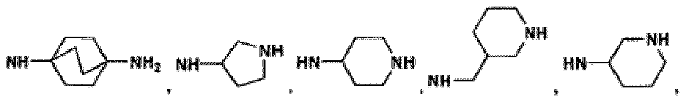

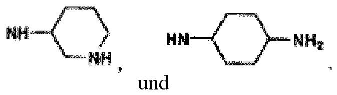

10. Verbindung nach Anspruch 8 oder ein Enantiomer, Diastereomer oder ein pharmazeutischannehmbares Salz davon, wobei NR₆R₇ ausgewählt ist aus: 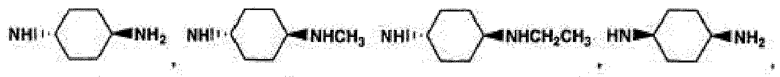 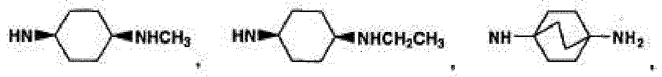 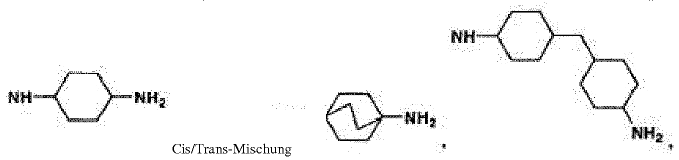 und

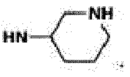

11. Verbindung nach Anspruch 5 oder ein Enantiomer, Diastereomer oder ein pharmazeutisch annehmbares Salz davon, wobei R₅ Wasserstoff ist und R₄ aus einem Phenyl-, Pyridyl-, Pyrimidinyl-, Cyclohexyl- und Piperidinylring ausgewählt ist, von denen jeder Ring optional durch eine oder zwei Gruppen, T₁ und/oder T₂, substituiert ist.

12. Verbindung nach Anspruch 1 oder 5 oder ein Enantiomer, Diastereomer oder ein pharmazeutisch annehmbares Salz davon, wobei:
R₁ und R₂ unabhängig aus Wasserstoff, Halogen, OR₁₀, Cyano, C₁₋₄Alkyl, CO₂R₁₀ und C(O)NR₁₀R₁₁ ausgewählt sind;
R₃ aus (i) Wasserstoff, Halogen, Nitro, Cyano, OR₁₀, NR₁₀R₁₁, CO₂R₁₀ und C(=O)R₁₀, (ii) C₁₋₄Alkyl, substituiertem C₁₋₄Alkyl, Cycloalkyl, Aryl und Heteroaryl ausgewählt ist; und
R₁₀ und R₁₁ unabhängig aus (i) Wasserstoff, C₁₋₄Alkyl und substituiertem C₁₋₄Alkyl ausgewählt sind; oder (ii) R₁₀ und R₁₁ zusammen mit dem Stickstoffatom, an dem sie beide angefügt sind, kombiniert werden, um ein optional substituiertem 5-, 6- oder 7-gliedriges Heteroaryl oder Heterocyclo zu bilden.

13. Verbindung nach Anspruch 1 oder 5, wobei die Verbindung die Formel (Ia) aufweist, 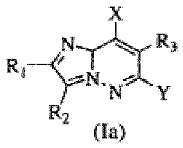 oder ein Enantiomer, Diastereomer oder ein pharmazeutisch annehmbares Salz davon.

14. Verbindung, ausgewählt aus dem Folgenden:
(i)
*N*⁶-(*trans*-4-Aminocyclohexyl)-*N*⁸-[4-(ethyloxy)phenyl]imidazo[1,2-*b*]pyridazine-6,8-diamin,
*N*⁶-(2-Aminoethyl)-*N*⁸-(4-(ethyloxy)phenyl)imidazo[1,2-*b*]pyridazin-6,8-diamin;
*N*⁶-(4-Aminobutyl)-*N*⁸-(4-(ethyloxy)phenyl)imidazo[1,2-*b*]pyridazin-6,8-diamin;
7-Chlor-*N*-(4-(ethyloxy)phenyl)-6-(1-piperazinyl)imidazo[1,2-*b*]pyridazin-8-amin;
*N*⁶-(*trans*-4-Aminocyclohexyl)-*N*⁸-(4-(methyloxy)phenyl)imidazo[1,2-*b*]pyridazin-6,8-diamin;
*N*⁶-(*trans*-4-Aminocyclohexyl)-*N*⁸-(3-(ethyloxy)phenyl)imidazo[1,2-*b*]pyridazin-6,8-diamin;
*N⁶*-(*trans*-4-Aminocyclohexyl)-*N⁸*-phenylimidazo[1,2-*b*]pyridazin-6,8-diamin;
*N⁶*-(*trans*-4-Aminocyclohexyl)-*N⁸-*(3-(methyloxy)phenyl)imidazo[1,2-b]pyridazin-6,8-diamin;
*N⁶*-(*trans*-4-Aminocyclohexyl)-*N⁸*-(3,4-dimethylphenyl)imidazo[1,2-*b*]pyridazin-6,8-diamin;
*N⁶*-(*trans*-4-Aminocyclohexyl)-*N⁸*-(4-(phenyloxy)phenyl)imidazo[1,2-*b*]pyridazin-6,8-diamin;
*N⁶*-(*trans*-4-Aminocyclohexyl)-*N⁸*-(4-(butyloxy)phenyl)imidazo[1,2-*b*]pyridazin-6,8-diamin;
*N⁶*-(*trans*-4-Aminocyclohexyl)-*N⁸*-4-biphenylylimidazo[1,2-*b*]pyridazin-6,8-diamin;
*N⁶*-(*trans*-4-Aminocyclohexyl)-*N⁸*-(4-methylphenyl)imidazo[1,2-*b*]pyridazin-6,8-diamin;
*N⁶*-(*trans*-4-Aminocyclohexyl)-*N⁸-*(3,4-bis(methyloxy)phenyl)imidazo[1,2-*b*]pyridazin-6,8-diamin;
*N⁶*-(*trans*-4-Aminocyclohexyl)-*N⁸*-(4-((phenylmethyl)oxy)phenyl)imidazo[1,2-b]pyridazin-6,8-diamin;
*N⁶*-(*trans*-4-Aminocyclohexyl)-*N⁸*-(4-(propyloxy)phenyl)imidazo[1,2-*b*]pyridazin-6,8-diamin;
*N⁶*-(*trans*-4-Aminocyclohexyl)-*N⁸*-pyridin-3-ylimidazo[1,2-*b*]pyridazin-6,8-diamin;
*N⁶*-(*trans-*4-Aminocyclohexyl)-*N⁸*-(2-methyl-1*H*-indol-5-yl)imidazo[1,2-b]pyridazin-6,8-diamin;
*N⁶*-(*trans*-4-Aminocyclohexyl)-*N⁸*-methyl-*N⁸*-phenylimidazo[1,2-*b*]pyridazin-6,8-diamin;
*N⁶*-(*trans*-4-Aminocyclohexyl)-*N⁸*-[2-(methyloxy)phenyl]imidazo[1,2-*b*]pyridazin-6,8-diamin;
*N⁶*-(*trans*-4-Aminocyclohexyl)-*N⁸*-(2-methylphenyl)imidazo[1,2-*b*]pyridazin-6,8-diamin;
*N⁶*-(*trans*-4-Aminocyclohexyl)-*N⁸*-(2,3-dimethylphenyl)imidazo[1,2-*b*]pyridazin-6,8-diamin;
*N⁶*-(*trans*-4-Aminocyclohexyl)-*N⁸*-(2,4-dimethylphenyl)imidazo[1,2-*b*]pyridazin-6,8-diamin;
*N⁶*-(*trans*-4-Aminocyclohexyl)-*N⁸*-(2,5-dimethylphenyl)imidazo[1,2-*b*]pyridazin-6,8-diamin;
*N⁶*-(*trans*-4-Aminocyclohexyl)-*N⁸*-(3-methylphenyl)imidazo[1,2-*b*]pyridazin-6,8-diamin;
*N⁶*-(*trans*-4-Aminocyclohexyl)-*N⁸*-(3,5-dimethylphenyl)imidazo[1,2-*b*]pyridazin-6,8-diamin;
*N⁶*-(*trans*-4-Aminocyclohexyl)-*N⁸*-[3-(dimethylamino)phenyl]imidazo[1,2-*b*]pyridazin-6,8-diamin;
*N⁶*-(*trans*-4-Aminocyclohexyl)-*N⁸*-(2-methyl-1,3-benzothiazol-6-yl)imidazo[1,2-*b*]pyridazin-6,8-diamin;
*N⁶*-(*trans*-4-Aminocyclohexyl)-*N⁸*-(2-methyl-1,3-benzothiazol-5-yl)imidazo[1,2-*b*]pyridazin-6,8-diamin;
*N⁶*-(*trans*-4-Aminocyclohexyl)-*N⁸*-cyclopropylimidazo[1,2-*b*]pyridazin-6,8-diamin;
*N⁶*-(*trans*-4-Aminocyclohexyl)-*N⁸*-cyclohexylimidazo[1,2-*b*]pyridazin-6,8-diamin;
*N⁶*-(*trans*-4-Aminocyclohexyl)-*N⁸*-(cyclohexylmethyl)imidazo[1,2-*b*]pyridazin-6,8-diamin;
*N⁶*-(*trans*-4-Aminocyclohexyl)-*N⁸*-(1-methylethyl)imidazo[1,2-*b*]pyridazin-6,8-diamin;
*N⁶*-(*trans*-4-Aminocyclohexyl)-*N⁸*-(phenylmethyl)imidazo[1,2-*b*]pyridazin-6,8-diamin;
*N⁶*-(*trans*-4-Aminocyclohexyl)-*N⁸*-[(2-chlorophenyl)methyl]imidazo[1,2-b]pyridazin-6,8-diamin;
*N⁶*-(*trans*-4-Aminocyclohexyl)-*N⁸*-((4-chlorphenyl)methyl)imidazo[1,2-*b*]pyridazin-6,8-diamin;
*N⁶*-(*trans*-4-Aminocyclohexyl)-*N⁸*-((4-(methyloxy)phenyl)methyl)imidazo[1,2-*b*]pyridazin-6,8-diamin;
*N⁶*-(*trans*-4-Aminocyclohexyl)-*N⁸*-ethylimidazo[1,2-*b*]pyridazin-6,8-diamin;
*N⁶*-(*trans*-4-Aminocyclohexyl)-*N⁸*-(2-(methyloxy)ethyl)imidazo[1,2-*b*]pyridazin-6,8-diamin;
*N⁶*-(*trans*-4-Aminocyclohexyl)-*N⁸*-(2-(4-(methyloxy)phenyl)ethyl)imidazo[1,2-*b*]pyridazin-6,8-diamin;
*N⁶*-(trans-4-Aminocyclohexyl)-*N⁸*-2-propen-1-ylimidazo[1,2-*b*]pyridazin-6,8-diamin;
*N⁶*-(*trans*-4-Aminocyclohexyl)-*N⁸*-(3-methylbutyl)imidazo[1,2-*b*]pyridazin-6,8-diamin;
*N⁶*-(*trans*-4-Aminocyclohexyl)-*N⁸*-propylimidazo[1,2-*b*]pyridazin-6,8-diamin; *N⁶*-(*trans*-4-Aminocyclohexyl)-*N⁸*-(cyclopropylmethyl)imidazo[1,2-*b*]pyridazin-6,8-diamin;
*N⁶*-(*trans*-4-Aminocyclohexyl)-*N⁸*-((3-chlorphenyl)methyl)imidazo[1,2-*b*]pyridazin-6,8-diamin;
6-(3-Amino-1-piperidinyl)-*N*-(4-(ethyloxy)phenyl)imidazo[1,2-*b*]pyridazin-8-amin;
*N⁶*-(3-Aminopropyl)-*N⁸*-(4-(ethyloxy)phenyl)imidazo[1,2-*b*]pyridazin-6,8-diamin;
*N⁶*-(*trans*-4-Aminocyclohexyl)-*N⁸*-(2,6-difluorphenyl)imidazo[1,2-*b*]pyridazin-6,8-diamin;
*N⁶*-(4-((4-Aminocyclohexyl)methyl)cyclohexyl)-*N⁸*-(4-(ethyloxy)phenyl)imidazo[1,2-*b*]pyridazin-6,8-diamin;
2-(1-(8-((4-(Ethyloxy)phenyl)amino)imidazo[1,2-*b*]pyridazin-6-yl)-4-piperidinyl)ethanol;
*N⁶*-(*trans*-4-Aminocyclohexyl)-*N⁸*-1*H*-indol-5-ylimidazo[1,2-*b*]pyridazin-6,8-diamin;
*N⁶*-(*trans*-4-Aminocyclohexyl)-*N⁸*-(2-(ethyloxy)phenyl)imidazo[1,2-*b*]pyridazin-6,8-diamin;
6-(3-Amino-1-pyrrolidinyl)-*N*-(4-(ethyloxy)phenyl)imidazo[1,2-*b*]pyridazin-8-amin;
*N⁶*-(*trans*-4-Aminocyclohexyl)-*N⁸*-(2-phenylethyl)imidazo[1,2-*b*]pyridazin-6,8-diamin;
*N*-(4-(Ethyloxy)phenyl)-6-(1-piperazinyl)imidazo[1,2-*b*]pyridazin-8-amin;
*N⁶*-(2-(Dimethylamino)ethyl)-*N⁸*-(4-(ethyloxy)phenyl)imidazo[1,2-*b*]pyridazin-6,8-diamin;
*N⁸*-(4-(Ethyloxy)phenyl)-*N⁶*-(2-furanylmethyl)imidazo[1,2-*b*]pyridazin-6,8-diamin;
*N*-(4-(Ethyloxy)phenyl)-6-(4-methyl-1,4-diazepan-1-yl)imidazo[1,2-*b*]pyridazin-8-amin;
2-((6-((*trans*-4-Aminocyclohexyl)amino)imidazo[1,2-*b*]pyridazin-8-yl)amino)phenol;
*N⁶*-(*trans*-4-Aminocyclohexyl)-*N⁸*-(3-((phenylmethyl)oxy)phenyl)imidazo[1,2-*b*]pyridazin-6,8-diamin;
3-((6-((*trans*-4-Aminocyclohexyl)amino)imidazo[1,2-*b*]pyridazin-8-yl)amino)phenol;
4-((6-((*trans*-4-Aminocyclohexyl)amino)imidazo[1,2-*b*]pyridazin-8-yl)amino)phenol;
*N⁶*-(*trans*-4-Aminocyclohexyl)imidazo[1,2-*b*]pyridazin-6,8-diamin;
3-((6-((*trans*-4-Aminocyclohexyl)amino)imidazo[1,2-*b*]pyridazin-8-yl)amino)benzonitril;
4-((6-((*trans*-4-Aminocyclohexyl)amino)imidazo[1,2-*b*]pyridazin-8-yl)amino)benzonitril;
3-((6-((*trans*-4-Aminocyclohexyl)amino)imidazo[1,2-*b*]pyridazin-8-yl)amino)benzoesäure;
*N⁶*-(*trans*-4-Aminocyclohexyl)-*N⁸*-1*H*-pyrazol-3-ylimidazo[1,2-*b*]pyridazin-6,8-diamin;
4-((6-((*trans*-4-Aminocyclohexyl)amino)imidazo[1,2-*b*]pyridazin-8-yl)amino)benzoesäure;
4-((6-((*trans*-4-Aminocyclohexyl)amino)imidazo[1,2-*b*]pyridazin-8-yl)amino)-*N,N*-dimethylbenzensulfonamid;
*N⁶*-(*trans*-4-Aminocyclohexyl)-*N⁸*-(4-(1*H*-tetrazol-5-yl)phenyl)imidazo[1,2-*b*]pyridazin-6,8-diamin;
*N⁶*-(*trans*-4-(Ethylamino)cyclohexyl)-*N⁸*-phenylimidazo[1,2-*b*]pyridazin-6,8-diamin;
*N⁶*-(*trans*-4-(Methylamino)cyclohexyl)-*N⁸*-phenylimidazo[1,2-*b*]pyridazin-6,8-diamin;
*N⁶*-(*trans*-4-Aminocyclohexyl)-*N⁸*-(2-(phenyloxy)phenyl)imidazo[1,2-*b*]pyridazin-6,8-diamin;
*N⁶*-(*trans*-4-Aminocyclohexyl)-*N⁸*-(4'-chlor-4-biphenylyl)imidazo[1,2-*b*]pyridazin-6,8-diamin;
*N⁶*-(*trans*-4-Aminocyclohexyl)-*N⁸*-(2'-methyl-4-biphenylyl)imidazo[1,2-*b*]pyridazin-6,8-diamin;
*N⁶*-(*trans*-4-Aminocyclohexyl)-*N⁸*-(3'-chlor-4-biphenylyl)imidazo[1,2-*b*]pyridazin-6,8-diamin;
*N⁶*-(*trans*-4-Aminocyclohexyl)-*N⁸*-(4-(phenylmethyl)phenyl)imidazo[1,2-*b*]pyridazin-6,8-diamin;
*N⁶*-(*trans*-4-Aminocyclohexyl)-*N⁸*-(4-(4-morpholinyl)phenyl)imidazo[1,2-*b*]pyridazin-6,8-diamin;
*N⁶*-(*trans*-4-Aminocyclohexyl)-*N⁸*-(3'-chlor-3-biphenylyl)imidazo[1,2-*b*]pyridazin-6,8-diamin;
*N⁶*-(*trans*-4-Aminocyclohexyl)-*N⁸*-(4-(1-methylethyl)phenyl)imidazo[1,2-*b*]pyridazin-6,8-diamin;
*N⁶*-(*trans*-4-Aminocyclohexyl)-*N⁸*-(4-butylphenyl)imidazo[1,2-*b*]pyridazin-6,8-diamin;
*N⁶*-(*trans*-4-Aminocyclohexyl)-*N⁸*-(5,6,7,8-tetrahydro-1-naphthalenyl)imidazo[1,2-*b*]pyridazin-6,8-diamin;
*N⁶*-(*trans*-4-Aminocyclohexyl)-*N⁸*-1-naphthalenylimidazo[1,2-*b*]pyridazin-6,8-diamin;
*N⁶*-(*trans*-4-Aminocyclohexyl)-*N⁸*-(3-(phenylmethyl)phenyl)imidazo[1,2-*b*]pyridazin-6,8-diamin;
*N⁶*-(*trans*-4-Aminocyclohexyl)-*N⁸*-(4-propylphenyl)imidazo[1,2-*b*]pyridazin-6,8-diamin;
*N⁶*-(*trans*-4-Aminocyclohexyl)-*N⁸*-(4'-methyl-4-biphenylyl)imidazo[1,2-*b*]pyridazin-6,8-diamin;
*N⁶*-(*trans*-4-Aminocyclohexyl)-*N⁸*-(3-(1-methylethyl)phenyl)imidazo[1,2-*b*]pyridazin-6,8-diamin;
*N⁶*-(*trans*-4-Aminocyclohexyl)-*N⁸*-(3-((1-methylethyl)oxy)phenyl)imidazo[1,2-*b*]pyridazin-6,8-diamin;
*N⁶*-(*trans*-4-Aminocyclohexyl)-*N⁸*-(3,5-bis(methyloxy)phenyl)imidazo[1,2-*b*]pyridazin-6,8-diamin;
*trans-N*-(8-(6-Methyl-3,4-dihydro-1(2*H*)-chinolinyl)imidazo[1,2-*b*]pyridazin-6-yl)-1,4-cyclohexanediamin;
*N⁶*-(*trans*-4-Aminocyclohexyl)-*N⁸*-2-naphthalenylimidazo[1,2-*b*]pyridazin-6,8-diamin;
*N⁶*-(*trans*-4-Aminocyclohexyl)-*N⁸*-(3-(methylsulfanyl)phenyl)imidazo[1,2-*b*]pyridazin-6,8-diamin;
*N⁶*-(*trans*-4-Aminocyclohexyl)-*N⁸*-(3-ethylphenyl)imidazo[1,2-*b*]pyridazin-6,8-diamin;
*N⁶*-(*trans*-4-Aminocyclohexyl)-*N⁸*-(4-ethylphenyl)imidazo[1,2-*b*]pyridazin-6,8-diamin;
*N⁶*-(*trans*-4-Aminocyclohexyl)-*N⁸*-(4-(methylsulfanyl)phenyl)imidazo[1,2-*b*]pyridazin-6,8-diamin;
*N⁶*-(*trans*-4-Aminocyclohexyl)-*N⁸*-9*H*-fluoren-2-ylimidazo[1,2-*b*]pyridazin-6,8-diamin;
*N⁶*-(*trans*-4-Aminocyclohexyl)-*N⁸*-(2-ethylphenyl)imidazo[1,2-*b*]pyridazin-6,8-diamin;
*N⁶*-(*trans*-4-Aminocyclohexyl)-*N⁸*-(4-cyclohexylphenyl)imidazo[1,2-*b*]pyridazin-6,8-diamin;
*N⁶*-(*trans*-4-Aminocyclohexyl)-*N⁸*-(4-(1,1-dimethylethyl)phenyl)imidazo[1,2-*b*]pyridazin-6,8-diamin;
*N⁶*-(*trans*-4-Aminocyclohexyl)-*N⁸*-(3-(phenyloxy)phenyl)imidazo[1,2-*b*]pyridazin-6,8-diamin;
*N⁶*-(*trans*-4-Aminocyclohexyl)-*N⁸*-3-biphenylylimidazo[1,2-*b*]pyridazin-6,8-diamin;
*N⁶*-(*trans*-4-Aminocyclohexyl)-*N⁸*-(4-((1-methylethyl)oxy)phenyl)imidazo[1,2-*b*]pyridazin-6,8-diamin;
*N⁶*-(*trans*-4-Aminocyclohexyl)-*N⁸*-(2-chlorphenyl)imidazo[1,2-*b*]pyridazin-6,8-diamin;
*N⁶*-(*trans*-4-Aminocyclohexyl)-*N⁸*-(4-chlorphenyl)imidazo[1,2-*b*]pyridazin-6,8-diamin;
*N⁶*-(*trans*-4-Aminocyclohexyl)-*N⁸*-(3-chlorphenyl)imidazo[1,2-*b*]pyridazin-6,8-diamin;
*N⁶*-(*trans*-4-Aminocyclohexyl)-*N⁸*-(4-chlor-1-naphthalenyl)imidazo[1,2-*b*]pyridazin-6,8-diamin;
*N⁶*-(*trans*-4-Aminocyclohexyl)-*N⁸*-3-chinolinylimidazo[1,2-*b*]pyridazin-6,8-diamin;
*N⁶*-(*trans*-4-Aminocyclohexyl)-*N⁸*-(3,5-dichlorphenyl)imidazo[1,2-*b*]pyridazin-6,8-diamin;
*N⁶*-(*trans*-4-Aminocyclohexyl)-*N⁸*-(3-(trifluormethyl)phenyl)imidazo[1,2-*b*]pyridazin-6,8-diamin;
*N⁶*-(*trans*-4-Aminocyclohexyl)-*N⁸*-(4-chlor-2-fluorophenyl)imidazo[1,2-*b*]pyridazin-6,8-diamin;
*N⁶*-(*trans*-4-Aminocyclohexyl)-N⁸-(2-fluor-5-methylphenyl)imidazo[1,2-*b*]pyridazin-6,8-diamin;
*N⁶*-(*trans*-4-Aminocyclohexyl)-*N⁸*-(4-chlor-3-methylphenyl)imidazo[1,2-*b*]pyridazin-6,8-diamin;
*N⁶*-(*trans*-4-Aminocyclohexyl)-*N⁸*-(5-phenyl-2-pyridinyl)imidazo[1,2-*b*]pyridazin-6,8-diamin;
*N⁶*-(*trans*-4-Aminocyclohexyl)-*N⁸*-(3-fluor-4-methylphenyl)imidazo[1,2-*b*]pyridazin-6,8-diamin;
*N⁶*-(*trans*-4-Aminocyclohexyl)-*N⁸*-(2-methyl-4-pyridinyl)imidazo[1,2-*b*]pyridazin-6,8-diamin;
*N⁶*-(trans-4-Aminocyclohexyl)-*N⁸*-(4-fluor-3-methylphenyl)imidazo[1,2-*b*]pyridazin-6,8-diamin;
*N⁶*-(*trans*-4-Aminocyclohexyl)-*N⁸*-(2-fluor-4-methylphenyl)imidazo[1,2-*b*]pyridazin-6,8-diamin;
*N⁶*-(*trans*-4-Aminocyclohexyl)-*N⁸*-(3-((trifluormethyl)oxy)phenyl)imidazo[1,2-*b*]pyridazin-6,8-diamin;
*N⁶*-(*trans*-4-Aminocyclohexyl)-*N⁸*-(4-methyl-3-(trifluormethyl)phenyl)imidazo[1,2-*b*]pyridazin-6,8-diamin;
*N⁶*-(*trans*-4-Aminocyclohexyl)-*N⁸*-(4-ethyl-2-pyridinyl)imidazo[1,2-*b*]pyridazin-6,8-diamin;
*N⁶*-(*trans*-4-Aminocyclohexyl)-*N⁸*-(4-(1*H*-1,2,4-triazol-1-yl)phenyl)imidazo[1,2-*b*]pyridazin-6,8-diamin;
*N⁶*-(*trans*-4-Aminocyclohexyl)-*N⁸*-(4-(1*H*-pyrrol-1-yl)phenyl)imidazo[1,2-*b*]pyridazin-6,8-diamin;
*N⁶*-(*trans*-4-Aminocyclohexyl)-*N⁸*-(4-(4,5-dichlor-1*H*-imidazol-1-yl)phenyl)imidazo[1,2-*b*]pyridazin-6,8-diamin;
*N⁶*-(*trans*-4-Aminocyclohexyl)-*N⁸*-(4-(1*H*-pyrazol-1-yl)phenyl)imidazo[1,2-*b*]pyridazin-6,8-diamin;
*N⁶*-(*trans*-4-Aminocyclohexyl)-*N⁸-*(4-(3,5-dimethyl-1*H*-pyrazol-1-yl)phenyl)imidazo[1,2-*b*]pyridazin-6,8-diamin;
*N⁶*-(*trans*-4-Aminocyclohexyl)-*N⁸*-(4-(4-methyl-4*H*-1,2,4-triazol-3-yl)phenyl)imidazo[1,2-*b*]pyridazin-6,8-diamin;
*N⁶*-(*trans-*4-Aminocyclohexyl)-*N⁸*-(4-(1*H*-imidazol-1-yl)phenyl)imidazo[1,2-*b*]pyridazin-6,8-diamin;
*N⁶*-(*trans*-4-Aminocyclohexyl)-*N⁸*-(4-(1-methyl-1*H*-imidazol-2-yl)phenyl)imidazo[1,2-*b*]pyridazin-6,8-diamin;
*N⁶*-(*trans*-4-Aminocyclohexyl)-*N⁸*-(4-(2-methyl-1,3-thiazol-4-yl)phenyl)imidazo[1,2-*b*]pyridazin-6,8-diamin;
*N⁶*-(*trans*-4-Aminocyclohexyl)-*N⁸*-(4-(5-methyl-2-furanyl)phenyl)imidazo[1,2-*b*]pyridazin-6,8-diamin;
*N⁶*-(*trans*-4-Aminocyclohexyl)-*N⁸*-(4-(2-ethyl-2*H*-tetrazol-5-yl)phenyl)imidazo[1,2-*b*]pyridazin-6,8-diamin;
4-((6-((*trans*-4-Aminocyclohexyl)amino)imidazo[1,2-*b*]pyridazin-8-yl)amino)-3-hydroxy-*N,N*-dimethylbenzensulfonamid ;
*N⁶*-(*trans*-4-Aminocyclohexyl)-*N⁸*-(2,3-dihydro-1*H*-inden-5-yl)imidazo[1,2-*b*]pyridazin-6,8-diamin;
3-((6-((*trans*-4-Aminocyclohexyl)amino)imidazo[1,2-*b*]pyridazin-8-yl)amino)-*N,N*-dimethylbenzensulfonamid ;
4-((6-((*trans*-4-Aminocyclohexyl)amino)imidazo[1,2-*b*]pyridazin-8-yl)amino)-*N,N*-dimethylbenzamid;
*N⁶*-(*trans*-4-((2-Chlor-4-pyrimidinyl)amino)cyclohexyl)-*N⁸*-phenylimidazo[1,2-*b*]pyridazin-6,8-diamin;
*N⁶*-(3-Aminocyclopentyl)-*N⁸*-phenylimidazo[1,2-*b*]pyridazin-6,8-diamin;
*N⁶*-(*trans*-4-Aminocyclohexyl)-*N⁸*-(3-(4-morpholinylsulfonyl)phenyl)imidazo[1,2-*b*]pyridazin-6,8-diamin;
3-((6-((*trans*-4-Aminocyclohexyl)amino)imidazo[1,2-*b*]pyridazin-8-yl)amino)-*N,N*-diethylbenzamid
3-((6-((*trans*-4-Aminocyclohexyl)amino)imidazo[1,2-*b*]pyridazin-8-yl)amino)-*N-*methyl-*N*-phenylbenzensulfonamid
4-((6-((*trans*-4-Aminocyclohexyl)amino)imidazo[1,2-*b*]pyridazin-8-yl)amino)-2-hydroxy-*N,N*-dimethylbenzensulfonamid
*N⁶*-(4-Aminobicyclo[2.2.2]oct-1-yl)-*N⁸*-phenylimidazo[1,2-*b*]pyridazin-6,8-diamin;
*N*-(4-((6-((*trans*-4-Aminocyclohexyl)amino)imidazo[1,2-*b*]pyridazin-8-yl)amino)phenyl)methansulfonamid;
*N⁶*-(*trans*-4-Aminocyclohexyl)-*N⁸*-(4-(3-(dimethylamino)-1-pyrrolidinyl)phenyl)imidazo[1,2-*b*]pyridazin-6,8-diamin;
*N⁶*-(*trans*-4-Aminocyclohexyl)-*N⁸*-(4-(1-pyrrolidinylsulfonyl)phenyl)imidazo[1,2-*b*]pyridazin-6,8-diamin;
4-((6-((*trans*-4-Aminocyclohexyl)amino)imidazo[1,2-*b*]pyridazin-8-yl)amino)benzensulfonsäure;
4-((6-((*trans*-4-Aminocyclohexyl)amino)imidazo[1,2-*b*]pyridazin-8-yl)amino)-*N,N*-diethylbenzensulfonamid;
4-((6-((*trans*-4-Aminocyclohexyl)amino)imidazo[1,2-*b*]pyridazin-8-yl)amino)-*N-*propylbenzensulfonamid;
4-((6-((*trans*-4-Aminocyclohexyl)amino)imidazo[1,2-*b*]pyridazin-8-yl)amino)-*N-*ethylbenzensulfonamid;
4-((6-((*trans*-4-Aminocyclohexyl)amino)imidazo[1,2-*b*]pyridazin-8-yl)amino)-*N-*methylbenzensulfonamid;
*N⁶*-(*trans*-4-Aminocyclohexyl)-*N⁸*-(4-aminophenyl)imidazo[1,2-*b*]pyridazin-6,8-diamin;
4-((6-((*trans*-4-Aminocyclohexyl)amino)imidazo[1,2-*b*]pyridazin-8-yl)amino)benzensulfonamid;
3-((6-((*trans*-4-Aminocyclohexyl)amino)imidazo[1,2-*b*]pyridazin-8-yl)amino)benzensulfonamid;
3-((6-((*trans*-4-Aminocyclohexyl)amino)imidazo[1,2-*b*]pyridazin-8-yl)amino)benzamid;
4-((6-((*trans*-4-Aminocyclohexyl)amino)imidazo[1,2-*b*]pyridazin-8-yl)amino)benzamid;
*N⁶*-(*trans*-4-Aminocyclohexyl)-*N⁸*-(4-(aminomethyl)phenyl)imidazo[1,2-*b*]pyridazin-6,8-diamin;
6-((6-((*trans*-4-Aminocyclohexyl)amino)imidazo[1,2-*b*]pyridazin-8-yl)amino)-1,2-dihydro-3*H*-indazol-3-on;
*N⁶*-(*trans*-4-Aminocyclohexyl)-*N⁸*-(3-(aminomethyl)phenyl)imidazo[1,2-*b*]pyridazin-6,8-diamin;
*N⁶*-(trans-4-Aminocyclohexyl)-*N⁸*-(2,6-difluorphenyl)imidazo[1,2-*b*]pyridazin-6,8-diamin;
*N⁶*-(*trans*-4-Aminocyclohexyl)-7-chlor-*N⁸*-[4-(ethyloxy)phenyl]imidazo[1,2-*b*]pyridazin-6,8-diamin;
*N⁶*-(3-Aminopropyl)-*N⁸*-[4-(ethyloxy)phenyl]imidazo[1,2-*b*]pyridazin-6,8-diamin;
*N⁶*-(*trans*-4-Aminocyclohexyl)-*N⁸*-(2-fluorphenyl)imidazo[1,2-*b*]pyridazin-6,8-diamin;
*N⁶*-(*trans*-4-Aminocyclohexyl)-*N⁸*-(2,6-difluorphenyl)imidazo[1,2-*b*]pyridazin-6,8-diamin;
*N⁶*-(*trans*-4-Aminocyclohexyl)-*N⁸*-(2,4-difluorphenyl)imidazo[1,2-*b*]pyridazin-6,8-diamin;
*N⁶*-(*trans*-4-Aminocyclohexyl)-*N⁸*-(3-fluorphenyl)imidazo[1,2-*b*]pyridazin-6,8-diamin;
*N⁶*-(*trans*-4-Aminocyclohexyl)-*N⁸*-(4-fluorphenyl)imidazo[1,2-*b*]pyridazin-6,8-diamin;
*N⁶*-(*trans*-4-Aminocyclohexyl)-*N⁸*-(3,4-difluorphenyl)imidazo[1,2-*b*]pyridazin-6,8-diamin;
*N⁶*-(*trans*-4-Aminocyclohexyl)-*N⁸*-(2,5-difluorphenyl)imidazo[1,2-*b*]pyridazin-6,8-diamin;
*N⁶*-(*trans*-4-Aminocyclohexyl)-*N⁸*-(2,3-difluorphenyl)imidazo[1,2-*b*]pyridazin-6,8-diamin;
*N⁶*-(*trans*-4-Aminocyclohexyl)-*N⁸*-(3,5-difluorphenyl)imidazo[1,2-*b*]pyridazin-6,8-diamin;
*N⁶*-(*trans*-4-Aminocyclohexyl)-*N⁸*-(3-iodophenyl)imidazo[1,2-*b*]pyridazin-6,8-diamin;
*N⁶*-(*trans*-4-Aminocyclohexyl)-*N⁸*-[4-(trifluormethyl)phenyl]imidazo[1,2-*b*]pyridazin-6,8-diamin;
*N⁶*-(*trans*-4-Aminocyclohexyl)-*N⁸*-pyridin-2-ylimidazo[1,2-*b*]pyridazin-6,8-diamin;
*N⁶*-(*trans*-4-Aminocyclohexyl)-*N⁸*-(4-methylpyridin-2-yl)imidazo[1,2-*b*]pyridazin-6,8-diamin;
*N⁶*-(*trans*-4-Aminocyclohexyl)-*N⁸*-(5-methylpyridin-2-yl)imidazo[1,2-*b*]pyridazin-6,8-diamin;
*N⁶*-(*trans*-4-Aminocyclohexyl)-*N⁸*-(4,6-dimethylpyridin-2-yl)imidazo[1,2-*b*]pyridazin-6,8-diamin;
*N⁶*-(*trans*-4-Aminocyclohexyl)-*N⁸*-pyrimidin-2-ylimidazo[1,2-*b*]pyridazin-6,8-diamin;
*N⁶*-(*trans*-4-Aminocyclohexyl)-*N⁸*-[4-(ethyloxy)phenyl]-7-methylimidazo[1,2-*b*]pyridazin-6,8-diamin;
*N⁶*-(*trans*-4-Aminocyclohexyl)-7-ethyl-*N⁸*-[4-(ethyloxy)phenyl]imidazo[1,2-*b*]pyridazin-6,8-diamin;
*N⁶*-(*trans*-4-Aminocyclohexyl)-7-methyl-*N⁸*-phenylimidazo[1,2-*b*]pyridazin-6,8-diamin;
*N⁶*-(*trans*-4-Aminocyclohexyl)-*N⁸*-(3,4-dimethylphenyl)-7-methylimidazo[1,2-*b*]pyridazin-6,8-diamin;
*N⁶*-(*trans*-4-Aminocyclohexyl)-7-methyl-*N⁸*-(4-methylphenyl)imidazo[1,2-*b*]pyridazin-6,8-diamin;
*N⁶*-(*trans*-4-Aminocyclohexyl)-7-methyl-*N⁸*-[3-(methyloxy)phenyl]imidazo[1,2-*b*]pyridazin-6,8-diamin;
*N⁶*-(*trans*-4-Aminocyclohexyl)-*N⁸*-biphenyl-4-yl-7-methylimidazo[1,2-*b*]pyridazin-6,8-diamin;
*N⁶*-(*trans*-4-Aminocyclohexyl)-7-methyl-*N⁸*-[4-(propyloxy)phenyl]imidazo[1,2-*b*]pyridazin-6,8-diamin;
4-((6-((*trans*-4-Aminocyclohexyl)amino)-7-methylimidazo[1,2-*b*]pyridazin-8-yl)amino)benzoesäure;
4-((6-((4-Aminocyclohexyl)amino)-7-methylimidazo[1,2-*b*]pyridazin-8-yl)amino)-*N,N*-dimethylbenzensulfonamid;
*N*-(4-((6-((*trans*-4-Aminocyclohexyl)amino)-7-methylimidazo[1,2-*b*]pyridazin-8-yl)amino)phenyl)-*N*-methylacetamid;
*N⁶*-(*trans*-4-Aminocyclohexyl)-7-ethyl-*N⁸*-phenylimidazo[1,2-*b*]pyridazin-6,8-diamin;
*N⁶*-(*trans*-4-Aminocyclohexyl)-7-chlor-*N⁸*-[4-(ethyloxy)phenyl]imidazo[1,2-*b*]pyridazin-6,8-diamin;
*N⁸*-[4-(Ethyloxy)phenyl]-*N⁶*-piperidin-3-ylimidazo[1,2-*b*]pyridazin-6,8-diamin;
*N⁸*-[4-(Ethyloxy)phenyl]-*N⁶*-pyrrolidin-3-ylimidazo[1,2-*b*]pyridazin-6,8-diamin;
*N⁸*-[4-(Ethyloxy)phenyl]-*N⁶*-piperidin-4-ylimidazo[1,2-*b*]pyridazin-6,8-diamin;
6-(3-Amino-1-piperidinyl)-*N*-(4-(ethyloxy)phenyl)imidazo[1,2-*b*]pyridazin-8-amin;
*N⁸*-(4-(Ethyloxy)phenyl)-7-methyl-*N⁶*-3-piperidinylimidazo[1,2-*b*]pyridazin-6,8-diamin;
*N⁸*-Phenyl-*N⁶*-3-piperidinylimidazo[1,2-*b*]pyridazin-6,8-diamin;
6-[(3*S*)-3-Aminopyrrolidin-1-yl]-*N*-[4-(ethyloxy)phenyl]imidazo[1,2-*b*]pyridazin-8-amin;
*N⁶*-(*cis*-4-Aminocyclohexyl)-*N⁸*-[4-(ethyloxy)phenyl]-7-methylimidazo[1,2-*b*]pyridazin-6,8-diamin;
*N⁶*-(*cis*-4-Aminocyclohexyl)-*N⁸*-(5-methyl-2-pyridinyl)imidazo[1,2-*b*]pyridazin-6,8-diamin;
*N⁶*-(*cis*-4-Aminocyclohexyl)-*N⁸*-phenylimidazo[1,2-*b*]pyridazin-6,8-diamin;
*N⁶*-(*cis*-4-Aminocyclohexyl)-7-methyl-*N⁸*-phenylimidazo[1,2-*b*]pyridazin-6,8-diamin;
*N⁶*-(*cis*-4-Aminocyclohexyl)-*N⁸*-(4-(ethyloxy)phenyl)imidazo[1,2-*b*]pyridazin-6,8-diamin;
*N⁶*-(*cis*-4-Aminocyclohexyl)-*N⁸*-(3,4-dimethylphenyl)imidazo[1,2-*b*]pyridazin-6,8-diamin;
*N⁶*-(*cis*-4-Aminocyclohexyl)-*N⁸*-(5-phenyl-2-pyridinyl)imidazo[1,2-*b*]pyridazin-6,8-diamin;
4-((6-((*cis*-4-Aminocyclohexyl)amino)imidazo[1,2-*b*]pyridazin-8-yl)amino)-*N,N-*dimethylbenzensulfonamid;
*N⁶*-(*cis*-4-Aminocyclohexyl)-*N⁸*-(4-methylphenyl)imidazo[1,2-*b*]pyridazin-6,8-diamin;
*N⁶*-(*cis*-4-Aminocyclohexyl)-*N⁸*-(4,6-dimethyl-2-pyridinyl)imidazo[1,2-*b*]pyridazin-6,8-diamin;
*N⁶*-(*cis*-4-Aminocyclohexyl)-*N⁸*-(4-(1*H*-pyrazol-1-yl)phenyl)imidazo[1,2-*b*]pyridazin-6,8-diamin;
*N⁶*-(*cis*-4-Aminocyclohexyl)-*N⁸*-(4-(4-morpholinylcarbonyl)phenyl)imidazo[1,2-*b*]pyridazin-6,8-diamin;
*N⁶*-(*trans*-4-Aminocyclohexyl)-7-methyl-*N⁸*-(2-methylphenyl)imidazo[1,2-*b*]pyridazin-6,8-diamin;
*N⁶*-(*trans*-4-Aminocyclohexyl)-*N⁸*-(2-fluorphenyl)-7-methylimidazo[1,2-*b*]pyridazin-6,8-diamin;
*N⁶*-(*cis*-4-Aminocyclohexyl)-7-methyl-*N⁸*-(2-methylphenyl)imidazo[1,2-*b*]pyridazin-6,8-diamin;
*N⁶*-(*cis*-4-Aminocyclohexyl)-*N⁸*-(2-fluorphenyl)-7-methylimidazo[1,2-*b*]pyridazin-6,8-diamin;
*N*-(6-((*trans*-4-Aminocyclohexyl)amino)imidazo[1,2-*b*]pyridazin-8-yl)benzamid;
1-(6-((*trans*-4-Aminocyclohexyl)amino)imidazo[1,2-*b*]pyridazin-8-yl)-3-phenylharnstoff;
*N,N*-Bis(4-trans-aminocyclohexyl)imidazo[1,2-*b*]pyridazin-6,8-diamin;
*N⁶*-(*trans*-4-Aminocyclohexyl)-*N⁸*-(4-ethyloxyphenyl)-7-phenylimidazo[1,2-*b*]pyridazin-6,8-diamin;
*N⁶*-(*trans*-4-Aminocyclohexyl)-*N⁸*-(phenyl)-7-phenylimidazo[1,2-*b*]pyridazin-6,8-diamin;
*N⁶*-(*cis*-4-Aminocyclohexyl)-*N⁸*-(phenyl)-7-phenylimidazo[1,2-*b*]pyridazin-6,8-diamin;
*N⁶*-(*cis*-4-Aminocyclohexyl)-*N⁸*-(4-ethoxyphenyl)-7-phenylimidazo[1,2-*b*]pyridazin-6,8-diamin;
4-((6-((*trans*-4-Aminocyclohexyl)amino)imidazo[1,2-*b*]pyridazin-8-yl)amino)-*N-*(2-(4-pyridinyl)ethyl)benzamid
4-((6-((*trans*-4-Aminocyclohexyl)amino)imidazo[1,2-*b*]pyridazin-8-yl)amino)-*N-*(2-furanylmethyl)benzamid;
4-((6-((*trans*-4-Aminocyclohexyl)amino)imidazo[1,2-*b*]pyridazin-8-yl)amino)-*N-*(1*H*-imidazol-4-ylmethyl)benzamid;
4-((6-((*trans*-4-Aminocyclohexyl)amino)imidazo[1,2-*b*]pyridazin-8-yl)amino)-*N-*(3-pyridinylmethyl)benzamid;
*N⁶*-(*trans*-4-Aminocyclohexyl)-*N⁸*-(4-((4-phenyl-1-piperidinyl)carbonyl)phenyl)imidazo[1,2-*b*]pyridazin-6,8-diamin;
*N⁶*-(*trans*-4-Aminocyclohexyl)-*N⁸*-(4-(1-pyrrolidinylcarbonyl)phenyl)imidazo[1,2-*b*]pyridazin-6,8-diamin;
*N⁶*-(*trans*-4-Aminocyclohexyl)-*N⁸*-(4-(1-piperidinylcarbonyl)phenyl)imidazo[1,2-*b*]pyridazin-6,8-diamin;
4-((6-((*trans*-4-Aminocyclohexyl)amino)imidazo[1,2-*b*]pyridazin-8-yl)amino)-*N-*(phenylmethyl)benzamid;
4-((6-((*trans*-4-Aminocyclohexyl)amino)imidazo[1,2-*b*]pyridazin-8-yl)amino)-*N-*(3-(methyloxy)phenyl)benzamid;
*N⁶*-(*trans*-4-Aminocyclohexyl)-*N⁸*-(4-((3-phenyl-1-pyrrolidinyl)carbonyl)phenyl)imidazo[1,2-*b*]pyridazin-6,8-diamin;
3-((6-((*trans*-4-Aminocyclohexyl)amino)imidazo[1,2-*b*]pyridazin-8-yl)amino)-*N-*phenylbenzamid;
4-((6-((*trans*-4-Aminocyclohexyl)amino)imidazo[1,2-*b*]pyridazin-8-yl)amino)-*N-*(2-hydroxyethyl)benzamid;
4-((6-((*trans*-4-Aminocyclohexyl)amino)imidazo[1,2-*b*]pyridazin-8-yl)amino)-*N-*(3-(2-oxo-1-pyrrolidinyl)propyl)benzamid; *N⁶*-(*trans*-4-Aminocyclohexyl)-*N⁸*-(4-(4-morpholinylcarbonyl)phenyl)imidazo[1,2-*b*]pyridazin-6,8-diamin;
4-((6-((*trans*-4-Aminocyclohexyl)amino)imidazo[1,2-*b*]pyridazin-8-yl)amino)-*N-*methyl-*N*-(2-(2-pyridinyl)ethyl)benzamid;
4-((6-((*trans*-4-Aminocyclohexyl)amino)imidazo[1,2-*b*]pyridazin-8-yl)amino)-*N,N*-diethylbenzamid;
4-((6-((*trans*-4-Aminocyclohexyl)amino)imidazo[1,2-*b*]pyridazin-8-yl)amino)-*N-*cyclopropylbenzamid;
4-((6-((*trans*-4-Aminocyclohexyl)amino)imidazo[1,2-*b*]pyridazin-8-yl)amino)-*N-*(cyclohexylmethyl)benzamid;
4-((6-((trans-4-Aminocyclohexyl)amino)imidazo[1,2-*b*]pyridazin-8-yl)amino)-*N-*3-pyridinylbenzamid;
4-((6-((*trans*-4-Aminocyclohexyl)amino)imidazo[1,2-*b*]pyridazin-8-yl)amino)-*N-*(1-methyl-1*H*-pyrazol-5-yl)benzamid;
4-((6-((*trans*-4-Aminocyclohexyl)amino)imidazo[1,2-*b*]pyridazin-8-yl)amino)-*N-*(1-(4-fluorphenyl)-2-oxo-1,2-dihydro-3-pyridinyl)benzamid;
4-((6-((*trans*-4-Aminocyclohexyl)amino)imidazo[1,2-*b*]pyridazin-8-yl)amino)-*N-*phenylbenzamid;
4-((6-((*trans*-4-Aminocyclohexyl)amino)imidazo[1,2-*b*]pyridazin-8-yl)amino)-*N-*cyclohexylbenzamid;
4-((6-((*trans*-4-Aminocyclohexyl)amino)imidazo[1,2-*b*]pyridazin-8-yl)amino)-*N-*(4-pyridinylmethyl)benzamid;
1-(4-((6-((trans-4-Aminocyclohexyl)amino)imidazo[1,2-*b*]pyridazin-8-yl)amino)phenyl)-3-phenylharnstoff;
1-(4-((6-((*cis*-4-Aminocyclohexyl)amino)-7-methylimidazo[1,2-*b*]pyridazin-8-yl)amino)phenyl)-3-phenylharnstoff;
1-(4-((6-((*trans*-4-Aminocyclohexyl)amino)-7-methylimidazo[1,2-*b*]pyridazin-8-yl)amino)phenyl)-3-phenylharnstoff;
*N*-(4-((6-((*trans*-4-Aminocyclohexyl)amino)imidazo[1,2-*b*]pyridazin-8-yl)amino)phenyl)-1-(4-fluorphenyl)-2-oxo-1,2-dihydro-3-pyridincarboxamid;
*N*-(4-((6-((*trans*-4-Aminocyclohexyl)amino)imidazo[1,2-*b*]pyridazin-8-yl)amino)-phenyl)-1-(4-fluorphenyl)-2-oxo-1,2-dihydro-3-pyridincarboxamid;
*N*-(4-((6-((*trans*-4-Aminocyclohexyl)amino)imidazo[1,2-*b*]pyridazin-8-yl)amino)-2-ethylphenyl)-1-(4-fluorphenyl)-2-oxo-1,2-dihydro-3-pyridincarboxamid;
*N*-(4-((6-((*trans*-4-Aminocyclohexyl)amino)imidazo[1,2-*b*]pyridazin-8-yl)amino)phenyl)benzamid;
*N*-(4-((6-((*trans*-4-Aminocyclohexyl)amino)imidazo[1,2-*b*]pyridazin-8-yl)amino)phenyl)acetamid;
3-((6-((*trans*-4-Aminocyclohexyl)amino)-7-methylimidazo[1,2-*b*]pyridazin-8-yl)amino)phenol;
*N*-(4-((6-Chlorimidazo[1,2-*b*]pyridazin-8-yl)amino)phenyl)-1-(4-fluorphenyl)-2-oxo-1,2-dihydro-3-pyridincarboxamid;
1-(4-Fluorphenyl)-*N*-(4-(imidazo[1,2-*b*]pyridazin-8-ylamino)phenyl)-2-oxo-3-piperidincarboxamid;
1-(4-Fluorphenyl)-*N*-(4-(imidazo[1,2-*b*]pyridazin-8-ylamino)phenyl)-2-oxo-1,2-dihydro-3-pyridincarboxamid;
6-((*trans*-4-Aminocyclohexyl)amino)-8-((4-(ethyloxy)phenyl)amino)imidazo[1,2-*b*]pyridazin-3-carbonitril;
6-((*trans*-4-Aminocyclohexyl)amino)-8-(phenylamino)imidazo[1,2-*b*]pyridazin-3-carbonitril;
6-((4-*trans*-Aminocyclohexyl)amino)-7-methyl-8-(phenylamino)imidazo[1,2-*b*]pyridazin-3-carbonitril;
6-((*trans*)-4-Aminocyclohexylamino)-7-ethyl-8-(phenylamino)imidazo[1,2-*b*]pyridazin-3-carbonitril;
6-((*trans*-4-Aminocyclohexyl)amino)-8-anilino-7-isopropylimidazo[1,2-*b*]pyridazin-3-carbonitril;
*N⁶*-(*trans*-4-Aminocyclohexyl)-*N⁸*-(4-(ethyloxy)phenyl)-3-fluorimidazo[1,2-*b*]pyridazin-6,8-diamin;
*N⁶*-(*trans*-4-Aminocyclohexyl)-*N⁸*-(4-(ethyloxy)phenyl)-3-methylimidazo[1,2-*b*]pyridazin-6,8-diamin;
*N⁶*-(*trans*-4-Aminocyclohexyl)-*N⁸*-(4-(ethyloxy)phenyl)-2,3-dimethylimidazo[1,2-*b*]pyridazin-6,8-diamin;
N-(6-((trans-4-Aminocyclohexyl)amino)imidazo[1,2-b]pyridazin-8-yl)benzensulfonamid;
6-((*trans*-4-Aminocyclohexyl)oxy)-8-anilinoimidazo[1,2-*b*]pyridazin-3-carbonitril;
6-((*trans*-4-Aminocyclohexyl)amino)-8-anilinoimidazo[1,2-*b*]pyridazin-3-carboxamid;
*N⁶*-(*trans*-4-Aminocyclohexyl)-7-ethyl-*N⁸*-phenylimidazo[1,2-*b*]pyridazin-6,8-diamin; und
*N⁶*-(*trans*-4-Aminocyclohexyl)-7-ethyl-*N⁸*-[4-(ethyloxy)phenyl]imidazo[1,2-*b*]pyridazin-6,8-diamin;
ii) oder ein Enantiomer, Diastereomer oder ein pharmazeutisch annehmbares Salz von (i) davon.

15. Pharmazeutische Zusammensetzung, umfassend eine oder mehrere Verbindungen nach Anspruch 1 oder 14 oder einen pharmazeutisch annehmbaren Träger oder ein Verdünnungsmittel.

16. Verbindung nach Anspruch 1 oder 14 zur Verwendung bei der Behandlung, in einem Säugetier, von Pankreatitis (akut oder chronisch), Asthma, Allergien, Atemnotsyndrom bei Erwachsenen, chronisch-obstruktiver Lungenerkrankung, Glomerulonephritis, rheumatoider Arthritis, systemischem Lupus erythematodes, Sklerodermie, chronischer Thyreoiditis, Basedow-Krankheit, Autoimmungastritis, Diabetes, Autoagglutinationsanämie, Autoimmunneutropenie, Thrombozytopenie, atopischer Dermatitis, chronischer aktiver Hepatitis, Myasthenia gravis, Multipler Sklerose, entzündlicher Darmerkrankung, Colitis ulcerosa, Crohn-Krankheit, Psoriasis, Graft-versus-host-Krankheit, durch Endotoxin ausgelöster entzündlicher Reaktion, Tuberkulose, Atherosklerose, Muskeldegeneration, Kachexie, Psoriasisarthritis, Reiter-Syndrom, Gicht, traumatischer Arthritis, Rötelnarthritis, akuter Synovitis, Pankreasinselzellenkrankheit; Krankheiten, charakterisiert durch massive Neutrophileninfiltration; rheumatoider Spondylitis, Gichtarthritis und anderen arthritischen Zuständen, zerebraler Malaria, chronischer entzündlicher Lungenerkrankung, Silikose, Lungensarkoidose, Knochenresorptionskrankheit, Transplantatabstoßung, Fieber und Myalgien aufgrund von Infektion, Kachexie im Anschluss an Infektion, Meloidbildung, Bildung von Narbengewebe, Colitis ulcerosa, Pyrese, Influenza, Osteoporose, Osteoarthritis, akuter myeloischer Leukämie, chronischer myeloischer Leukämie, metastatischem Melanom, Kaposi-Sarkom, multiplem Myelom, Sepsis, septischem Schock und Shigellose; Alzheimer-Krankheit, Parkinson-Krankheit, zerebraler Ischämie oder durch traumatische Verletzung verursachter neurodegenerativer Krankheit; angiogenen Störungen einschließlich von soliden Tumoren, okularer Neovaskularisation und infantilen Hämangiomen; akuter Hepatitisinfektion (einschließlich von Hepatitis A, Hepatitis B und Hepatitis C), HIV-Infektion und CMV-Retinitis, AIDS, ARC oder Malignität und Herpes; Schlaganfall, myokardialer Ischämie, Ischämie bei Schlaganfall-Herzanfällen, Organ-Hyposie, vaskulärer Hyperplasie, kardialer und renaler Reperfusionsverletzung, Thrombose, kardialer Hypertrophie, Thrombin-induzierter Blutplättchenaggregation, Endotoxinämie und/oder toxischem Schocksyndrom, mit Prostaglandin-Endoperoxidasesynthase-2 assoziierten Zuständen, Ödeme, Analgesie, neuromuskulärem Schmerz, Kopfschmerz, durch Krebs verursachtem Schmerz, Zahnschmerz, Arthritisschmerz, Equinem infektiösem Anämievirus; Felinem Immunschwächevirus, Bovinem Immunschwächevirus, Caninem Immunschwächevirus und Pemphigus vulgaris.

17. Verbindung zur Verwendung bei der Behandlung nach Anspruch 16, wobei der Zustand aus Crohns und Colitis ulcerosa, Transplantat-Abstoßung, rheumatoider Arthritis, Psoriasis, Spondylitis ancylopoetica, Psoriasisarthritis und Pemphigus vulgaris ausgewählt ist.

18. Verbindung zur Verwendung bei der Behandlung nach Anspruch 16, wobei der Zustand aus Ischämiereperfusionsverletzung einschließlich von zerebraler IschämieReperfusionsverletzung, die aus Schlaganfall entsteht, und kardialer IschämieReperfusionsverletzung, die aus Myokardinfarzierung entsteht, ausgewählt ist.

19. Verbindung zur Verwendung bei der Behandlung nach Anspruch 16, wobei der Zustand multiples Myelom ist.

## Revendications

1. Composé selon la formule (I), 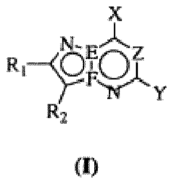 ou un énantiomère, un diastéréomère ou un sel pharmaceutiquement acceptable de celui-ci, dans lequel:
E est C; F est N;
X est NR₄R₅;
Z est CR₃;
Y est sélectionné parmi hydrogène, halogène, nitro, cyano, SR₈, S(O)*ₚ*R₈, OR₈, NR₆R₇, CO₂R₈, C(=O)R₈, O-C(=O)R₈, C(=O)NR₈R₉, cycloalkyle, cycloalcényle, cycloalcynyle, hétérocyclo, aryle et hétéroaryle, à la condition que si Y est hydrogène alors R₄ est phényle substitué par un groupe carboxamido;
R₁ et R₂ sont sélectionnés indépendamment parmi (i) hydrogène, alkyle, halogène, nitro, cyano, SR₁₀, OR₁₀, NR₁₀R₁₁, NR₁₀C(=O)R₁₁, CO₂R₁₀, C(=O)R₁₀, -O-C(=O)R₁₀, C(=O)NR₁₀R₁₁;
R₃ est sélectionné parmi hydrogène, halogène, alkyle, alkyle substitué, alcényle, alcényle substitué, alcynyle, alcynyle substitué, nitro, cyano, SR₁₃, OR₁₃ NR₁₃R₁₄, NR₁₃C(=O)R₁₄, CO₂R₁₃, C(=O)R₁₃, -O-C(=O)R₁₃, -C(=O)NR₁₃R₁₄, cycloalkyle, hétérocyclo, aryle et hétéroaryle;
R₄, R₅, R₆ et R₇ sont sélectionnés indépendamment parmi hydrogène, alkyle, alkyle substitué, alcényle, alcényle substitué, alcynyle, alcynyle substitué, OR₁₅, SR₁₅, C(=O)R₁₅, CO₂R₁₅, C(=O)NR₁₅R₁₆, C(W)OR₁₆, S(O)*ₚ*R₁₇, SO₂NR₁₅R₁₆, cycloalkyle, hétérocyclo, aryle et hétéroaryle; ou bien (ii) R₄ est pris ensemble avec R₅ et l'atome d'azote auquel ils sont attachés tous les deux et/ou R₆ est pris ensemble avec R₇ et l'atome d'azote auquel ils sont attachés tous les deux pour former un hétéroaryle ou un hétérocyclo;
R₈, R₉, R₁₀, R₁₁, R₁₃, R₁₄, R₁₅ et R₁₆ sont sélectionnés indépendamment à chaque occurrence parmi (i) hydrogène, alkyle, alkyle substitué, alcényle, alcényle substitué, alcynyle, alcynyle substitué, cycloalkyle, aryle, hétéroaryle et hétérocyclo; ou bien (ii) ensemble avec l'atome d'azote auquel ils sont attachés, R₈ est pris ensemble avec R₉, et/ou R₁₀ est pris ensemble avec R₁₁, et/ou R₁₃ est pris ensemble avec R₁₄, et/ou R₁₅ est pris ensemble avec R₁₆ pour former un hétéroaryle ou un hétérocyclo;
R₁₇ est sélectionné parmi alkyle, alkyle substitué, alcényle, alcényle substitué, alcynyle, alcynyle substitué, cycloalkyle, aryle, hétéroaryle et hétérocyclo;
W est à chaque occurrence O, S, N, CN ou NH; et
*p* est 1 ou 2;
avec les restrictions suivantes:
(1) si X est NH(Me), N(Me)₂, NH(phényle non substitué) ou NHNH₂, alors Y est autre qu'hydrogène ou halogène; et
(2) les composés suivants sont exclus:

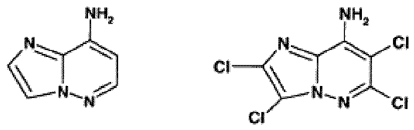

2. Composé selon la revendication 1, ayant la formule (Ia), 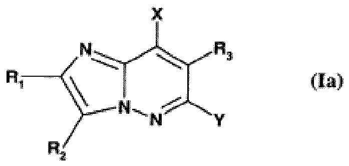 ou un énantiomère, un diastéréomère ou un sel pharmaceutiquement acceptable de celui-ci.

3. Composé selon la revendication 2 ou un énantiomère, un diastéréomère ou un sel pharmaceutiquement acceptable de celui-ci, dans lequel:
R₄ est -AM;
R₅ est hydrogène ou C₁₋₄ alkyle;
ou bien R₄ et R₅ ensemble avec l'atome d'azote auquel ils sont attachés, forment un cycle hétéroaryle ou hétérocyclo monocyclique à 5, 6 ou 7 chaînons ou un cycle hétéroaryle ou hétérocyclo bicyclique de 7 à 11 chaînons, chaque cycle optionnellement substitué par un à trois groupes T₁, T₂ et/ou T₃;
A est une liaison, C₁₋₃ alkylène, C₂₋₄ alcénylène, C₂₋₄ alkynylène, -C(O)- ou -SO₂-;
M est (i) hydrogène, alkyle, alcoxy ou alcényle; ou bien (ii) cycloalkyle, hétérocyclo, aryle ou hétéroaryle, chaque groupe optionnellement substitué par un à trois groupes T₁, T₂ et/ou T₃;
T₁, T₂ et T₃ sont sélectionnés indépendamment parmi (i) halogène, alkyle, alkyle substitué, alcényle, alcényle substitué, alcynyle, alcynyle substitué, nitro, cyano, SO₃H SR₁₉, S(O)*ₚ*R₂₁, S(O)*ₚ*NR₁₉R₂₀, NR₁₉ S(O)*ₚ*R₂₁, OR₁₉, NR₁₉R₂₀, NR₁₉C(=O)₂₀, NR₁₉C(=O)NR₁₉R₂₀, CO₂R₁₉, C(=O)R₁₉, -O-C(=O)R₁₉, -C(=O)NR₁₉R₂₀, cycloalkyle, hétérocyclo, aryle et hétéroaryle, où *p* est un ou 2; et/ou (ii) deux groupes T₁ et T₂ situés sur des atomes cycliques adjacents sont pris ensemble avec les atomes cycliques auxquels ils sont attachés pour former un cycloalkyle, un aryle, un hétéroaryle ou un hétérocyclo fusionné;
R₁₉, R₂₀ et R₂₁ sont sélectionnés indépendamment à chaque occurrence parmi (i) hydrogène, alkyle, alkyle substitué, alcényle, alcényle substitué, cycloalkyle, aryle, hétéroaryle et hétérocyclo; ou bien (ii) R₁₉ et R₂₀ avec l'atome d'azote auquel ils sont attachés tous les deux forment un hétéroaryle ou un hétérocyclo; et
R₂₁ est sélectionné à chaque occurrence parmi alkyle, alkyle substitué, alcényle, alcényle substitué, alcynyle, alcynyle substitué, cycloalkyle, aryle, hétéroaryle et hétérocyclo.

4. Composé selon la revendication 2 ou un énantiomère, un diastéréomère ou un sel pharmaceutiquement acceptable de celui-ci, dans lequel:
Y est hydrogène, halogène, OR₈ ou NR₆R₇;
R₆ est sélectionné parmi hydrogène ou C₁₋₄ alkyle optionnellement substitué par un à trois groupes sélectionnés parmi halogène, C₁₋₄ alkyle, nitro, cyano, amino, C₁₋₄ alcoxy et OH;
R₇ et R₈ sont sélectionnés indépendamment parmi alkyle, cycloalkyle, hétérocyclo, aryle et hétéroaryle, chaque groupe desquels est optionnellement substitué par un à trois groupes T₄, T₅ et/ou T₆;
ou bien R₆ et R₇ avec l'atome d'azote auquel ils sont attachés forment un cycle hétéroaryle ou hétérocyclo, chaque cycle est optionnellement substitué par un à trois groupes T₄, T₅ et/ou T₆;
T₄, T₅ et T₆ sont sélectionnés indépendamment parmi (i) halogène, alkyle, alkyle substitué, alcényle, alcényle substitué, alcynyle, alcynyle substitué, nitro, cyano, SR₁₉, OR₁₉, NR₁₉R₂₀, NR₁₉C(=O)R₂₀, CO₂R₁₉, C(=O)R₁₉, -O-C(=O)R₁₉, -C(=O)NR₁₉R₂₀, cycloalkyle, hétérocyclo, aryle et hétéroaryle; et/ou (ii) deux groupes T₄ et T₅ substitués sur des atomes cycliques adjacents sont pris ensemble avec les atomes cycliques auxquels ils sont attachés pour former un cycloalkyle, un hétérocyclo, un aryle ou un hétéroaryle fusionné; et R₁₉ et R₂₀ sont sélectionnés indépendamment à chaque occurrence parmi (i) hydrogène, alkyle, alkyle substitué, alcényle, alcényle substitué, cycloalkyle, aryle, hétéroaryle et hétérocyclo; ou bien (ii) R₁₉ et R₂₀ ensemble avec l'atome d'azote auquel ils sont attachés tous les deux se combinent pour former un hétéroaryle ou un hétérocyclo.

5. Composé de la formule (Ia): 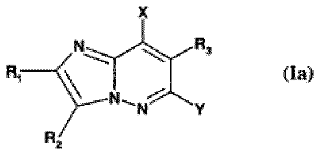 ou un énantiomère, un diastéréomère ou un sel pharmaceutiquement acceptable de celui-ci, dans lequel:
X est NR₄R₅;
Y est hydrogène, halogène, OR₈ ou NR₆R₇;
R₁ et R₂ sont sélectionnés indépendamment parmi (i) hydrogène, alkyle, halogène, nitro, cyano, SR₁₀, OR₁₀, NR₁₀R₁₁, NR₁₀C(=O)R₁₁, CO₂R₁₀, C(=O)R₁₀, -O-C(=O)R₁₀, C(=O)NR₁₀R₁₁,
R₃ est sélectionné parmi hydrogène, halogène, alkyle, alkyle substitué, alcényle, alcényle substitué, alcynyle, alcynyle substitué, nitro, cyano, SR₁₃, OR₁₃, NR₁₃R₁₄, NR₁₃C(=O)R₁₄, CO₂R₁₃, C(=O)R₁₃, -O-C(=O)R₁₃, -C(=O)NR₁₃R₁₄, cycloalkyle, hétérocyclo, aryle et hétéroaryle;
R₄ est -AM;
R₅ est hydrogène ou C₁₋₄ alkyle;
ou bien R₄ et R₅ avec l'atome d'azote auquel ils sont attachés forment un cycle hétéroaryle ou hétérocyclo monocyclique à 5, 6 ou 7 chaînons ou un cycle hétéroaryle ou hétérocyclo bicyclique de 7 à 11 chaînons, chaque cycle optionnellement substitué par un à trois groupes T₁, T₂ et/ou T₃;
A est une liaison, C₁₋₃ alkylène, C₂₋₄ alcénylène, C₂₋₄ alcynylène, -C(O)- ou -SO₂-; M est (i) hydrogène, NR₁₅R₁₆, alkyle, alcoxy ou alcényle; ou bien (ii) cycloalkyle, hétérocyclo, aryle ou hétéroaryle, chaque cycle optionnellement substitué par un à trois groupes T₁, T₂ et/ou T₃;
R₆ est sélectionné parmi hydrogène ou C₁₋₄ alkyle optionnellement substitué par un à trois groupes sélectionnés parmi halogène, C₁₋₄ alkyle, nitro, cyano, amino, C₁₋₄ alcoxy et OH;
R₇ est sélectionné parmi alkyle, cycloalkyle, hétérocyclo, aryle et hétéroaryle, chaque groupe desquels est optionnellement substitué par un à trois groupes T₄, T₅ et/ou T₆;
ou bien R₆ et R₇ ensemble avec l'atome d'azote auquel ils sont attachés forment un cycle hétéroaryle ou hétérocyclo, chaque cycle est optionnellement substitué par un à trois groupes T₄, T₅ et/ou T₆;
R₈ est sélectionné parmi alkyle, cycloalkyle, hétérocyclo, aryle et hétéroaryle, chaque groupe desquels est optionnellement substitué par un à trois groupes T₄, T₅ et/ou T₆;
R₁₀, R₁₁, R₁₃ et R₁₄ sont sélectionnés indépendamment à chaque occurrence parmi (i) hydrogène, C₁₋₄ alkyle et C₁₋₄ alkyle substitué; ou bien (ii) R₁₀ et R₁₁ ensemble avec l'atome d'azote auquel ils sont attachés tous les deux et/ou R₁₃ et R₁₄ ensemble avec l'atome d'azote auquel ils sont attachés tous les deux se combinent pour former un hétéroaryle ou un hétérocyclo à 5, 6 ou 7 chaînons optionnellement substitué;
R₁₅ et R₁₆ sont sélectionnés indépendamment parmi (i) hydrogène, alkyle, alkyle substitué, alcényle, alcényle substitué, alcynyle, alcynyle substitué, cycloalkyle, aryle, hétéroaryle et hétérocyclo; ou bien (ii) ensemble avec l'atome d'azote auquel ils sont attachés R₁₅ est pris ensemble avec R₁₆ pour former un hétéroaryle ou un hétérocyclo;
T₁, T₂ et T₃ sont sélectionnés indépendamment parmi (i) halogène, alkyle, alkyle substitué, alcényle, alcényle substitué, alcynyle, alcynyle substitué, nitro, cyano, SO₃H SR₁₉, S(O)*ₚ*R₂₁, S(O)*ₚ*NR₁₉R₂₀, NR₁₉ S(O)*ₚ*R₂₁, OR₁₉, NR₁₉R₂₀, NR₁₉C(=O)₂₀, NR₁₉C(=O)NR₁₉R₂₀, CO₂R₁₉, C(=O)R₁₉, -O-C(=O)R₁₉, -C(=O)NR₁₉R₂₀, cycloalkyle, hétérocyclo, aryle et hétéroaryle, où *p* est un ou 2; et/ou (ii) deux groupes T₁ et T₂ situés sur des atomes cycliques adjacents sont pris ensemble avec les atomes cycliques auxquels ils sont attachés pour former un cycloalkyle, aryle, hétéroaryle ou hétérocyclo fusionné;
T₄, T₅ et T₆ sont sélectionnés indépendamment parmi (i) halogène, alkyle, alkyle substitué, alcényle, alcényle substitué, alcynyle, alcynyle substitué, nitro, cyano, SR₁₉, OR₁₉, NR₁₉R₂₀, NR₁₉C(=O)R₂₀, CO₂R₁₉, C(=O)R₁₉, -O-C(=O)R₁₉, -C(=O)NR₁₉R₂₀, cycloalkyle, hétérocyclo, aryle et hétéroaryle; et/ou (ii) deux groupes T₄ et T₅ substitués sur des atomes cycliques adjacents sont pris ensemble avec les atomes cycliques auxquels ils sont attachés pour former un cycloalkyle, hétérocyclo, aryle ou hétéroaryle fusionné; et
R₁₉ et R₂₀ sont sélectionnés indépendamment à chaque occurrence parmi (i) hydrogène, alkyle, alkyle substitué, alcényle, alcényle substitué, cycloalkyle, aryle, hétéroaryle et hétérocyclo; ou bien (ii) R₁₉ et R₂₀ ensemble avec l'atome d'azote auquel ils sont attachés tous les deux forment un cycle hétéroaryle ou hétérocyclo; et
R₂₁ est sélectionné à chaque occurrence parmi alkyle, alkyle substitué, alcényle, alcényle substitué, alcynyle, alcynyle substitué, cycloalkyle, aryle, hétéroaryle et hétérocyclo;
avec les restrictions suivantes:
(1) si X est NH(Me), N(Me)₂, NH(phényle non substitué) ou NHNH₂, alors Y est autre qu'hydrogène ou halogène; et
(2) les composés suivants sont exclus:

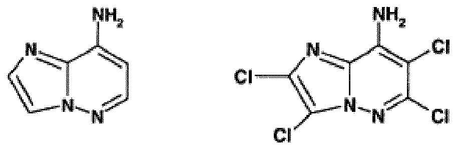

6. Composé selon la revendication 5 ou un énantiomère, un diastéréomère ou un sel pharmaceutiquement acceptable de celui-ci, dans lequel:
R₄ est -AM;
A est une liaison, -C(O)- ou -S(O)₂- ou C₁₋₃ alkylène;
M est (i) hydrogène, -NH(aryle), C₁₋₆ alkyle, C₂₋₄ alcényle ou -OC₁₋₄ alkyle ou bien (ii) C₃₋₆ cycloalkyle, phényle, fluorényle, 1-naphtyle ou 2-naphtyle, chaque groupe optionnellement substitué par un à trois groupes T₁, T₂ et/ou T₃; ou bien (iii) un cycle hétéroaryle ou hétérocyclo monocyclique à 5, 6 ou 7 chaînons ou bicyclique de 7 à 11 chaînons, chaque cycle optionnellement substitué par un à trois groupes T₁, T₂ et/ou T₃; et
T₁, T₂ et T₃ sont sélectionnés indépendamment parmi (i) C₁₋₄ alkyle, C₁₋₄ alkyle substitué, C₁₋₄ alkyloxy, C₁₋₄ alkyloxy substitué, C₁₋₄ alkyloxy substitué, C₁₋₄ alkylthio, phénoxy, -NR₁₉R₂₀, halogène, hydroxy, cyano, SO₃H, COOH, -C(O)(R₁₉), C(O)NR₁₉R₂₀, NR₁₉C(O)R₂₀, S(O)₂R₂₁, S(O)₂NR₁₉R₂₀ et NR₁₉(C(O)NR₁₉R₂₀; et/ou (ii) phényle, cyclopropyle, cyclohexyle, tétrazolyle, imidazolyle, pyrazolyle, triazolyle, thiazolyle, furyle et morpholinyle, chaque groupe desquels est optionnellement substitué comme la valence le permet, par de un à trois groupes R₂₂, R₂₃ et/ou R₂₄; et/ou (iii) deux groupes T₁, T₂ substitués sur des atomes cycliques adjacents sont pris ensemble avec les atomes cycliques auxquels ils sont attachés pour former un cycloalkyle fusionné de cinq à sept chaînons, un phényle fusionné ou bien un hétérocyclo ou un hétéroaryle fusionné à 5 ou 6 chaînons, chaque groupe desquels est optionnellement substitué comme la valence le permet, par un à trois groupes R₂₂, R₂₃ et/ou R₂₄; et
R₁₉ et R₂₀ sont sélectionnés indépendamment à chaque occurrence parmi (i) hydrogène, -(CH₂)ᵥOH et C₁₋₄ alkyle; ou bien (ii) -(CH₂)ᵥcyclohexyle, -(CH₂)ᵥphényle, - (CH₂)ᵥmorpholinyle, -(CH₂)ᵥpyridyle, -(CH₂)ᵥpyrazolyle, -(CH₂)ᵥcyclopropyle, - (CH₂)ᵥpyrrolidinyle, -(CH₂)ᵥpipéridinyle, -(CH₂)ᵥfuryle, -(CH₂)ᵥimidazolyle, - (CH₂)ᵥpyrimidinyle, -(CH₂)ᵥpipérazinyle et -(CH₂)ᵥpyradizinyle, chaque groupe desquels est optionnellement substitué comme la valence le permet, par de un à trois groupes R₂₂, R₂₃ et/ou R₂₄; ou bien R₁₉ et R₂₀ sont pris ensemble avec l'atome d'azote auquel ils sont attachés tous les deux pour former un pyrrolidinyle, morpholinyle, pipéridinyle, pyradazinyle ou pipérazinyle, chaque groupe desquels est optionnellement substitué comme la valence le permet, par de un à trois groupes R₂₂, R₂₃ et/ou R₂₄;
R₂₁ est sélectionné à chaque occurrence parmi (i) -(CH₂)ᵥOH et C₁₋₄ alkyle; ou bien (ii) -(CH₂)ᵥcyclohexyle, -(CH₂)ᵥphényle, -(CH₂)ᵥmorpholinyle, -(CH₂)ᵥpyridyle, - (CH₂)ᵥpyrazolyle, -(CH₂)ᵥcyclopropyle, -(CH₂)ᵥpyrrolidinyle, -(CH₂)ᵥpipéridinyle, - (CH₂)ᵥfuryle, -(CH₂)ᵥimidazolyle, -(CH₂)ᵥpyrimidinyle, -(CH₂)ᵥpipérazinyle et - (CH₂)ᵥpyradizinyle, chaque groupe desquels est optionnellement substitué comme la valence le permet, par de un à trois groupes R₂₂, R₂₃ et/ou R₂₄; R₂₂, R₂₃ et R₂₄ sont sélectionnés indépendamment à chaque occurrence parmi (C₁₋₄)alkyle, (C₂₋₄)alcényle, halogène, hydroxy, cyano, nitro, CF₃, =O, O(C₁₋₄ alkyle), OCF₃, C(=O)H, C(=O)(C₁₋₄ alkyle), CO₂H, CO₂(C₁₋₄ alkyle), NHCO₂(C₁₋₄ alkyle), -S(C₁₋₄ alkyle), -NH₂, NH(C₁₋₄ alkyle), N(C₁₋₄ alkyle)₂, N(C₁₋₄ alkyle)₃⁺, SO₂(C₁₋₄ alkyle), C(=O)(C₁₋₄ alkylène)NH₂, C(=O)(C₁₋₄ alkylène)NH(alkyle), -C(=O)(C₁₋₄ alkylène)N(C₁₋₄ alkyle)₂ et optionnellement substitués phényle; et v est 0, 1, 2 ou 3.

7. Composé selon la revendication 6 ou un énantiomère, un diastéréomère ou un sel pharmaceutiquement acceptable de celui-ci, dans lequel NR₄R₅ est sélectionné parmi: 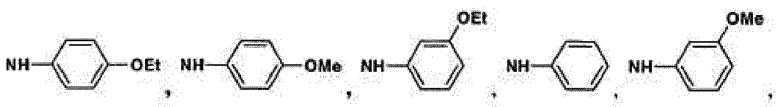 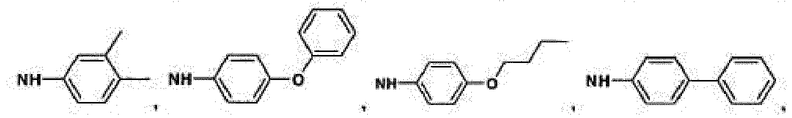 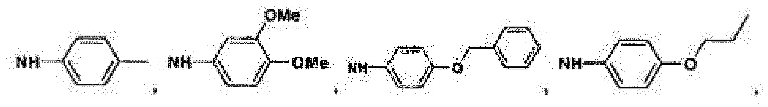 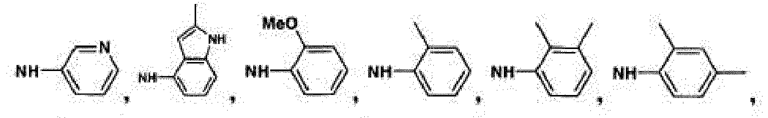 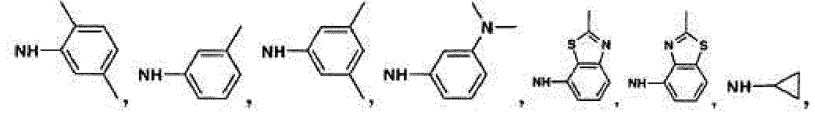 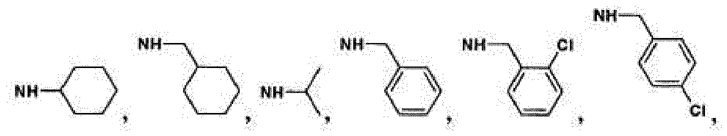 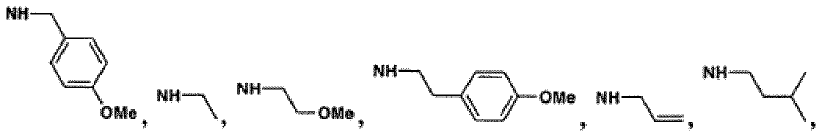 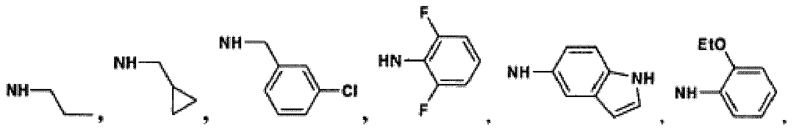 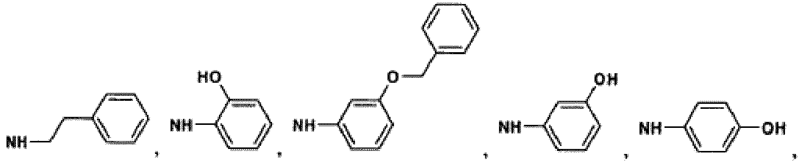 **NH₂,**

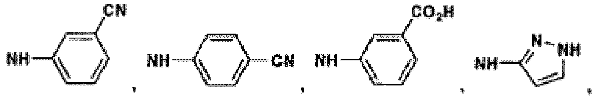

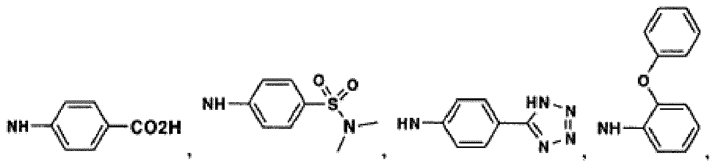

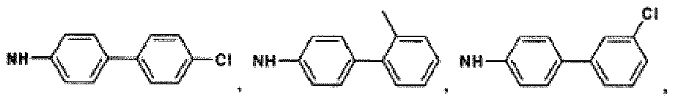

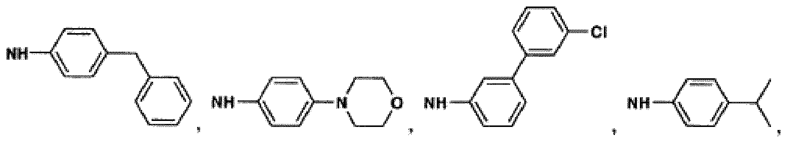

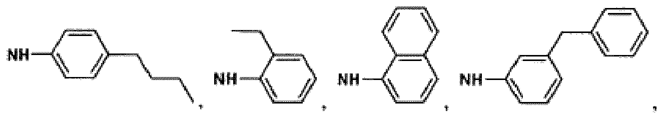

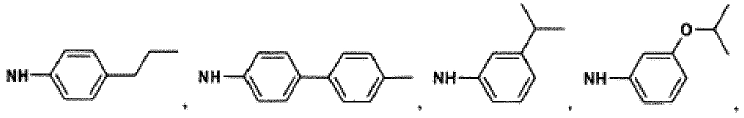

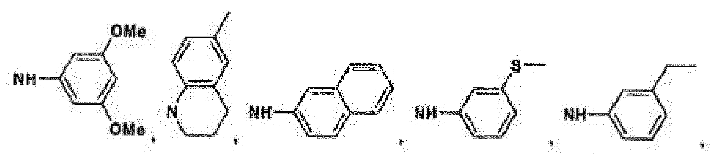

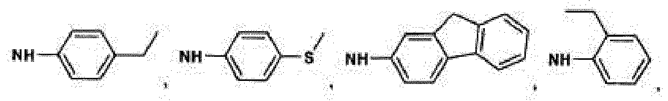

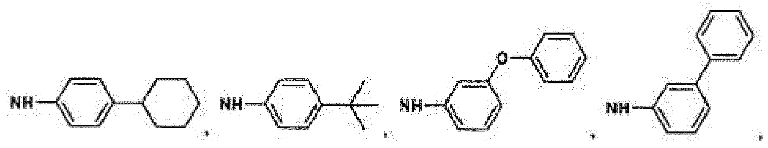

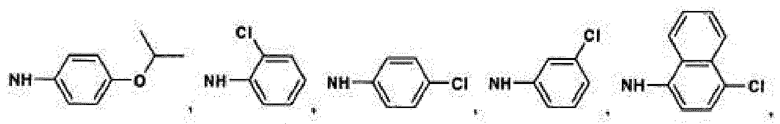

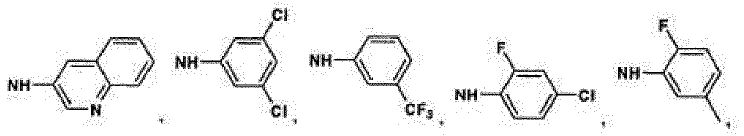

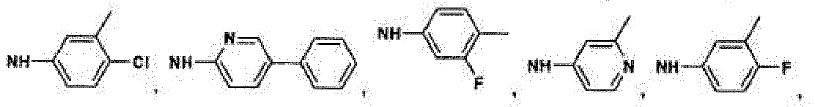

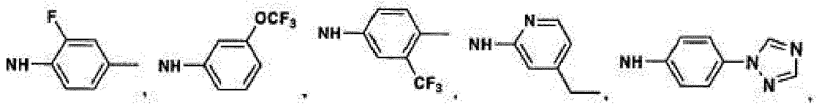

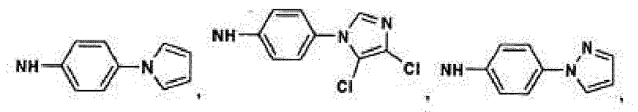

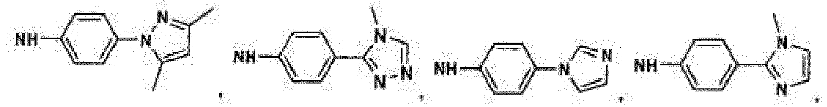

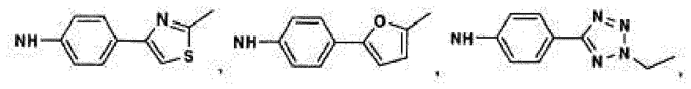

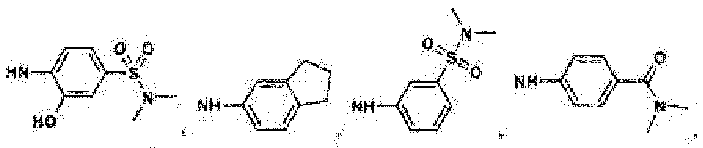

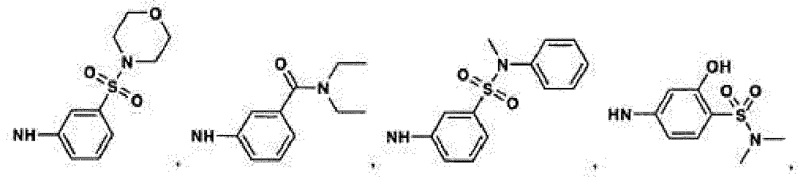

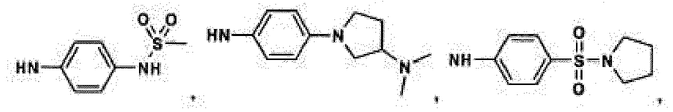

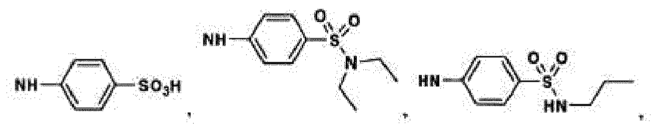

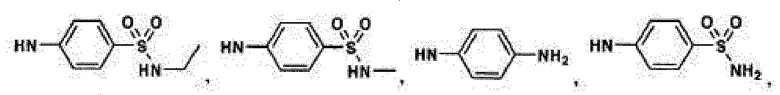

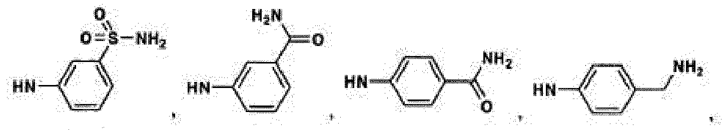

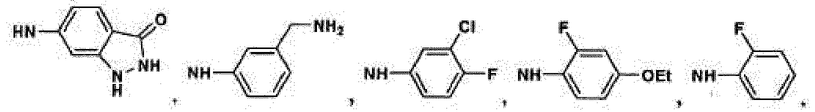

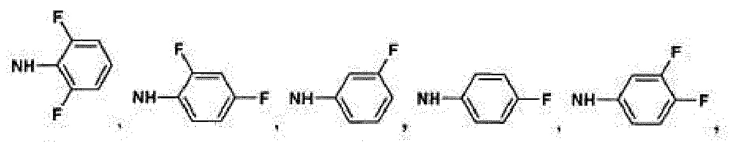

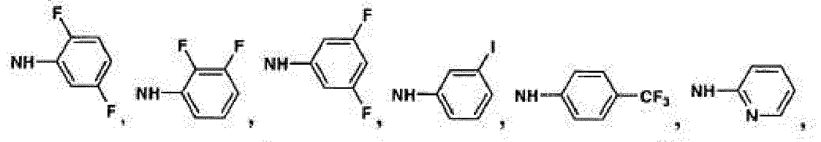

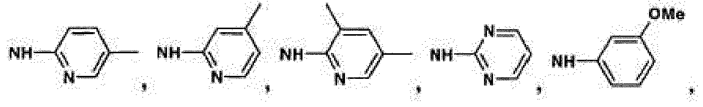

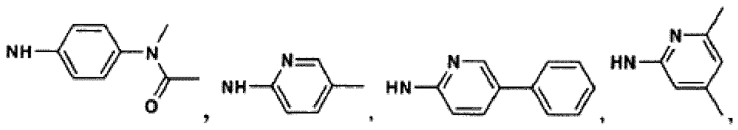

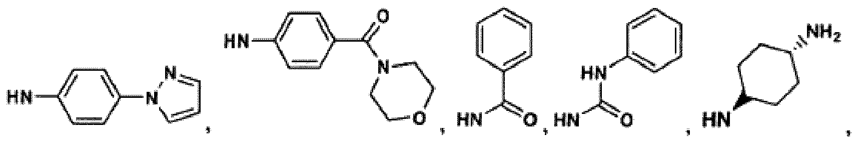

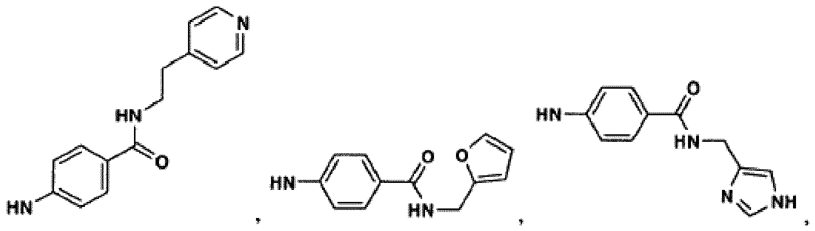

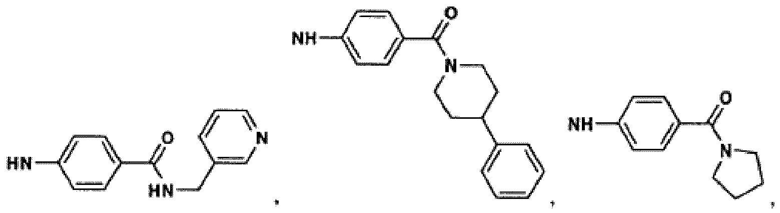

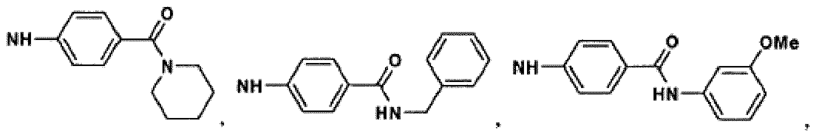

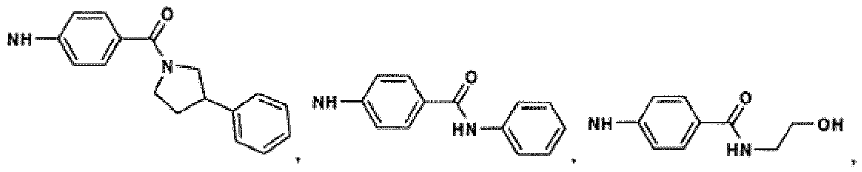

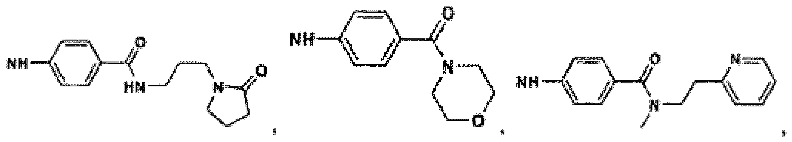

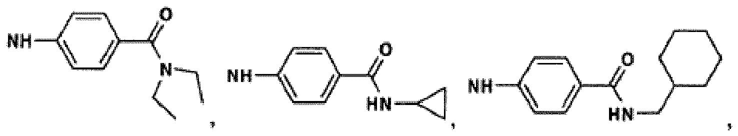

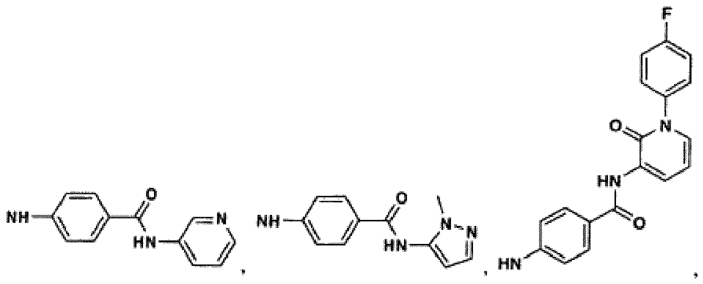

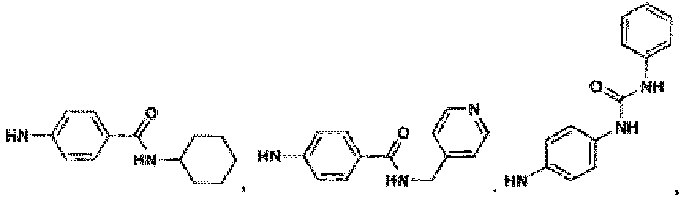

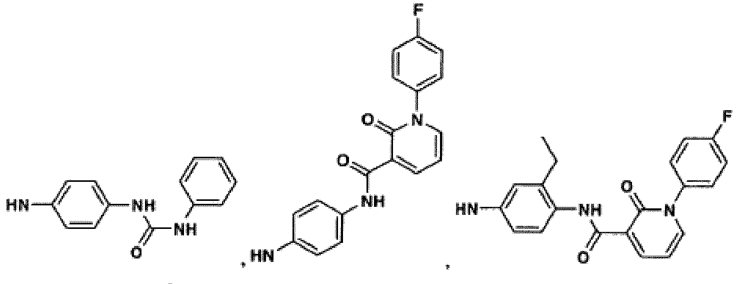

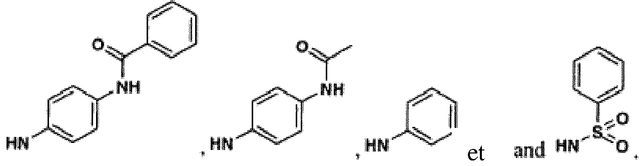

8. Composé selon la revendication 5 ou un énantiomère, un diastéréomère ou un sel pharmaceutiquement acceptable de celui-ci, dans lequel:
Y est NR₆R₇;
R₆ est sélectionné parmi hydrogène ou C₁₋₄ alkyle;
R₇ est sélectionné parmi C₁₋₄ alkyle, cyclopentyle, cyclohexyle, bicyclo[2.2.2]octyle, pyrrolidinyle et pipéridinyle, chaque groupe desquels est optionnellement substitué par un à trois groupes T₄, T₅ et/ou T₆;
ou bien R₆ et R₇ ensemble avec l'atome d'azote auquel ils sont attachés forment un pipérazinyle, pipéridinyle, pyrrolidinyle ou diazépanyle, chaque groupe desquels est optionnellement substitué par un à trois groupes T₄, T₅ et/ou T₆; et
T₄, T₅ et T₆ sont sélectionnés indépendamment parmi (i) C₁₋₄alkyle, OH, NH₂, NH(C₁₋₄ alkyle), furyle et N(C₁₋₄ alkyle)₂ et NH(pyrimidinyle) où le pyrimidinyle est substitué par halogène; ou bien (ii) C₁₋₄ alkyle substitué par cyclohexyle ou OH, où le cyclohexyle est substitué par NH₂.

9. Composé selon la revendication 8 ou un énantiomère, un diastéréomère ou un sel pharmaceutiquement acceptable de celui-ci, dans lequel NR₆R₇ est sélectionné parmi:
NH-(CH₂)₂-NH₂, NH-(CH₂)₄-NH₂.

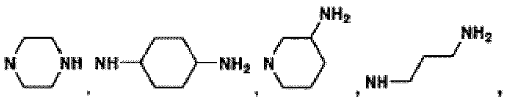

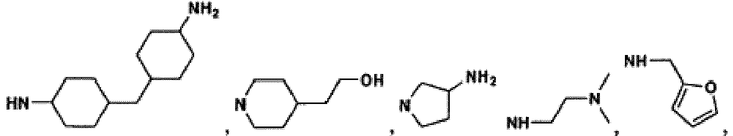

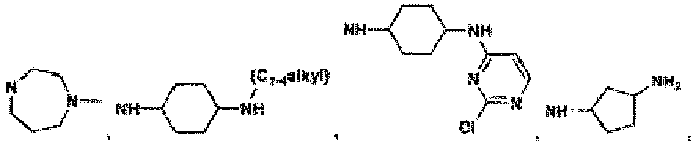

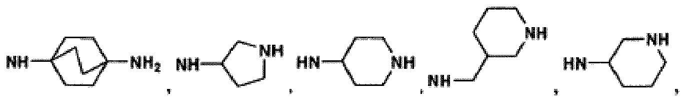

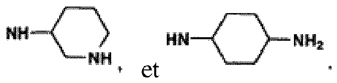

10. Composé selon la revendication 8 ou un énantiomère, un diastéréomère ou un sel pharmaceutiquement acceptable de celui-ci, dans lequel NR₆R₇ est sélectionné parmi: 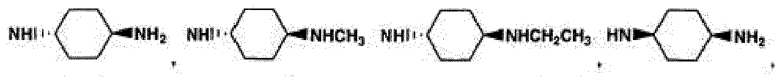 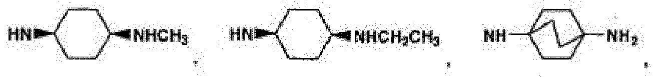 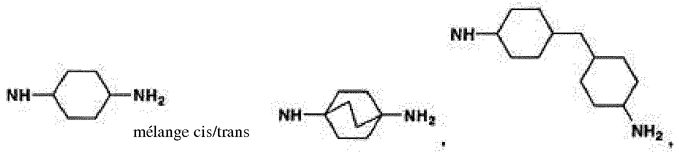 et

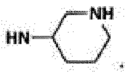

11. Composé selon la revendication 5 ou un énantiomère, un diastéréomère ou un sel pharmaceutiquement acceptable de celui-ci, dans lequel R₅ est hydrogène et R₄ est sélectionné parmi un cycle phényle, pyridyle, pyrimidinyle, cyclohexyle et pipéridinyle, chaque cycle optionnellement substitué par un à deux groupes T₁ et/ou T₂.

12. Composé selon la revendication 1 ou 5 ou un énantiomère, un diastéréomère ou un sel pharmaceutiquement acceptable de celui-ci, dans lequel:
R₁ et R₂ sont sélectionnés indépendamment parmi hydrogène, halogène, OR₁₀, cyano, C₁₋₄ alkyle, CO₂R₁₀ et C(O)NR₁₀R₁₁;
R₃ est sélectionné parmi (i) hydrogène, halogène, nitro, cyano, OR₁₀, NR₁₀R₁₁, CO₂R₁₀ et C(=O)R₁₀, (ii) C₁₋₄ alkyle, C₁₋₄ alkyle substitué, cycloalkyle, aryle et hétéroaryle; et
R₁₀ et R₁₁ sont sélectionnés indépendamment parmi (i) hydrogène, C₁₋₄ alkyle et C₁₋₄ alkyle substitué; ou bien (ii) R₁₀ et R₁₁ ensemble avec l'atome d'azote auquel ils sont attachés tous les deux se combinent pour former un hétéroaryle ou un hétérocyclo à 5, 6 ou 7 chaînons optionnellement substitué.

13. Composé selon la revendication 1 ou 5, où le composé a la formule (Ia), 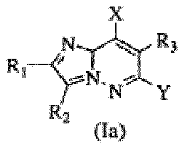 ou un énantiomère, un diastéréomère ou un sel pharmaceutiquement acceptable de celui-ci.

14. Composé sélectionné parmi les suivants:
(i)
*N*⁶-(*trans*-4-aminocyclohexyl])-*N*⁸-[4-(éthyloxy)phényl]imidazo[1,2-*b*]pyridazine-6,8-diamine,
*N*⁶-(2-aminoéthyl)-*N*⁸-(4-(éthyloxy)phényl)imidazo [1,2-*b*]pyridazine-6,8-diamine;
*N*⁶-(4-aminobutyl)-*N*⁸-(4-(éthyloxy)phényl)imidazo [1,2-*b*]pyridazine-6,8-diamine;
7-chloro-*N*-(4-(éthyloxy)phényl)-6-(1-pipérazinyl)imidazo[1,2-*b*]pyridazin-8-amine;
*N*⁶-(*trans*-4-aminocyclohexyl)-*N*⁸-(4-(méthyloxy)phényl)imidazo[1,2-*b*]pyridazine-6,8-diamine;
*N*⁶-(*trans*-4-aminocyclohexyl)-*N*⁸-(3-(éthyloxy)phényl)imidazo[1,2-*b*]pyridazine-6,8-diamine;
*N*⁶-(*trans-4-*aminocyclohexyl)-*N*⁸-phénylimidazo[1,2-*b*]pyridazine-6,8-diamine;
*N*⁶-(*trans*-4-aminocyclohexyl)-*N*⁸-(3-(méthyloxy)phény)imidazo[1,2-*b*]pyridazine-6,8-diamine;
*N*⁶-(*trans*-4-aminocyclohexyl)-*N*⁸-(3,4-diméthylphényl)imidazo[1,2-*b*]pyridazine-6,8-diamine;
*N*⁶-(*trans*-4-aminocyclohexyl)-*N*⁸-(4-(phényloxy)phényl)imidazo[1,2-*b*]pyridazine-6,8-diamine;
*N*⁶-(*trans*-4-aminocyclohexyl)-*N*⁸-(4-(butyloxy)phényl)imidazo[1,2-*b*]pyridazine-6,8-diamine;
*N*⁶-(*trans*-4-aminocyclohexyl)-*N*⁸-4-biphénylylimidazo[1,2-*b*]pyridazine-6,8-diamine;
*N*⁶-(*trans*-4-aminocyclohexyl)-*N*⁸-(4-méthylphényl)imidazo[1,2-*b*]pyridazine-6,8-diamine;
*N*⁶-(*trans*-4-aminocyclohexyl)-*N*⁸-(3,4-bis(méthyloxy)phényl)imidazo[1,2-*b*]pyridazine-6,8-diamine;
*N*⁶-(*trans*-4-aminocyclohexyl)-*N*⁸-(4-((phénylméthyl)oxy)phényl)imidazo[1,2-*b*]pyridazine-6,8-diamine;
*N*⁶-(*trans*-4-aminocyclohexyl)-*N*⁸-(4-(propyloxy)phényl)imidazo[1,2-*b*]pyridazine-6,8-diamine;
*N*⁶-(*trans*-4-aminocyclohexyl)-*N*⁸-pyridin-3-ylimidazo[1,2-*b*]pyridazine-6,8-diamine;
*N*⁶-(*trans*-4-aminocyclohexyl)-*N*⁸-(2-méthyl-1*H*-indo-5-y)imidazo[1,2-*b*]pyridazine-6,8-diamine;;
*N*⁶-(*trans*-4-aminocyclohexyl)-*N*⁸-méthyl-*N*⁸-phénylimidazo[ 1,2-*b*]pyridazine-6,8-diamine;;
*N*⁶-(*trans*-4-aminocyclohexyl)-*N*⁸-[2-(méthyloxy)phényl]imidazo[1,2-*b*]pyridazine-6,8-diamine;
*N*⁶-(*trans*-4-aminocyclohexyl)-*N*⁸-(2-méthylphényl)imidazo[1,2-*b*]pyridazine-6,8-diamine;
*N*⁶-(*trans*-4-aminocyclohexyl)-*N*⁸-(2,3-diméthylphényl)imidazo[1,2-*b*]pyridazine-6,8-diamine;
*N*⁶-(*trans*-4-aminocyclohexyl)-*N*⁸-(2,4-diméthylphényl)imidazo[1,2-*b*]pyridazine-6,8-1diamine;
*N*⁶-(*trans*-4-aminocyclohexyl)-*N*⁸-(2,5-diméthylphényl)imidazo[1,2-*b*]pyridazine-6,8-diamine;
*N*⁶*-*(*trans*-4-aminocyclohexyl)-*N*⁸-(3-méthylphényl)imidazo[1,2-*b*]pyridazine-6,8-diamine;
*N*⁶-(*trans*-4-aminocyclohexyl)-*N*⁸-(3,5-diméthylphényl)imidazo[1,2-*b*]pyridazine-6,8-diamine;
*N*⁶-(*trans*-4-aminocyclohexyl)-*N*⁸[3-(diméthylamino)phényl]imidazo[1,2-*b*]pyridazine-6,8-diamine;
*N*⁶-(*trans*-4-aminocyclohexyl)-*N*⁸-(2-méthyl-1,3-benzothiazol-6-yl)imidazo[1,2-*b*]pyridazine-6,8-diamine;
*N*⁶-(*trans*-4-aminocyclohexyl)-*N*⁸-(2-méthyl-1,3-benzothiazol-5-yl)imidazo[1,2-*b*]pyridazine-6,8-diamine;
*N*⁶-(*trans*-4-aminocyclohexyl)-*N*⁸-cyclopropylimidazo[1,2-*b*]pyridazine-6,8-diamine;
*N*⁶-(*trans*-4-aminocyclohexyl)-*N*⁸-cyclohexylimidazo[1,2-*b*]pyridazine-6,8-diamine;
*N*⁶-(*trans*-4-aminocyclohexyl)-*N*⁸-(cyclohexylméthyl)imidazo[1,2-*b*]pyridazine-6,8-diamine;
*N*⁶-(*trans*-4-aminocyclohexyl)-*N*⁸-(1-méthyléthyl)imidazo[1,2-*b*]pyridazine-6,8-diamine;
*N*⁶-(trans-4-aminocyclohexyl)-*N*⁸-(phénylméthyl)imidazo[1,2-*b*]pyridazine-6,8-diamine;
*N*⁶-(trans-4-aminocyclohexyl)-*N*⁸-[(2-chlorophényl)méthyl]imidazo[1,2-*b*]pyridazine-6,8-diamine;
*N*⁶-(trans-4-aminocyclohexyl)-*N*⁸((4-chlorophényl)méthyl)imidazo[1,2-*b*]pyridazine-6,8-diamine;
*N*⁶-(trans-4-aminocyclohexyl)-*N*⁸((4-(méthyloxy)phényl)méthyl)imidazo[1,2-*b*]pyridazine-6,8-diamine;
*N*⁶-(trans-4-aminocyclohexyl)-*N*⁸-éthylimidazo[1,2-*b*]pyridazine-6,8-diamine;
*N*⁶-(*trans*-4-aminocyclohexyl)-*N*⁸-(2-(méthyloxy)éthyl)imidazo[1,2-*b*]pyridazine-6,8-diamine;
*N*⁶-(*trans*-4-aminocyclohexyl)-*N*⁸-(2-(4-(méthyloxy)phényl)éthyl)imidazo[1,2-*b*]pyridazine-6,8-diamine;
*N*⁶-(*trans*-4-aminocyclohexyl)-*N*⁸-2-propén-1-ylimidazo[1,2-*b*]pyridazine-6,8-diamine;
*N*⁶-(*trans*-4-aminocyclohexyl)-*N*⁸-(3-méthylbutyl)imidazo[1,2-*b*]pyridazine-6,8-diamine;
*N*⁶-(*trans*-4-aminocyclohexyl)-*N*⁸-propylimidazo[1,2-*b*]pyridazine-6,8-diamine;
*N*⁶-(*trans*-4-aminocyclohexyl)-*N*⁸-(cyclopropylméthyl)imidazo[ 1,2-*b*]pyridazine-6,8-diamine;
*N*⁶-(*trans*-4-aminocyclohexyl)-*N*⁸((3-chlorophényl)méthyl)imidazo[1,2-*b*]pyridazine-6,8-diamine;
6-(3-amino-1-pipéridinyl)-*N*-(4-(éthyloxy)phényl)imidazo [1,2-*b*]pyridazin-8-amine
*N*⁶-(3-aminopropyl)-*N*⁸-(4-(éthyloxy)phényl)imidazo[1,2-*b*]pyridazine-6,8-diamine;
*N*⁶-(*trans*-4-aminocyclohexyl)-*N*⁸-(2,6-difluorophényl)imidazo[1,2-*b*]pyridazine-6,8-diamine;
*N*⁶-(4-((4-aminocyclohexyl)méthyl)cyclohexyl)-*N*⁸-(4-(éthyloxy)phényl)imidazo[1,2-*b*]pyridazine-6,8-diamine;
2-(1-(8-((4-(éthyloxy)phényl)amino)imidazo[1,2-*b*]pyridazin-6-y])-4-pipéridinyl)éthanol;
*N*⁶-(*trans*-4-aminocyclohexyl)-*N*⁸-1*H*-indol-5-ylimidazo[1,2-*b*]pyridazine-6,8-diamine;
*N*⁶-(*trans*-4-aminocyclohexyl)-*N*⁸-(2-(éthyloxy)phényl)imidazo[1,2-*b*]pyridazine-6,8-diamine;
6-(3-amino-1-pyrrolidinyl)-*N*-(4-(éthyloxy)phényl)imidazo[1,2-*b*]pyridazin-8-amine
*N*⁶-(*trans*-4-aminocyclohexyl)-*N*⁸-(2-phényléthyl)imidazo[1,2-*b*]pyridazine-6,8-diamine;
*N*-(4-(éthyloxy)phényl)-6-(1-pipérazinyl)imidazo[1,2-*b*]pyridazin-8-amine *N*⁶-(2-(diméthylamino)éthyl)-*N*⁸-(4-(éthyloxy)phényl)imidazo[1,2-*b*]pyridazine-6,8-diamine;
*N*⁸-(4-(éthyloxy)phényl)-*N*⁶-(2-furanylméthyl)imidazo[1,2-*b*]pyridazine-6,8-diamine;
*N*-(4-(éthyloxy)phényl)-6-(4-méthyl-1,4-diazépan-1-yl)imidazo[1,2-*b*]pyridazin-8-amine;
2-((6-((*trans*-4-aminocyclohexyl)amino)imidazo[1,2-*b*]pyridazin-8-yl)amino)phénol;
*N*⁶-(*trans*-4-aminocyclohexyl)-*N*⁸-(3-((phénylméthyl)oxy)phényl)imidazo[1,2-*b*]pyridazine-6,8-diamine;
3-((6-((*trans*-4-aminocyclohexyl)amino)imidazo[1,2-*b*]pyridazin-8-yl)amino)phénol;
4-((6-((*trans*-4-aminocyclohexyl)amino)imidazo[1,2-*b*]pyridazin-8-yl)amino)phénol;
*N*⁶-(*trans*-4-aminocyclohexyl)imidazo[1,2-*b*]pyridazine-6,8-diamine;
3-((6-((*trans*-4-aminocyclohexyl)amino)imidazo[1,2-*b*]pyridazin-8-yl)amino)benzonitrile;
4-((6-((*trans*-4-aminocyclohexyl)amino)imidazo[1,2-*b*]pyridazin-8-yl)amino)benzonitrile;
acide 3-(6-((*trans*-4-aminocyclohexyl)amino)imidazo[1,2-*b*]pyridazin-8-yl)amino)benzoïque;
*N*⁶-(*trans*-4-aminocyclohexyl)-*N*⁸-1*H*-pyrazol-3-ylimidazo[1,2-*b*]pyridazine-6,8-diamine;
acide 4-((6-((trans-4-aminocyclohexyl)amino)imidazo[1,2-*b*]pyridazin-8-yl)amino)benzoïque;
4-((6-((*trans*-4-aminocyclohexyl)amino)imidazo[1,2-*b*]pyridazin-8-yl)amino)-*N,N*-diméthylbenzènesulfonamide;
*N*⁶-(*trans*-4-aminocyclohexyl)-*N*⁸-(4-(1*H*-tétrazol-5-yl)phényl)imidazo[1,2-*b*]pyridazine-6,8-diamine;
*N*⁶-(*trans*-4-(éthylamino)cyclohexyl)-*N*⁸-phénylimidazo[1,2-*b*]pyridazine-6,8-diamine;
*N*⁶-(*trans*-4-(méthylamino)cyclohexyl)-*N*⁸-phénlimidazo[1,2-*b*]pyridazine-6,8-diamine;
*N*⁶-(*trans*-4-aminocyclohexyl)-*N*⁸-(2-(phényloxy)phényl)imidazo[1,2-*b*]pyridazine-6,8-diamine;
*N*⁶-(*trans*-4-aminocyclohexyl)-*N*⁸-(4'-chloro-4-biphénylyl)imidazo[1,2-*b*]pyridazine-6,8-diamine;
*N*⁶-(trans-4-aminocyclohexyl)-*N*⁸-(2'-méthyl-4-biphénylyl)imidazo[1,2-*b*]pyridazine-6,8-diamine;
*N*⁶-(*trans*-4-aminocyclohexyl)-*N*⁸-(3'-chloro-4-biphénylyl)imidazo[1,2-*b*]pyridazine-6,8-diamine;
*N*⁶-(*trans*-4-aminocyclohexyl)-*N*⁸-(4-(phénylméthyl)phényl)imidazo[1,2-*b*]pyridazine-6,8-diamine;
*N*⁶-(*trans*-4-aminocyclohexyl)-*N*⁸-(4-(4-morpholinyl)phényl)imidazo[1,2-*b*]pyridazine-6,8-diamine;
*N*⁶-(*trans*-4-aminocyclohexyl)-*N*⁸-(3'-chloro-3-biphénylyl)imidazo[1,2-*b*]pyridazine-6,8-diamine;
*N*⁶-(*trans*-4-aminocyclohexyl)-*N*⁸-(4-(1-méthyléthyl)phényl)imidazo[1,2-*b*]pyridazine-6,8-diamine;
*N*⁶-(*trans*-4-aminocyclohexyl)-*N*⁸-(4-butylphényl)imidazo[1,2-*b*]pyridazine-6,8-diamine;
*N*⁶-(*trans*-4-aminocyclohexyl)-*N*⁸-(5,6,7,8-tétrahydro-1-naphtalényl)imidazo[1,2-*b*]pyridazine-6,8-diamine;
*N*⁶-(*trans*-4-aminocyclohexyl)-*N*⁸-1-naphtalénylimidazo[1,2-*b*]pyridazine-6,8-diamine;
*N*⁶-(*trans*-4-aminocyclohexyl)-*N*⁸-(3-(phénylméthyl)phényl)imidazo[1,2-*b*]pyridazine-6,8-diamine;
*N*⁶-(*trans*-4-aminocyclohexyl)-*N*⁸-(4-propylphényl)imidazo[1,2-*b*]pyridazine-6,8-diamine;
*N*⁶-(*trans*-4-aminocyclohexyl)-*N*⁸-(4'-méthyl-4-biphénylyl)imidazo[1,2-*b*]pyridazine-6,8-diamine;
*N*⁶-(*trans*-4-aminocyclohexyl)-*N*⁸-(3-(1-méthyléthyl)phényl)imidazo[1,2-*b*]pyridazine-6,8-diamine;
*N*⁶-(*trans*-4-aminocyclohexyl)-*N*⁸-(3-((1-méthyléthyl)oxy)phényl)imidazo[1,2-*b*]pyridazine-6,8-diamine;
*N*⁶-(*trans*-4-aminocyclohexyl)-*N*⁸-(3,5-bis(méthyloxy)phényl)imidazo[1,2-*b*]pyridazine-6,8-diamine;
*trans*-*N*-(8-(6-méthyl-3,4-dihydro-1 (2*H*)-quinolinyl)imidazo[1,2-*b*]pyridazin-6-yl)-1,4-cyclohexanediamine;
*N*⁶-(*trans*-4-aminocyclohexyl)-*N*⁸-2-naphtalénylimidazo[1,2-*b*]pyridazine-6,8-diamine;
*N*⁶-(trans-4-aminocyclohexyl)-*N*⁸-(3-(méthylsulfanyl)phényl)imidazo[1,2-*b*]pyridazine-6,8-diamine;
*N*⁶-(*trans*-4-aminocyclohexyl)-*N*⁸-(3-éthylphényl)imidazo[1,2-*b*]pyridazine-6,8-diamine;
*N*⁶-(*trans*-4-aminocyclohexyl)-*N*⁸-(4-éthylphényl)imidazo[1,2-*b*]pyridazine-6,8-diamine;
*N*⁶-(*trans*-4-aminocyclohexyl)-*N*⁸-(4-(méthylsulfanyl)phényl)imidazo[1,2-*b*]pyridazine-6,8-diamine;
*N*⁶-(*trans*-4-aminocyclohexyl)-*N*⁸-9H-fluorén-2-ylimidazo[1,2-*b*]pyridazine-6,8-diamine;
*N*⁶-(trans-4-aminocyclohexyl)-*N*⁸-(2-éthylphényl)imidazo[1,2-*b*]pyridazine-6,8-diamine;
*N*⁶-(*trans*-4-aminocyclohexyl)-*N*⁸-(4-cyclohexylphényl)imidazo[1,2-*b*]pyridazine-6,8-diamine;
*N*⁶-(*trans*-4-aminocyclohexyl)-*N*⁸-(4-(1,1-diméthyléthyl)phényl)imidazo[1,2-*b*]pyridazine-6,8-diamine;
*N*⁶-(*trans*-4-aminocyclohexyl)-*N*⁸-(3-(phényloxy)phényl)imidazo[1,2-*b*[pyridazine-6,8-diamine;
*N*⁶-(*trans*-4-aminocyclohexyl)-*N*⁸-3-biphénylylimidazo[1,2-*b*]pyridazine-6,8-diamine;
*N*⁶-(*trans*-4-aminocyclohexyl)-*N*⁸-(4-((1-méthyléthyl)oxy)phényl)imidazo[1,2-*b*]pyridazine-6,8-diamine;
*N*⁶-(*trans*-4-aminocyclohexyl)-*N*⁸-(2-chlorophényl)imidazo[1,2-*b*]pyridazine-6,8-diamine;
*N*⁶-(trans-4-aminocyclohexyl)-*N*⁸-(4-chlorophényl)imidazo[1,2-*b*]pyridazine-6,8-diamine;
*N*⁶-(*trans*-4-aminocyclohexyl)-*N*⁸-(3-chlorophényl)imidazo[1,2-*b*]pyridazine-6,8-diamine;
*N*⁶-(*trans*-4-aminocyclohexyl)-*N*⁸-(4-chloro-1-naphtalényl)imidazo[1,2-*b*]pyridazine-6,8-diamine;
*N*⁶-(*trans*-4-aminocyclohexyl)-*N*⁸-3-quinolinylimidazo[1,2-*b*]pyridazine-6,8-diamine;
*N*⁶-(*trans*-4-aminocyclohexyl)-*N*⁸-(3,5-dichlorophényl)imidazo[1,2-*b*]pyridazine-6,8-diamine;
*N*⁶-(*trans*-4-aminocyclohexyl)-*N*⁸-(3-(trifluorométhyl)phényl)imidazo[1,2-*b*]pyridazine-6,8-diamine;
N⁶-(trans-4-aminocyclohexyl)-*N*⁸-(4-chloro-2-fluorophényl)imidazo[1,2-*b*]pyridazine-6,8-diamine;
*N*⁶-(*trans*-4-aminocyclohexyl)-*N*⁸-(2-fluoro-5-méthylphényl)imidazo[1,2-*b*]pyridazine-6,8-diamine;
*N*⁶-(*trans*-4-aminocyclohexyl)-*N*⁸-(4-chloro-3-méthylphényl)imidazo[1,2-*b*]pyridazine-6,8-diamine;
*N*⁶-(*trans*-4-aminocyclohexyl)-*N*⁸-(5-phényl-2-pyridinyl)imidazo[1,2-*b*]pyridazine-6,8-1diamine;
*N*⁶-(*trans*-4-aminocyclohexyl)-*N*⁸-(3-fluoro-4-méthylphényl)imidazo[1,2-*b*]pyridazine-6,8-diamine;
*N*⁶-(*trans*-4-aminocyclohexyl)-*N*⁸-(2-méthyl-4-pyridinyl)imidazo[1,2-*b*]pyridazine-6,8-diamine;
*N*⁶-(*trans*-4-aminocyclohexyl)-*N*⁸-(4-fluoro-3-méthylphényl)imidazo[1,2-*b*]pyridazine-6,8-diamine;
*N*⁶-(*trans*-4-aminocyclohexyl)-*N*⁸-(2-fluoro-4-méthylphényl)imidazo[1,2-*b*]pyridazine-6,8-diamine;
*N*⁶-(*trans*-4-aminocyclohexyl)-*N*⁸-(3-((trifluorométhyl)oxy)phényl)imidazo[1,2-*b*]pyridazine-6,8-diamine;
*N*⁶-(*trans*-4-aminocyclohexyl)-*N*⁸-(4-méthyl-3-(trifluorométhyl)phényl)imidazo[1,2-*b*]pyridazine-6,8-diamine;
*N*⁶-(*trans*-4-aminocyclohexy])-*N*⁸-(4-éthyl-2-pyridinyl)imidazo[1,2-*b*]pyridazine-6,8-diamine;
*N*⁶-(*trans*-4-aminocyclohexy])-*N*⁸-(4-(1*H*-1,2,4-triazol-1-yl)phényl)imidazo[1,2-*b*]pyridazine-6,8-diamine;
*N*⁶-(*trans*-4-aminocyclohexyl)-*N*⁸-(4-(1*H*-pyrrol-1-yl)phényl)imidazo[1,2-*b*]pyridazine-6,8-diamine;
*N*⁶-(*trans*-4-aminocyclohexyl)-*N*⁸-(4-(4,5-dichloro-1*H*-imidazol-1-yl)phényl)imidazo[1,2-*b*]pyridazine-6,8-diamine;
*N*⁶-(*trans*-4-aminocyclohexyl)-*N*⁸-(4-(1*H*-pyrazol-1-yl)phényl)imidazo[1,2-*b*]pyridazine-6,8-diamine;
*N*⁶-(*trans*-4-aminocyclohexyl)-*N*⁸-(4-(3,5-diméthyl-1*H*-pyrazol-1-yl)phényl)imidazo[1,2-*b*]pyridazine-6,8-diamine;
*N*⁶-(*trans*-4-aminocyclohexyl)-*N*⁸-(4-(4-méthyl-4*H*-1,2,4-triazol-3-yl)phény])imidazo[1,2-*b*]pyridazine-6,8-diamine;
*N*⁶-(*trans*-4-aminocyclohexyl)-*N*⁸-(4-(1*H*-imidazol-1-yl)phényl)imidazo[1,2-*b*]pyridazine-6,8-diamine;
*N*⁶-(*trans*-4-aminocyclohexyl)-*N*⁸-(4-(1-méthyl-1*H*-imidazol-2-yl)phényl)imidazo[1,2-*b*]pyridazine-6,8-diamine;
*N*⁶-(*trans*-4-aminocyclohexyl)-*N*⁸-(4-(2-méthyl-1,3-thiazol-4-yl)phényl)imidazo[1,2-*b*]pyridazine-6,8-diamine;
*N*⁶-(*trans*-4-aminocyclohexyl)-*N*⁸-(4-(5-méthyl-2-furanyl)phényl)imidazo[1,2-*b*]pyridazine-6,8-diamine;
*N*⁶-(*trans*-4-aminocyclohexyl)-*N*⁸-(4-(2-éthyl-2*H*-tétrazol-5-yl)phény])imidazo[1,2-*b*]pyridazine-6,8-diamine;
4-((6-((*trans*-4-aminocyclohexyl)amino)imidazo[1,2-*b*]pyridazin-8-yl)amino)-3-hydroxy-*N,N*-diméthylbenzènesulfonamide;
*N*⁶-(*trans*-4-aminocyclohexy])-*N*⁸-(2,3-dihydro-1*H*-indén-5-yl)imidazo[1,2-*b*]pyridazine-6,8-diamine;
3-((6-((*trans*-4-aminocyclohexyl)amino)imidazo[1,2-*b*]pyridazin-8-yl)amino)-*N,N*-diméthylbenzènesulfonamide;
4-(6-((*trans*-4-aminocyclohexyl)amino)imidazo[1,2-*b*]pyridazin-8-yl)amino)-*N,N-*diméthylbenzamide;
*N*⁶-(*trans*-4-((2-chloro-4-pyrimidinyl)amino)cyclohexyl)-*N*⁸-phénylimidazo[1,2-*b*]pyridazine-6,8-diamine;
*N*⁶-(3-aminocyclopentyl)-*N*⁸-phénylimidazo[1,2-*b*]pyridazine-6,8-diamine;
*N*⁶-(*trans*-4-aminocyclohexyl)-*N*⁸(3-(4-morpholinylsulfonyl)phényl)imidazo[ 1,2-*b*]pyridazine-6,8-diamine;
3-((6-((*trans*-4-aminocyclohexyl)amino)imidazo[1,2-*b*]pyridazin-8-yl)amino)-*N*,*N*-diéthylbenzamide;
3-((6-((*trans*-4-aminocyclohexyl)amino)imidazo[1,2-*b*]pyridazin-8-yl)amino)-*N-*méthyl-*N*-phénylbenzènesulfonamide;
4-((6-((*trans*-4-aminocyclohexyl)amino)imidazo[1,2-*b*]pyridazin-8-yl)amino)-2-hydroxy-*N,N*-diméthylbenzènesulfonamide;
*N*⁶-(4-aminobicyclo[2.2.2]oct-1-yl)-N⁸-phénylimidazo[1,2-*b*]pyridazine-6,8-diamine;
*N*-(4-((6-((*trans*-4-aminocyclohexyl)amino)imidazo[1,2-*b*]pyridazin-8-yl)amino)phényl)méthanesulfonamide;
*N*⁶-(*trans*-4-aminocyclohexyl)-*N*⁸-(4-(3-(diméthylamino)- 1 - pyrrolidinyl)phényl)imidazo[1,2-*b*]pyridazine-6,8-diamine;
*N*⁶-(*trans*-4-aminocyclohexyl)-*N*⁸(4-(1-pyrrolidinylsulfonyl)phényl)imidazo[1,2-*b*]pyridazine-6,8-diamine;
acide 4-((6-((*trans*-4-aminocyclohexyl)amino)imidazo[1,2-*b*]pyridazin-8-yl)amino)benzènesulfonique;
4-((6-((*trans*-4-aminocyclohexyl)amino)imidazo[1,2-*b*]pyridazin-8-yl)amino)-*N,N*-diéthylbenzènesulfonamide;
4-((6-((*trans*-4-aminocyclohexyl)amino)imidazo[1,2-*b*]pyridazin-8-yl)amino)-*N-*propylbenzènesulfonamide;
4-((6-((*trans*-4-aminocyclohexyl)amino)imidazo[1,2-*b*]pyridazin-8-yl)amino)-*N-*éthylbenzènesulfonamide;
4-((6-((*trans*-4-aminocyclohexyl)amino)imidazo[1,2-*b*]pyridazin-8-yl)amino)-*N-*méthylbenzènesulfonamide;
*N*⁶-(*trans*-4-aminocyclohexyl)-*N*⁸-(4-aminophényl)imidazo[1,2-b]pyridazine-6,8-diamine;
4-((6-((*trans*-4-aminocyclohexyl)amino)imidazo[1,2-*b*]pyridazin-8-yl)amino)benzènesulfonamide;
3-((6-((*trans*-4-aminocyclohexyl)amino)imidazo[1,2-*b*]pyridazin-8-yl)amino)benzènesulfonamide;
3-((6-((*trans*-4-aminocyclohexyl)amino)imidazo[1,2-*b*]pyridazin-8-yl)amino)benzamide;
4-(6-((*trans*-4-aminocyclohexyl)amino)imidazo[1,2-*b*]pyridazin-8-yl)amino)benzamide;
*N*⁶-(*trans*-4-aminocyclohexyl)-*N*⁸-(4-(aminométhyl)phényl)imidazo[1,2-*b*]pyridazine-6,8-diamine;
6-((6-((*trans*-4-aminocyclohexyl)amino)imidazo[1,2-*b*]pyridazin-8-yl)amino)-1,2-dihydro-3*H*-indazol-3-one;
*N*⁶-(*trans*-4-aminocyclohexyl)-*N*⁸-(3-(aminométhyl)phényl)imidazo[1,2-*b*]pyridazine-6,8-diamine;
*N*⁶-(*trans*-4-aminocyclohexyl)-*N*⁸-(2,6-difluorophényl)imidazo[1,2-*b*]pyridazine-6,8-diamine;
*N*⁶-(*trans*-4-aminocyclohexyl)-7-chloro-*N*⁸-[4-(éthyloxy)phényl]imidazo[1,2-*b*]pyridazine-6,8-diamine;
*N*⁶-(3-aminopropyl)-*N*⁸-[4-(éthyloxy)phényl]imidazo[1,2-*b*lpyridazine-6,8-diamine;
*N*⁶-(*trans*-4-aminocyclohexyl)-*N*⁸-(2-fluorophényl)imidazo[1,2-*b*]pyridazine-6,8-diamine;
*N*⁶-(*trans*-4-aminocyclohexyl)-*N*⁸-(2,6-difluorophényl)imidazo[1,2-*b*]pyridazine-6,8-diamine;
*N*⁶-(*trans*-4-aminocyclohexyl)-*N*⁸-(2,4-difluorophényl)imidazo[1,2-*b*]pyridazine-6,8-diamine;
*N*⁶-(*trans*-4-aminocyclohexyl)-*N*⁸-(3-fluorophényl)imidazo[1,2-*b*]pyridazine-6,8-diamine;
*N*⁶-(*trans*-4-aminocyclohexyl)-*N*⁸-(4-fluorophényl)imidazo[1,2-*b*]pyridazine-6,8-diamine;
*N*⁶-(*trans*-4-aminocyclohexyl)-*N*⁸-(3,4-difluorophényl)imidazo[1,2-*b*]pyridazine-6,8-diamine;
*N*⁶-(*trans*-4-aminocyclohexyl)-*N*⁸-(2,5-difluorophényl)imidazo[1,2-*b*]pyridazine-6,8-diamine;
*N*⁶-(*trans*-4-aminocyclohexyl)-*N*⁸-(2,3-difluorophényl)imidazo[1,2-*b*]pyridazine-6,8-diamine;
*N*⁶-(*trans*-4-aminocyclohexyl)-*N*⁸-(3,5-difluorophényl)imidazo[1,2-*b*]pyridazine-6,8-diamine;
*N*⁶-(*trans*-4-aminocyclohexyl)-*N*⁸-(3-iodophényl)imidazo[1,2-*b*]pyridazine-6,8-diamine;
*N*⁶-(*trans*-4-aminocyclohexyl)-*N*⁸-[4-(trifluorométhyl)phényl]imidazo[1,2-*b*]pyridazine-6,8-diamine;
*N*⁶-(*trans*-4-aminocyclohexyl)-*N*⁸-pyridin-2-ylimidazo[1,2-*b*]pyridazine-6,8-diamine;
*N*⁶-(*trans*-4-aminocyclohexyl)-*N*⁸-(4-méthylpyridin-2-yl)imidazo[1,2-*b*]pyridazine-6,8-diamine;
*N*⁶-(*trans*-4-aminocyclohexyl)-*N*⁸-(5-méthylpyridin-2-yl)imidazo[1,2-*b*]pyridazine-6,8-diamine;
*N*⁶-(*trans*-4-aminocyclohexyl)-*N*⁸-(4,6-diméthylpyridin-2-yl)imidazo[1,2-*b*]pyridazine-6,8-diamine;
*N*⁶-(*trans*-4-aminocyclohexyl)-*N*⁸-pyrimidin-2-ylimidazo[1,2-*b*]pyridazine-6,8-diamine;
*N*⁶-(*trans*-4-aminocyclohexyl)-N⁸-[4-(éthyloxy)phényl]-7-méthylimidazo[1,2-*b*]pyridazine-6,8-diamine;
*N*⁶-(*trans*-4-aminocyclohexyl)-7-éthyl-*N*⁸-[4-(éthyloxy)phényl]imidazo[1,2-*b*]pyridazine-6,8-diamine;
*N*⁶-(*trans*-4-aminocyclohexyl)-7-méthyl-*N*⁸-phénylimidazo[1,2-*b*]pyridazine-6,8-diamine;
*N*⁶-(*trans*-4-aminocyclohexyl)-*N*⁸-(3,4-diméthylphényl)-7-méthylimidazo[ 1,2-*b*]pyridazine-6,8-diamine;
*N*⁶-(*trans*-4-aminocyclohexyl)-7-méthyl-*N*⁸-(4-méthylphényl)imidazo[1,2-*b*]pyridazine-6,8-diamine;
*N*⁶-(*trans*-4-aminocyclohexyl)-7-méthyl-*N*⁸-[3-(méthyloxy)phényl]imidazo[1,2-*b*]pyridazine-6,8-diamine;
*N*⁶-(*trans*-4-aminocyclohexyl)-*N*⁸-biphényl-4-yl-7-méthylimidazo[1,2-*b*]pyridazine-6,8-diamine;
*N*⁶-(*trans*-4-aminocyclohexyl)-7-méthyl-*N*⁸-[4-(propyloxy)phényllimidazo[1,2-*b*]pyridazine-6,8-diamine;
acide 4-((6-((*trans*-4-aminocyclohexyl)amino)-7-méthylimidazo[1,2-*b*]pyridazin-8-yl)amino)benzoïque;
4-((6-((4-aminocyclohexyl)amino)-7-méthylimidazo[1,2-*b*]pyridazin-8-yl)amino)-*N,N*-diméthylbenzènesulfonamide;
*N*-(4-((6-((*trans*-4-aminocyclohexyl)amino)-7-méthylimidazo[ 1,2-*b*]pyridazin-8-yl)amino)phényl)-*N*-méthylacétamide;
*N*⁶-(*trans*-4-aminocyclohexyl)-7-éthyl-*N*⁸-phénylimidazo[1,2-*b*]pyridazine-6,8-diamine;
*N*⁶-(*trans*-4-aminocyclohexyl)-7-chloro-*N*⁸-[4-(éthyloxy)phényl]imidazo[1,2-*b*]pyridazine-6,8-diamine;
*N*⁸-[4-(éthyloxy)phényl]-*N*⁶-pipéridin-3-ylimidazo[1,2-*b*]pyridazine-6,8-diamine;
*N*⁸-[4-(éthyloxy)phényl]-*N*⁶-pyrrolidin-3-ylimidazo[1,2-*b*]pyridazine-6,8-diamine;
*N*⁸-[4-(éthyloxy)phényl]-*N*⁶-pipéridin-4-ylimidazo[1,2*-b*]pyridazine-6,8-diamine;
6-(3-amino-1-pipéridinyl)-*N*-(4-(éthyloxy)phényl)imidazo[1,2-*b*]pyridazin-8-amine;
*N*⁸-(4-(éthyloxy)phényl)-7-méthyl-*N*⁶-3-pipéridinylimidazo[1,2-*b*]pyridazine-6,8-diamine;
*N*⁸-phényl-*N*⁶-3-pipéridinylimidazo[1,2-*b*]pyridazine-6,8-diamine; 6-[(3*S*)-3-aminopyrrolidin-1-yl]-*N*-[4-(éthyloxy)phényl]imidazo[1,2-*b*]pyridazin-8-amine;
*N*⁶-(*cis*-4-aminocyclohexyl)-*N*⁸-[4-(éthyloxy)phényl]-7-méthylimidazo[1,2-*b*]pyridazine-6,8-diamine;
*N*⁶-(*cis*-4-aminocyclohexyl)-*N*⁸-(5-méthyl-2-pyridinyl)imidazo[1,2-*b*]pyridazine-6,8-diamine;
*N*⁶-(*cis*-4-aminocyclohexyl)-*N*⁸-phénylimidazo[1,2-*b*]pyridazine-6,8 -diamine; *N*⁶-(*cis*-4-aminocyclohexyl)-7-méthyl-*N*⁸-phénylimidazo[1,2-*b*]pyridazine-6,8-diamine;
*N*⁶-(*cis*-4-aminocyclohexyl)-*N*⁸-(4-(éthyloxy)phényl)imidazo[1,2-*b*]pyridazine-6,8-diamine;
*N*⁶-(*cis*-4-aminocyclohexyl)-*N*⁸-(3,4-diméthylphényl)imidazo[1,2-*b*]pyridazine-6,8-diamine;
*N*⁶-(*cis*-4-aminocyclohexyl)-*N*⁸-(5-phényl-2-pyridinyl)imidazo[1,2-*b*]pyridazine-6,8-diamine;
4-((6-((*cis*-4-aminocyclohexyl)amino)imidazo[1,2-*b*]pyridazin-8-yl)amino)-*N,N-*diméthylbenzènesulfonamide;
*N*⁶-(*cis*-4-aminocyclohexyl)-*N*⁸-(4-méthylphényl)imidazo[1,2-*b*]pyridazine-6,8-diamine;
*N*⁶-(*cis*-4-aminocyclohexyl)-*N*⁸-(4,6-diméthyl-2-pyridinyl)imidazo[1,2-*b*]pyridazine-6,8-diamine;
*N*⁶-(*cis*-4-aminocyclohexyl)-*N*⁸-(4-(1*H*-pyrazol-1-yl)phényl)imidazo[1,2-*b*]pyridazine-6,8-diamine;
*N*⁶-(*cis*-4-aminocyclohexyl)-*N*⁸-(4-(4-morpholinylcarbonyl)phényl)imidazo[1,2-*b*]pyridazine-6,8-diamine;
*N*⁶-(*trans*-4-aminocyclohexyl)-7-méthyl-*N*⁸-(2-méthylphényl)imidazo[1,2-*b*]pyridazine-6,8-diamine;
*N*⁶-(*trans*-4-aminocyclohexyl)-*N*⁸-(2-fluorophényl)-7-méthylimidazo[1,2-*b*]pyridazine-6,8-diamine;
*N*⁶-(cis-4-aminocyclohexyl)-7-méthyl-*N*⁸-(2-méthylphényl)imidazo[1,2-*b*]pyridazine-6,8-diamine;
*N*⁶-(cis-4-aminocyclohexyl)-*N*⁸-(2-fluorophényl)-7-méthylimidazo[1,2-*b*]pyridazine-6,8-diamine;
*N*-(6-((*trans*-4-aminocyclohexyl)amino)imidazo[1,2-*b*]pyridazin-8-yl)benzamide;
1-(6-((*trans*-4-aminocyclohexyl)amino)imidazo[1,2-*b*]pyridazin-8-y1)-3-phénylurée;
*N,N*'-bis(4-*trans*-aminocyclohexyl)imidazo[1,2-*b*]pyridazine-6,8-diamine;
*N*⁶-(*trans*-4-aminocyclohexyl)-*N*⁸-(4-éthyloxyphényl)-7-phénylimidazo[1,2-*b*]pyridazine-6,8-diamine;
*N*⁶-(trans-4-aminocyclohexyl)-*N*⁸-(phényl)-7-phénylimidazo[1,2-*b*]pyridazine-6,8-diamine;
*N*⁶-(cis-4-aminocyclohexyl)-*N*⁸-(phényl)-7-phénylimidazo[1,2-*b*]pyridazine-6,8-diamine;
*N*⁶-(*cis*-4-aminocyclohexyl)-*N*⁸-(4-éthoxyphényl)-7-phénylimidazo[1,2-*b*]pyridazine-6,8-diamine;
4-((6-((*trans*-4-aminocyclohexyl)amino)imidazo[1,2-*b*]pyridazin-8-yl)amino)-*N-*(2-(4-pyridinyl)éthyl)benzamide;
4-((6-((*trans*-4-aminocyclohexyl)amino)imidazo[1,2-*b*]pyridazin-8-yl)amino)-*N-*(2-furanylméthyl)benzamide;
4-((6-((*trans*-4-aminocyclohexyl)amino)imidazo[1,2-*b*]pyridazin-8-yl)amino)-*N-*(1*H*-imidazol-4-ylméthyl)benzamide;
4-((6-((*trans*-4-aminocyclohexyl)amino)imidazo[1,2-*b*]pyridazin-8-yl)amino)-*N-*(3-pyridinylméthyl)benzamide;
*N*⁶-(*trans*-4-aminocyclohexyl)-*N*⁸-(4-((4-phényl-1-pipéridinyl)carbonyl)phényl)imidazo[1,2-*b*]pyridazine-6,8-diamine;
*N*⁶-(*trans*-4-aminocyclohexyl)-*N*⁸-(4-(1-pyrrolidinylcarbonyl)phényl)imidazo [1,2-*b*]pyridazine-6,8-diamine;
*N*⁶-(*trans*-4-aminocyclohexyl)-*N*⁸-(4-(1-pipéridinylcarbonyl)phényl)imidazo[1,2-*b*]pyridazine-6,8-diamine;
4-((6-((*trans*-4-aminocyclohexyl)amino)imidazo[1,2-*b*]pyridazin-8-yl)amino)-*N-*(phénylméthyl)benzamide;
4-((6-((*trans*-4-aminocyclohexyl)amino)imidazo[1,2-*b*]pyridazin-8-yl)amino)-*N-*(3-(méthyloxy)phény)benzamide;
*N*⁶-(*trans*-4-aminocyclohexyl)-*N*⁸-(4-((3-phényl-1-pyrrolidinyl)carbonyl)phényl)imidazo[1,2-*b*]pyridazine-6,8-diamine;
3-((6-((*trans*-4-aminocyclohexyl)amino)imidazo[1,2-*b*]pyridazin-8-yl)amino)-*N-*phénylbenzamide;
4-((6-((*trans*-4-aminocyclohexyl)amino)imidazo[1,2-*b*]pyridazin-8-yl)amino)-*N-*(2-hydroxyéthyl)benzamide;
4-((6-((*trans*-4-aminocyclohexyl)amino)imidazo[1,2-*b*]pyridazin-8-yl)amino)-*N-*(3-(2-oxo-1-pyrrolidinyl)propyl)benzamide;
*N*⁶-(*trans*-4-aminocyclohexyl)-*N*⁸-(4-(4-morpholinylcarbonyl)phényl)imidazo[1,2-*b*]pyridazine-6,8-diamine;
4-((6-((*trans*-4-aminocyclohexyl)amino)imidazo[1,2-*b*]pyridazin-8-yl)amino)-*N-*méthyl-*N*-(2-(2-pyridinyl)éthyl)benzamide;
4-((6-((*trans*-4-aminocyclohexyl)amino)imidazo[1,2-*b*]pyridazin-8-yl)amino)-*N,N*-diéthylbenzamide;
4-((6-((*trans*-4-aminocyclohexyl)amino)imidazo[1,2-*b*]pyridazin-8-yl)amino)-*N-*cyclopropylbenzamide;
4-((6-((*trans*-4-aminocyclohexyl)amino)imidazo[1,2-*b*]pyridazin-8-yl)amino)-*N-*(cyclohexylméthyl)benzamide;
4-((6-((*trans*-4-aminocyclohexyl)amino)imidazo[1,2-*b*]pyridazin-8-yl)amino)-*N-*3-pyridinylbenzamide;
4-((6-((*trans*-4-aminocyclohexyl)amino)imidazo[1,2-*b*]pyridazin-8-yl)amino)-*N-*(1-méthyl-1*H*-pyrazol-5-yl)benzamide;
4-((6-((*trans*-4-aminocyclohexyl)amino)imidazo[1,2-*b*]pyridazin-8-yl)amino)-*N-*(1-(4-fluorophényl)-2-oxo-1,2-dihydro-3-pyridinyl)benzamide;
4-((6-((*trans*-4-aminocyclohexyl)amino)imidazo[1,2-*b*]pyridazin-8-yl)amino)-*N-*phénylbenzamide;
4-((6-((*trans*-4-aminocyclohexyl)amino)imidazo[1,2-*b*]pyridazin-8-yl)amino)-*N-*cyclohexylbenzamide;
4-((6-((*trans*-4-aminocyclohexyl)amino)imidazo[1,2-*b*]pyridazin-8-yl)amino)-*N-*(4-pyridinylméthyl)benzamide;
1-(4-((6-((*trans*-4-aminocyclohexyl)amino)imidazo[1,2-b]pyridazin-8-yl)amino)phényl)-3-phénylurée;
1-(4-((6-((*cis*-4-aminocyclohexyl)amino)-7-méthylimidazo[1,2-*b*]pyridazin-8-yl)amino)phényl)-3-phénylurée;
1-(4-((6-((*trans*-4-aminocyclohexyl)amino)-7-méthylimidazo[1,2-*b*]pyridazin-8-yl)amino)phényl)-3-phénylurée;
*N*-(4-((6-((*trans*-4-aminocyclohexyl)amino)imidazo[1,2-*b*]pyridazin-8-yl)amino)phényl)-1-(4-fluorophényl)-2-oxo-1,2-dihydro-3-pyridinecarboxamide;
*N*-(4-((6-((*trans*-4-aminocyclohexyl)amino)imidazo[ 1,2-*b*]pyridazin-8-yl)amino)-phényl)-1-(4-fluorophényl)-2-oxo-1,2-dihydro-3-pyridinecarboxamide;
*N*-(4-((6-((*trans*-4-aminocyclohexyl)amino)imidazo[1,2-*b*]pyridazin-8-yl)amino)-2-éthylphényl)-1-(4-fluorophényl)-2-oxo-1,2-dihydro-3-pyridinecarboxamide;
*N*-(4-((6-((trans-4-aminocyclohexyl)amino)imidazo[1,2-*b*]pyridazin-8-yl)amino)phényl)benzamide;
*N*-(4-((6-((trans-4-aminocyclohexyl)amino)imidazo[1,2-*b*]pyridazin-8-yl)amino)phényl)acétamide;
3-((6-((trans-4-aminocyclohexyl)amino)-7-méthylimidazo[1,2-*b*]pyridazin-8-yl)amino)phénol;
*N*-(4-((6-chloroimidazo[1,2-*b*]pyridazin-8-yl)amino)phényl)-1-(4-fluorophényl)-2-oxo-1,2-dihydro-3-pyridinecarboxamide;
1-(4-fluorophényl)-*N*-(4-(imidazo[1,2-*b*]pyridazin-8-ylamino)phényl)-2-oxo-3-pipéridinecarboxamide;
1-(4-fluorophény])-*N*-(4-(imidazo[1,2-*b*]pyridazin-8-ylamino)phényl)-2-oxo-1,2-dihydro-3-pyridinecarboxamide;
6-((*trans*-4-aminocyclohexyl)amino)-8-((4-(éthyloxy)phényl)amino)imidazo [ 1,2-*b*]pyridazine-3-carbonitrile;
6-((*trans*-4-aminocyclohexyl)amino)-8-(phénylamino)imidazo[1,2-*b*]pyridazine-3-carbonitrile;
6-((4-*trans*-aminocyclohexyl)amino)-7-méthyl-8-(phénylamino)imidazo[1,2-*b*]pyridazine-3-carbonitrile;
6-((*trans*)-4-aminocyclohexylamino)-7-éthyl-8-(phénylamino)imidazo[1,2-*b*]pyridazine-3-carbonitrile;
6-((*trans*-4-aminocyclohexyl)amino)-8-anilino-7-isopropylimidazo[ 1,2-*b*]pyridazine-3-carbonitrile;
*N*⁶-(*trans*-4-aminocyclohexyl)-*N*⁸-(4-(éthyloxy)phényl)-3-fluoroimidazo[1,2-*b*]pyridazine-6,8-diamine;
*N*⁶-(*trans*-4-aminocyclohexyl)-*N*⁸-(4-(éthyloxy)phényl)-3-méthylimidazo[1,2-*b*]pyridazine-6,8-diamine;
*N*⁶-(*trans*-4-aminocyclohexyl)-*N*⁸-(4-(éthyloxy)phényl)-2,3-diméthylimidazo[ 1,2-*b*]pyridazine-6,8-diamine;
*N*-(6-((*trans*-4-aminocyclohexyl)amino)imidazo[ 1,2-*b*]pyridazin-8-yl)benzènesulfonamide;
6-((*trans*-4-aminocyclohexyl)oxy)-8-anilinoimidazo[1,2-*b*]pyridazine-3-carbonitrile;
6-((*trans*-4-aminocyclohexyl)amino)-8-anilinoimidazo[ 1,2-*b*]pyridazine-3-carboxamide;
*N*⁶-(*trans*-4-aminocyclohexyl)-7-éthyl-*N*⁸-phénylimidazo[1,2-*b*]pyridazine-6,8-diamine;
et
*N*⁶-(*trans*-4-aminocyclohexyl)-7-éthyl-*N*⁸-[4-(éthyloxy)phényl]imidazo[1,2-*b*]pyridazine-6,8-diamine;
ii) ou un énantiomère, un diastéréomère ou un sel pharmaceutiquement acceptable de (i), de celui-ci.

15. Une composition pharmaceutique comprenant un ou plusieurs composés selon la revendication 1 ou 14 et un véhicule ou diluant pharmaceutiquement acceptable.

16. Composé selon la revendication 1 ou 14, à utiliser chez un mammifère dans le traitement de la pancréatite (aiguë ou chronique), de l'asthme, d'allergies, du syndrome de détresse respiratoire de l'adulte, maladie pulmonaire obstructive chronique, glomérulonéphrite, polyarthrite rhumatoïde, lupus érythémateux disséminé, sclérodermie, thyroïdite chronique, maladie de Basedow, gastrite auto-immune, diabète, anémie hémolytique auto-immune, neutropénie auto-immune, thrombocytopénie, dermatite atopique, hépatite active chronique, myasthénie grave, sclérose en plaques, maladie inflammatoire de l'intestin, colite ulcéreuse, maladie de Crohn, psoriasis, réaction du greffon contre l'hôte, réaction inflammatoire induite par endotoxine, tuberculose, athérosclérose, dégénérescence musculaire, cachexie, arthrite psoriasique, syndrome de Reiter, goutte, arthrite traumatique, arthrite au cours de la rubéole, synovite aiguë, maladie des cellules β pancréatiques; maladies **caractérisées par** une infiltration massive de neutrophiles; spondylarthrite rhumatoïde, arthrite goutteuse et autres conditions arthritiques, paludisme cérébral, maladie pulmonaire inflammatoire chronique, silicose, sarcoïdose pulmonaire, maladie de résorption osseuse, rejets d'allogreffe, fièvre et myalgies dues à une infection, cachexie secondaire à une infection, formation de chéloïdes, formation de tissu cicatriciel, colite ulcéreuse, fièvre, grippe, ostéoporose, ostéoarthrite, leucémie aiguë myéloïde, leucémie chronique myéloïde, mélanome métastasique, sarcome de Kaposi, myélome multiple, septicémie, choc septique et shigellose; maladie d'Alzheimer, maladie de Parkinson, ischémies cérébrales ou maladie neurodégénérative causées par une lésion traumatique; troubles angiogéniques tumeurs solides comprises, néovascularisation oculaire et hémangiomes infantiles; infection d'hépatite aiguë (y compris hépatite A, hépatite B et hépatite C), infection par le VIH et rétinite à CMV, SIDA, SAS ou malignité et herpès; attaque, ischémie du myocarde, ischémie dans une attaque, attaques cardiaques, hypoxie d'un organe, hyperplasie vasculaire, lésion de reperfusion cardiaque et rénale, thrombose, hypertrophie cardiaque, agrégation plaquettaire induite par la thrombine, endotoxémie et/ou syndrome de choc toxique, conditions associées à la prostaglandine-endoperoxydase synthase de type 2, oedème, analgésie, douleur neuromusculaire, céphalée, douleur causée par un cancer, douleur dentaire, douleur arthritique, virus de l'anémie infectieuse des équidés; virus d'immunodéficience féline, virus d'immunodéficience bovine, virus d'immunodéficience canine et pemphigus vulgaire.

17. Composé à utiliser dans le traitement selon la revendication 16, où la condition est sélectionnée parmi la maladie de Crohn et la colite ulcéreuse, le rejet d'allogreffe, la polyarthrite rhumatoïde, le psoriasis, la spondylite ankylosante, l'arthrite psoriasique et le pemphigus vulgaire.

18. Composé à utiliser dans le traitement selon la revendication 16, où la condition est sélectionnée parmi une lésion d'ischémie-reperfusion, y compris lésion d'ischémie-reperfusion cérébrale provenant d'une attaque et d'ischémie-reperfusion cardiaque provenant d'un infarctus du myocarde.

19. Composé à utiliser dans le traitement selon la revendication 16, où la condition est le myélome multiple.
